# EUROPÄISCHE PATENTSCHRIFT

(11) **EP 1 917 251 B1**
(45) Veröffentlichungstag und Bekanntmachung des Hinweises auf die Patenterteilung: **03.04.2013**
(21) Anmeldenummer: 06791604.9
(22) Anmeldetag: 21.08.2006
(51) Int. Cl.: C07D 233/88, C07D 409/04, C07D 405/04, C07D 233/70, A61K 31/13, A61P 25/00

(54) **5-RING-HETEROAROMATEN-VERBINDUNGEN UND IHRE VERWENDUNG ALS BINDUNGSPARTNER FÜR 5-HT5-REZEPTOREN**
5-RING HETEROAROMATIC COMPOUNDS AND THEIR USE AS BINDING PARTNERS FOR 5-HT5 RECEPTORS
COMPOSES 5-CYCLE-HETEROAROMATES ET LEUR UTILISATION EN TANT QUE PARTENAIRES DE LIAISON DES RECEPTEURS 5-HT5

(30) Priorität: 21.08.2005 DE 102005040600; 24.08.2005 US 711014 P; 09.02.2006 DE 102006005917
(43) Veröffentlichungstag der Anmeldung: 07.05.2008
(60) Teilanmeldung: 10183318.4
(73) Patentinhaber: Abbott GmbH & Co. KG, 65205 Wiesbaden (DE)
(72) Erfinder: AMBERG, Wilhelm, 68239 Mannheim (DE); NETZ, Astrid, 68199 Mannheim (DE); KLING, Andreas, 68239 Mannheim (DE); OCHSE, Michael c/o Abbott GmbH & Co. KG, 67061 Ludwigshafen (DE); LANGE, Udo, 13593 Berlin (DE); HAUPT, Andreas, 68723 Schwetzingen (DE); GARCIA-LADONA, Francisco Javier, 76780 Kandel (DE); WERNET, Wolfgang, 67434 Neustadt (DE)
(74) Vertreter: Reitstötter - Kinzebach
(86) Internationale Anmeldenummer: PCT/EP2006/008223
(87) Internationale Veröffentlichungsnummer: WO 2007/022947

(56) Entgegenhaltungen:
- WO-A-00/41696
- WO-A-01/81326
- US-B1- 6 303 638

## Beschreibung

### Gebiet der Erfindung

Die vorliegende Erfindung betrifft 5HT5 Rezeptorliganden, Methoden zur Herstellung solcher Verbindungen und von Zwischenprodukten für deren Herstellung, sowie pharmazeutische Zusammensetzungen und Behandlungsmethoden von Krankheiten, die durch eine 5-HT5-Rezeptoraktivität moduliert werden, insbesondere zur Behandlung von neurodegenerativen und neuropsychiatrischen Störungen sowie den damit zusammenhängender Anzeichen, Symptomen und Fehlfunktionen.

### Hintergrund der Erfindung

Der Neurotransmitter Serotonin (5-Hydroxytryptamin oder "5-HT") ist Gegenstand erheblicher Forschungstätigkeiten, und Abweichungen in der Prozessierung von Serotonin sind Bestandteil von verschiedenen Krankheitsbildern. Serotonin übt seine Wirkung im Kardiovaskularen, Gastrointestinalen und Zentralen Nervensystem aus, indem es an verschiedene 5-HT Rezeptortypen bindet. Wenigstens sieben verschiedene Rezeptorklassen vermitteln die physiologischen Aktivitäten von Serotonin. Sie werden entsprechend einer international anerkannten Klassifikation mit 5-HT1, 5-HT2, 5-HT3, 5-HT4, 5-HT5, 5-HT6 und 5-HT7 bezeichnet. Die meisten dieser Klassen umfassen darüber hinaus weitere unterscheidbare Rezeptorsubtypen, so gehören zur 5-HT1-Klasse Rezeptoren, die sich wiederum in wenigstens fünf Unterklassen einteilen lassen, und als 5-HT1A, 5-HT1B, 5-HT1C 5-HT1D und 5-HT1E bezeichnet werden (Boess, Martin; Neuropharmacology 33:275-317 (1994).

Die Eigenschaften, Funktion und Pharmakologie dieser Rezeptorsubtypen wurden z.B. zusammengefasst von: (a) Kennet G.A., Serotonin Receptors and Thier Function", TOCRIS Review (http://www.tocris.com/serotonin.htm), publiziert Mai, 1997; (b) Peroutka, S.J., 1994, "Molecular Biology of Serotonin (5-HT) Receptors, Synapse 18, 241-260 und Current Drug Targets - CNS & Neurological Disorders 2004, 3, Heft 1.

Die 5-HT5-Klasse wurde erstmals beschrieben von Plassat et al., The EMBO Journal Bd. 11 Nr. 13, S. 4779-4786 (1992). Man unterscheidet 5-HT5A- und 5-HT5B-Rezeptoren (Erlander et al., Proc. Natl. Acad. Sci. USA 90:3452-3456 (1993). 5-HT5 Rezeptoren gehören zur Familie der G-Protein gekoppelten Rezeptoren und sind negativ an Adenylyl Cyclase gekuppelt. 5-HT5 Rezeptoren wurden geklont ausgehend von Maus, Meerschweinchen, Ratte und Human cDNA. Trotz einer hohen Interspezies-Homologie bestehen zwischen 5-HT5 Rezeptoren und anderen 5-HT-Rezeptoren nur geringe Sequenzhomologien.

5-HT5-Rezeptoren konnten mit Hilfe molekularbiologischer Techniken z.B. im Hippocampus, im Zerebellum, Hypothalamus, Thalamus, und Striatum, Cortex lokalisiert werden. Mittels immunhistochemischer Methoden konnte gezeigt werden, dass 5-HT5-Rezeptoren von Neuronen in verschiedenen Hirnregionen exprimiert werden (Oliver et al. Brain Res 2000, 867, 131-142 ; Pasqualetti et al. Mol Brain Res 1998, 56, 1-8)) Diese 5-HT5-Rezeptoren können zum einen direkt oder indirekt wichtige Funktionen des Hirns modulieren, andererseits aber auch an Mechanismen beteiligt sein, die bei neuropathologischen, neurodegenerativen und neuropsychiatrischen Erkrankungen involviert sind. 5HT5-Rezeptoren wurden auch in Astrocyten lokalisiert (Carson et al., GLIA 17:317-326 (1996). Reaktive Astrocyten wurden in Zusammenhang mit reaktiver Gliosis bei einer Reihe von pathologischen Gehirnveränderungen und neuropsychiatrischen Erkrankungen beobachtet. In-vitro Untersuchungen an kultivierten Astrocyten zeigten 5-HT5-Rezeptor-vermittelte Antworten. Aus diesem Grund wird einerseits vermutet, dass 5-HT5-Rezeptor an Erholungsprozessen des Gehirns nach Störungen beteiligt sind, andererseits ist aber auch nicht auszuschließen, dass sie zur Schadensentstehung oder sogar zu einer Schadensvermehrung beitragen.

5-HT5 Rezeptoren zeigen eine hohe Affinität zu verschiedenen Antidepressiva und Antipsychotika. Bisherige Studien deuten auf eine Rolle von 5-HT5 Rezeptoren bei folgenden Krankheitsbildern:
Psychose, Depression, chronische Schizophrenie, andere psychotischen Erkrankungen, Angstzustände, bipolare Störungen, Demenz, insbesondere Alzheimer Demenz, demyelinisierende Erkrankungen, insbesondere Multiple Sklerose, Ischämie, Hirnschlag und Migräne.

Die Verwendung von 5-HT5-Rezeptor Liganden allgemein zur Behandlung von Migräne und anderen cerebrovaskulären Erkrankungen ist in WO 00/041472, zur Behandlung von neurodegenerativen und neuropsychiatrischen Erkrankungen in WO 00/041696 beschrieben.

Imidazolverbindungen wurden bisher nicht als 5-HT5 Liganden verwendet.

Substiuierte Imidazole, Triazole und Pyrazole sind z.B. bekannt als Kinase Inhibitoren, als Inhibitoren von verschiedenen Subtypen der Phosphodiesterase, als Angiotensinrezeptorantagonisten und als Polymeraseinhibitoren.

WO2005028448 beschreibt benzylsubstituierte Benzimidazole als Tyrosinkinase Inhibitoren. EP545845 **(**US5314903**)** beschreibt Herstellung und Verwendung substituierter *N*-Alkylphenyl-Aminobenzimidazole als Calcium-Kanal-Blocker. WO-9724334 **(**US6352985**,** US6166219**,** EP882718**)** beschreibt "Carbonyl" substituierte N-Alkylphenyl-Alkylaminobenzimidazole und ihre Verwendung als PDE5 Inhibitoren. In EP111993 wird die Herstellung von "Carbonyl" substituierten N-Alkylaminobenzimidazolen und ihre Verwendung als Antivirale Verbinungen beschrieben. WO9855120 beschreibt Benzoyl substituierte N-Alkyl-aminobenzimidazole und Ihre Verwendung als Antivirale Verbindungen und W09962908 beschreibt u.a. Benzimidazolderivate und Ihre Verwendung als Zelladhäsionsinhibitoren. WO200272549 und WO200276960 befassen sich mit funktionalisierten Indolen und Benzimidazolen und deren Verwendung als Kinase- inhibitoren. Im Russian Journal of General Chemistry 2004, 74 (5), 738-743 schließlich werden Benzoyl substituierte Benzimidazole beschrieben. W0200288094 beschreibt die Herstellung von N,N' disubstituierten Iminobezimidazolen und deren Verwendung als Thrombininhibitoren, und W0200253158 nennt u.a. die Darstellung und Verwendung von N,N' disubstituierten Imidazolium Verbindungen zur Behandlung von Glaucoma.

WO2001081326 beschreibt 5-Ring Heteroaromaten mit Phenethylseitenketten als Modulatoren des □4□2 receptors. WO2001098276 und WO2000031067 befassen sich mit Imidazolen als Haarfärbemittel, und in US6303638 Pyridyl substituierte Imidazole. W09215577 nennt Imidazoltoluylpyrrole als Angiotensinrezeptorantagonisten. Imidazolone werden in Journal of Medicinal Chemistry 1982, 17 (6), 547-556 als Blutblättchenaggregationshemmer beschrieben. Imidazole als Zwischenprodukte werden in WO9736875 genannt. In Heterocycles 2003, 60 (3), 583-598, Journal of Natural Products 2002, 65 (8), 1190-1193 and 1991, 54 (6). 1509-1515, Journal of Organic Chemistry 1999, 64 (7), 2540-2544, Tetrahedron 1989, 45 (7), 2193- 2200, werden Imidazol-Naturstoffe ihre Wirkung und Synthese beschrieben.
Benzylsubstituierte Aminotriazole sind in JP2002275165 zur Behandlung von Autoimmunkrankheiten und Phenylsustituierte Triazole sind als Insektizide und Glycintransporter in JP02091062**,** EP285893 und WO2001087855 beschrieben. Synthesebeispiele zu Phenylsubstituierten Triazolen finden sich in Compte Rendues des Seances de l'Academie des Sciences, Serie C: Sciences Chimiques 1978, 287 (4), 121-123, Synthesis 1979, (5), 359-361 und Chemistry&Industry, 1978, (3), 92-94,
WO2002004424 beschreibt Pyrazole und deren Verwendung zur Behandlung von HIV Infektionen,

### Zusammenfassung der Erfindung

Die Aufgabe bestand darin Verbinungen zur Verfügung zu stellen, die die Funktion der 5HT5 Rezeptoren modulieren.

Die vorliegende Erfindung betrifft nun Verbinungen, die die Funktion der 5HT5 Rezeptoren modulieren und damit die Behandlung neuropathologischer, neuropsychiatrischer und neurodegenerativer Störungen zu ermöglichen. Die Erfindung betrifft auch pharmazeutische Zusammensetzungen, die diese Verbindungen enthalten und die Verwendung dieser Verbindungen und Zusammensetzungen zur Behandlung von Krankheiten bei denen der 5HT5 Rezeptor beteiligt ist.

Gemäß einem Aspekt der vorliegenden Erfindung wird mindestens eine 5-Ring Heteroaromaten-Verbindung der allgemeinen Formel I 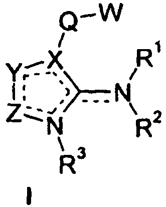 , und/oder entsprechende enantiomere, diastereomere und/ oder tautomere Formen davon und/oder pharmazeutisch annehmbare Salze davon, wobei die angegebenen Reste die folgenden Definitionen besitzen
- **W:**: einen Rest der allgemeinen Formel **W1** oder **W2** 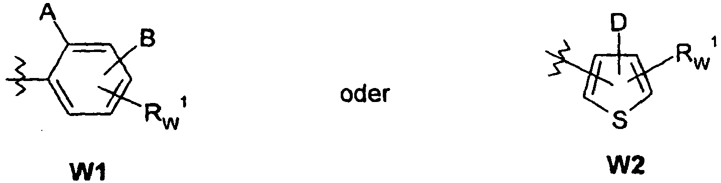 worin
- **A:**: NO₂, NH₂, CN, CF₃, OCF₃, CHF₂, OCHF₂, COOH, O-CH₂-COOH, Halogen, SH, oder jeweils gegebenenfalls substituiertes O-C₁-C₆-Alkyl, S-C₁-C₆-Alkyl, -O-CO-C₁-C₆-Alkyl, -O-CO-Aryl, -O-CO-Hetaryl, -O-COO-C₁-C₆-Alkyl oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-AlkylenHetaryl oder C₁-C₄-Alkylen-Aryl, oder jeweils gegebenenfalls substituiertes O-R_{A}1, CO-R_{A}¹, S-R_{A}¹, SO-R_{A}¹, CO-O-R_{A}¹, NR_{A}⁴-CO-O-R_{A}¹_{,} O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²RA³oder CO-NR_{A}²R_{A}³;
- **R_{A}¹:**: jeweils gegebenenfalls substituiertes C₁-C₆-Alkyl, C₂-C₆-Alkenyl, C₂-C₆-Alkinyl,C₃-C₇-Cycloalkyl,C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl, C₁-C₆-Alkylen-Hetaryl;
- **R_{A}²:**: Wasserstoff, oder
jeweils gegebenenfalls substituiertes C₁-C₆-Alkyl,C₂-C₆-Alkenyl, C₂-C₆-Alkinyl, C₁-C₄-Alkylen-C₃-C₇-Cycloalky), C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-AlkylenHetaryl, CO-O-C₁-C₆-Alkyl, CO-O-Aryl, CO-O-C₁-C₄-Alkylen-Ary), CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
- **R_{A}³**:: jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl,C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-AlkylenHetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste **R_{A}²** und **R_{A}³** bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome aus der Gruppe O, N, S enthalten kann; wobei gegebenenfalls zwei an diesem Heterocyclus substituierte Reste zusammen einen 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls 3 bis 7-gliedriger substituierter Cyclus ankondensiert sein kann;
- **R_{A}⁴**:: Wasserstoff, oder
jeweils gegebenenfalls substituiertes C₁-C₆-Alkyl, C₂-C₆-Alkenyl, C₂-C₁₂-Alkinyl, C₃-C₇-Cycloalkyl, Aryl, C₁-C₄-Alkylen-Aryl, C₁-C₆-Alkylen-O-C₁-C₆-Alkyl, Hetaryl;
- **B:**: Wasserstoff oder unabhängig von Rest **A** wie Rest **A** definiert ist,
oder zwei der Reste **A**, **B** oder **R_{w}¹** bilden jeweils unabhängig voneinander zusammen mit den anhängenden C-Atomen einen 3 bis 7-gliedrigen, jeweils gegebenenfalls substituierten, gesättigten, ungesättigten oder aromatischen Carbocyclus oder einen jeweils gegebenenfalls 3 bis 7-gliedrigen substituierten, gesättigten, ungesättigten oder aromatischen Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann;
- **R_{w}¹**:: Wasserstoff, OH, Halogen, NO₂, NH₂, CN, CF₃, CHF₂, CH₂F, OCF₃, OCHF₂, OCH₂F oder jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
gegebenenfalls mit der Maßgabe, dass wenn W = W1, dann R_{w}¹ oder B nicht ein in 4-Position (para-Position) zur Veknüpfungsposition mit Q stehender gegebenenfalls substituierter N-Pyrrolidinyl-Rest sein soll;
**D**: Wasserstoff oder unabhängig von A wie Rest **A** definiert ist;
**Q**: einen Rest der allgemeinen Formel **Q1** 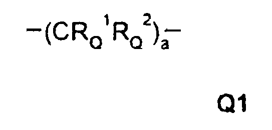 mit dem Index
**R_{Q}¹**, **R_{Q}²** unabhängig voneinander: Wasserstoff, Halogen, OH, oder
**R¹, R²** unabhängig voneinander:
   Wasserstoff, OH, CN, oder jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
   **R¹** und **R²** bilden zusammen mit dem Stickstoff einen 5 bis 7-gliedrigen, jeweils gegebenenfalls substituierten, gesättigten oder ungesättigten Heterocyclus, der ein, zwei oder drei weitere verschiedene oder gleiche Heteroatome aus der Gruppe O, N, S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen jeweils gegebenenfalls substituierten Carbocyclus oder Heterocyclus bilden können, wobei der Heterocyclus bis zu drei verschiedene oder gleiche Heteroatome ausgewählt aus der Gruppe bestehend aus O, N, und S enthalten kann und wobei der gebildete Cyclus gegebenenfalls substituiert sein kann;
**R³** Freies Elektronenpaar oder Wasserstoff;
**X,Y,Z** X = N, Y = C R4, Z = C R⁶, oder
   X = C, Y = NR⁵, Z = CR⁶, oder
   X = C, Y = CR⁴, Z = NR⁷, oder
   X = N, Y = N, Z = CR⁶, oder
   X = N, Y = N, Z = NR⁷
   **oder**
**X,Y,Z** X = N, Y = CR⁴, Z = CR⁶, oder
   X = C, Y = NR⁵, Z = CR⁶, oder
   X = C, Y = CR⁴, Z = NR⁷, oder
   X = N, Y = N, Z = CR⁶, oder
   X = N , Y = CR⁴ , Z = N
**R⁴, R⁶** jeweils unabhängig voneinander einen Rest, ausgewählt aus der Gruppe bestehend aus Wasserstoff und der gleichen oder verschiedenen der Gruppen **1.), 3.), 4.) 5.)** oder **6.)** und
**R⁵, R⁷** jeweils unabhängig voneinander einen Rest, ausgewählt aus der gleichen oder verschiedenen der Gruppen **2.), 3.) 4.)** oder **5.),** wobei die Gruppen 1.) bis 6.) wie folgt definiert sind:
   1.) Wasserstoff, NO₂, NH₂, CN, CF₃, CHF₂, OCF₃, OCHF₂, COOH, **O-**CH₂-COOH, Halogen, oder
      jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C,-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes
      O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}6^{R}_{Z,Y};
   **2.)** Freies Elektronenpaar oder Wasserstoff, CH₂-CF₃, CH₂-CHF₂, jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes
      CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   **3.)** Phenyl, 1-Naphthyl oder 2-Naphthyl, die jeweils mit einem, zwei oder drei Resten ausgewählt aus R_{Z,Y}¹, R_{Z,Y}² und **R_{Z,Y}³** substituiert sind oder sein können, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, NO₂, NH₂, OH, CN, CF₃, CHF₂, OCF₃, OCHF₂, COOH, O-CH₂-COOH, SH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
      O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}7-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ oder CO-NR_{Z,Y}⁶K_{Z,Y}⁷, oder
      jeweils zwei der Reste ausgewählt aus der Gruppe bestehend aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei gegebenenfalls jeweils gegebenenfalls zwei an diesem Carbo- oder Heterocyclus substituierte Reste zusammen einen 3 bis 7-gliedrigen, anellierten, gesättigten, ungesättigten oder aromatischen Carbocyclus oder Heterocyclus bilden können, wobei der Heterocyclus 1, 2, oder bis zu drei verschiedene oder gleiche Heteroatome ausgewählt aus der Gruppe bestehend aus O, N; S enthalten kann und wobei der gebildete Cyclus gegebenenfalls substituiert sein kann und/oder an diesem Cyclus ein weiterer, 3 bis 7-gliedriger, gegebenenfalls substituierter Cyclus ankondensiert sein kann;
      **R_{Z,Y}⁴** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Hetaryl, Aryl, C₁-C₄-AlkylenHetaryl, Hetaryl, C₁-C₄-Alkylen-Aryl, oder C₁-C₆-Alkyl, das jeweils gegebenenfalls substituiert ist mit einem Substituenten ausgewählt aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, bedeutet;
      **R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, Benzofuranyl, Dihydrobenzofuranyl, Indanyl, oder jeweils gegebenenfalls 1, 2 oder bis zu 3-fach substituiertes Aryl oder Hetaryl, wobei mit Substituenten unabhängig voneinander ausgewählt aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, sowie COOH, O-CH₂-COOH, SH, oder
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder jeweils gegebenenfalls substituiertes O-R_{A}¹,CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³oder CO-NR_{A}²R_{A}³, bedeutet;
      **R_{Z,Y}⁶** Wasserstoff, oder
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, CO-O-C₁-C₄-Alkylen-Aryl, SO₂-C₁-C₆-Alkyl, SO₂-Aryl, SO₂-Hetaryl, SO₂-C₁-C₄-AlkylenAryl oder SO₂-C₁-C₄-Alkylen-Hetaryl; bedeutet;
      **R_{Z,Y}⁷** Wasserstoff oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-AlkylenAryl oder SO₂-C₁-C₄-Alkylen-Hetaryl; bedeutet;
      oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann, bilden; und jeweils gegebenenfalls zwei an diesem Heterocyclus substituierte Reste zusammen einen 3 bis 7-gliedrigen, anellierten, gesättigten, ungesättigten oder aromatischen Carbocyclus oder Heterocyclus bilden können, wobei der Heterocyclus 1, 2 oder bis zu drei verschiedene oder gleiche Heteroatome ausgewählt aus der Gruppe bestehend aus O, N, und S enthalten kann, und der gebildete Cyclus gegebenenfalls substituiert sein kann oder an diesem Cyclus ein weiterer, gegebenenfalls substituierter 3 bis 7-gliedriger Cyclus ankondensiert sein kann;
   **4.)** einen jeweils gegebenenfalls substituierten 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** wie vorstehend definiert substituiert sein kann;
   **5.)** einen jeweils gegebenenfalls substituierten C5-C18-bi- oder tricyclischen, gesättigten Kohlenwasserstoffrest.
   **6.) R⁴** und **R⁶** bilden zusammen mit X und Y einen jeweils gegebenenfalls substituierten C₆-C₁₀-gliedrigen gesättigten, ungesättigten oder aromatischen Carbocyclus, bevorzugt einen Benzo-Rest, die jeweils mit einem, zwei oder bis zu drei Resten substituiert sein können, welche jeweils unabhängig voneinander ausgewählt sind aus **R_{Z,Y}⁸.**
      **R_{Z,Y}⁸**: Wasserstoff, NO₂, NH_{2.} OH, CN, CF₃, CHF₂, OCF₃, OCHF₂, COOH, O-CH₂-COOH, SH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-AlkylenHetaryl, oder jeweils gegebenenfalls substituierten O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}5, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷
   , gegebenenfalls mit einer, zwei oder drei der Maßgaben ausgewählt aus Gruppe bestehend aus den nachstehend genannten Maßgaben (i), (ii) und (III):
   (i) mit der Maßgabe (i), dass wenn R⁴ und R⁶ die in der Gruppe 6.) genannten Bedeutungen haben, dann die Reste **A, B, Q, R_{Q}¹** und **R_{Q}²** wie folgt definiert sind:
      **A** sitzt in W1 wie angegeben in 2 Position und bedeutet NH₂. OCF₃, OCHF₂, O-CH₂-COOH, O-C₁-C₆-Alkyl, S-C₁-C₆-Alkyl, oder O-R_{A}¹, CO-R_{A}¹, S-R_{A}¹, SO-R_{A}¹, SO₂-R_{A}¹, SO₂-NR_{A}²R_{A}³;
      **B** Sitzt in W1 in 6 Position und bedeutet Halogen, CN, CF₃,-CHF₂, OCF₃, OCHF₂, oder
         jeweils gegebenenfalls substituiertes C₁-C₆-Alkyl oder C₂-C₆-Alkenyl, -O-CH₂-COO-R_{A}¹, - O-R_{A}¹, S-R_{A}¹, NR_{A}²R_{A}³, -NR_{A}⁴-CO-R_{A}¹ oder-CO-NR_{A}²R_{A}³; NR_{A}⁴-SO₂-R_{A}¹;
         oder B bildet zusammen **R_{w}¹** einen der Reste -(CH₂)₃-, -O-CH₂-O-, -O-(CH₂) ₂-O-, -CH=CH-CH=CH-, -O-(CH₂)₂-,-(CH₂)₂-O-;
      **Q** -CR_{Q}¹R_{Q}²-
      **R_{Q}¹**, **R_{Q}²** unabhängig voneinander:
      Wasserstoff, Halogen, gegebenenfalls substituiertes C₁-C₆-Alkyl;
      und/oder
   (ii) mit der Maßgabe (ii), dass wenn W = W1, dann R_{w}¹ oder B nicht ein in 4-Position (para-Position) zur Veknüpfungsposition mit Q stehender gegebenenfalls substituierter N-Pyrrolidinyl-Rest sein soll;
      und/oder
   (iii) mit der Maßgabe (iii), dass die Summe aus a, b und c 1, 2, 3, 4 oder 5 ist, dann wenn X = N, Y = CR⁴, und Z = CR⁶; bereitgestellt

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5 Ring-Heteroaromaten-Verbindung der allgemeinen Formel I wie vorstehend ausgeführt oder nach Anspruch 1, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{w}¹, D, Q, a, R_{Q}¹, R_{Q}², R₁, R₂, R₃, X, Y, Z**, soweit nachstehend nichts anderslautendes ausgeführt ist die gleiche Bedeutung wie vorstehend ausgeführt oder in Anspruch 1 haben und die nachfolgenden Reste folgende Definitionen besitzen:
**R⁴, R⁶** jeweils unabhängig voneinander einen Rest, ausgewählt aus der Gruppe bestehend aus Wasserstoff und der gleichen oder verschiedenen der Gruppen **1.), 3.), 4.)** oder **5.)** und
**R⁵, R⁷** jeweils unabhängig voneinander einen Rest, ausgewählt aus der gleichen oder verschiedenen der Gruppen **2.), 3.) 4.)** oder **5.)**
wobei die Gruppen 1.) - 5.) jeweils die wie vorstehend ausgeführt oder in Anspruch 1 genannten Bedeutungen haben sollen; bereilgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 4, und/oder entsprechende enantiomere, diastereomere und/ oder tautomere Formen davon und/oder pharmazeutisch annehmbare Salze davon, wobei die Reste **Q, R_{Q}¹, R_{Q}², R₁, R₂, R₃, X, Y, Z, R₄, R₅, R₆, R_{7,} R_{Z,Y}¹, R_{Z,Y}², R_{Z,Y}³, R_{Z,Y}⁴, R_{Z,Y}⁵, R_{Z,Y}⁶, R_{Z,Y}⁷_{.} R_{Z,Y}⁶** soweit nachstehend nichts anderslautendes ausgeführt ist die gleiche Bedeutung wie vorstehend ausgeführt oder in einem der Ansprüche 1 oder 2 genannt haben und die nachfolgenden Reste folgende Definition besitzen:
**W**: einen Rest der allgemeinen Formel **W1, W21** oder **W22** 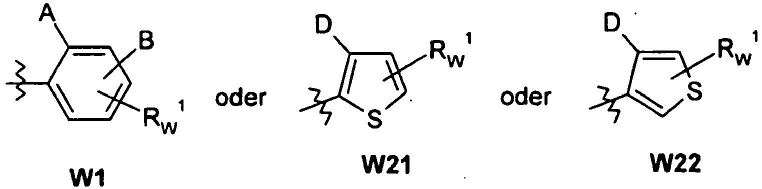 bedeutet, worin die Rest A, B, D und R_{w}¹ unabhängig voneinander und jeweils unabhängig von ihrem jeweiligen Auftreten eine der nachstehend genannten Bedeutungen aufweisen können:
**A**: Halogen, NH₂, CN, CF₃, OCF₃, OCHF₂, COOH, O-CH₂-COOH, SH, O-C₁-C₆-Alkyl, S-C₁-C₆-Alkyl, C₁-C₆-Alkyl oder jeweils gegebenenfalls substituiertes O-R_{A}¹, CO-R_{A}¹, S-R_{A}¹, O-CH₂-COO-R_{A}¹, NR^{A}₂R_{A}³, CONH₂, SO₂NH₂, SO₂-R_{A}¹, NR_{A}⁴-SO₂-R_{A}¹, SO₂-NRA²RA³ oder CO-NR_{A}²R_{A}³;
**R_{A}¹**: jeweils gegebenenfalls substituiertes C₁-C₆-Alkyl, C₃-C₇-Cycloalkyl, Phenyl oder Benzyl;
**R_{A}²; R_{A}³** unabhängig voneinander Wasserstoff, oder
   jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₆-Alkyl, SO₂-Aryl, SO₂-Hetaryl, oder SO₂-C₁-C₄-Alkylen-Aryl;
   oder beide Reste **R_{A}²** und **R_{A}³** bilden zusammen mit dem Stickstoff einen jeweils gegebenenfalls substituierten 5-oder 6-gliedrigen gesättigten oder ungesättigten Ring, der ein oder bis zu zwei gleiche oder verschiedene Heteroatome ausgewählt aus der Gruppe bestehend aus O und N enthalten kann.
**R_{A}⁴:** Wasserstoff oder einen gegebenenfalls substituierten C₁-C₆-Alkylrest;
**B**: Wasserstoff, Halogen, CN, CF₃, -CHF₂, OCF₃, OCHF₂, oder jeweils gegebenenfalls substituiertes C₁-C₆-Alkyl oder C₂-C₆-Alkenyl, oder jeweils gegebenenfalls substituierten -O-CH₂-COO-R_{A}1, - O-R_{A}¹, S-R_{A}¹, NR_{A}²R_{A}³, -NR_{A}⁴-CO-R_{A}¹ oder-CO-NR₁R_{A}³; NR_{A}⁴-SO₂-R_{A}¹;
   oder B bildet zusammen R_{w}¹ einen der Reste -(CH₂)₃-, -O-CH₂-O-.- O-(CH₂)₂-_{O}-, -CH=CH-CH=CH-, -O-(CH₂)₂-, -(CH₂)₂-O-;
**R_{w}¹**: Wasserstoff, F, Cl, CN, CF₃, O-CF₃, oder
   jeweils gegebenenfalls substituiertes C₁-C₆-Alkyl, Aryl, C₁-C₆-Alkylamino oder C₁-C₆-Dialkylamino;
**D**: Wasserstoff oder unabhängig von A wie Rest A definiert ist; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 5, und/oder entsprechende enantiomere, diastereomere und/ oder tautomere Formen davon und/oder pharmazeutisch annehmbare Salze davon wobei die Reste **W, A, R_{A}¹, R_{A}², R_{A}³, R_{A}⁴, B, R_{w}¹, O, Q, a, R_{Q}¹, R_{Q}², X, Y, Z, R₄, R_{5,} R_{6,} R₇, R_{Z,Y}¹**, **R_{Z,Y}², R_{Z,Y}³, R_{Z,Y}⁴. R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 3 genannt haben und die nachfolgenden Reste folgende Definition besitzen:
**R¹, R²**, unabhängig voneinander:
   Wasserstoff, OH, CN, C₁-C₅-Alkyl, C₁-C₆-Alkylen-O-C₁-C₆-Alkyl, substituiertes Aryl, Benzyl, CO-C₁-C₆-Alkyl, CO-Aryl, oder CO-C₁-C₄-Alkylen-Aryl;
**R³** Freies Elektronenpaar oder Wasserstoff; bereitgestellt.

Jede dieser vorstehend genannten Definitionen einer Variablen kann mit beliebigen der vorstehend genannten Definitionen der restlichen Variablen. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 6, und/oder entsprechende enantiomere, diastereomere und/ oder tautomere Formen davon und/oder pharmazeutisch annehmbare Salze davon wobei die Reste **W, A, R_{A}¹, R_{A}², R_{A}³, R_{A}⁴, B, R_{w}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 4 genannt haben und die nachfolgenden Reste folgende Definition besitzen:
**X, Y, Z** X=N,Y=CR⁴,Z=CR⁶.oder
   X=C.Y=CR⁴.Z=NR⁷,oder
   X=N.Y=NR⁵,Z=CR⁶
   oder
X, Y, Z x=N,Y=CR⁴,Z=CR⁶,oder
   X=C.Y=CR⁴,Z=NR⁷,oder
   X=N,Y=N,Z=CR⁶
bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 7, und/oder entsprechende enantiomere, diastereomere und/ oder tautomere Formen davon und/oder pharmazeutisch annehmbare Salze davon , wobei die Reste W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸ soweit nachstehend nichts anderslautendes ausgeführt ist die gleiche Bedeutung wie vorstehend ausgeführt oder in einem der Ansprüche 1 bis 5 genannt haben und die nachfolgenden Reste folgende Definition besitzen:
X, Y, Z X = N, Y = CR⁴, Z = CR⁶, oder
   X = N, Y = NR⁵, Z = CR⁶
   oder
X,Y,Z X = N, Y = CR⁴, Z=CR⁶, oder
   X = N, Y = N, Z = CR⁶
bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 6, und/oder entsprechende enantiomere, diastereomere und/ oder tautomere Formen davon und/oder pharmazeutisch annehmbare Salze davon , wobei die Reste **W, A, R_{A}¹, R_{A}², R_{A}³, R_{A}⁴, B, R_{w}¹, D, Q, a, R_{Q}¹, R_{Q}²**, **R₁, R₂, R₃, X, Y, Z**, soweit nachstehend nichts anderslautendes ausgeführt ist die gleiche Bedeutung wie vorstehend ausgeführt oder in einem der Ansprüche 1 bis 8 genannt haben und die nachfolgenden Reste folgende Definition besitzen:
**R⁴, R⁶** jeweils unabhängig voneinander einen Rest, ausgewählt aus der Gruppe bestehend aus Wasserstoff oder der gleichen oder verschiedenen der Gruppen **1.), 3.), 4.)** oder **5.)** und
**R⁵, R⁷** jeweils unabhängig voneinander einen Rest, ausgewählt aus der gleichen oder verschiedenen der Gruppen **2.), 3.)** oder **4.)** oder **5.)**
   wobei die Gruppen 1.), 2.), 3.) 4.) und 5.) wie folgt definiert sind:
   1.) H, CN, CF₃, CHF₂, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, oder jeweils gegebenenfalls substituiertes
      O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   2.) Freies Elektronenpaar oder Wasserstoff,
      jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₆-Alkyl oder jeweils gegebenenfalls substituiertes SO₂-R_{Z,Y}⁵, SO₂NH₂, CONH₂, SO₂-NR_{Z,Y}⁶R_{Z,Y}⁷ oder CO-**NR_{Z,Y}⁶R_{Z,Y}⁷**;
   3.) Phenyl, 1-Naphthyl oder 2-Naphthyl, die jeweils mit **R_{Z,Y}¹, R_{Z,Y}²** und **R_{Z,Y}³** substituiert sind, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, NH₂, CN, CF₃, CHF₂, OCF₃, OCHF₂, O-CH₂-COOH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
      O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷, oder
      jeweils zwei der Reste aus **R_{Z,Y}¹, R_{Z,Y}²** oder **R_{Z,Y}³** bilden zusammen mit den Ringatomen, die diese Substituenten tragen, einen einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
      **R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, Hetaryl, C₁-C₄-AlkylenAryl, oder
      C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
      **R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁C₄-Alkylen-Heterocycloalkyl, Heterocycloalkyl oder C₁-C₆-Alkylen-O-C₁-C₆-Alkyl, Dioxymethylenphenyl, oder jeweils gegebenenfalls 1-, 2- oder bis zu 3-fach substituiertes Aryl oder Hetaryl, wobei dabei mit Substituenten unabhängig voneinder ausgewählt aus der Gruppe bestehend aus Halogen, NO₂, NH₂, OH, CN, CF₃, CHF₂, OCF₃, OCHF₂, NH-(C₁-C₆-Alkyl) oder N(C₁-C₆-Alkyl)₂, COOH, O-CH₂-COOH, SH, oder jeweils gegebenenfalls substituiertes C₁-C₆-Alkyl, C₃-C₇-Cycloalkyl, Aryl, Hetaryl, Heterocycloalkyl, oder jeweils gegebenenfalls substituiertes
      O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
      **R_{Z,Y}⁶** Wasserstoff, oder
      jeweils gegebenenfalls substituiertes C₁-C₆-Alkyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₆-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-AlkylenHetaryl;
      **R_{Z,Y}⁷** Wasserstoff oder gegebenenfalls substituiertes C₁-C₆-Alkyl;
      oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
   4.) einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** substituiert sein kann, wobei die Reste **R_{Z,Y}¹, R_{Z,Y}²,** und **R_{Z,Y}³** wie vorstehend definiert sein sollen;
   5.) einen gegebenenfalls substituierten C₅-C₁₀- bi- oder tricyclischen, gesättigten Kohlenwasserstoffrest; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formen I wie vorstehend ausgeführt oder nach einem der Ansprüche 1 bis 7, und/oder entsprechende enantiomere, diastereomere und/ oder tautomere Formen davon und/oder pharmazeutisch annehmbare Salze davon, wobei die Reste **Q, a**, **R_{Q}¹, R_{Q}²**, **R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 9 genannt haben und die nachfolgenden Reste folgende Definition besitzen:
**W**: einen Rest der allgemeinen Formel 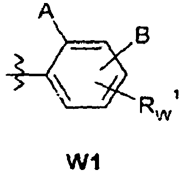
**A:** F. Cl, OCF₃. OCHF₂, jeweils gegebenenfalls substituiertes C₁-C₆-Alkyl, O-C₁-C₈-Alkyl, S-C₁-C₆-Alkyl oder OR_{A}¹;
**B:** Wasserstoff, F, Cl, CF₃, OCF₃, OCHF₂, jeweils gegebenenfalls substituiertes C₁-C₆-Alkyl, O-C₁-C₆-Alkyl oder S-C₁-C₆-Alkyl;
**R_{w}¹:** Wasserstoff, F, Cl, CN, CF₃ oder O-CF₃;
**R_{A}¹:** jeweils gegebenenfalls substituiertes C₁-C₆-Alkyl oder Phenyl; bereitgestellt

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 8, und/oder entsprechende enantiomere, diastereomere und/ oder tautomere Formen davon und/oder pharmazeutisch annehmbare Salze davon wobei die Reste **W, A, R_{A}¹, R_{A}², R_{A}³, R_{A}⁴, B, R_{w}¹, D, R₁, R₂, R₃, X, Y, Z, R⁴, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 10 genannt haben und die nachfolgenden Reste folgende Definition besitzen:
**Q:** -**CR_{Q}¹R_{Q}²**-;
**R_{Q}^{1,} R_{Q}²** jeweils unabhängig voneinander Wasserstoff, F, oder CH₃ : bereitgestellt

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 9, und/oder entsprechende enantiomere, diastereomere und/ oder tautomere Formen davon und/oder pharmazeutisch annehmbare Salze davon; wobei die Reste **W, A, R_{A}¹, R_{A}², R_{A}³, R_{A}⁴, B, R_{w}¹, D, Q, a, R_{Q}¹, R_{Q}², X, Y, Z, R₄, R₅, R₆, R₇, R_{Z,Y}, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 9 genannt haben und die nachfolgenden Reste folgende Definition besitzen:
**R¹, R²** unabhängig voneinander:
   Wasserstoff. OH, CN, O-Methyl, O-Phenyl, Acetyl. Benzoyl. O-Acetyl, oder O-Benzoyl;
**R³** Freies Elektronenpaar oder Wasserstoff; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 10, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{w}¹, D, Q, a, R_{Q}¹, R_{Q}², X, Y, Z, R₄, R₅, R₆, R₇, R_{Z,Y}¹. R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 10 genannt haben und die nachfolgenden Reste die folgende Definition besitzen:
**R¹, R²** Wasserstoff
**R³** Freies Elektronenpaar oder Wasserstoff: bereitgestellt

Jede dieser vorstehend genannten Definitionen einer Variablen kann mit beliebigen der vorstehend genannten Definitionen der restlichen Variablen. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 11, entsprechende enantiomere, diastereomere und/ oder tautomere Formen davon und/oder pharmazeutisch annehmbare Salze davon wobei die Reste **W, A, R_{A}¹, R_{A}², R_{A}³, R_{A}⁴, B, R_{w}¹, D, Q, a**, **R_{Q}¹, R_{Q}², R₁, R₂, R₃, R₄, R₅, R₆, R₇, R_{Z,Y}¹**, **R_{Z,Y}^{Z}, R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie vorstehend ausgeführt oder in einem der Ansprüche 1 bis 11 genannt haben und die nachfolgenden Reste die folgende Definition besitzen:
**X, Y, Z** X = N, Y = CR⁴, Z = CR⁶; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 12, und/oder entsprechende enantiomere, diastereomere und/ oder tautomere Formen davon und/oder pharmazeutisch annehmbare Salze davon wobei die Reste **W, A, R_{A}¹, R_{A}², R_{A}³, R_{A}⁴, B, R_{w}¹, D, Q, a**, **R_{Q}¹, R_{Q}², R₁, R₂, R₃, X, Y, Z** soweit nachstehend nichts anderslautendes ausgeführt ist die gleiche Bedeutung wie vorstehend ausgeführt oder in einem der Ansprüche 1 bis 12 genannt haben und die nachfolgenden Reste folgende Definition besitzen:
**R⁴, R⁶** jeweils unabhängig voneinander einen Rest? ausgewählt aus der gleichen oder verschiedenen der Gruppen **1.), 3.), 4.)** oder **5.)** und
**R⁵, R⁷** einen Rest, ausgewählt aus der gleichen oder verschiedenen der Gruppen **2.) oder 3.),** wobei die Gruppen 1.), 2.), 3.) 4.) und 5.) wie folgt definiert sind:
   1.) Wasserstoff, Cf₃, CHF₂, oder jeweils gegebenenfalls substituiertes C₁-C₁₀-Alkyl, C₃-C₇-Cycloalkyl, C₁-C₆-Alkylen-C₃-C₇-Cycloalkyl? C₁-C₄-Alkylen-Aryl, C₁-C₄-AlkylenHetaryl, oder jeweils gegebenenfalls substituiertes NR_{Z,Y}⁷-CO-O-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁸R_{Z,Y}⁷ oder CO-NR_{Z,Y}⁵R_{Z,Y}⁷;
   2.) Freies Elektronenpaar oder Wasserstoff, oder
      jeweils gegebenenfalls substituiertes C₁-C₁₀-Alkyl, C₃-C₇-Cycloalkyl;
   3.) Phenyl oder Naphthyl, die jeweils mit **R_{Z,Y}¹, R_{Z,Y}²** und **R_{Z,Y}³** substituiert sind, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, CN, NH2, CF₃, CHF₂, OCF₃, OCHF₂, O-CH₂-COOH, Halogen, oder
      jeweils gegebenenfalls substituiertes Aryl, Hetaryl, Heterocycloalkyl, C₁-C₈-Alkyl, C₃-C₇-Cycloalkyl oder jeweils gegebenenfalls substituiertes O-R_{Z,Y}⁴, NR_{Z,Y}⁶R_{Z,Y}⁷, SO₂-R_{Z,Y}⁶R_{Z,Y}⁷, NR_{Z,Y}-SO₂-R_{Z,Y}⁵ oder
      jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend O, N, und S enthalten kann; wobei
      **R_{Z,Y}⁴** jeweils gegebenenfalls substituiertes Hetaryl, Aryl,
      oder
      C₁-C₆-Alkyl, das gegebenenfalls substituiert ist mit einem Substituenten ausgewählt aus der Gruppe, bestehend aus Halogen, CN, CF₃, CHF₂, OCF₃, OCHF₂, gegebenenfalls substituiertes NH-(C₁-C₆-Alkyl) und gegebenenfalls substituiertes N(C₁-C₆-Alkyl)₂;
      **R_{Z,Y}⁵** gegebenenfalls substituiertes C₁-C₆-Alkyl, oder
      jeweils gegebenenfalls 1-, 2- oder bis zu 3-fach substituiertes Aryl oder Hetaryl, wobei dabei substituiert mit Substituenten unabhängig voneinder ausgewählt aus der Gruppe bestehend aus Halogen, NH₂, CN, CF₃, CHF₂, OCF₃, OCHF₂, NH-(C₁-C₆-Alkyl) oder N(C₁-C₆-Alkyl)₂, oder
      gegebenenfalls substituiertes C₁-C₆-Alkyl, oder jeweils gegebenenfalls substituiertes
      O-R_{A}¹, NR_{A}²R_{A}³, SO₂NH₂, SO₂-R_{A}¹, NR_{A}⁴-SO₂-R_{A}¹, oder SO₂-N R 2R 3.
      **R_{Z,Y}⁶** Wasserstoff, oder
      jeweils gegebenenfalls substituiertes C₁-C₆-Alkyl, Aryl, C₁-C₄-Alkylen-Aryl, C₁-C₄-Alkylen-Hetaryl, SO₂-C₁-C₆-Alkyl, SO₂-Aryl, SO₂-Hetaryl oder SO₂-C₁-C₄-Alkylen-Aryl;
      **R_{Z,Y}⁷** Wasserstoff oder gegebenenfalls substituiertes C₁-C₆-Alkyl;
      oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
   4.) einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit R_{Z,Y}¹, R_{Z,Y}² und R_{Z,Y}³ wie vorstehend definiert substituiert sein kann;
   5.) gegebenenfalls substituiertes Adamantyl;
   bedeuten; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel wie vorstehend ausgeführt oder nach einem der Ansprüche 1 bis 13, und/oder entsprechende enantiomere, diastereomere und/ oder tautomere Formen davon und/oder pharmazeutisch annehmbare Salze davon wobei die Reste **R_{Q}1, R_{Q}²**, **R₄, R₅, R₆, R₇, R_{Z,Y}¹, R_{Z,Y}², R_{Z,Y}³, R_{Z,Y}⁴, R_{Z,Y}⁵, R_{Z,Y}⁸, R_{Z,Y}⁷, R_{Z,Y}⁸** soweit nachstehend nichts anderslautendes ausgeführt ist, die wie vorstehend ausgeführt oder in einem der Ansprüche 1 bis 15 genannten Definitionen besitzen und die nachfolgenden Reste die folgenden Definitionen besitzen:
**W**: einen Rest der allgemeinen Formel 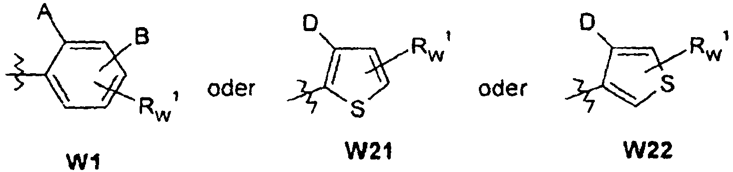
**A**: NH₂, OCF₃, OCHF₂, COOH, O-CH₂-COOH, SH, oder jeweils gegebenenfalls substituiertes O-C₁-C₆-Alkyl, S-C₁-C₆-Alkyl. C₁-C₆-Alkyl, oder jeweils gegebenenfalls substituiertes
   O-R_{A}¹, CO-R_{A}¹, S-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, SO₂-NR_{A}²R_{A}³ oder CO-NR_{A}²R_{A}³; wobei
**R_{A}¹** jeweils gegebenenfalls substituiertes C₁-C₅-Alkyl, C₃-C₇-Cycloalkyl, Phenyl oder Benzyl;
**R_{A}²; R_{A}³** unabhängig voneinander
   Wasserstoff, oder jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₄-Alkyl, SO₂-Aryl, SO₂-Hetaryl, oder SO₂-C₁-C₄-Alkylen-Aryl;
   oder beide Reste **R_{A}²** und **R_{A}³** bilden zusammen mit dem Stickstoff einen jeweils gegebenenfalls substituierten 5-oder 6-gliedrigen gesättigten oder ungesättigten Ring, der ein oder bis zu zwei gleiche oder verschiedene Heteroatome ausgewählt aus der Gruppe bestehend aus O und N enthalten kann;
**R_{A}⁴**; Wasserstoff oder einen gegebenenfalls substituierten C₁-C₆-Alkylrest;
**B**: Wasserstoff, Halogen, CN, CF₃, -CHF₂, OCF₃, OCHF₂, oder jeweils gegebenenfalls substituiertes C₁-C₆-Alkyl oder C₂-C₆-Alkenyl, oder jeweils gegebenenfalls substituiertes -O-CH₂-COO-R_{A}¹, - O-R_{A}¹, S-R_{A}¹, NR_{A}²R_{A}³, -NR_{A}⁴-CO-R_{A}¹ oder-CO-NR_{A}²R_{A}³; NR_{A}⁴-SO₂-R_{A}¹;
   oder B bildet zusammen mit **R_{w}¹** einen der Reste -(CH₂)₃-, -O-CH₂-O-, -O-(CH₂)₂-O-, -CH=CH-CH=CH-, -O-(CH₂)₂-, -(CH₂)₂-O-;
**R_{w}¹**: Wasserstoff, F, Cl, CN, CF₃, O-CF₃, oder
   jeweils gegebenenfalls substituiertes C₁-C₆-Alkyl, Aryl, C₁-C₆-Alkylamino oder C₁-C₆-Dialkylamino;
**Q**:
**R_{Q}¹**, **R_{Q}²**, unabhängig voneinander:
   Wasserstoff, Halogen, OH, gegebenenfalls substituiertes C₁-C₆-Alkyl;
**R¹**, **R²**, unabhängig voneinander:
   Wasserstoff, OH, CN, oder jeweils gegebenenfalls substituiertes C₁-C₆-Alkyl, C₁-C₆-Alkylen-O-C₁-C₆-Alkyl, substituiertes Aryl, Benzyl, CO-C₁-C₆-Alkyl, CO-Aryl, oder CO-C₁-C₄-Alkylen-Aryl;
**R³** Freies Elektronenpaar oder Wasserstoff;
**X**, **Y**, **Z** X = N, Y = CR⁴, Z = CR⁶, oder
   X = C, Y = CR⁴, Z = NR⁷, oder
   X=N, Y=N, Z=CR⁶
**R⁴**, **R⁶** jeweils unabhängig voneinander einen Rest, ausgewählt aus der gleichen oder verschiedenen der Gruppen **1.)**, **3.)**, **4.)** oder **5.)** und
**R⁵**, **R⁷** jeweils unabhängig voneinander einen Rest, ausgewählt aus der gleichen oder verschiedenen der Gruppen **2.), 3.)** oder **4.)** oder **5.),** mit den folgenden Bedeutungen für die Gruppen 1.) bis 5.):
   1.) Wasserstoff, CN, CF₃, CHF₂, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, oder C₁-C₄-Alkylen-Hetaryl, oder
      jeweils gegebenenfalls substituiertes O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   2.) Freies Elektronenpaar oder Wasserstoff,
      jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₆-Alkyl oder SO₂-R_{Z,Y}⁵, SO₂NH₂, CONH₂, oder jeweils gegebenenfalls substituiertes SO₂-NR_{Z,Y}⁶R_{Z,Y}⁷ oder CO-NR_{Z,Y}⁶R_{Z,Y}⁷;
   3.) Phenyl, 1-Naphthyl oder 2-Naphthyl, die jeweils mit **R_{Z,Y}¹, R_{Z,Y}²** und **R_{Z,Y}³** substituiert sind oder sein können, wobei
      **R_{Z,Y}¹, R_{Z,Y}²** und **R_{Z,Y}³** jeweils unabhängig voneinander einen Substituenten ausgewählt aus der folgenden Gruppe darstellen:
      Wasserstoff, NH₂, CN, CF₃, CHF₂, OCF₃, OCHF₂, O-CH₂-COOH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
      O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}-SO₂-R_{Z,Y}⁵, SO₂NH₂, SO₂-NR_{Z,Y}⁶R_{Z,Y}⁷, CO₂NH₂, oder CO-NR_{Z,Y}⁶R_{Z,Y}⁷, oder
      jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
      **R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, Hetaryl, C₁-C₄-AlkylenAryl, oder
      C₁-C₆-Alkyl, das gegebenenfalls substituiert ist mit einem Substituenten ausgewählt aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) und gegebenenfalls substituiertes N(C₁-C₆-Alkyl)₂;
      **R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, oder jeweils gegebenenfalls 1-, 2- oder bis zu 3-fach substituiertes Aryl oder Hetaryl, wobei substituiert mit Substituenten
      unabhängig voneinder ausgewählt aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, COOH, O-CH₂-COOH, SH, oder
      jeweils gegebenenfalls substituiertes C₁-C₆-Alkyl, C₃-C₇-Cycloalkyl, Aryl, Hetaryl, Heterocycloalkyl, oder
      O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
      **R_{Z,Y}⁶** Wasserstoff, oder
      jeweils gegebenenfalls substituiertes C₁-C₆-Alkyl, C₃-C₇-Cycloalkyl, C₁-C₄-Alkylen-C₃-C₇-Cycloalkyl, C₁-C4-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-AlkylenHetaryl;
      **R_{Z},_{Y}⁷** Wasserstoff oder gegebenenfalls substituiertes C₁-C₆-Alkyl;
      oder die Reste R_{Z,Y}⁵ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
   4.) einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** wie vorstehend definiert substituiert sein kann;
   5.) einen gegebenenfalls substituierten C₅-C₁₀- bi- oder tricyclischen, gesättigten Kohlenwasserstoffrest;
   bedeuten, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegende Erfindung wird mindestens eine 5-Ring-Heteroaromaten-Verbindung der allgemeinen Formel I wie vorstehend ausgeführt oder nach einem der Ansprüche 1 bis 14, und/oder entsprechende enantiomere, diastereomere und/ oder tautomere Formen davon und/oder pharmazeutisch annehmbare Salze davon, wobei die Reste W, A, R_{A}¹, R_{A}², R_{A}³, R_{A}⁴, B, R_{W}¹, D, Q, a, R_{Q}¹, R_{Q}², R₁ R₂, R₃, X, Y, Z soweit nachstehend nichts anderslautendes ausgeführt ist, die wie vorstehend ausgeführt oder in einem der Ansprüche 1 bis 16 genannten Bedeutungen besitzen und die nachfolgenden Reste die folgenden Definitionen besitzen:
**W:** einen Rest der allgemeinen Formel 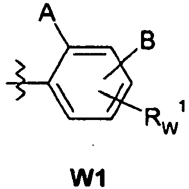
**A:** F, Cl OCF₃, OCHF₂, jeweils gegebenenfalls substituiertes C₁-₆-Alkyl, O-C₁-C₆-Alkyl oder S-C₁-C₆-Alkyl oder gegebenenfalls substituiertes OR_{A}¹;
**R_{A}¹:** jeweils gegebenenfalls substituiertes C₁-C₆-Alkyl oder Phenyl
**B:** Wasserstoff, F, Cl, CF₃, OCF₃, OCHF₂, oder jeweils gegebenenfalls substituiertes C₁-C₆-Alkyl, O-C₁-C₆-Alkyl oder S-C₁-C₆-Alkyl;
**R_{w}¹:** Wasserstoff, F, Cl, CN, CF₃ oder O-CF₃;
**Q:** -CR_{Q}¹R_{Q}²-;
**R_{Q}^{1,} R_{Q}²** jeweils unabhängig voneinander Wasserstoff, F, CH₃;
**R¹, R²** unabhängig voneinander:
   Wasserstoff, OH, CN, O-Methyl-, O-Phenyl, Acetyl, Benzoyl, O-Acetyl, O-Benzoyl;
**R³** Freies Elektronenpaar oder Wasserstoff
**X,Y,Z** X = N, Y = CR⁴, Z = CR⁶, _{oder}
   X=N,Y=N,Z=CR⁶
**R⁴, R⁶** jeweils unabhängig voneinander einen Rest, ausgewählt aus der gleichen oder verschiedenen der Gruppen **1.), 3.), 4.)** oder **5.)** und
**R⁵** einen Rest, ausgewählt aus der gleichen oder verschiedenen der Gruppen **2.)** oder **3.),** wobei die Gruppen 1.) bis 5.) bedeuten:
   1.) Wasserstoff, CN, CF₃, CHF₂, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, oder jeweils gegebenenfalls substituiertes
      O-R_{Z,Y}⁴, NR_{Z,Y}⁶R_{Z,Y}⁷, CO-OR_{Z,Y}⁵, NR_{Z,Y}-CO-O-R_{Z,Y}⁵, O-CH₂-COO-R_{Z,Y}⁵,NR_{Z,Y}⁷-CO-R_{Z,Y}⁵, SO₂-R_{Z,Y}⁵ NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   2.) Freies Elektronenpaar oder Wasserstoff,
      jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₄-Alkyl oder
      SO₂-R_{Z,Y}⁵, SO₂NH₂, CONH₂, gegebenenfalls substituiertes SO₂-NR_{Z,Y}⁶R_{Z,Y}⁷ oder gegebenenfalls substituiertes CO-NR_{Z,Y}⁶R_{Z,Y}⁷;
   3.) Phenyl, 1-Naphthyl oder 2-Naphthyl, die jeweils mit **R_{Z,Y}¹, R_{Z,Y}²** und **R_{Z,Y}³** substituiert sein können, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, NH₂, CN, CF₃, CHF₂, OCF₃, OCHF₂, O-CH₂-COOH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
      O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷ oder CO-NR_{Z,Y}⁶R_{Z,Y}⁷, oder
      jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
      **R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, Hetaryl, C₁-C₄-AlkylenAryl, oder
      C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) und gegebenenfalls substituiertes N(C₁-C₆-Alkyl)₂;
      **R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, oder jeweils gegebenenfalls 1-, 2- oder bis zu 3-fach substituiertes Aryl oder Hetaryl, wobei substituiert mit Substituenten unabhängig voneinder ausgewählt aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, COOH, O-CH₂-COOH, SH, oder
      jeweils gegebenenfalls substituiertes C₁-C₆-Alkyl, C₃-C₇-Cycloalkyl, Aryl, Hetaryl, Heterocycloalkyl, oder jeweils gegebenenfalls substituiertes
      O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
      **R_{Z,Y}⁶** Wasserstoff, oder
      jeweils gegebenenfalls substituiertes C₁-C₆-Alkyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-AlkylenHetaryl;
      **R_{Z,Y}⁷** Wasserstoff oder gegebenenfalls substituiertes C₁-C₆-Alkyl;
      oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
   4.) einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** wie vorstehend definiert substituiert sein kann;
   5.) gegebenenfalls substituiertes Adamantyl; bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend ausgeführt oder nach einem der Ansprüche 1 bis 15, und/oder entsprechende enantiomere, diastereomere und/ oder tautomere Formen davon und/oder pharmazeutisch annehmbare Salze davon, wobei die Reste W, A, R_{A}¹, R_{A}², R_{A}³, R_{A}⁴, B, R_{W}¹, D, Q, a, R_{Q}¹, R_{Q}², R₁, R₂, R₃, X, Y, Z soweit nachstehend nichts anderslautendes ausgeführt ist, die wie vorstehend ausgeführt oder in einem der Ansprüche 1 bis 15 genannten Bedeutungen besitzen und und die nachfolgenden Reste die folgenden Definitionen besitzen:
**W:** einen Rest der allgemeinen Formel 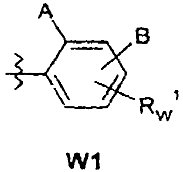
**A:** OCF₃, OCHF₂, OCH₃, Methyl, O-Ethyl, O-Propyl oder O-iPropyl;
**B:** Wasserstoff, F. Cl, CF₃, OCF₃, OCHF₂, gegebenenfalls substituiertes C₁-C₆-Alkyl oder gegebenenfalls substituiertes O-C₁-C₆-Alkyl;
**R_{W}¹:** Wasserstoff, F, Cl;
**Q:** -CH₂-;
**R¹, R²** Wasserstoff,
**R³** Freies Elektronenpaar oder Wasserstoff
**X** N
**Y** CR⁴
**Z** CR⁶
**R⁴, R⁶** jeweils unabhängig voneinander einen Rest, ausgewählt aus der gleichen oder verschiedenen der Gruppen **1.), 2.)** oder **3.),** wobei die Gruppen 1.) bis 3.) bedeuten:
   **1.)** Wasserstoff, CF₃, CHF₂, oder jeweils gegebenenfalls substituiertes C₁-C₁₀-Alkyl, oder
   **2.)** Phenyl, 1-Naphthyl oder 2-Naphthyl, die jeweils mit **R_{Z,Y}¹, R_{Z,Y}²** und **R_{Z,Y}³** substituiert sind, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, CN, CF₃, CHF₂, OCF₃, OCHF₂, O-CH₂-COOH, Halogen, oder
      jeweils gegebenenfalls substituiertes C₁-C₆-Alkyl, Aryl, oder jeweils gegebenenfalls substituiertes O-R_{Z,Y}⁴, NR_{Z,Y}⁶R_{Z,Y}⁷,NR_{Z,Y}⁷-SO₂-R_{Z,Y}⁵ oder
      jeweils zwei der Reste aus **R_{Z,Y}¹, R_{Z,Y}²** oder **R_{Z,Y}³** bilden zusammen einen der Reste
      -O-CH₂-O-, -O-CH₂-CH₂-O-, -O-CH₂-CH₂- oder -CH₂-CH₂-CH₂-;
      **R**_{Z,Y}⁴ jeweils gegebenenfalls substituiertes Hetaryl, Aryl, oder C₁-C₆-Alkyl;
      **R**_{Z,Y}⁵ gegebenenfalls substituiertes C₁-C₆-Alkyl, oder
      jeweils gegebenenfalls 1-, 2- oder bis zu 3-fach substituiertes Aryl oder Hetaryl, wobei substituiert mit Substituenten unabhängig voneinder ausgewählt aus der Gruppe bestehend aus Halogen, NH₂, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, oder gegebenenfalls substituiertes C₁-C₆-Alkyl, oder
      jeweils gegebenenfalls substituiertes O-R_{A}¹, NR_{A}²R_{A}³, SO₂NH₂, SO₂-R_{A}¹, NR_{A}⁴-SO₂-R_{A}¹, oder SO₂-NR_{A}²R_{A}³;
      **R**_{Z,Y}⁶ Wasserstoff, oder
      jeweils gegebenenfalls substituiertes C₁-C₆-Alkyl, Aryl, SO₂-C₁-C₆-Alkyl, SO₂-Aryl;
      **R_{Z,Y}⁷** Wasserstoff oder gegebenenfalls substituiertes C₁-C₆-Alkyl;
   3.) Benzothiophenyl, Benzofuranyl, Chinolinyl, Isochinolinyl, Thienyl, Furanyl, Pyridinyl, Pyrimidinyl, oder Thiazolyl die jeweils mit **R_{Z,Y}¹**, **R_{Z,Y}²** und **R_{Z,Y}³** substituiert sein können, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird mindestens eine 5-Ring-Heteroaromaten-Verbindung der allgemeinen Formel I wie vorstehend beschrieben, und/oder entsprechende enantiomere, diastereomere und/ oder tautomere Formen davon und/oder pharmazeutisch annehmbare Salze davon, wobei die Reste R¹, R², R³, Q, W, X, Y und Z die wie vorstehend beschrieben genannten Bedeutungen haben sollen, zur Verwendung als Arzneimittel, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird eine pharmazeutische Zusammensetzung, enthaltend mindestens eine 5-Ring-Heteroaromaten-Verbindung der allgemeinen Formel I wie vorstehend beschrieben, und/oder entsprechende enantiomere, diastereomere und/ oder tautomere Formen davon und/oder pharmazeutisch annehmbare Salze davon, wobei die Reste R¹, R², R³, Q, W, X, Y und Z die wie vorstehend beschrieben genannten Bedeutungen haben sollen, sowie gegebenenfalls mindestens einen pharmazeutisch annehmbaren Träger und/oder Verdünnungsmittel, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I 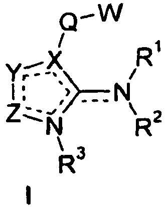 , und/oder entsprechende enantiomere, diastereomere und/ oder tautomere Formen davon und/oder pharmazeutisch annehmbare Salze davon, wobei die Reste R¹, R², R³, Q, W, X, Y und Z wie folgt definiert sind:
**W:** einen Rest der allgemeinen Formel **W11** oder **W2** 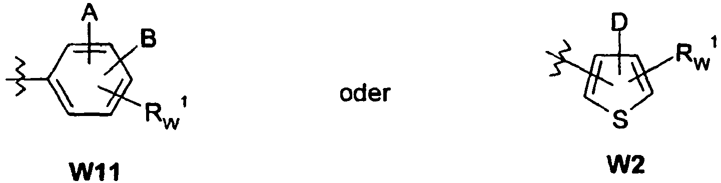 worin
**A:** NO₂, NH₂, CN, CF₃, OCF₃, CHF₂, OCHF₂, COOH, O-CH₂-COOH, Halogen, SH, oder jeweils gegebenenfalls substituiertes O-C₁-C₆-Alkyl, S-C₁-C₆-Alkyl, -O-CO-C₁-C₆-Alkyl, -O-CO-Aryl, -O-CO-Hetaryl, -O-COO-C₁-C₆-Alkyl oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-AlkylenHetaryl oder C₁-C₄-Alkylen-Aryl, oder jeweils gegebenenfalls substituiertes
   O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
**R_{A}¹:** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²**: Wasserstoff, oder
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-AlkylenHetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³**: jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-AlkylenHetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   oder die Reste **R_{A}²** und **R_{A}³** bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann; wobei gegebenenfalls zwei an diesem Heterocyclus substituierte Reste zusammen einen jeweils gegebenenfalls substituierten 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome ausgewählt aus der Gruppe bestehend aus O, N, und S enthalten kann, und wobei der dadurch gebildete Cyclus gegebenenfalls substituiert sein kann oder an diesem Cyclus ein weiterer, gegebenenfalls 3 bis 7-gliedriger jeweils gegebenenfalls substituierter Cyclus ankondensiert sein kann;
**R_{A}⁴**: Wasserstoff, oder
   jeweils gegebenenfalls substituiertes C₁-C₆-Alkyl, C₂-C₆-Alkenyl, C₂-C₁₂-Alkinyl, C₃-C₇-Cycloalkyl, Aryl, C₁-C₄-Alkylen-Aryl, C₁-C₆-Alkylen-O-C₁-C₆-Alkyl, Hetaryl;
**B:** Wasserstoff, OH oder unabhängig von **A** wie Rest **A** definiert ist,
   oder zwei der Reste **A, B** oder **R_{W}¹** bilden jeweils unabhängig voneinander zusammen mit den anhängenden C-Atomen einen 3 bis 7-gliedrigen, jeweils gegebenenfalls substituierten, gesättigten, ungesättigten oder aromatischen Carbocyclus oder einen jeweils gegebenenfalls substituierten 3 bis 7-gliedrigen jeweils gegebenenfalls substituierten, gesättigten, ungesättigten oder aromatischen Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen jeweils
   gegebenenfalls substituierten 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome ausgewählt aus der Gruppe bestehend aus O, N, und S enthalten kann und wobei der gebildete Cyclus gegebenenfalls substituiert sein kann;
**R_{W}¹:** Wasserstoff, OH, Halogen, NO₂, NH₂, CN, CF₃, CHF₂, CH₂F, OCF₃, OCHF₂, OCH₂F oder
   jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D:** unabhängig von A wie Rest **A** definiert ist;
**Q**: einen Rest der allgemeinen Formel **Q1** 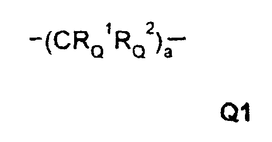 mit dem Index
   a = 1,
R_{Q}¹, R_{Q}², unabhängig voneinander :
   Wasserstoff, Halogen, OH, oder
   gegebenenfalls substituiertes C₁-C₅-Alkyl,
R¹, R² unabhängig voneinander:
   Wasserstoff, OH, CN, oder
   jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₅-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₅-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
   **R¹** und **R²** bilden zusammen mit dem Stickstoff einen 5 bis 7-gliedrigen, jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen jeweils gegebenenfalls substituierten Carbocyclus oder Heterocyclus bilden können, wobei der Heterocyclus ein, zwei oder bis zu drei verschiedene oder gleiche Heteroatome ausgewählt aus der Gruppe bestehend aus O, N, und S enthalten kann und wobei der gebildete Cyclus gegebenenfalls substituiert sein kann;
**R³** Freies Elektronenpaar oder Wasserstoff;
**X, Y, Z** X = N, Y = CR⁴, Z = CR⁶, oder
   X = C, Y = NR⁵,Z = CR⁶, oder
   X = C, Y = CR⁴ Z = NR⁷, oder
   X = N, Y = N, Z = CR⁶, oder
   X = N, Y = CR⁴, Z = N
**R⁴ , R⁶** jeweils unabhängig voneinander einen Rest, ausgewählt aus der gleichen oder verschiedenen Gruppen **1.), 3.), 4.) 5.)** oder **6.)** und
**R⁵, R⁷** jeweils unabhängig voneinander einen Rest, ausgewählt aus der gleichen oder verschiedenen Gruppen **2.), 3.) 4.)** oder **5.),** wobei die Gruppen 1.) bis 6.) wie folgt definiert sind:
   **1.)** H, NO₂, NH₂, CN, CF₃, CHF₂, OCF₃, OCHF₂, COOH, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes
      O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷CO-O-R_{Z,Y}⁵, O-CH₂-COO-R_{Z,Y}⁵, NR_{Z,Y}⁷-CO-R_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   **2.)** Freies Elektronenpaar oder Wasserstoff, CH₂-CF₃, CH₂-CHF₂, jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryₗ, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes
      CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
   **3.)** Phenyl, 1-Naphthyl oder 2-Naphthyl, die jeweils mit **R_{Z,Y}¹, R_{Z,Y}²** und **R_{Z,Y}³** substituiert sein können, wobei
      **R_{Z,Y}¹, R_{Z,Y}²** und **R_{Z,Y}³** jeweils unabhängig voneinander einen Substituenten aus der folgenden Gruppe darstellen:
      Wasserstoff, NO₂, NH₂, OH, CN, CF₃, CHF₂, OCF₃, OCHF₂, COOH, O-CH₂-COOH, SH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
      O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}⁷-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ oder CO-NR_{Z,Y}⁶R_{Z,Y}⁷, oder
      jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen, anellierten, gesättigten, ungesättigten oder aromatischen jeweils gegebenenfalls substituierten Carbocyclus oder Heterocyclus bilden können, wobei der Heterocyclus ein, zwei oder bis zu drei verschiedene oder gleiche Heteroatome ausgewählt aus der Gruppe bestehend aus O, N, und S enthalten kann und wobei der gebildete Cyclus gegebenenfalls substituiert sein kann und/oder an diesem Cyclus ein weiterer, 3 bis 7-gliedrigen, gegebenenfalls substituierter Cyclus ankondensiert sein kann;
      **R_{Z,Y}⁴** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Hetaryl, Aryl, C₁-C₄-AlkylenHetaryl, Hetaryl, C₁-C₄-Alkylen-Aryl, oder
      C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂;
      **R_{Z,Y}⁵** jeweils gegebenenfalls substituiertes C₁-C₆-Alkyl, C₂-C₆-Alkenyl, C₂-C₆-Alkinyl, C3-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, Benzofuranyl, Dihydrobenzofuranyl, Indanyl, oder jeweils gegebenenfalls 1-, 2- oder bis zu 3-fach substituiertes Aryl oder Hetaryl, wobei substituiert mit Substituenten unabhängig voneinander ausgewählt aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, COOH, O-CH₂-COOH, SH, oder
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder jeweils gegebenenfalls substituiertes
      O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-RA¹, SO₂-NR_{A}²R_{A}³ oder CO-NR_{A}²R_{A}³;
      **R_{Z,Y}⁶** Wasserstoff, oder
      jeweils gegebenenfalls substituiertes C₁-C₆-Alkyl, C₂-C₆-Alkenyl, C₂-C₆-Alkinyl_{;} C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, CO-O-C₁-C₄-Alkylen-Aryl, SO₂-C₁-C₆-Alkyl, SO₂-Aryl, SO₂-Hetaryl, SO₂-C₁-C₄-AlkylenAryl oder SO₂-C₁-C₄-Alkylen-Hetaryl;
      **R_{Z,Y}⁷** Wasserstoff oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-AlkylenAryl oder SO₂-C₁-C₄-Alkylen-Hetaryl;
      oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann, bilden; und jeweils gegebenenfalls zwei an diesem Heterocyclus substituierte Reste zusammen einen 3 bis 7-gliedrigen, anellierten, gesättigten, ungesättigten oder aromatischen jeweils gegebenenfalls substituierten Carbocyclus oder Heterocyclus bilden können, wobei der Heterocyclus bis zu drei verschiedene oder gleiche Heteroatome ausgewählt aus der Gruppe bestehend aus O, N, und S enthalten kann, und der gebildete Cyclus gegebenenfalls substituierte sein kann und/oder an diesem Cyclus ein weiterer, gegebenenfalls substituierter 3 bis 7-gliedriger Cyclus ankondensiert sein kann;
   4.) einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹**, **R_{Z,Y}²** und **R_{Z,Y}³** wie vorstehend definiert substituiert sein kann;
   5.) einen jeweils gegebenenfalls substituierten C₅-C₁₈- bi- oder tricyclischen, gesättigten Kohlenwasserstoffrest
   6.) **R⁴** und **R⁶** bilden zusammen mit X und Y einen C₆-C₁₀-gliedrigen gesättigten, ungesättigten oder aromatischen gegebenenfalls substituierten Carbocyclus, bevorzugt einen gegebenenfalls substituierten Benzo-Rest, die jeweils mit einem, zwei oderbis zu drei Resten substituiert sein können, unabhängig voneinander ausgewählt aus der Gruppe **R_{Z,Y}⁸**.
      **R_{Z,Y}⁸**: Wasserstoff, NO₂, NH₂, OH, CN, CF₃, CHF₂, OCF₃, OCHF₂, COOH, O-CH₂-COOH, SH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-AlkylenHetaryl, oder jeweils gegebenenfalls substituiertes
      O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷
   zur Herstellung eines Medikaments zur Behandlung und/oder Prophylaxe von ZNS Erkrankungen oder von ZNS bezogenen Erkrankungen, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/ oder tautomere Formen davon und/oder pharmazeutisch annehmbare Salze davon , wobei die Reste R¹, R², R³, Q, W, X, Y und Z wie vorstehend beschrieben definiert sind, zur Herstellung eines Medikaments zur Behandlung und/oder Prophylaxe von ZNS Erkrankungen und/oder von ZNS bezogenen Erkrankungen, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird dieVerwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon, wobei die Reste R¹, R², R³, Q, W, X, Y und Z wie vorstehend ausgeführt definiert sind, zur Herstellung eines Medikaments zur Behandlung und/oder Prophylaxe von neuropathologischen, neuropsychiatrischen und/oder neurodegenerativen Störungen, Symptomen und/oder Fehlfunktionen, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon , wobei die Reste R¹, R², R³, Q, W, X, Y und Z wie vorstehend ausgeführt definiert sind, zur Herstellung eines Medikamentes zur Behandlung und/oder Prophylaxe von Migräne und/oder Gehirnschädigungen, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegende Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon _{,} wobei die Reste R¹, R², R³, Q, W, X, Y und Z wie vorstehend ausgeführt definiert sind, zur Herstellung eines Medikamentes zur Behandlung und/oder Prophylaxe, on neuropathologischen, neuropsychiatrischen und/oder neurodegenerativen Krankheiten ausgewählt aus der Gruppe bestehend aus cerebraler Ischämie, Schlaganfall, Epilepsie und Anfälle im allgemelnen, Psychosen, Schizophrenie, Autismus, OCD-Syndrom, cognitiven Erkrankungen, Aufmerksamkeitsstörungen, Depressionen, bipolaren und/oder unipolaren Depressionen, Angstzuständen, Demenz, seniler Demenz, Alzheimer Demenz, demyelinisierenden Erkrankungen, Multipler Sklerose und Gehirntumoren, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel 1 wie vorstehend beschrieben, und/oder entsprechende enantiomere, diastereomere und/oder tautomere Formen davon und/oder pharmazeutisch annehmbare Salze davon , wobei die Reste R¹, R², R³, Q, W, X, Y und Z wie vorstehend ausgeführt definiert sind, zur Herstellung eines Medikamentes zur Behandlung und/oder Prophylaxe von Krankheiten ausgewählt aus der Gruppe bestehend aus cerebrovaskulären Störungen, Schmerz, Schmerz-bedingten Störungen, Abhängigkeit, Drogen-bedingten Störungen, Amnesie, Alkoholmissbrauch, Drogenmissbrauch, Störungen des circadianen Rhythmus und Cushing Syndrom, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon , wobei die Reste R¹, R², R³, Q, W, X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/ oder tautomere Formen davon und/oder pharmazeutisch annehmbare Salze davon, wobei die Reste R¹, R², R³, Q, W, X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden, bereitgestellt, wobei die Modulation der 5-HT5-Rezeptoraktivität ausgewählt ist aus der Gruppe bestehend aus Antagonisierung (Antagonist), Agonisierung (Agonist), partielle Agonisierung (partieller Agonist), inverse Agonisierung (inverser Agonist), partielle inverse Agonisierung (partieller inverser Agonist). Bevorzugt sind Substanzen mit antagonister Wirkung auf den 5-HT5-Rezeptor, also Antagonisten oder partielle Agonisten. Besonders bevorzugt sind Antagonisten des 5-HT5-Rezeptor.

Im Sinne der Erfindung bedeuten die Begriffe "Agonist" eine Substanz,die am Rezeptor (hier: 5-HT5-Rezeptor) einem dem physiologischen Liganden gleichartigen Effekt hervorruft, "Antagonist" eine Substanz, die die biologische Wirkung eines Agonisten reduziert oder aufhebt. "partieller Agonist" eine Substanz, die am Rezeptor eine submaximale Wirkung hervorruft, wobei in Abwesenheit eines Agonisten der partielle Agonist agonistisch und in Anwesenheit eines Agonisten der partielle Agonist antagonistisch wirken kann, und "inverser Agonist" eine Substanz, die eine negative Wirkung hervorruft, "kompetitiver Antagonist".eine Substanz mit Affinität zum Rezeptor, reversibler Bindung an den Rezeptor (Konkurrenz zum Agonisten) und keiner intrinsischen Aktivität am Rezeptor (relative Wirkstärke: Fähigkeit einer Substanz, bei gleichem Rezeptorbesatz einen Effekt auszulösen), und "nicht-kompetitiver Antagonist" eine Substanz mit allosterischer Bindung am Rezeptor und Beeinflussung der Wirkstärke (und gegebenenfalls Agonist-Bindung) durch Konformationsänderung des Rezeptors.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon, wobei die Reste R¹, R², R³, Q, W, X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor beruht, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon , wobei die Reste R¹, R², R³, Q, W, X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor beruht, bereitgestellt, wobei die Modulation der 5-HT5A-Rezeptoraktivität ausgewählt ist aus der Gruppe bestehend aus Antagonisierung (Antagonist), kompetitive Antagonisierung (kompeitiver Antagonist), nicht-kompetitive Antagonisierung ("mixed-type inhibition") (nicht-kompetitiver Antagonist), Agonisierung (Agonist), partielle Agonisierung (partieller Agonist), inverse Agonisierung (inverser Agonist), partielle inverse Agonisierung (partieller inverser Agonist). Bevorzugt sind Substanzen mit antagonister Wirkung auf den 5-HT5A-Rezeptor, also Antagonisten oder partielle Agonisten. Besonders bevorzugt sind Antagonisten des 5-HT5A-Rezeptor.

Jede dieser vorstehend genannten Definitionen einer Variablen kann mit beliebigen der vorstehend genannten Definitionen der restlichen Variablen. Dies gilt insbesondere für die Kombination von bevorzugten Definitionen einer Variablen mit beliebigen oder bevorzugten Definitionen der restlichen Variablen.

Gemäß einem weiteren Aspekt der vorliegende Erfindung wird die Verwendung von mindestens einer 5-Ring-Heteroaromaten-Verbindung der allgemeinen Formel I wie vorstehend beschrieben, und/oder entsprechende enantiomere, diastereomere und/ oder tautomere Formen davon und/oder pharmazeutisch annehmbare Salze davon, wobei die Reste R¹, R², R³, Q, W,

X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor mit einer Bindungsaffinität (Ki) von kleiner oder gleich 600 nM beruht, bereitgestellt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einem weiteren Aspekt der vorliegenden Erfindung wird die Verwendung von mindestens einer 5-Ring-Heteroaromaten-Verbindung der allgemeinen Formel I wie vorstehend beschrieben, und/oder entsprechende enantiomere, diastereomere und/oder tautomere Formen davon und/oder pharmazeutisch annehmbare Salze davon , wobei die Reste R¹, R², R³, Q, W, X, Y und Z wie vorstehend ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor mit einer Bindungsaffinität (Ki) von kleiner oder gleich 600 nM beruht, bereitgestellt, wobei die Modulation der 5-HT5A-Rezeptoraktivität ausgewählt ist aus der Gruppe bestehend aus Antagonisierung (Antagonist), Agonisierung (Agonist), partielle Agonisierung (partieller Agonist), inverse Agonisierung (inverser Agonist), partielle inverse Agonisierung (partieller inverser Agonist). Bevorzugt sind Substanzen mit antagonister Wirkung auf den 5-HT5A-Rezeptor, also Antagonisten oder partielle Agonisten. Besonders bevorzugt sind Antagonisten des 5-HT5A-Rezeptor.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

Gemäß einer bevorzugten Ausführungsform Verwendung von mindestens einer 5-Ring-Heteroaromaten-Verbindung der allgemeinen Formel I wie vorstehend beschrieben und die Reste R¹, R², R³, Q, W, X, Y und Z wie vorstehend ausgeführt definiert sind, bereitgestellt, wobei die Behandlung und/oder Prophylaxe in einem menschlichen oder nicht menschlichen Säugetier erfolgt.

Jede dieser vorstehend genannten Definitionen einer Variablen kann mit beliebigen der vorstehend genannten Definitionen der restlichen Variablen kombiniert werden. Dies gilt insbesondere für die Kombination von bevorzugten Definitionen einer Variablen mit beliebigen oder bevorzugten Definitionen der restlichen Variablen.

### Ausführliche Beschreibung der Erfindung

In bevorzugten Ausführungsformen besitzen die Reste der Formeln I die folgenden Bedeutungen:
W ist wie vorstehend definiert und stellt vorzugsweise W1 dar.
A befindet sich in der 2-Position am Ring, ist wie vorstehend definiert und bedeutet vorzugsweise
Halogen, NH₂, CN, CF₃, CHF₂, OCF₃, OCHF₂, oder jeweils gegebenenfalls substituiertes O-C₁-C₆-Alkyl, S-C₁-C₆-Alkyl, oder jeweils gegebenenfalls substituiertes
O-R_{A}¹, S-R_{A}¹, O-CH₂-COO-R_{A}¹, NR_{A}²R_{A}³, SO₂NH₂. SO₂-R_{A}¹, NR_{A}⁴-SO₂-R_{A}¹, oder SO₂-NR_{A}²R_{A}³

Besonders bevorzugt für A ist: F, Cl, OCF₃, OCHF₂, jeweils gegebenenfalls substituiertes C₁-C₆-Alkyl, O-C₁-C₆-Alkyl oder S-C₁-C₆-Alkyl oder O-R_{A}1; Noch bevorzugter ist A = F, Cl, OCF₃, OCHF₂, jeweils gegebenenfalls substituiertes C₁-C₆-Alkyl, O-C₁-C₆-Alkyl, O-Benzyl, O-Phenyl oder S-C₁-C₆-Alkyl. Davon noch bevorzugter ist A = F, Cl, OCF₃, OCHF₂, C₁-C₆-Alkyl, O-C₁-C₆-Alkyl oder S-C₁-C₆-Alkyl. Am meisten bevorzugt ist A = Methyl, OCF₃, OCH₃, O-Ethyl, O-n-Propyl oder O-i-Propyl.

R_{A}¹ ist wie vorstehend definiert und bedeutet vorzugsweise jeweils gegebenenfalls substituiertes C₁-C₆-Alkyl, C₃-C₇-Cycloalkyl, Phenyl oder Benzyl. Noch bevorzugter ist R_{A}¹ Methyl, Ethyl, n-Propyl oder i-Propyl. Am meisten bevorzugt ist R_{A}¹ Methyl oder Ethyl.

R_{A}² ist wie vorstehend definiert und bedeutet vorzugsweise
Wasserstoff oder
jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₄-Alkyl, SO₂-Aryl, SO₂-Hetaryl, oder SO₂-C₁-C₄-Alkylen-Aryl.
Noch bevorzugter ist R_{A}² Wasserstoff, C₁-C₆-Alkyl, Phenyl oder Benzyl.

In einer Ausführungsform ist R_{A}² vorzugsweise Wasserstoff, Methyl, Ethyl, n-Propyl, i-Propyl oder Phenyl.

R_{A}³ ist wie vorstehend definiert und bedeutet vorzugsweise jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₄-Alkyl, SO₂-Aryl, SO₂-Hetaryl, oder SO₂-C₁-C₄-AlkylenAryl.

Noch bevorzugter ist R_{A}³ C₁-C₆-Alkyl, Phenyl oder Benzyl, am meisten bevorzugt Methyl , Ethyl, n-Propyl oder i-Propyl oder Phenyl.

Die beiden Reste R_{A}² und R_{A}³ können auch, wie vorstehend beschrieben, zusammen mit dem Stickstoff einen 3-7-gliedrigen Heterocyclus bilden. Dabei bilden beide Reste R_{A}² und R_{A}³ vorzugsweise zusammen einen jeweils gegebenenfalls substituierten 5-oder 6-gliedrigen gesättigten oder ungesättigten Ring, der ein oder zwei weitere gleiche oder verschiedene Heteroatome aus der Gruppe O, N und S enthalten kann.

R_{A}⁴ ist wie vorstehend definiert und bedeutet vorzugsweise Wasserstoff oder einen gegebenenfalls substituierten C₁-C₆-Alkyl-Rest. Am meisten bevorzugt ist R_{A}⁴ Wasserstoff, Methyl, Ethyl, n-Propyl oder i-Propyl.

B ist wie vorstehend definiert und bedeutet vorzugsweise Wasserstoff, Halogen, CN, CF₃, CHF₂, OCF₃, OCHF₂,
jeweils gegebenenfalls substituiertes C₁-C₆-Alkyl oder C₂-C₆-Alkenyl, -O-CH₂-COO-R_{A}¹, O-R_{A}¹, S-R_{A}¹, NR_{A}²R_{A}³, -NR_{A}⁴-CO-R_{A}¹ oder -CO-NR_{A}⁴-R_{A}¹.

Besonders bevorzugt ist B Wasserstoff, Halogen, OH, OCF₃, OCHF₂, jeweils gegebenenfalls substituiertes C₁-C₆-Alkyl, O-R_{A}¹ oder S-R_{A}¹.

Noch bevorzugter ist B Wasserstoff, Halogen, OCF₃, OCHF₂, jeweils gegebenenfalls substituiertes C₁-C₆-Alkyl, O-C₁-C₆-Alkyl, O-Benzyl, O-Phenyl oder S-C₁-C₆-Alkyl. Davon noch bevorzugter ist B Wasserstoff, F, Cl, OCF₃, OCHF₂, oder jeweils gegebenenfalls substituiertes C₁-C₆-Alkyl, O-C₁-C₆-Alkyl oder S-C₁-C₆-Alkyl. Am meisten bevorzugt ist B Wasserstoff, OCF₃, OCH₃, O-Ethyl, O-n-Propyl oder O-i-Propyl.

B befindet sich vorzugsweise in der 5- oder 6-Position am Ring, noch bevorzugter in der 6-Position.

R_{w}¹ ist wie vorstehend definiert und bedeutet vorzugsweise Wasserstoff, F, Cl, CN, CF₃, CHF₂, O-CF₃, OCHF₂, jeweils gegebenenfalls substituiertes C₁-C₆-Alkyl, OC₁-C₆-Alkyl, Aryl, C₁-C₆-Alkylamino oder C₁-C₆-Dialkylamino. Noch bevorzugter ist R_{w}¹ Wasserstoff, F, Cl, CN, CF₃ oder O-CF₃, OMe, am meisten bevorzugt Wasserstoff.

R_{w}¹ befindet sich vorzugsweise in der 4- oder 5-Position am Ring, noch bevorzugter in der 5-Position.

Vorzugsweise gilt für B und R_{w}¹ die Maßgabe, dass wenn **W = W1,** dann ist **R_{w}¹** und /oder **B** nicht ein in 4-Position (para-Position) zur Veknüpfungsposition mit **Q** stehender gegenbenenfalls substituierter N-Pyrrolidinyl-Rest;
D ist wie vorstehend definiert und bedeutet vorzugsweise
Halogen, OH, CN, CF₃, CHF₂, OCF₃, OCHF₂,
jeweils gegebenenfalls substituiertes C₁-C₆-Alkyl oder C₂-C₆-Alkenyl,
-O-CH₂-COO-R_{A}¹, O-R_{A}¹, S-R_{A}¹, NR_{A}²R_{A}³, -NR_{A}⁴-CO-R_{A}¹ oder -CO-NR_{A}⁴-R_{A}¹.

Besonders bevorzugt ist D
Halogen, OH, OCF₃, OCHF₂, gegebenenfalls substituiertes C₁-C₆-Alkyl, O-R_{A}¹ oder S-R_{A}¹.

Noch bevorzugter ist D
Halogen. OH, OCF₃, OCHF₂ jeweils gegebenenfalls substituiertes C₁-C₆-Alkyl, O-C₁-C₆-Alkyl, O-Benzyl, O-Phenyl oder S-C₁-C₆-Alkyl.

Davon noch bevorzugter ist D
OH, F, Cl, OCF₃, OCHF₂, oder jeweils gegebenenfalls substituiertes C₁-C₆-Alkyl, O-C₁-C₆-Alkyl oder S-C₁-C₆-Alkyl. Am meisten bevorzugt ist D OH, OCF₃, OCH₃, O-Ethyl, O-n-Propyl oder O-i-Propyl.

D befindet sich vorzugsweise in der 3 oder 4-Position am Ring, noch bevorzugter in der 3-Position.

Insgesamt stellt W vorzugsweise einen Rest dar, der sich aus den bevorzugten Kombinationen von A, B, R_{A}¹, R_{A}², R_{A}³, R_{A}⁴ und R_{w}¹ zusammensetzt.

Q ist wie vorstehend definiert

Q ist vorzugsweise CH₂-.

R_{Q}¹, R_{Q}² sind wie vorstehend definiert und bedeuten vorzugsweise unabhängig voneinander Wasserstoff, Halogen, OH oder gegebenenfalls substituiertes C₁-C₆-Alkyl, noch bevorzugter Wasserstoff, F oder CH₃. Am meisten bevorzugt sind R_{Q}¹, R_{Q}² gleichzeitig Wasserstoff.

R¹, R² sind wie vorstehend definiert und bedeuten vorzugsweise unabhängig voneinander Wasserstoff, OH, CN, oder jeweils gegebenenfalls substituiertes C₁-C₆-Alkyl, OC₁-C₆-Alkyl, O-Aryl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, noch bevorzugter Wasserstoff, OH, CN, O-Methyl, O-Phenyl, Acetyl, Benzoyl. Besonders bevorzugt sind R¹ Wasserstoff, und der zweite Rest R² Wasserstoff, OH, Acetyl, Benzoyl, am meisten bevorzugt sind alle Reste R¹ und R² Wasserstoff.
R³ ist Wasserstoff oder ein freies Elektronenpaar,
X, Y, Z sind wie vorstehend definiert und bedeuten vorzugsweise X = N, Y = CR⁴, Z = CR⁶, oder X = C, Y = CR⁴, Z = NR⁷, oder X = N, Y = NR⁵, Z = CR⁶, oder X = N, Y = N, Z = CR⁶ Besonders bevorzugt sind X = N, Y = CR⁴, Z = CR⁶, oder X = C, Y = CR⁴, Z = NR⁷. Gleichermaßen besonders bevorzugt sind X = N, Y = N, Z = CR⁶Am meisten bevorzugt sind X = N, Y = CR⁴, Z = CR⁶.

R⁴ ,R⁵ ,R⁶ und R⁷ können jeweils unabhängig voneinander aus einer der Gruppen 1-6 ausgewählt werden, wobei die Auswahl grundsätzlich aus der gleichen oder verschiedenen Gruppen erfolgen kann. Die Aufteilung nach Gruppen dient dabei nur der besseren Übersichtlichkeit der möglichen substituenten und bedeutet keinen Auswahlcharakter. Die Gruppen 1-6 sind vorstehend definiert und besitzen vorzugsweise die folgenden Definitionen:

Für die Gruppe 1.) sind R⁴ und/oder R⁶ vorzugsweise jeweils unabhängig voneinander jeweils ein Rest ausgewählt aus der Gruppe, bestehend aus Wasserstoff, F, Cl, CN, CF₃, CHF₂, O-CH₂-COOH, jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-AlkylenHetaryl. Besonders bevorzugt sind Wasserstoff, C₁-C₆-Alkyl, z.B. Methyl, Ethyl, n-Propyl, i-Propyl oder tert-Butyl, Cyclopentyl- oder Cyclohexyl, oder CF₃.

Für die Gruppe 2.) sind R⁵ und/oder R⁷ ein freies Elektonenpaar oder wie vorstehend definiert, vorzugsweise Wasserstoff,
jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, COO-C₁-C₆-Alkyl oder SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷. Besonders bevorzugt sind Wasserstoff, C₁-C₆-Alkyl, z.B. Methyl, Ethyl, n-Propyl, i-Propyl oder tert-Butyl, Cyclopentyl- oder Cyclohexyl, SO₂-R_{Z,Y}⁵, SO₂NH₂ oder SO₂-NR_{Z,Y}⁶R_{Z,Y}⁷

Für die Gruppe 3.) sind R⁴, R⁵, R⁶, R⁷ vorzugsweise jeweils unabhängig voneinander jeweils ein Rest ausgewählt aus der Gruppe, Phenyl, 1-Naphthyl oder 2-Naphthyl, besonders bevorzugt Phenyl, jeweils gegebenenfalls substituiert mit **R_{Z,Y}¹, R_{Z,Y}²** und/oder **R_{Z,Y}**³

**R_{Z,Y}¹, R_{Z,Y}²** und **R_{Z,Y}³** sind wie vorstehend definiert und bedeuten vorzugsweise jeweils unabhängig voneinander einen Substituenten aus der folgenden Gruppe: Wasserstoff, NO₂, CN, CF₃, CHF₂, OCF₃, OCHF₂, O-CH₂-COOH, Halogen, oder jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷ oder CO-NR_{Z,Y}⁶R_{Z,Y}⁷.

In einer Ausführungsform sind Wasserstoff, CN, CF₃, CHF₂, OCF₃, OCHF₂, Cl und F oder jeweils gegebenenfalls substituiertes Aryl, Hetaryl, Heterocycloalkyl, C₁-C₆-Alkyl, C₃-C₇-Cycloalkyl, O-R_{Z,Y}⁴, NR_{Z,Y}⁶R_{Z,Y}⁷, NR_{Z,Y}⁷-SO₂-R_{Z,Y}⁵ bevorzugt.

Es ist auch möglich, dass jeweils zwei der Reste aus **R_{Z,Y}¹, R_{Z,Y}²** oder **R_{Z,Y}³** zusammen einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome aus der Gruppe O, N, S enthalten kann.

In einer Ausführungsform sind R⁴ und R⁵ Wasserstoff oder ein freies Elektronenpaar, R⁶ und R⁷ Phenyl und R_{Z,Y}¹, R_{Z,Y}² oder R_{Z,Y}³ vorzugsweise jeweils Wasserstoff oder zwei der Substituenten sind Wasserstoff und der dritte Substituent ist ein Rest außer Wasserstoff, vorzugsweise CN oder NR_{Z,Y}⁷-SO₂-R_{Z,Y}⁵.

**R_{Z,Y}⁴** ist wie vorstehend definiert und bedeutet vorzugsweise jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, Hetaryl, C₁-C₄-Alkylen-Aryl, oder
C₁-C₆-Alkyl, das gegebenenfalls substituiert ist mit einem Substituenten aus der Gruppe, bestehend aus Halogen, NO₂, NH₂, OH, CN, CF₃, CHF₂, OCHF₃, OCHF₂, gegebenenfalls substituiertes NH-(C₁-C₆-Alkyl) und gegebenenfalls substituiertes N(C₁-C₆-Alkyl)₂, am meisten bevorzugt Methyl oder Ethyl oder CH₂NH-(C₁-C₆-Alkyl) oder CH₂N(C₁-C₆-Alkyl)₂;
**R_{Z,Y}⁵** ist wie vorstehend definiert und bedeutet vorzugsweise jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, oder
jeweils gegebenenfalls 1 bis 3-fach substituiertes Aryl oder Hetaryl, mit Substituenten unabhängig voneinder ausgewählt aus der Gruppe bestehend aus Halogen, NO₂, NH₂, OH, CN, CF₃, CHF₂, OCF₃, OCHF₂, NH-(C₁-C₆-Alkyl) oder N(C₁-C₆-Alkyl)₂, COOH, O-CH₂-COOH, SH, oder jeweils gegebenenfalls substituiertes C₁-C₆-Alkyl, C₃-C₇-Cycloalkyl, Aryl, Hetaryl, Heterocycloalkyl, oder jeweils gegebenenfalls substituiertes O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NRA⁴-SO₂-R_{A}¹, oder SO₂-NR_{A}²R_{A}³;

Am meisten bevorzugt ist **R_{Z,Y}⁵** Methyl, Ethyl, isopropyl, Cyclohexyl oder jeweils gegebenenfalls 1-, 2- oder bis zu 3-fach substituiertes Aryl oder Hetaryl, mit Substituenten unabhängig voneinder ausgewählt aus der Gruppe bestehend aus Halogen, NH₂, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, oder gegebenenfalls substituiertes C₁-C₆-Alkyl, oder O-R_{A}¹, NR_{A}²R_{A}³, SO₂NH₂, SO₂-R_{A}¹, NR_{A}⁴-SO₂-R_{A}¹, oder SO₂-NR_{A}²R_{A}³;

R_{Z,Y}⁶ ist wie vorstehend definiert und bedeutet vorzugsweise
Wasserstoff, oder jeweils gegebenenfalls substituiertes C₁-C₆.Alkyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-AlkylenHetaryl, noch bevorzugter Wasserstoff, oder jeweils gegebenenfalls substituiertes C₁-C₆-Alkyl, Aryl, C₁-C₄-Alkylen-Aryl, C₁-C₄-Alkylen-Hetaryl, SO₂-C₁-C₆-Alkyl, SO₂-Aryl, SO₂-Hetaryl oder SO₂-C₁-C₄-Alkylen-Aryl.

Davon noch bevorzugter ist Wasserstoff, C₁-C₆-Alkyl, Phenyl, oder Benzyl, am meisten bevorzugt Wasserstoff, Methyl, Ethyl, oder Phenyl.

**R_{Z,Y}⁷** ist wie vorstehend definiert und bedeutet vorzugsweise Wasserstoff oder gegebenenfalls substituiertes C₁-C₆-Alkyl.
Noch bevorzugter sind Wasserstoff oder Methyl,

Es ist ebenfalls bevorzugt, dass die beiden Reste R_{Z,Y}⁶ und R_{Z,Y}⁷ zusammen mit dem Stickstoff einen 3 bis 7-gliedrigen, jeweils gegebenenfalls substituierten, gesättigten oder ungesättigten Heterocyclus, der ein, zwei oder drei weitere verschiedene oder gleiche Heteroatome aus der Gruppe O, N, S enthalten kann;

Noch bevorzugter bilden die beiden Reste R_{Z,Y}⁶ und R_{Z,Y}⁷ zusammen mit dem Stickstoff einen 5- oder 6-gliedrigen, gegebenenfalls substituierten, gesättigten Heterocyclus, der ein weiteres Heteroatom O, N, oder S, vorzugsweise O oder N enthalten kann, bilden. Bevorzugt sind ein 5-gliedriger gesättigter Heterocyclus mit einem N und ein 6-gliedriger gesättigter Heterocyclus mit 2 N oder 1 N und 1 O.

Für die Gruppe 4.) sind die Reste R⁴, R⁵ ,R⁶, R⁷ wie vorstehend definiert vorzugsweise einen 5- oder 6-gliedrigen aromatischen Heterocyclus der ein, zwei oder drei verschiedene oder gleiche Heteroatome aus der Gruppe O, N, S enthalten kann und der jeweils mit **R_{Z,Y}¹, R_{Z,Y}²** und **R_{Z,Y}³** substituiert sein kann; Vorzugsweise sind R⁴ und/oder R⁶ jeweils unabhängig voneinander ein jeweils gegebenenfalls substituiertes Rest ausgewählt aus der Gruppe, bestehend 2-Pyrrolyl, 3-Pyrrolyl, Benzothiophenyl, Benzofuranyl, Chinolinyl; Isochinolinyl, die jeweils gegebenenfalls mit 1 oder 2 Substituenten substituiert sein können; oder jeweils gegebenenfalls mit 1 oder 2 Substituenten substituiertes 2-Thienyl, oder 3-Thienyl, wobei die Substituenten ausgewählt sind aus der Gruppe, bestehend aus Halogen, insbesondere Cl, -NO₂, -NH₂, -OH, -CN, -CF₃,-OCF₃, -CHF₂. O-CHF₂, oder jeweils gegebenenfalls substituiertes C₁-C₆-Alkyl, insbesondere Methyl oder Ethyl, O-C₁-C₆-Alkyl, NH-(C₁-C₆-Alkyl) und N(C₁-C₆-Alkyl)₂, NHCO-C₁-C₆-Alkyl, NHSO₂-C₁-C₄-Alkyl und SO₂-C₁-C₄-Alkyl.

Besonders bevorzugt sind für R⁴ und/oder R⁶ jeweils unabhängig voneinander jeweils gegebenenfalls substituiertes Benzothiophenyl, Benzofuranyl, Chinolinyl, Isochinolinyl, 2-Thienyl, oder 3-Thienyl, wobei die beiden Letzteren vorzugsweise substituiert sind mit Halogen, insbesondere Cl, C₁-C₆-Alkyl, insbesondere Methyl oder Ethyl.

In einer Ausführungsform sind R⁴ und/oder R⁵ vorzugsweise jeweils unabhängig voneinander jeweils 2-Pyridyl, 3-Pyridyl, 4-Pyridyl, 2-Pyrimidyl, 4-Pyrimidyl, 5-Pyrimidyl, 6-Pyrimidyl, 2-Thienyl, 3-Thienyl, Benzothiophenyl, Benzofuranyl, Benzimidazolyl, Chinolinyl oder Isochinolinyl, noch bevorzugter 2-Pyridyl, 3-Pyridyl, 4-Pyridyl, 2-Thienyl, 3-Thienyl, Benzothiophenyl, Benzofuranyl, Chinolinyl, oder Isochinolinyl, die jeweils gegebenenfalls mit 1 oder 2 Resten substituiert sein können. Die Reste sind vorzugsweise ausgewählt aus der Gruppe, bestehend aus Halogen, insbesondere Cl oder F, -NO₂, -NH₂, -OH, -CN, -CF₃,-OCF₃, -CHF₂. O-CHF₂,- C₁-C₆-Alkyl, insbesondere Methyl oder Ethyl, O-C₁-C₆-Alkyl, NH-(C₁-C₆-Alkyl) und N(C₁-C₆-Alkyl)₂, NHCO-C₁-C₆-Alkyl, NHSO₂-C₁-C₄-Alkyl und SO₂-C₁-C₄-Alkyl, am meisten bevorzugt Halogen, insbesondere Cl oder F, C₁-C₆-Alkyl, insbesondere Methyl oder Ethyl.

Dabei sind jeweils gegebenenfalls substituiertes 2-Pyridyl, 3-Pyridyl, 4-Pyridyl oder 2-Pyrimidyl, inbesondere 2-Pyridyl, 3-Pyridyl oder 4-Pyridyl besonders bevorzugt.

Für die Gruppe 5.) sind R⁴, R⁵ ,R⁶, R⁷ vorzugsweise gegebenenfalls substituiertes Adamantyl.

Für die Gruppe 6.) sind die Reste **R⁴** und **R⁶** wie vorstehend definiert und bilden bevorzugt mit X und Y einen Benzo-Rest, der mit einem bis drei Resten substituiert sein kann, die unabhängig voneinander ausgewählt werden können aus der Gruppe **R_{Z,Y}⁸.**

**R_{Z,Y}⁸:** ist wie vorstehend definiert und bedeutet vorzugsweise Wasserstoff, NH₂, OH, OCF₃, OCHF₂, O-CH₂-COOH, SH, Halogen, oder jeweils gegebenenfalls substituiertes Aryl, C₁-C₆-Alkyl, C₃-C₇-Cycloalkyl, oder C₁-C₄-Alkylen-Aryl, oder jeweils gegebenenfalls substituiertes
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷

Vorzugsweise gilt die die Maßgabe, dass wenn R⁴ und R⁶ die in der Gruppe 6.) genannten Bedeutungen haben, dann sind die Reste **A, B, Q, Rₐ¹** und **Rₐ²** wie folgt definiert
**A** ist wie in Anspruch 1 unter **R_{Z,Y}⁸** definiert,sitzt wie in W1 angegeben in Bezug auf Q in 2 Position und bedeutet vorzugsweise OCF₃, OCHF₂, O-C₁-C₆-Alkyl, oder O-R_{A}¹, S-R_{A}¹, SO₂-R_{A}¹. Bevorzugter ist A = F, Cl, OCF₃, OCHF₂, C₁-C₆-Alkyl, O-C₁-C₆-Alkyl oder S-C₁-C₆-Alkyl. Am meisten bevorzugt ist A = Methyl, OCF₃, OCH₃, O-Ethyl, O-n-Propyl oder O-i-Propyl.
**B** ist wie in Anspruch 1 unter **R_{z,y}⁸** definiert, sitzt in Bezug auf Q in 6 Position und bedeutet vorzugsweise Halogen, CF₃, CHF₂, OCF₃, OCHF₂, O- C₁-C₆-Alkyl, S-C₁-C₆-Alkyl, gegebenenfalls substituiertes C₁-C₆-Alkyl, oder O-R_{A}¹, S-R_{A}¹. Bevorzugter ist B = F, Cl, OCF₃, C₁-C₆-Alkyl, O-C₁-C₆-Alkyl oder S-C₁-C₆-Alkyl. Am meisten bevorzugt ist B = F, Cl, Methyl, OCF₃, OCH₃, O-Ethyl, O-n-Propyl oder O-i-Propyl. Q ist wie in Anspruch 1 unter R_{Z,Y}⁸ definiert und bedeutet vorzugsweise CH₂
**R_{Q}¹** und **R_{Q}²** sind wie in Anspruch 1 unter R_{Z,Y}⁸ definiert,und bedeuten bevorzugt unabhängig voneinander Wasserstoff und Methyl. Am meisten Bevorzugt sind RQ¹ und R_{Q}² gleichzeitig Wasserstoff.

Vorzugsweise werden die beiden Reste R⁶ und R⁷ aus der Gruppe 1.) bis 4.) noch bevorzugter 1.) bis 3.) einschließlich der bevorzugten Ausführungsformen davon, ausgewählt, und die Reste R⁴ und R⁵ aus der Gruppe 1.),oder 2.) einschließlich der jeweiligen bevorzugten Ausführungsformen davon, ausgewählt. Dabei bedeutet der Rest R⁴ Methyl oder Wasserstoff und R⁵ ist vorzugsweise ein freies Elektronenpaar.

In einer bevorzugten Ausführungsform wird der Rest R⁶ aus der Gruppe 1.) ausgewählt, einschließlich der bevorzugten Ausführungsformen aus der Gruppe 1.), der Rest R⁴ bedeutet Methyl oder Wasserstoff und R⁵ ist vorzugsweise ein freies Elektronenpaar.

In einer weiteren bevorzugten Ausführungsform wird der Reste R⁶ aus der Gruppe 4.) ausgewählt, einschließlich der bevorzugten Ausführungsformen aus der Gruppe 4.), und der Rest R⁴ bedeutet Methyl oder Wasserstoff und R⁵ ist vorzugsweise ein freies Elektronenpaar.

Noch in einer bevorzugten Ausführungsform wird der Rest R⁷ aus der Gruppe 2.) ausgewählt, einschließlich der bevorzugten Ausführungsformen aus der Gruppe 2.), und der Rest R⁴ bedeutet Methyl oder Wasserstoff und R⁵ ist vorzugsweise ein freies Elektronenpaar.

Die vorstehend erläuterten Ausführungsformen eines jeden Restes, einschließlich der bevorzugten Ausführungsformen, sind mit den jeweiligen Ausführungsformen, einschließlich der bevorzugten Ausführungsformen, der verbleibenden anderen Reste beliebig kombinierbar.

In der vorliegenden Erfindung besitzen die verwendeten Ausdrücke die nachstehend erläuterten Bedeutungen:

Der Ausdruck "Alkyl" ist gleichbedeutend für eine unsubstituierte oder gegebenenfalls substituierte geradkettige oder verzweigte gesättigte Kohlenwasserstoffkette mit der jeweils angegebenen Anzahl von Kohlenstoffatomen, vorzugsweise 1, 2, 3, 4, 5, 6, 7, 8, 9 oder 10 Kohlenstoffatome, besonders bevorzugt 1, 2, 3, 4, 5 oder 6, noch bevorzugter 1, 2, 3 oder 4 Kohlenstoffatomen, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, n-Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl oder 1-Ethyl-2-methylpropyl, vorzugsweise Methyl, Ethyl, Propyl, n-Butyl oder i-Butyl. Der Begriff Alkyl" soll auch Halogen substituiertes Alkyl ("Haloalkyl") umfassen.

Der Ausdruck "Alkylen" ist gleichbedeutung für eine unsubstituierte oder gegebenenfalls substituierte geradkettige oder verzweigte Alkylgruppe, die wie vorstehend definiert ist, bei der ein Wasserstoffatom-durch eine Bindung ersetzt ist. Insbesondere sind Methylen, Eth-1,2-ylen, Prop-1,2-ylen, Prop-1,3-ylen, But-1,2-ylen, But-1,3-ylen, But-2,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, 2-Methylbut-1,3-ylen, 2-Ethylprop-1,3-ylen, Hex-3,4-ylen, 3-Methylpent-2,4-ylen, Hept-3,5-ylen, 2-Ethylpent-1,3-ylen, 3-Ethylhept-3,5-ylen, etc., vorzugsweise Methylen, Eth-1,2-ylen und Prop-1,2-ylen; zu nennen. Der Begriff ,Alkylen" soll auch Halogen substituiertes Alkylen ("Haloalkylen") umfassen.

Der Ausdruck "Cycloalkyl" ist gleichbedeutend ein unsubstituierter oder gegebenenfalls substituierter verzweigter oder unverzweigter gesättigter Kohlenwasserstoffring mit 3, 4, 5, 6 oder 7, vorzugsweise 3, 4, 5 oder 6 Kohlenstoffatomen, wie Cyclopropyl, Cyclobutyl, Cyclopentyl, Cyclohexyl oder Cycloheptyl. Der Begriff "Cycloalkyl" soll auch Halogen substituiertes Cycloalkyl ("Halocycloalkyl") umfassen.

Der Ausdruck "Alkylen-O-Alkyl" ist gleichbedeutend für eine im Alkylen- und/oder Alkylrest unsubstituierte oder gegebenenfalls substituierte geradkettige oder verzweigte gesättigte Alkyletherkette, die insgesamt 2 bis 12 Kohlenstoffatome und ein Sauerstoffatom enthält, wobei sowohl der Alkylenrest als auch der Alkylrest unabhängig voneinander 1, 2, 3, 4, 5 oder 6, noch bevorzugter 1, 2, 3 oder 4, am meisten bevorzugt 1 oder 2 Kohlenstoffatome enthalten, wobei beide Reste wie vorstehend definiert sind. Bevorzugte Beispiele von Alkylen-O-Alkyl beinhalten Methoxymethylen, Ethoxymethylen, t-Butoxymethylen, Methoxyethylen oder Ethoxyethylen. Der Begriff "Alkylen-O-Alkyl" soll auch Halogen substituiertes Alkylen-O-Alkyl im Sinne von "Haloalkylen-O-Alkyl" oder "Alkylen-O-Haloalkyl" oder Haloalkylen-O-Haloalkyl" umfassen.

Der Ausdruck "Thioalkyl" ist gleichbedeutend für eine unsubstituierte oder gegebenenfalls substituierte geradkettige oder verzweigte Alkylensulfanylkette, die 1, 2, 3, 4, 5 oder 6 Kohlenstoffatome und ein Schwefelatom enthält. Vorzugsweise enthält der Alkylenrest 1, 2, 3 oder 4, noch bevorzugter 1 oder 2 Kohlenstoffatome, wobei Alkylen wie vorstehend definiert ist. Beispiele von Thioalkyl beinhalten Thiomethyl oder Thio-tert-butyl. Der Begriff "Thioalkyl" soll auch Halogen substituiertes Thioalkyl ("Halothioalkyl") umfassen.

Der Ausdruck "Alkenyl" ist gleichbedeutend für eine unsubstituierte oder gegebenenfalls substituierte verzweigte oder unverzweigte Kohlenwasserstoffkette, enthaltend mindestens eine Doppelbindung, mit 2, 3, 4, 5 oder 6, vorzugsweise 2, 3 oder 4 Kohlenstoffatomen. Vorzugsweise enthält Alkenyl eine oder zwei Doppelbindungen, am meisten bevorzugt eine Doppelbindung. Beispiele der Alkenylgruppen sind jene, wie sie vorstehend für Alkyl genannt werden, wobei diese Gruppen eine oder zwei Doppelbindungen enthalten, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl=3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl,2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl oder 3-Methyl-2-pentenyl. Der Begriff "Alkenyl" soll auch Halogen substituiertes Alkenyl ("Haloalkenyl") umfassen.

Der Ausdruck "Alkinyl" ist gleichbedeutend für eine unsubstituierte oder gegebenenfalls substituierte verzweigte oder unverzweigte Kohlenwasserstoffkette, enthaltend mindestens eine Dreifachbindung mit 2, 3, 4, 5 oder 6, vorzugsweise 2, 3 oder 4 Kohlenstoffatomen. Vorzugsweise enthält Alkinyl eine oder zwei Dreifachbindungen, am meisten bevorzugt eine Dreifachbindung. Beispiele der Alkenylgruppen sind jene, wie sie vorstehend für Alkyl genannt werden, wobei diese Gruppen eine oder zwei Dreifachbindungen enthalten, wie beispielsweise Ethinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, 1-Methyl-2-propinyl oder 1-Methyl-2-butinyl. Der Begriff "Alkinyl" soll auch Halogen substituiertes Alkinyl ("Haloalkinyl") umfassen.

Der Ausdruck "Heterocycloalkyl" ist gleichbedeutend ein unsubstituierter oder gegebenenfalls substituierter gesättigter Alkylring oder ein Alkylring, an den ein weiterer unsubstituierter oder gegebenenfalls substituierter gesättigter Alkylring anelliert ist, mit vorzugsweise 3, 4, 5, 6, 7, 8, 9 oder 10 Ringatomen insgesamt, noch bevorzugter 3, 4, 5 oder 6 Ringatomen, am meisten bevorzugt 5 oder 6 Ringatomen, wobei dieser Heterocycloalkyl mindestens ein Heteroatom, vorzugsweise 1, 2 oder drei gleiche oder verschiedene Heteroatome ausgewählt aus der Gruppe bestehend aus O, N und S, enthält und 1, 2, 3, 4, 5 oder 6, vorzugsweise 1, 2, 3, 4 oder 5 Kohlenstoffatome enthält. Vorzugsweise enthält Heterocycloalkyl 1 oder 2 gleiche oder verschiedene Heteroatome, die vorzugsweise ausgewählt sind aus der Gruppe bestehend aus N und O. Beispiele einer Heterocycloalkylgruppe beinhalten beispielsweise N-Pyrrolidinyl, N-Piperidinyl, N-Hexahydroazepinyl, N-Morpholinyl oder N-Piperazinyl, wobei bei Heterocyclen, die Aminogruppen enthalten, wie beispielsweise N-Piperazinyl, diese Aminogruppen durch gängige Reste, wie beispielsweise Methyl, Benzyl, Boc (tert.-Butoxycarbonyl), Benzyloxycarbonyl, Tosyl (p-Toluolsulfonyl), -SO₂-C₁-C₄-Alkyl, -SO₂-Phenyl oder -SO₂-Benzyl ersetzt sein können. Der Begriff "Heterocycloalkyl" soll auch Halogen substituiertes Heterocycloalkyl ("Haloheterocycloalkyl") umfassen.

Der Ausdruck "Aryl" ist gleichbedeutend ein unsubstituierter oder gegebenenfalls substituierter aromatischer mono-, bi- oder polycyclischer Rest mit vorzugsweise 6, 7, 8, 9, 10, 11, 12, 13, 14, 15, 16, 17, 18, 19 oder 20 Kohlenstoffatomen, noch bevorzugter 6, 7, 8, 9 oder 10 Kohlenstoffatomen und wird vorzugsweise ausgewählt aus Phenyl, Biphenyl, Naphthyl, Tetrahydronaphthyl, Fluorenyl, Indenyl und Phenanthrenyl, noch bevorzugter aus Phenyl und Naphthyl, wie 1-Naphthyl oder 2-Naphthyl. Am meisten bevorzugt ist Phenyl.

Der Ausdruck "Alkylenaryl" ist gleichbedeutend ein über C₁-C₆-, noch bevorzugter C₁-C₄-Alkylen gebundenes, im Aryl- und/oder Alkylenrest gegebenenfalls substituiertes Aryl, wobei Alkylen und Aryl wie vorstehend definiert sind. Alkylenaryl ist insbesondere im Arylrest gegebenenfalls substituiertes Benzyl oder Phenethyl. Der Begriff Alkenylaryl" soll auch Halogen substituiertes Alkenylaryl ("Haloalkenylaryl") umfassen.

Der Ausdruck "Aryloxy" oder "-O-Aryl" ist gleichbedeutend ein über Sauerstoff gebundenes unsubstituierter oder gegebenenfalls substituierter Aryl, das wie vorstehend definiert ist, insbesondere -O-Phenyl.

Der Ausdruck "Hetaryl" (oder auch "Heteroaryl" oder "Heteroaromat") ist gleichbedeutend ein unsubstituierter oder gegebenenfalls substituierter mono-, bi- oder tricyclischer aromatischer Ring, enthaltend wenigstens ein Heteroatom, vorzugsweise 1, 2 oder 3 gleiche oder verschiedene Heteroatome, noch bevorzugter 1 oder 2 gleiche oder verschiedene Heteroatome ausgewählt aus der Gruppe bestehend aus O, N und S und vorzugsweise 1, 2, 3, 4, 5, 6, 7, 8, 9, 10, 11 oder 12, noch bevorzugter 1, 2, 3, 4, 5 oder 6 Kohlenstoffatome. Der aromatische Ring ist vorzugsweise 5- oder 6-gliedrig. Hetaryl umfasst außerdem die mit Aryl anellierten Derivate davon, nämlich einen aromatischen Rest mit vorzugsweise 6, 7, 8, 9, 10, 11, 12, 13, 14, 15, 16, 17, 18, 19 oder 20 Kohlenstoffatomen, noch bevorzugter 6, 7, 8, 9 oder 10 Kohlenstoffatomen, am meisten bevorzugt Phenyl, der mit diesem aromatischen Ring, enthaltend wenigstens ein Heteroatom, anelliert ist. Hetaryl kann auch ausgewählt werden aus einem aromatischen Rest mit vorzugsweise 6, 7, 8, 9, 10, 11, 12, 13, 14, 15, 16, 17, 18, 19 oder 20, noch bevorzugter 6, 7, 8, 9 oder 10 Kohlenstoffatomen, am meisten bevorzugt Phenyl, mit einer Heterocycloalkylgruppe, die daran anelliert ist. Dabei ist die Heterocycloalkylgruppe wie vorstehend definiert.

Hetaryl wird vorzugsweise ausgewählt aus 2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl, Triazinyl, Indolinyl, Benzothienyl, Naphthothienyl, Benzofuranyl, Chromenyl, Indoxyl, Isoindolyl, Indazolyl, Chinolyl, Isochinolyl, Phthalazinyl, Chinoxalinyl, Benzimidazolyl und Benzoxazolyl, 2,3-Dihydro-1,4-benzodioxinyl, 1,3-Benzodioxolyl-, 2,1,3-Benzothiadiazolyl. Der Heterocyclus kann eine oder mehrere endo- oder exocyclische Doppelbindungen enthalten ("ungesättigter Heterocyclus") oder gesättigt sein ("gesättigter Heterocyclus"). Der Heterocyclus kann auch aromatisch sein, wobei im Falle von Ringgerüsten mit mehreren Ringen, insbesondere bei anellierten Ringen, der gesamte Heterocyclus oder nur ein oder mehrere seiner Teilringe aromatischen Charakter aufweisen kann ("aromatischer Heterocyclus").

Die, Begriffe "Pyridyl" und "Pyridinyl" bezeichnen im Zusammenhang mit der vorliegenden Erfindung ein und denselben Rest. Gleiches gilt für "Pyrimidyl" und "Pyrimidinyl".

Der Ausdruck "Alkylenhetaryl" ist bezeichnend für ein über C₁-C₆-, noch bevorzugter C₁-C₄-Alkylen gebundenes, im Alkenyl- und/oder Hetarylrest gegebenenfalls substituiertes Hetaryl, wobei Alkylen und Hetaryl wie hier definiert sind. Alkylenhetaryl ist vorzugsweise gegebenenfalls substituiertes -CH₂-Pyridyl,-CH₂-3-Pyridyl, -CH₂-4-Pyridyl, -CH₂-2-Thienyl, -CH₂-3-Thienyl, -CH₂-2-Thiazolyl, -CH₂-4-Thiazolyl, CH₂-5-Thiazolyl, -CH₂-CH₂-2-Pyridyl, -CH₂-CH₂-3-Pyridyl, - CH₂-CH₂-4-Pyridyl, -CH₂-CH₂-2-Thienyl, -CH₂-CH₂-3-Thienyl, -CH₂-CH₂-2-Thiazolyl, -CH₂-CH₂-4-Thiazolyl oder -CH₂-CH₂-5-Thiazolyl. Der Begriff "Alkenylhetaryl" soll auch Halogen substituiertes Alkenylhetaryl ("Haloalkenylhetaryl") umfassen.

Der Ausdruck "Carbocyclus" steht für ein gegebenenfalls substituiertes Ringgerüst aus Kohlenstoffatomen als Ringatome. Das Ringgerüst ist vorzugsweise mono-, bi- oder tricyclisch oder eine höhere Anzahl von Ringen aufweisen. Die Ringe können anelliert oder verbrückt sein. Die Anzahl der Ringkohtenstoffatome liegt vorzugsweise im Bereich zwischen 3 und 12 C-Atomen, besonders bevorzugt im Bereich von 6 bis 10 C-Atomen als Ringatome. Das Ringgerüst kann eine oder mehrere endo- oder exocyclische Doppelbindungen enthalten ("ungesättigter Carbocyclus") oder gesättigt sein ("gesättigter Carbocyclus"). Der Carbocyclus kann auch aromatisch sein, wobei im Falle von Ringgerüsten mit mehreren Ringen, insbesondere bei anellierten Ringen, der gesamte Carbocyclus oder nur einer oder mehrere seiner Teilringe einen aromatischen Charakter aufweisen kann ("aromatischer Carbocyclus").

Der Ausdruck "Cyclus" steht für ein gegebenenfalls substituiertes Ringsystem ausgewählt aus der Gruppe bestehend aus "Aryl", "Hetaryl", "Carbocyclus" und "Heterocyclus"

Ein "bi- oder tricyclischer, gesättigter Kohlenwasserstoffrest" ist ein unsubstituierter oder gegebenenfalls substituierter Bicycloalkyl- oder Tricycloalkylrest und besitzt 5, 6, 7, 8, 9, 10, 11, 12, 13, 14, 15, 16, 17 oder 18 Kohlenstoffatome. Bei einem Bicycloalkylrest enthält das Ringsystem vorzugsweise 5, 6, 7, 8, 9, 10, 11 oder 12, noch bevorzugter 6, 7, 8, 9 oder 10 Kohlenstoffatome, Bei einem Tricycloalkylrest enthält das Ringsystem vorzugsweise 6, 7, 8, 9, 10, 11, 12, 13, 14, 15 oder 16, noch bevorzugter 6, 7, 8, 9, 10, 11 oder 12 Kohlenstoffatome. Beispiele eines Bicycloalkylrestes beinhalten Indanyl, Camphyl und Norbornyl. Beispiele eines Tricycloalkylrestes beinhalten Adamantyl.

"Halogen" ist ein Halogenatom ausgewählt aus Fluor, Chlor, Brom oder lod, vorzugsweise Fluor, Chlor oder Brom, noch bevorzugter Fluor oder Chlor, am meisten bevorzugt Fluor.

Mit Halogen substituiertes Alkyl ("Haloalkyl") bezeichnet einen Alkylrest, wie vorstehend definiert, der partiell oder vollständig durch Fluor, Chlor, Brom und/oder lod substituiert ist, also z.B. CH₂F, CHF₂, CF₃, CH₂Cl, 2-Fluorethyl, 2-Chlorethyl, 2,2,2-Trifluorethyl. Gleiches gilt in analoger Weise für die Begriffe "Haloalkylen", "Haloalkenyl", "Haloalkinyl", "Haloalkenylaryl", "Haloalkenylhetaryl", "Haloalkylen-O-Alkyl" oder "Alkylen-O-Haloalkyl", Haloalkylen-O-Haloalkyl", "Halothioalkyl", "Halocycloalkyl".

Falls durch den Ausdruck "gegebenenfalls substituiert" erwähnt, können die Reste und Gruppen vorzugsweise ein- oder mehrfach, noch bevorzugter ein-, zwei- oder dreifach, am meisten bevorzugt ein- oder zweifach substituiert sein. Der Ausdruck "jeweils gegebenenfalls substituiert" soll verdeutlichen, dass nicht nur der direkt darauf folgende Rest sondern alle in der jeweiligen Gruppe genannten Reste gleich oder verschieden substituiert sein können.
Beispiele der geeigneten Substituenten für die Ausdrücke "gegebenenfalls substituiert" oder "jeweils gegebenenfalls substituiert" beirihalten: Halogen, CN, CF₃, CHF₂, OCF₃, OCHF₂, NO₂, NH₂, OH, COOH, jeweils verzweigtes oder unverzweigtes, gegebenenfalls substituiertes C₁-C₆-Alkyl, C₃-C₇-Cycloalkyl, C₁-C₆-Alkylen-O-C₁-C₆-Alkyl oder C₁-C₆-Thioalkyl, O-C₁-C₆-Alkyl, N(C₁-C₆-Alkyl)(C₁-C₆-Alkyl), NH(C₁-C₆-Alkyl), Aryl, -O-Aryl, C₁-C₆-Alkylen-O-Aryl, Hetaryl, Heterocycloalkyl, C₁-C₄-Alkylen-Aryl, C₁-C₄-Alkylen-Hetaryl, C₁-C₄-Alkylen-C₃-C₇-Cycloalkyl, C₁-C₄-Alkylen-Heterocycloalkyl, NHCO-C₁-C₄-Alkyl, NH-SO₂-C₁-C₄-Alkyl, CO-C₁-C₆-Alkyl, COO-C₁-C₆-Alkyl, O-CO-C₁-C₆-Alkyl, SO₂-C₁-C₆-Alkyl, im Arylrest gegebenenfalls substituiertes NHCO-Aryl, NHSO₂-Aryl, CONH₂, SO₂NH₂, SO₂-Aryl, SO-C₁-C₆-Alkyl, SO₂-C₁-C₆-Alkyl, SO-Aryl, N-Pyrrolidinyl, N-Piperidinyl, und N-Morpholinyl. Bevorzugte Substituenten sind F, Cl, CF₃, OCF₃, NH₂, NO₂, OH, COOH, C₁-C₄-Alkyl, Methoxy, Acetyl, NH-Acetyl und SO₂NH₂.

Das Präfix "C₁-C₆" bedeutet, dass der nachstehend genannte Rest, wie beispielsweise der Rest "Alkyl" in "C₁-C₆-Alkyl" ein, zwei, drei, vier, fünf oder sechs Kohlenstoffatome aufweisen kann. Gleiches gilt in analoger Weise für die Bedeutung der weiteren in der vorliegenden Beschreibung und den Ansprüchen verwendeten Präfixe, wie beispielsweise "C₃-C₇" (3, 4, 5, 6 oder 7 Kohlenstoffatome), "C₁-C₄" (1, 2, 3 oder 4 Kohlenstoffatome), "C₂-C₆" (2, 3, 4, 5 oder 6 Kohlenstoffatome) usw.

Der Ausdruck "3- bis 7-gliedriger" Carbocyclus, Heterocyclus oder Ring bezieht sich auf die gesamte Anzahl der Ringglieder, also auf einen Ring mit 3, 4, 5, 6 oder 7 Ringgliedern insgesamt. Im Falle von miteinander anellierten Ringsystemen, wobei anelliert sowohl benachbarte (vicinale) wie geminale (also spriroverbrückte Ringsysteme) bedeuten kann, bedeutet die Angabe "3- bis 7-gliedrig" die gesamt Anzahl der Ringglieder einschließlich der Ringglieder, die Teil des angrenzenden anellierten Ringsystems sind. Gleiches gilt in analoger Weise für die Ausdrücke "5- bis 7-gliedrig", "5- oder 6-gliedrig", "4- bis 7-gliedrig" usw.

Generell gilt, dass ein in Klammern gesetzter Rest, wie z.B. der Rest "(C₁-C₆-Alkyl)" in dem Ausdruck "N(C₁-C₆-Alkyl)₂", zusammen mit einem dem Klammerausruck zugeordneten Zahlenwert das dem Zahlenwert entsprechende mehrfache Auftreten des jeweiligen Restes bedeutet, also im Falle des vorstehend genannten Beispiels für einen Rest "N(C₁-C₆-Alkyl)(C₁-C₆-Alkyl)" steht, wobei die mehrfach auftretenden Reste jeweils unabhängig voneinander die gleiche oder verschiedene Bedeutungen haben können. Gleiches gilt in analoger Weise für alle Ausdrücke gemäß dem Schema "(Rest)ₓ" mit x = eine ganze Zahl gleich oder größer zwei.

### Optische Isomere - Diastereomere - Geometrische Isomere - Tautomere

Die erfindungsgemäßen Verbindungen der allgemeinen Formel I bzw. deren Salze können mindestens ein asymmetrisches Zentrum besitzen und können als Racemate und racemische Gemische, einzelne Enantiomere, diastereomere Gemische und einzelne Diastereomere vorliegen. Die vorliegende Erfindung umfasst alle diese stereoisomeren Formen der erfindungsgemäßen Verbindungen der allgemeinen Formel I.

Die erfindungsgemäßen Verbindungen der allgemeinen Formel I können in ihre einzelnen Stereoisomere durch herkömmliche Verfahren aufgespalten werden durch z.B. fraktionierte Kristallisation aus einem geeigneten Lösungsmittel, z.B. Methanol oder Ethylacetat oder einem Gemisch davon oder durch chirale Chromatographie unter Verwendung einer optisch aktiven stationären Phase. Die absolute Konfiguration kann durch Röntgenkristallographie der kristallinen Produkte oder kristallinen Zwischenprodukte ermittelt werden, die, falls notwendig, mit einem Reaktionsmittel derivatisiert werden, das ein asymmetrisches Zentrum einer bekannten absoluten Konfiguration enthält.

Alternativ kann ein beliebiges Stereoisomer einer erfindungsgemäßen Verbindung der allgemeinen Formel I erhalten werden durch stereospezifische Synthese unter Verwendung von optisch reinen Ausgangsmaterialien oder Reaktionsmitteln mit bekannter absoluter Konfiguration oder durch asymmetrische Synthesemethoden.

Bevorzugt ist die Verwendung einer enantiomeren- bzw. diastereomerenreinen Verbindung.

Insbesondere können die erfindungsgemäßen Verbindungen der allgemeinen Formel 1 auch als verschiedene Tautomere vorliegen, wobei, wie es für den Fachmann ersichtlich ist, die Art der Tautomerie von der Natur der Reste abhängt. Auch andere Tautomere, wie Keto-Enol-Tautomere, können vorliegen. Alle einzelnen möglichen Tautomere sowie Gemische davon sind durch die erfindungsgemäßen Verbindungen der allgemeinen Formel I umfasst.

### Salze

Der Begriff "pharmazeutisch annehmbare Salze" bezieht sich auf Salze, die aus pharmazeutisch annehmbaren physiologisch verträglichen Basen oder Säuren, einschließlich anorganischen oder organischen Basen und anorganischen oder organischen Säuren, hergestellt werden.

Salze, die sich aus anorganischen Basen ableiten beinhalten Aluminium, Ammonium, Calcium, Kupfer, Eisen(II), Eisen(III), Lithium, Magnesium, Mangan, Kalium, Natrium, Zink und Ähnliche. Besonders bevorzugt sind die Ammonium-, Calcium-, Lithium-, Magnesium-, Kalium- und Natriumsalze. Salze, die sich von pharmazeutisch annehmbaren organischen nicht toxischen Basen ableiten, beinhalten Salze von primären, sekundären und tertiären Aminen, substituierten Aminen, einschließlich natürlich vorkommenden substituierten Aminen, cyclischen Aminen und basischen lonenaustauscherharzen, wie Arginin, Betain, Coffein, Cholin, N,N'-Dibenzylethylendiamin, Diethylamin, 2-Diethylaminomethanol, 2-Dimethylaminoethanol, Ethanolamin, Ethylendiamin, N-Ethylmorpholin, N-Ethylpiperidin, Glucamin, Glucosamin, Histidin, Hydrabamin, Isopropylamin, Lysin, Methylglucamin, Morpholin, Piperazin, Piperidin, Polyaminharze, Procain, Purine, Theobromin, Triethylamin, Trimethylamin, Tripropylamin, Tromethamin und ähnliche.

Wenn die erfindungsgemäßen Verbindungen der allgemeinen Formel I basisch sind, können Salze aus pharmazeutisch annehmbaren physiologisch verträglichen Säuren, einschließlich anorganischer und organischer Säuren, hergestellt werden. Solche Säuren beinhalten unter anderem Essigsäure (Acetat), Benzolsulfonsäure, Benzoesäure, Kampfersulfonsäure, Citronensäure, Ethansulfonsäure, Ameisensäure, Fumarsäure, Gluconsäure, Glutaminsäure, Bromwasserstoffsäure, Chlorwasserstoffsäure, Milchsäure, Äpfelsäure, Maleinsäure, Mandelsäure, Methansulfonsäure, Malonsäure, Salpetersäure, Pantotheninsäure, Phosphorsäure, Propionsäure, Bernsteinsäure, Schwefelsäure, Weinsäure, p-Toluolsulfonsäure, Trifluoressigsäure und ähnliche. Besonders bevorzugt sind Essigsäure, Citronensäure, Fumarsäure, Bromwasserstoffsäure, Chlorwasserstoffsäure, Maleinsäure, Phosphorsäure, Schwefelsäure und Weinsäure.

Wenn Bezug genommen wird auf die erfindungsgemäßen Verbindungen der allgemeinen Formel I, soll dies bedeuten, dass auch die pharmazeutisch annehmbaren Salze davon beinhaltet sind.

### Verwendung, Einsatzgebiete und Wirkungen

Gegenstand der Erfindung ist auch die Verwendung der 5-Ring Heteroaromaten der Formel I oder IA zur Herstellung eins Medikaments zur von:
- Depressionen und/oder bipolaren Störungen wie zum Beispiel dysthemische Störungen, jahreszeitlich bedingte Störungen und/oder psychotische Störungen.
- Angst und/oder stress-bedingte Störungen wie zum Beispiel generelle Angststörungen, Panikstörungen, Zwangsstörungen, posttraumatische Störungen, akute Stress-Störungen und/oder soziale Phobie
- Gedächtnisstörungen und/oder Alzheimer Krankheit
- Schizophrenie, Psychosen, psychotische Störungen und/oder psychotisch bedingte Störungen
- Cerebrovaskuläre Störungen
- Schmerz und/oder Schmerz-bedingte Störungen, Abhängigkeit und Drogenbedingte Störungen, einschließlich Medikamenten-bedingte Störungen
- Amnesie
- Alkohol- und/oder Drogenmissbrauch, einschließlich
   Medikamentenmißbrauch
- Störungen des circadianen Rythmus
   und/oder
- Cushing Syndrom.

Der Begriff "Störung" im erfindungsgemäßen Sinne bezeichnet Anomalien, die in der Regel als krankhafte Zustände angesehen werden und sich in Form bestimmter Anzeichen, Symptome und/oder Fehlfunktionen zu erkennen geben können. Die Behandlung kann auf einzelne Störungen sprich Anomalien bzw. krankhafte Zustände gerichtet sein, es können aber auch mehrere gegebenenfalls ursächlich miteinander verbundene Anomalien zu Mustern, d.h. Syndromen, zusammengefasst sein, die erfindungsgemäß behandelt werden können. Dieser Zustand kann vorübergehend, fortschreitend oder dauerhaft bestehen.

Verbindungen der vorliegenden Erfindung können verwendet werden zur Behandlung oder Prävention von verschiedenen Erkrankungen, an deren Entstehung und/oder Verlauf 5-HT5-Rezeptoren beteiligt sind, d.h. Erkrankungen, die durch eine 5-HT5-Rezeptoraktivität moduliert werden, wie mentale Störungen. Beispiele solch mentaler Störungen sind nach dem "American Psychatric Assiciation. DSM-IV, Diagnostic and Statistical Manual of Mental Disorders, 4th ed., 1994: Aufmerksamkeitsstörungen und sozial störendes Verhalten; Lernstörungen, Delir, Demenz und amnestische und andere kognitive Störungen; Störungen im Zusammenhang mit verschiedenen Substanzen, wie zum Beispiel Störungen im Zusammenhang mit Alkoholkonsum und Alkohol-induzierte Störungen, Entzugserscheinungen; Schizophrenie und andere psychotische Störungen wie z.B. schizophrenieforme Störung, schizoaffektive Störung, und wahnhafte Störung; Substanz-induzierte Psychosen; paranoide Störungen; Neuroleptika-induzierte Störungen; affektive Störungen, wie zum Beispiel depressive Störungen (Major Depression, dysthemische Störung, jahreszeitlich bedingte Störung, nicht näher bezeichnete depressive Störung), bipolare Störungen (bipolar I Störung, bipolar **II** Störung, zyklothyme Störung, nicht näher bezeichnete bipolare Störung, Substanz (Amphetamin oder amphetaminähnliche Substanzen) induzierte affektive Störung, nicht näher bezeichnete affektive Störung); Störungen im Zusammenhang mit Stress, wie zum Beispiel akute Belastungsstörung; Angststörungen, wie zum Beispiel Panikstörungen ohne Agoraphobie, Panikstörung mit Agoraphobie, Agoraphobie ohne Panikstörung in der Vorgeschichte, spezifische Phobie, soziale Phobie, Zwangsstörung, posttraumatische Belastungsstörung, akute Belastungsstörung, generalisierte Angststörung, substanzinduzierte Angststörung; somatoforme Störungen wie zum Beispiel Somatisierungsstörung, nicht näher bezeichnete somatoforme Störung, Konversionsstörung, Schmerzstörung; Eßstörungen; Schlafstörungen wie zum Beispiel primäre Schlafstörungen (Dyssomnie, Parasomnie), Schlafstörungen im Zusammenhang mit einer anderen mentalen Störung.

Gegenstand der Erfindung ist insbesondere auch die Verwendung der 5-Ring Heteroaromaten I für die Behandlung neuropathologischer, neuropsychiatrischer und neurodegenerativer Störungen.

Unter neuropathologischen Störungen versteht man Störungen, die von neurologischen Defiziten begleitet sind, d. h. einen durch neurologische Ausfallerscheinungen gekennzeichneten Zustand.

Erfindungsgemäß bevorzugt ist die Behandlung neurodegenerativer und/oder neuropsychiatrischer Störungen. Diese Störungen treten insbesondere bei neuropathologischen, in der Regel Gehirnschädigungen verursachenden Krankheitsbildern auf, beispielsweise cerebraler Ischämie, Schlaganfall, Epilepsie und Anfällen im allgemeinen, chronischer Schizophrenie, anderen psychotischen Erkrankungen, Depression, Angstzuständen, bipolaren Störungen, Demenz, insbesondere Alzheimer Demenz, demyelinisierenden Erkrankungen, insbesondere Multipler Sklerose, Gehirntumore und generelle Entzündungsprozesse. Eine weitere neuropathologische Störung ist Migräne, sowie die damit zusammenhängenden Anzeichen, Symptome und Fehlfunktionen.

Gemäß eines weiteren Aspekts der vorliegenden Erfindung werden neuropathologische Störungen behandelt, die mit einer glialen Reaktion einhergehen. Die erfindungsgemäße Verwendung betrifft insbesondere die Modulation einer glialen Reaktion. Eine vorteilhafte Wirkung der Bindungspartner zeigt sich bei der präventiven oder akuten Behandlung neurologischer Defizite, die an Patienten beobachtet werden, die unter psychiatrischen Erkrankungen leiden, wie Epilepsie, Psychose, z.B. Psychosen vom akuten exogenen Reaktionstyp oder Begleitpsychosen organischer bzw. exogener Ursache, z.B. nach Trauma, vor allem Hirnläsionen und diffusen Hirnschädigungen, bei Stoffwechselstörungen, Infektionen, und Endokrinopathien; endogene Psychosen, wie Schizophrenie sowie schizotype und wahnhafte Störungen; affektive Störungen, wie Depression, Manie bzw. manisch-depressive Zustände; sowie Mischformen der zuvor geschilderten Psychosen; seniler Demenz und seniler Demenz vom Alzheimer Typ, sowie bei der Behandlung oder Prävention von Demyelinisationsvorgängen.

Wirksam sind die erfindungsgemäßen 5-Ring Heteroaromaten insbesondere im Hinblick auf die Behandlung ischämischer Schäden, z.B. infolge von Hirn- und Rückenmarkstrauma sowie Gefäßverschluß oder Herzversagen. Zu nennen ist hier vor allem der Schlaganfall (Synonym: Apoplexia cerebri, cerebraler oder apoplektischer Insult, Gehirnschlag). Erfindungsgemäß behandelbar sind transitorisch-ischämische Attacken, reversible ischämische neurologische Defizite, prolongierte reversible ischämische neurologische Defizite, partiell reversible ischämische neurologische Symptomatiken und auch persistierende komplette Hirninfarkte. Besonders vorteilhaft ist erfindungsgemäß die Behandlung akuter Formen.

Den erfindungsgemäß bevorzugt behandelten Formen neuropathologischer Störungen liegen eine oder mehrere der nachfolgend aufgezählten Veränderungen von Nervengeweben zugrunde: Degeneration oder Absterben von Neuronen, insbesondere der Ganglienzellen, zB. Tigrolyse, Kernmembranunschärfe, Zellschrumpfung, Zytoplasmavakuolisierung und -inkrustation, Parenchymnekrosen des Gehirns, Hirnödeme, durch Sauerstoffmangel verursachte Veränderungen von Neuronen, Atrophie, morphologische Veränderungen, wie Demyelinisierungen, insbesondere ein Markscheidenzerfall, perivaskuläre Infiltrate, gliöse Proliferation und/oder Glianarben; Degeneration der Substantia nigra.

Die erfindungsgemäß zu behandelnde Indikation ist häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten. Durch die erfindungsgemäße Behandlung neuropathologischer, neuropsychiatrischer oder neurodegenerativer Störungen bzw. den ihr zugrunde liegenden Zuständen lassen sich eine Reihe weiterer Anzeichen, Symptome und/oder Fehlfunktionen behandeln, die mit diesen Störungen zusammenhängen, d.h. insbesondere die oben beschriebenen Erkrankungszustände begleiten. Hierzu gehören beispielsweise Schocklunge; Hirnnervenausfälle, z.B. retrobulbäre Neuritis, Augenmuskellähmungen, skandierende Sprache, spastische Lähmungen, Kleinhirnsymptome, Sensibilitäts-, Blasen- und Mastdarmstörungen, Euphorie, Demenz; Hypo- und Akinese, fehlende Mitbewegung, kleinschrittiger Gang, Beugehaltung von Rumpf und Gliedern, Pro-, Retro- und Lateropulsion, Tremor, Mimikarmut, monotone Sprache, Depressionen, Apathie, labile oder starre Affektivität, erschwerte Spontaneität und Entschlußkraft, verlangsamtes Denken, verarmte Assoziationsfähigkeit; Muskelatrophie.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Der Begriff "Bindungspartner für 5-HT5-Rezeptoren" beschreibt Substanzen, welche an 5-HT5-Rezeptoren binden und daher auch als 5-HT5-Rezeptorliganden bezeichnet werden können.

Unter Bindung versteht man jede molekulare Wechselwirkung zwischen dem Bindungspartner und dem Rezeptor, insbesondere unter physiologischen Bedingungen. Dies sind in der Regel klassische Wechselwirkungen, zu denen elektrostatische Anziehung, Wasserstoffbrücken-Bindung, hydrophobe Bindungen, van-der-Waals-Kräfte oder metallkomplexartige koordinative Bindungen gehören. Zusätzlich zu den vorstehend genannten, reversiblen molekularen Wechselwirkungen können auch irreversible Wechselwirkungen zwischen Bindungspartner und Rezeptor in Betracht kommen, wie z.B. kovalente Bindungen.

Erfindungsgemäße 5-Ring Heteroaromaten können die Bindung von Vergleichsbindungspartnern, wie 5-HT (5-Hydroxytryptamin) oder 5-CT (5-Carboxamidotryptamin), an 5-HT5-Rezeptoren kompetitiv hemmen. Unter kompetitiver Hemmung versteht man, dass die erfindungsgemäßen Verbindungen der Formel 1 mit einem Vergleichsbindungspartner, im vorliegenden Fall Z.B. 5-HT oder 5-CT, um die Bindung an den Rezeptor konkurrieren.

Gemäß einer weiteren Ausführungsform hemmen erfindungsgemäße 5-Ring Heteroaromaten die Bindung von Vergleichsbindungspartnern, wie 5-HT (5-Hydroxytryptamin) oder 5-CT (5-Carboxamidotryptamin), an 5-HT5-Rezeptoren nicht-kompetitiv. Unter nicht-kompetitiver Hemmung versteht man, dass erfindungsgemäße 5-Ring Heteroaromaten über ihre Bindung an den Rezeptor die Bindung eines Vergleichsbindungspartner, im vorliegenden Fall z.B. 5-HT oder 5-CT, modulieren, insbesondere dessen Bindungsaffinität verringern.

Zumindest für den Fall der kompetitiven Hemmung, also der reversiblen Bindung, gilt der Grundsatz, dass die Verdrängung eines Bindungspartners durch einen anderen mit abnehmender Bindungsaffinität des einen bzw. zunehmender Bindungsaffinität des anderen im Hinblick auf den Rezeptor zunimmt. Zweckmäßigerweise besitzen daher erfindungsgemäße 5-Ring Heteroaromaten eine hohe Bindungsaffinität für 5-HT5-Rezeptoren. Eine derartige Bindungsaffinität gestattet einerseits eine wirksame Verdrängung natürlich vorkommender Bindungspartner für 5-HT5-Rezeptoren, wie beispielsweise Serotonin (5-Hydroxytryptamin, 5-HT) selbst, wobei die erforderliche Konzentration an erfindungsgemäßem 5-Ring Heteroaromat zur Bindung einer bestimmten Menge dieses Bindungspartners an 5-HT5-Rezeptoren mit zunehmender Bindungsaffinität abnimmt: Im Hinblick auf die medizinische Anwendung werden daher 5-Ring Heteroaromaten bevorzugt, deren Bindungsaffinität so groß ist, dass diese als Wirkstoff im Rahmen einer wirksamen medizinischen Behandlung in vertretbaren Mengen verabreicht werden können.

Eine Möglichkeit, die Bindungsaffinität auszudrücken, bieten die oben angesprochenen Kompetitionsexperimente, mit denen man in-vitro diejenige Konzentration an erfindungsgemäßem 5-Ring Heteroaromat ermittelt, die einen anderen Vergleichsbindungspartner zu 50% von der Rezeptorbindungsstelle verdrängt (IC50-Werte). So lässt sich auch die kompetitive Hemmung der Bindung von 5-CT an 5-HT5-Rezeptoren dahingehend auswerten, dass bevorzugte erfindungsgemäße 5-Ring Heteroaromaten halbmaximale Hemmkonstanten IC50 von weniger als 10-5 M, vorzugsweise von weniger als 10-6 M und insbesondere von weniger als 10-7 M aufweisen. Die Bindungsaffinität erfindungsgemäßer 5-Ring Heteroaromaten kann auch über die Hemmkonstante Ki ausgedrückt werden, die man im allgemeinen ebenfalls mit Kompetitionsexperimenten in-vitro bestimmt. Für die Bindung an 5-HT5-Rezeptoren weisen erfindungsgemäße 5-Ring Heteroaromaten vorzugsweise Ki-Werte von weniger als 10-6 M, vorteilhafterweise von weniger als 10-7 M und insbesondere bevorzugt von weniger als 10-8 M auf.

Brauchbare Bindungspartner können mit einer geringeren, einer im wesentlichen gleichen, oder einer höheren Affinität an 5-HT5 binden als an einen bestimmten, von 5-HT5 verschiedenen Rezeptor. So gehören zu Bindungspartnern für 5-HT5-Rezeptoren im Hinblick auf die erfindungsgemäße Verwendung insbesondere diejenigen, deren Bindungsaffinität zu 5-HT5-Rezeptoren verglichen mit der Affinität zu 5-HT-Rezeptoren so hoch ist, dass sie für die erfindungsgemäße Verwendung in vorteilhafter Weise geeignet sind. Dies setzt nicht notwendigerweise eine vergleichsweise selektivere Bindung an 5-HT5-Rezeptoren voraus, wenngleich selektive Bindungspartner für 5-HT5-Rezptoren eine besondere Ausführungsform der vorliegenden Erfindung sind.

Beispielsweise kann man Bindungspartner verwenden, die hochaffin sowohl zu 5-HT5 als auch zu anderen 5-HT-Rezeptoren sind. Hochaffin bedeutet in diesem Zusammenhang Ki-Werte in der Regel im Bereich von 1•10⁻¹⁰ M bis 1•10⁻⁶ M. Gemäß einer besonderen Ausführungsform besitzen erfindungsgemäße 5-Ring Heteroaromaten im hochaffinen Bereich zu 5-HT-Rezeptoren ein Bindungsprofil, dass durch eine Bindungsaffinität zu 5-HT5 gekennzeichnet ist, die im Vergleich zu anderen Bindungsaffinitäten dieses Bereichs im wesentlichen gleich oder nur wenig geringer ist. Faktoren von 10 oder weniger können von Vorteil sein.

Erfindungsgemäße 5-Ring Heteroaromaten besitzen Bindungsaffinitäten für 5-HT5-Rezeptoren, die größer sind als für einen oder mehrere von 5-HT5 verschiedene 5-HT-Rezeptoren, also insbesondere den obengenannten 5-HT-Rezeptorklassen 5-HT1, 5-HT2, 5-HT3, 5-HT4, 5-HT6 und 5-HT7 zuzuordnenden Rezeptoren. Ist die Bindungsaffinität für 5-HT5-Rezeptoren eines Bindungspartners größer als die eines von 5-HT5 verschiedenen 5-HT-Rezeptors, so spricht man von einer in Bezug auf den von 5-HT5 verschiedenen 5-HT-Rezeptor selektiven Bindung dieser Bindungspartner an 5-HT5-Rezeptoren. Besondere Bindungspartner sind diejenigen, deren Bindungsaffinität für 5-HT5-Rezeptoren größer ist als für wenigstens einen 5-HT-Rezeptor. 5-Ring Heteroaromaten, deren Bindungsaffinität für 5-HT5-Rezeptoren größer ist als für sämtliche von 5-HT5 verschiedene 5-HT-Rezeptoren, stellen eine weitere besondere Klasse erfindungsgemäßer 5-Ring Heteroaromaten dar.

Unter Selektivität versteht man die Eigenschaft eines Bindungspartners, vorzugsweise an 5-HT5-Rezeptoren zu binden. Für die vorstehend geschilderte Selektivität ist maßgebend, dass sich die Bindungsaffinitäten für 5-HT5-Rezeptoren einerseits und für einen oder mehrere von 5-HT5 verschiedene 5-HT-Rezeptoren andererseits hinreichend unterscheiden. Bevorzugt sind Affinitätsunterschiede, wonach Bindungsaffinitäts-Verhältnisse von wenigstens 2, vorteilhafter von wenigstens 5, besonders vorteilhaft von wenigstens 10, vorzugsweise von wenigstens 20, besonders bevorzugt von wenigstens 50 und insbesondere von wenigstens 100 vorliegen.

Gemäß einer weiteren Ausführungsform binden erfindungsgemäße 5-Ring Heteroaromaten in Bezug auf einen oder mehrere von 5-HT5 verschiedene 5-HT-Rezeptoren selektiv an 5-HT5-Rezeptoren mit den oben beschriebenen vorteilhaften Bindungsaffinitäten.

Gemäß einer weiteren Ausführungsform binden erfindungsgemäße 5-Ring Heteroaromaten in Bezug auf alle von 5-HT5 verschiedenen 5-HT-Rezeptoren selektiv an 5-HT5-Rezeptoren mit den oben beschriebenen vorteilhaften Bindungsaffinitäten.

Besonders vorteilhaft sind 5-Ring Heteroaromaten der Formel I die mit den vorstehend beschriebenen Affinitäten und Selektivitäten an 5-HT5-Rezeptoren binden, die von Gliazellen und insbesondere von Astrocyten exprimiert werden. Erfingdungsgemäß ist die humane Rezeptorvariante ein bevorzugtes Target für die erfindungsgemäßen 5-Ring Heteroaromaten.

Die Bindung erfindungsgemäßer 5-Ring Heteroaromaten an 5-HT5-Rezeptoren ist an eine Effektorfunktion gekoppelt. Bindungspartner können agonistisch oder antagonistisch sowie teilagonistisch und/oder teilantagonistisch wirken. Als Agonisten werden erfindungsgemäß Verbindungen bezeichnet, die ganz oder teilweise die Aktivität von 5-HT an 5-HT5-Rezeptoren nachahmen. Als Antagonisten werden erfindungsgemäße 5-Ring Heteroaromaten bezeichnet, welche die agonistische Aktivität von 5-HT an 5-HT5-Rezeptoren blockieren können.

Gemäß einer besonderen Ausführungsform der vorliegenden Erfindung werden 5-Ring Heteroaromaten eingesetzt, deren Bindung zumindest an 5-HT5-Rezeptoren h5-HT5-transfizierter CHO- oder HEK 293- oder SHSY-5Y-Zellen eine Veränderung der Agonist-induzierten Stimulierung der GTP-Bindung an membrangebundene G-Proteine, eine Veränderung intrazellulärer Calcium-Spiegel, eine Veränderung der Agonist-induzierten Induktion der Phospholipase C-Aktivität und/oder eine Veränderung der cAMP-Produktion bewirkt. Was die Veränderung intrazellulärer Calcium-Spiegel angeht, so stellt die Verwendung von 5-Ring Heteroaromaten, die eine Erhöhung intrazellulärer Calcium-Spiegel bewirken, eine besondere Ausführungsform der Erfindung dar. Zu dieser Ausführungsform, gehören auch 5-Ring Heteroaromaten, die in bekannten Tiermodellen für neurodegenerative und neuropsychiatrische Vorgänge wirksam sind.

Bevorzugt sind 5-Ring Heteroaromaten, die auch in Bezug auf ihre Effektorfunktion im oben beschriebenen Sinn selektiv für 5-HT5-Rezeptoren sind.

### Darreichungsformen und Formulierung

Aufgrund ihrer pharmakologischen Eigenschaften sind die erfindungsgemäßen 5-Ring Heteroaromaten als Wirkstoffe für therapeutische Zwecke brauchbar. Dabei werden die erfindungsgemäßen 5-Ring Heteroaromaten vorzugsweise vor der Verabreichung in eine geeignete Darreichungsform gebracht. Ein weiterer Gegenstand der vorliegenden Erfindung sind daher auch Zusammensetzungen, insbesondere pharmazeutische Zusammensetzungen, die wenigstens eine erfindungsgemäße 5-Ring Heteroaromaten und sowie einen pharmazeutisch annehmbaren Träger oder Verdünnungsmittel enthalten.

Pharmazeutisch annehmbar sind die im Bereich der Pharmazie und angrenzenden Gebieten bekanntermaßen verwendbaren Träger oder Hilfstoffe, insbesondere die in einschlägigen Arzneibüchern (z.B. DAB (Deutsches Arzneimittelbuch), Ph. Eur. (Pharmacopoeia Europaea), BP (Baccalaureus Pharmaciae), NF (National Formulary), USP (United States Pharmacopoeia) gelisteten, und auch andere Träger, deren Eigenschaften einer physiologischen Anwendung nicht entgegenstehen.

Geeignete Träger und Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel, Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie es beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Beispiele geeigneter Träger und Verdünnungsmittel beinhalten Lactose, Dextrose, Sucrose, Sorbitol, Mannitol, Stärken, Gum Acacia, Calciumphosphat, Alginate, Tragant, Gelatine, Calciumsilicat, microkristalline Cellulose, Polyvinylpyrrolidon, Cellulose, Wassersirup, Methylcellulose, Methyl- and Propylhydroxybenzoate, Talkum, Magnesiumstearat and Mineralöl.

Die erfindungsgemäßen 5-Ring Heteroaromaten können formuliert werden, um eine sofortige oder eine verzögerte Freigabe des Wirkstoffes an den Patienten zu gewährleisten.

Beispiele geeigneter pharmazeutischer Zusammensetzungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser- Emulsionen, Suspensionen, beispielsweise Lotionen, lnjektions- und Infusionszubereitungen, Augen- und Ohrentropfen. Auch implantierte Abgabevorrichtungen können zur Verabreichung der erfindungsgemäßen 5-Ring Heteroaromaten verwendet werden. Ferner können auch Liposomen oder Mikrosphären zur Anwendung kommen.

Die erfindungsgemäßen Zusammensetzungen können beispielsweise auf üblichem Wege verabreicht werden.

Bei der Herstellung erfindungsgemäßen Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z.B. eine Granulierung, Komprimierung und ähnliches.

Die erfindungsgemäße Verwendung der erfindungsgemäßen Wirkstoffe beinhaltet im Rahmen der Behandlung ein Verfahren. Dabei wird dem zu behandelnden Individuum, vorzugsweise einem Säuger, insbesondere einem Menschen, und auch einem Nutz- oder Haustier, eine wirksame Menge wenigstens einer 5-Ring Heteroaromaten der Formel I, in der Regel der pharmazeutischen Praxis entsprechend formuliert, verabreicht.

Die Erfindung betrifft auch die Herstellung von Mitteln zur Behandlung eines Individuums, vorzugsweise eines Säugers, insbesondere eines Menschen, Nutz- oder Haustieres.

Die 5-Ring Heteroaromaten der Formel I oder die entsprechende pharmazeutische Zusammensetzung können oral, rektal, topisch, parenteral, einschließlich subkutan, intravenös und intramuskulär, okular, pulmonar oder nasal verabreicht werden. Bevorzugt ist eine orale Verabreichung.

Eine wirksame Dosierung des Wirkstoffes kann von der Art der 5-Ring Heteroaromaten, der Verabreichungsart, der zu behandelnden Erkrankung und der Schwere der zu behandelnden Erkrankung abhängig sein. Eine derartige wirksame Dosierung des Wirkstoffes kann von dem Fachmann auf dem Gebiet ohne Schwierigkeiten ermittelt werden.

Die Dosierung hängt vom Alter, Zustand und Gewicht des Patienten sowie von der Applikationsart ab. In der Regel beträgt die tägliche Wirkstoffdosis zwischen etwa 0,5 und 100 mg/kg Körpergewicht bei oraler Gabe und zwischen etwa 0,1 und 10 mg/kg Körpergewicht bei parenteraler Gabe.

### Herstellung der 5-Ring Heteroarmaten

Die erfindungsgemäßen 5-Ring Heteroaromaten lassen sich analog zu literaturbekannten Methoden herstellen, wie sie dem Fachmann bekannt sind. So ist die Synthese von Pyrazolen allgemein beschrieben in Synthetic Communications 2004, 34 (23), 4359; Heterocyclic Communications 2004, 10 (2-3), 163; Journal of Medicinal Chemistry 2004, 47 (24), 5872-5893; WO 2004037794; W02002004424 oder sie lassen sich analog zu Beispiel 4 herstellen. Hydrazine sind kommerziell erhältlich oder nach literaturbekannten Methoden-(z. B. Houben-Weyl, Methoden der organischen Chemie, Band X/2 und E16A Stuttgart, 1967 und 1994; M.B. Smith, J. March, March's Advanced Organic Chemistry, New York, 2001) herstellbar. Die Derivatisierung der freien Aminogruppe in III (z.B. Acylierung oder Alkielerung) lässt sich nach literaturbekannten Methoden durchführen, wie sie dem. Fachmann bekannt sind (siehe auch: Schema 1).

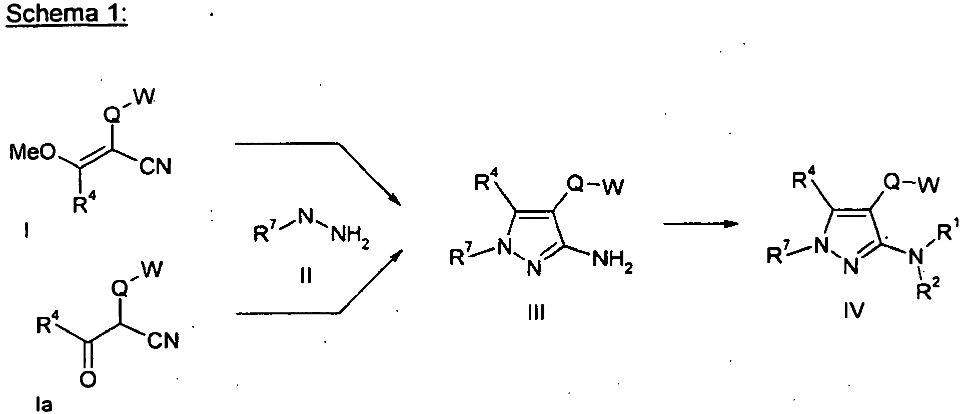

Die Synthese von Triazolen ist beschrieben in Journal of the Chemical Society Abstracts, 1950, 612-614, Journal of Medicinal Chemistry, 2002, 45(14), 2942-2952 oder sie lassen sich analog zu Beispiel 5 herstellen. Hydrazide und Thiohydrazide sind kommerziell erhältlich oder nach literaturbekannten Methoden (z. B. Houben-Weyl, Methoden der organischen Chemie, Band VIII, X/2 und E5, Stuttgart, 1952,1967 und 1985 bzw. E5, Stuttgart, 1985; M.B: Smith, J. March, March's Advanced Organic Chemistry, New York, 2001) herstellbar. Die nach dem in Schema 2 abgebildeten Syntheseweg verwendeten Aldehyde/Ketone VI und Amine XIV sind ebenfalls kommerziell erhältlich oder beispielsweise nach bekannten Vorschriften (z. B. Houben-Weyl, Methoden der organischen Chemie, Band XI/1, Stuttgart, 1957 und Houben-Weyl, Methoden der organischen Chemie, Band VII/1, VII2A, EIII Stuttgart, 1954, 1973, 1983) herstellbar. Auch in diesen Fällen lässt sich die Derivatisierung der freien Aminogruppe in VIII und XII (z.B. Acylierung oder Alkylierung) nach literaturbekannten Methoden durchführen, wie sie dem Fachmann bekannt sind (Schema 2 und 2a).

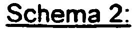

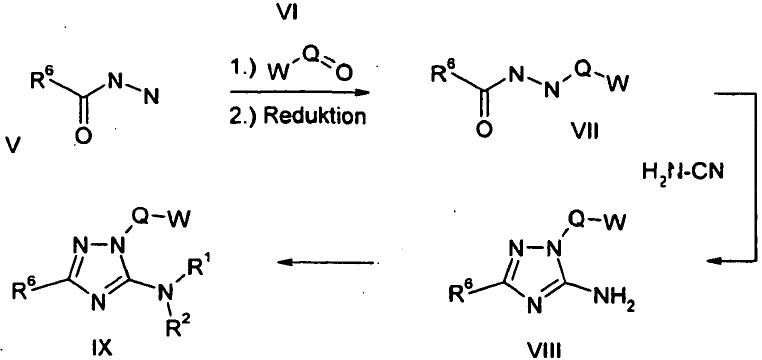

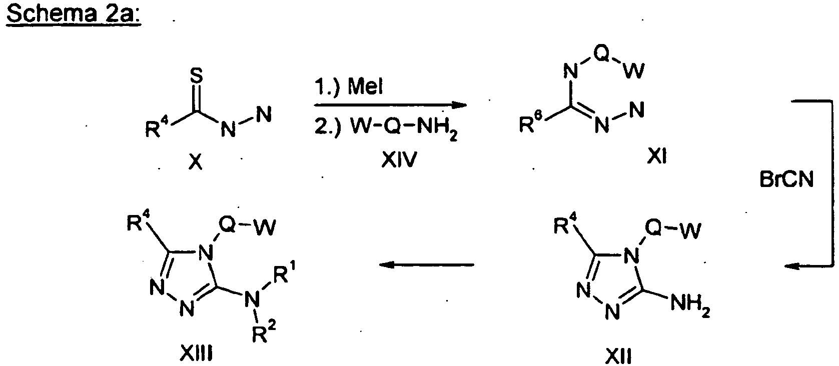

Für den Fall, dass es sich bei den 5-Ring Heteroaromaten um Imidazole und deren Zwischenprodukte handelt sind analoge Synthesen beschrieben in Journal of the Chemical Society - Abstracts, 1950, 2775-2784; Journal of Medicinal Chemistry 1993, 36 (22), 3337; Arzneimittelforschung 1977, 27 (10), 1889-1918; Tetrahedron 2004, 60 (18), 3987-3997; European Journal of Medicinal Chemistry 1992, 27 (7), 717-722; Arzneimittelforschung 1984, 34 (11A), 1612-1624, Journal of Organic Chemistry 1994, 59(24), 7299-7305; Tetrahedron 2002, 58 (49), 9865-9870; Journal of Organic Chemistry 1994, 59 (6), 1589-90 oder sie lassen sich analog zu den Beispielen 1 bis 3 herstellen. (Schema 3 und 3a). Die Synthese von Aminonitrilen XXI ist zum Beispiel über Streckersynthese möglich und zum Beispiel beschrieben in Houben-Weyl, Methoden der organischen Chemie, Band VIII und E5 Stuttgart, 1952 und 1985) sowie in Biorganic&Medicinal Chemistry Letters 2004, 14 (21), 5317-5322, Tetrahedron 2004, 60 (46) 10559 oder Synlett 2004, (4), 688-692.

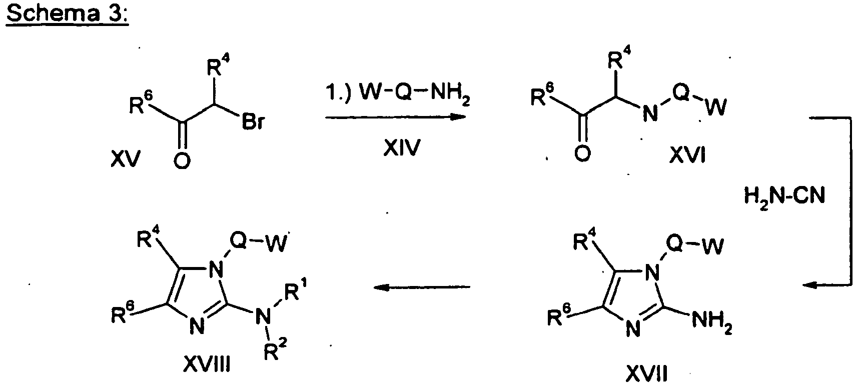

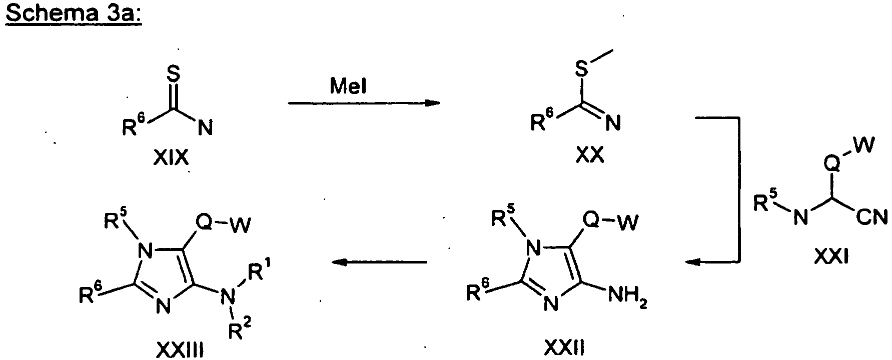

Die ebenfalls benötigten α-Haloketone und Thioamide sind kommerziell erhältlich oder nach der Literatur herstellbar (z. B. Houben-Weyl, Methoden der organischen Chemie, 4. Auflage, Band VII/2c, Stuttgart, 1977 + Houben-Weyl, Methoden der organischen Chemie, Band E5, Stuttgart, 1985).

Weitere Methoden zur Synthese der 5-Ring Heteroaromaten finden sich in Houben-Weyl, Methoden der organischen Chemie, Band E8b und E8c, Stuttgart, 1994; M.B. Smith, J. March, March's Advanced Organic Chemistry, New York, 2001 und in den weiter ober genannten genannten Literaturstellen zur Verwendung und Herstellung von Imidazolen, Triazolen und Pyrazolen.

Die erfindungsgemäßen 5-Ring Heteroaromaten können genauso wie die gegebenenfalls anfallenden Zwischenprodukte auf herkömmliche Art und Weise gewonnen sowie erforderlichenfalls aufgereinigt werden, beispielsweise durch Umkristallisieren aus üblichen organischen Lösungsmitteln, vorzugsweise einem kurzkettigen Alkohol wie Ethanol, oder mit Hilfe chromatographischer Techniken.

Je nach Einsatzstoffen fallen die erfindungsgemäßen Guanidinverbindungen der Formel in freier Form oder bereits als Säureadditionssalze an. Sowohl die Verbindungen in freier Form als auch verfahrensgemäß resultierende Salze dieser Verbindungen können in an sich bekannter Weise in gewünschte Säureadditionssalze bzw. in die freie Form überführt werden.

Die folgenden Beispiele veranschaulichen die Erfindung ohne sie einzuschränken. Es ist zu beachten, dass Bezeichnung und formelmäßige Darstellung von Salzen mit protoniertem Stickstoff lediglich eine von mehreren allesamt erfassten Möglichkeiten hinsichtlich der Ladungsverteilung wiedergibt. Dies gilt auch für tautomere Formen.

### Herstellungsbeispiele

### Beispiel 1: 4-(4-Bromo-phenyl)-1-(2-methoxy-benzyl)-1H-imidazo!-2-ylamin

### 1.1. 1-(4-Bromo-phenyl)-2-(2-methoxy-benzylamino)-ethanon

Eine Mischung aus 500 mg (1.8 mmol) 2-Bromo-1-(4-bromo-phenyl)-ethanon und 520 mg (3.8 mmol) 2-Methoxybenzylamin in 100 ml Diethylether wird 2 Std bei Raumtemperatur gerührt. Danach wird der entstandene Niederschlag abfiltriert, das Filtrat mit 1 M isopropanolischer HCl versetzt und mit Diethylether verdünnt. Der so erhaltene Niederschlag wird filtriert und ohne weitere Reinigung in der nächsten Stufe eingesetzt..

### 1.2. 4-(4-Bromo-phenyl)-1-(2-methoxy-benzyl)-1H-imidazol-2-ylamin

Eine Lösung des Ketoamins (670 mg, 1.8 mmol Rohprodukt aus 1.1) in 40ml TetrahydrofuranlWasser (1/1) wird mit gesättigter NaHC03 Lösung auf pH 4.5 eingestellt, mit 380 mg (9 mmol) Cyanamid versetzt und 2 Stunden unter Rückfluß gerührt. Nach beendeter Reaktion wird der Ansatz mit Ethylacetat versetzt und nacheinander mit 1 N NaOH und Wasser extrahiert. Die organische Phase wird getrocknet (MgS04) eingeengt und der Rückstand an Kieselgel mit Methylenchlorid/Methanol 50/1 getrennt, wobei 100mg des Zielprodukts als Feststoff erhalten wurden.
ESI-MS [M+H⁺] = 358 / 360
¹H-NMR (400 MHz, CDCl₃), δ (ppm): 3.90 (s, 3H), 4.45 (d, 2H), 5.60 (s, 2H), 6.90 (m, 2H), 7.0 - 7.1 (m, 2H), 7.25-7.35 (m, 1 H), 7.45 (d, 2H), 7.55 (d, 2H).

### Beispiel 2: 1-(2-Methoxy-benzyl)-4-(4-nitro-phenyl)-1H-imidazol-2-ylamin

### 2.1 N-[4-(4-Nitro-phenyl)-1H-imidazol-2-yl]-acetamid

Eine Lösung aus 5.0 g (20.5 mmol) 2-Bromo-1-(4-nitro-phenyl)-ethanon und 5.0 g (49.5 mmol) Acetylguanidin in 70 ml Acetonitril werden 4 Stunden bei 40°C und weiter 72Std bei Raumtemperatur gerührt. Der entstandene Niederschlag wird abgesaugt in Methanol aufgerührt erneut filtriert und dann getrocknet. Der so erhaltene Feststoff (0.7 g) wird ohne weitere Reinigung eingesetzt.
ESI-MS [M+H⁺] = 265

### 2.2 4-(4-Nitro-phenyl)-1H-imidazol-2-ylamin

N-[4-(4-Nitro-phenyl)-1 H-imidazol-2-yl]-acetamid 2.7g (11 mmol) werden in 30ml EtOH aufgenommen, mit 50ml halbkonzentrierter Salzsäure versetzt und 3 Stunden unter Rückfluß gerührt. Der Ansatz wird mit 6M NaOH alkalisch gestellt und mit Ethylacetat extrahiert. Die organische Phase wird getrocknet (MgS04) eingeengt. Der Rückstand (0.9 g) wird ohne Reinigung weiter eingesetzt.

### 2.3 1-(2-Methoxy benzyl)-4-(4-nitro-phenyl)-1H-imidazol-2-ylamin

Eine Mischung aus 4-(4-Nitro-phenyl)-1H-imidazol-2-ylamin (0.9g, 4.4 mmol), 2-Methoxybenzylchlorid (0.69g, 4.4 mmol) und Cs2C03 (1.6g, 4.9 mmol) in Dimethylformamid werden 18 Stunden bei Raumtemperatur gerührt. Der Ansatz wird mit halbkonzentrierter Kochsalzlösung versetzt und mit Ethylacetat extrahiert. Die organische Phase wird nochmals mit Wasser und gesättigter Kochsalzlösung gewaschen, eingeengt und der Rückstand an Kieselgel mit Methylenclorid/Methanol 50:1 gereinigt (100 mg roter Feststoff). ESI-MS [M+H⁺] = 325
¹H-NMR (400 MHz, CDCl₃), δ (ppm): 3.85 (s, 3H), 4.45 (d, 2H), 5.80 (s, 2H), 6.85-6.95 (m, 2H), 7.05 (d, 2H), 7.25-7.35 (m, 1H), 7.35 (s, 1 H), 7.80 (d, 2H), 8.10 (d, 2H).

### Beispiel 3: 5-(2-Methoxy-benzyl)-2-phenyl-3H-imdazol-4-ylamin

### 3.1 2-(Benzhydryliden-amino)-3-(2-methoxy-phenyl)-propionitril

Eine Lösung aus 13.7g (Benzhydrylidene-amino)-acetonitril (62 mmol) und 10.3 g 2-Methoxybenzylchlorid (66 mmol) in 150 ml Methylenchlorid wurde mit 75 ml 11 M NaOH versetzt und nach weiterer Zugabe von 1.4g Benzyltriethylammoniumchlorid (6.2 mmol) 3 Stunden bei Raumtemperatur kräftig gerührt. Nach Trennung der Phasen wurde dann die organische Phase mit Wasser gewaschen, über Na2SO4 getrocknet, filtriert und eingeengt. Der so erhaltene Rückstand (22.5g) wird ohne weitere Reinigung in der nächsten Stufe eingesetzt.

### 3.2 2-Amino-3-(2-methoxy-phenyl)-propionitril

Eine Lösung von 22.5 g 2-(Benzhydryliden-amino)-3-(2-methoxy-phenyl)-propionitril (66 mmol, Rohprodukt aus 3.1.) in 200 ml Tetrahydrofuran wurde mit 70ml 1M HCl versetzt, 3 Stunden bei Raumtemperatur gerührt und die Reaktionslösung anschließend am Rotationverdampfer weitgehend eingeengt. Nach Versetzen mit Methylenchlorid wird die organische Phase mehrmals mit 6M HCl extrahiert, die vereinigten salzsauren Phasen mit 50% NaOH alkalisch gestellt, mit Methylenchlorid extrahiert und dann die organsiche Phase über (Na2S04) getrocknet und eingeengt. Der ölige Rückstand (10.4g) wurde ohne weitere Reinigung eingesetzt.

### 3.3 5-(2-Methoxy-benzyl)-2-phenyl-3H-imidazol-4-ylamin

Eine Lösung aus 1.7 g (10 mmol) 2-Amino-3-(2-methoxy-phenyl)-propionitrit und 2.7g (10 mmol) Thiobenzimidsäuremethylester Hydroiodid in 30 ml Dioxan wurde 2 Stunden unter Rückfluß gerührt. Nach abtrennen vom entstandenen Niederschlag, wurde die Lösung eingengt, der Rückstand in Methylenchlorid, aufgenommen, mit Wasser gewaschen, dann die Organische Phase getrocknet (Na2S04), filtriert, eingeengt und der Rückstand 1.4 g in Essigsäureethylester/Heptan 19/1 verrührt. Der so erhaltene Niederschlag wurde abgetrennt, die Mutterlauge eingeengt (430 mg) und mittels präparativer HPLC (Fließmittel Wasser/Acetonitril/Essigsäure) getrennt. 131 mg Zielprodukt, das mit 1M etherischer HCl Lösung in das Hydrochlorid überführt wurden (92 mg).
ESI-MS [M+H⁺] = 280
¹H-NMR (400 MHz, d₆-DMSO), δ (ppm): 3.85 (s, 3H), 3.95 (s, 2H), 6.9 (t, 1 H), 6.78 (s, 1H), 7.05 (t, 2H), 7.35 (t, 1H), 7.5 (m, 3H), 7.9 (d, 2H). 13.9 (s, br, 1H).

### Beispiel 4: 4-(2-Methoxy benzyl)-1-phenyl-1H-pyrazol-3-ylamin

### 4.1 2-Methoxymethyl-3-(2-methoxy phenyl)-acrylonitril

Eine Mischung aus 5.2 g 2-Methoxybenzaldehyd (38 mmol) und 4.0 g Acrylnitril (5 ml, 76 mmol) wurde bei 10°C innerhalb von 1 Stunde in eine frisch hergestellte Natriummethylatlösung (1.3 g Na, 57 mmol in 100 ml Methanol) zugetropft und dann 18 stunden bei Raumtemperatur gerührt. Der Ansatz wurde anschließend mit Wasser verdünnt und mit Essigsäureethylester extrahiert. Trocknen und Einengen der organischen Phase erbrachten 10 g Rohprodukt, daß durch Chromatographie an Kieselgel (Fließmittel: Methylenchlorid/Methanol 1:19) gereinigt wurde (4.4 g Produkt als Gemisch von mindestens drei Isomeren)

### 4.2 4-(2-Methoxy-benzyl)-1-phenyl-1H-pyrazol-3-ylamin

Eine Lösung aus 0.6 ml Natriummethylat (30% in Methanol, 10.6 mmol) in 10 ml Methanol wurde bei Raumtemperatur mit 1 g (4.9 mmol) 2-Methoxymethyl-3-(2-methoxy-phenyl)-acrylonitril in 10 ml DMSO versetzt 3.5 Stunden auf 85°C erwärmt und dann mit 0.57 g Phenylhydrazin (4.9 mmol) versetzt. Der Ansatz wurde dann auf 125°C erwämt, das Methanol dabei über einen Wasserabscheider abgetrennt, anschließend nochmal 1 Stunde bei 115°C gerührt dann auf 5°C abgekühlt mit 50 ml Wasser versetzt und 1 Stunde gerührt. Der entstandene Niederschlag wurde abgesaugt, mit Wasser gewaschen, getrocknet (0.6 g) und dann mit 30 ml Diethylether verrührt (0.35 g Zielprodukt)
ESI-MS [M+H⁺] = 280
¹H-NMR (400 MHz, d₆-DMSO), δ (ppm): 3.65 (s, 2H), 3.85 (s, 3H), 4.9 (s, 2H), 6.85 (t, 1 H), 6.95 (d, 1H), 7.05 (t, 1 H), 7.15-7.25 (m, 2H), 7.35 (t, 2H), 7.55 (d, 2H), 7.85 (s, 1 H).

### Beispiel 5: 2-(2-Methoxy-benzyl)-5-phenyl-4H-[1,2,4]triazol-3-ylamin

### 5.1 Benzoesäure [1-(2-methoxy-phenyl)-methylidene]-hydrazid

Eine Suspension aus 1.5 g (7.3 mmol) 2-Methoxybenzaldehyd und 1 g (7.3 mmol) Benzhydrazid in 35 ml n-Hexan wurde über Nacht bei Raumtemperatur gerührt. Der Niederschlag wurde abgesaugt, getrocknet und ohne weitere Reinigung weiter eingesetzt (1.75 g).

### 5.2 Benzoesäure N-(2-methoxy-benzyl)-hydrazid

Zu einer Lösung aus 1.45 g (5.7 mmol, Rohprodukt aus 5.1) Benzoesäure [1-(2-methoxy-phenyl)-methylidenel-hydrazid in 10 ml Trifluoressigsäure wurden bei 0°C 1.8 ml (11.4 mmol) Triethylsilan gegeben, dann zunächst 2 Studen bei 0°C und anschließend 4 Stunden bei Raumtemperatur gerührt. Die gelbe Reaktionslösung wurde jetzt mit 120 ml 2N HCl versetzt, mit Methylenchlorid extrahiert, die organische Phase mit 1 N NaOH geschüttelt dann mit Na2SO4 getrocknet und am Rotationsverdampfer eingeengt. Ausrühren in Diisopropylether und anschließendes Trocknen erbrachte ca. 1.1 g Produkt als weißen Festkörper.

### 5.3 2-(2-Methoxy-benzyl)-5-phenyl-4H-[1,2,4]triazol-3-ylamin

Benzoesäure N'-(2-methoxy-benzyl)-hydrazid (200 mg, 0.78 mmol aus 5.2) wurde in einer Mischung aus 1.5 ml Dioxan und 1 ml Wasser gelöst, mit 164 mg (3.9 mmol) Cyanamid, gelöst in 1 ml Wasser, versetzt, mit 1N HCl auf pH 2 eingestellt und dann in der Mikrowelle 4 Stunden bei 120°C und 200 Watt Leistung gerührt. Dabei wurde der pH alle 30 minuten überprüft und falls nötig erneut auf pH 2 eingestellt. Anschließend wurde der entstande Festkörper abgesaugt, mit wenig Diethylether gewaschen und getrocknet (65 mg Zielprodukt als leicht gelblicher Festkörper)
ESI-MS [M+H⁺] = 281
¹H-NMR (400 MHz, d₆-DMSO), δ (ppm): 3.85 (s, 3H), 5.1 (s, 2H), 6.35 (s, 2H), 6.75 (d, 1 H), 6.90 (t, 1 H), 7.05 (d, 1H), 7.25-7.4 (m, 4H), 7.85 (d, 2H).

### Die Herstellung der Endprodukte der Formel I

Die Verbindungen 6 und 7 wurden durch Umsetzung geeigneter Ausgangsmaterialien analog zu Beispiel 4 die Verbindungen 8-40, 42-50, 52, 54 und 61 durch Umsetzung geeigneter Ausgangsmaterialien analog zu Beispiel 1 oder 2, die Verbindung 41 durch Umsetzung geeigneter Ausgangsmaterialien analog zu Anthony, Neville J.; Stokker, Gerald E.; Gomez, Robert P.; Solinsky, Kelly M.; Wai, John S.; Williams, Theresa M.; Young, Steven D.; Hutchihson, John H.; Halczenko, Wasyl; Biphenyl-substituted imidazoles useful as inhibitors of farnesyl-protein transferase, PCT Int. Appl. (1997), WO 9736875, die Verbindung 53 analog zu Beispiel 51, die Verbidnungen 56-60, 63-64 durch Umsetzung geeigneter Ausgangsmaterialien analog zu Beispiel 55 und die Verbindung 62 analog zu Beispiel 3 hergestellt:

### Beispiel 6:

### 4-(2-Methoxy-benzyl)-1-(4-methoxy-phenyl)-1H-pyrazol-3-ylamine

ESI-MS [M+H⁺] = 310

### Beispiel 7:

### 1-(4-Fluoro-phenyl)-4-(2-methoxy benzyl)-1H-pyrazol-3-ylamin

ESI-MS [M+H⁺] = 298

### Beispiel 8:

### 1-(2,6-Dimethoxy-benzyl)-4-phenyl-1H-imidazol-2-ylamin hydrochlorid

ESI-MS [M+H⁺] = 310

### Beispiel 9:

### N-[1-(2-Methoxy-benzyl)-4-phenyl-1H-imidazol-2-yl]-acetamid

ESI-MS [M+H⁺] = 322

### Beispiel 10:

### 1-(2-Methoxy-benzyl)-4-phenyl-1H-imidazol-2-ylamine hydrochlorid

ESI-MS [M+H⁺] = 280

### Beispiel 11:

### 4-tert-Butyl-1-(2-methoxy-benzyl)-1H-imidazol-2-ylamin

ESI-MS [M+H⁺] = 260

### Beispiel 12:

### 4-(4-Fluoro-phenyl)-1-(2-methoxy-benzyl)-1H-imidazol-2-ylamin

ESI-MS [M+H⁺] = 298

### Beispiel 13:

### 1-(2-Methoxy-benzyl)-4-thiophen-2-yl-1H-imidazol-2-ylamine

ESI-MS [M+H⁺] = 286

### Beispiel 14:

### 1-(2-Methoxy benzyl)-5-methyl-4-phenyl-1H-imidazol 2-ylamine

ESI-MS [M+H⁺] = 294

### Beispiel 15

### 4-Furan-2-yl-1-(2-methoxy-benzyl)-1H-imidazol-2-ylamine hydrochlorid

ESI-MS [M+H⁺] = 270

### Beispiel 16:

### 1-(2-Methoxy-benzyl)-4-naphthalen-2-yl-1H-imidazol-2-ylamine hydrochlorid

ESI-MS [M+H⁺] = 330

### Beispiel 17:

### 4-Benzo[b]thiophen-3-yl-1-(2-methoxy-benzyl)-1H-imidazol-2-ylamin hydrochorid

ESI-MS [M+H⁺] = 336

### Beispiel 18:

### 1-(2,6-Dimethoxy-benzyl)-4-(4-fluoro-phenyl)-1H-imidazol-2-ylamine

ESI-MS [M+H⁺] = 328

### Beispiel 19:

### 1-(2,6-Dimefhoxy-benzyl)-4-thiophen-2-yl-1H-imidazol-2-ylamine

ESI-MS [M+H⁺] = 316

### Beispiel 20:

### 1-(2-Methoxy-benzyl)-4-thiophen-3-yl-1H-imidazol-2-ylamine hyrochlorid

ESI-MS [M+H⁺] = 286

### Beispiel 21:

### 2-Amino-1-(2-methoxy-benzyl)-1H-imidazole-4-carboxylic acid ethyl ester

ESI-MS [M+H⁺] = 276

### Beispiel 22:

### 1-(2-Methoxy-benzyl)-4-(4-tritluoromethoxy-phenyl)-1H-imidazol-2-ylamine

ESI-MS [M+H⁺] = 364

### Beispiel 23:

### 1-(2-Methoxy benzyl)-4-(3-methoxy-phenyl)-1H-imidazol-2-ylamine

ESI-MS [M+H⁺] = 310

### Beispiel 24:

### 4-Adamantan-1-yl-1-(2-mefhoxy-benzyl)-1H-imidazol-2-ylamine

ESI-MS [M+H⁺] = 338

### Beispiel 25:

### 4-[2-Amino-1-(2,6-dimethoxy-benzyl)-1H-imidazol-4-yl]-benzonitrile hydrochlorid

ESI-MS [M+H⁺] = 335

### Beispiel 26:

### 1-(2,6-Dimethoxy-benzyl)-4-(2-methoxy-phenyl)-1H-imidazol-2-ylamine hydrochlorid

ESI-MS [M+H⁺] = 340

### Beispiel 27:

### 1-(2-Methoxy-benzyl)-4-p-tolyl-1H-imidazol-2-ylamine

ESI-MS [M+H⁺] = 294

### Beispiel 28:

### 1-(2-Chloro-6-methoxy-benzyl)-4-thiophen-3-yl-1H-imidazol-2-ylamine

ESI-MS [M+H⁺] = 320/322

### Beispiel 29:

### 1-(2-Chloro-6-methoxy-benzyl)-4-(4-trifluoromethioxy-phenyl)-1H-imidazol-2-ylamine

ESI-MS [M+H⁺] = 398/400

### Beispiel 30:

### 5-[2-Amino-1-(2-methoxy-benzyl)-1H-imidazol-4-yl]-isoxazole-3-carboxylic acid ethyl ester

ESI-MS [M+H⁺] = 343

### Beispiel 31:

### 1-(2-Fluoro-6-methoxy-benzyl)-4-thiophen-3-yl-1H-imidazol-2-ylamine

ESI-MS [M+H⁺] = 304

### Beispiel 32:

### 1-(2-Chloro-6-methoxy-benzyl)-4-(4-methoxy-phenyl)-1H-imidazol-2-ylamine

ESI-MS [M+H⁺] = 344

### Beispiel 33:

### 4-[2-Amino-1-(2-fluoro-6-methoxy-benzyl)-1H-imidazol-4-yl]-benzonitrile

ESI-MS [M+H⁺] = 323

### Beispiel 34:

### 1-(2-Chloro-6-methoxy-benzyl)-4-(4-chloro-phenyl)-1H-imidazol-2-ylamine

ESI-MS [M+H⁺] = 348/350/352

### Beispiel 35:

### 4-(2,4-Dichloro-phenyl)-1-(2-methoxy-benzyl)-1H-imidazol-2-ylamine hydrochloride

ESI-MS [M+H⁺] = 348/350/352

### Beispiel 36:

### 1-(2-Methoxy-benzyl)-4-(5-methyl-furan-2-yl)-1H-imidazol-2-ylamine

ESI-MS [M+H⁺] = 284

### Beispiel 37:

### 1-(2-Chloro-6-methoxy-benzyl)-4-thiophen-2-yl-1H-imidazot-2-ylamine

ESI-MS [M+H⁺] = 320

### Beispiel 38:

### 1-(2-Methoxy-benzyl)-4-(5-methyl-thiophen-2-yl)-1H-imidazol-2-ylamine

ESI-MS [M+H⁺] = 300

### Beispiel 39:

### 4-(4-Isopropyl-phenyl)-9-(2-methoxy-benzyl)-1H-imidazol-2-ylamine

ESI-MS [M+H⁺] = 322

### Beispiel 40:

### 1-(2,6-Dimethoxy benzyl)-4-(3,4-dimethyl-phenyl)-1H-imidazol-2-ylamine hydrochlorid

ESI-MS [M+H⁺] = 338

### Beispiel 41:

### 5-Benzyl-1-(2-methoxy-benzyl)-1H-imidazol-2-ylamine

ESI-MS [M+H⁺] = 294

### Beispiel 42:

### 4-Benzofuran-2-yl-1-(2-methoxy-benzyl)-1H-imidazol-2-ylamine

ESI-MS [M+H⁺] = 320

### Beispiel 43:

### 1-(2-Methoxy-benzyl)-4-naphthalen-1-yl-1H-imidazol-2-ylamine

ESI-MS [M+H⁺] = 330

### Beispiel 44:

### 1-(2-Methoxy-benzyl)-4-(4-phenoxy-phenyl)-1H-imidazol-2-ylamine hydrochlorid

ESI-MS [M+H⁺] = 372

### Beispiel 45:

### 1-(2-Methoxy benzyl)-4-(3-nitro-phenyl)-1H-imidazol-2-ylamine

ESI-MS [M+H⁺] = 325

### Beispiel 46:

### 4-(4-Difluoromethoxy phenyl)-1-(2-methoxy benzyl)-1H-imidazol-2-ylamine

ESI-MS [M+H⁺] = 346

### Beispiel 47:

### 1-(2-Methoxy-benzyl)-4-(2-phenyl-thiazo/-4-yl)-1H-imidazo/-2-ylamine

ESI-MS [M+H⁺] = 363

### Beispiel 48:

### 4-(4-Benzyloxy-phenyl)-1-(2-methoxy-benzyl)-1H-imidazol-2-ylamine

ESI-MS [M+H⁺] = 386

### Beispiel 49:

### 1-(2-Methoxy-benzyl)-4-thiazol-2-yl-1H-imidazol-2-ylamine hydrochlorid

ESI-MS [M+H⁺] = 287

### Beispiel 50:

### 5-[2-Amino-1-(2-methoxy-benzyl)-1H-imidazo/-4-yl]-thiophene-2-carbonitrile

ESI-MS [M+H⁺] = 311

### Beispiel 51:

### 4-(3-Amino-phenyl)-1-(2-methoxy-benzyl)-1H-imidazol-2-ylamin

*51.1 Eine Mischung aus 2.5 g 1-(2-Methoxy-benzyl)-4-(3-nifro-phenyl)-1H-imidazol 2-ylamine (7.7 mmol, Beispiel 45), 2.5 g Aktivkohle, 30 mg Eisen(III)chlorid (als Hexahydrat) in 100 ml Methanol und 20 ml Acetonitril wurde auf Rückfluß erhitzt, langsam mit 50 ml Hydrazin versetzt und anschließend 2 weitere Stunden unter Rückfluß gerührt Der Ansatz wurde filtriert eingeengt, in Essigsäureethylester aufgenommen, mit Wasser extrahiert, die organische Phase getrocknet (Na2SO4) und eingeengt. Der so erhaltene Rückstand wurde in Essigsäureethylester aufgerührt, abgesaugt und getrocknet (2g Zielprodukt)*
ESI-MS [M+H⁺] = 295

### Beispiel 52:

### 1-(2,6-Dimethoxy-benzyl)-4-(4-methyl-thiophen-2-yl)-1H-imidazol-2-ylamine

ESI-MS [M+H⁺] = 330

### Beispiel 53:

### 4-(4-Amino-phenyl)-1-(2-mefhoxy benzyl)-1H-imidazol-2-ylamine hydrochlorid

ESI-MS [M+H⁺] = 295

### Beispiel 54:

### 4-[2-Amino-1-(2-methoxy-4-nitro-benzyl)-1H-imidazol-4-yl]-benzonitrile

ESI-MS [M+H⁺] = 350

### Beispiel 55:

### N-(3-[2-Amino-1-(2-methoxy-benzyl)-1H-imidazol-4-yl]-phenyl)-4-isopropyl-benzenesulfonamide

*55.1 Eine Lösung aus 100 mg 4-(3-Amino-phenyl)-1-(2-methoxy-benzyl)-1H-imidazol-2-ylamin (0. 34 mmol, Beispiel 51) in 3 ml Pyridin wurde bei 0°C mit 74 mg 4-i-Propylbenzolsolfonsäurechlorid versetztund anschließend 18 Stunden bei Raumtemperatur gerührt. Der Ansatz wurde anschließend mit Methylenchlorid verdünnt, mit 1 M NaOH sowie Wasser extrahiert, dann die organische Phase getrocknet (Na2S04), eingeengt und der so erhaltene Rückstand in Essigsäureethylester aufgerührt, abgesaugt und getrocknet (130 mg Zielprodukt)*
ESI-MS [M+H⁺] = 477

### Beispiel 56:

### N-(3-[2-Amino-1-(2-methoxy-benzyl)-1H-imidazol-4-yl]-phenyl)-benzenesulfonamide

ESI-MS [M+H⁺] = 435

### Beispiel 57:

### N-{3-[2-Amino-1-(2-methoxy-benzyl)-1H-imidazol-4-yl]-phenyl}-methanesulfonamide

ESI-MS.[M+H⁺] = 373

### Beispiel 58:

### N-{3-[2-Amino-1-(2-methoxy-benzyl)-1H-imidazol-4-yl]-phenyl}-benzamide

ESI-MS [M+H⁺] = 399

### Beispiel 59:

### N-{4-[2-Amino-1-(2-methoxy-benzyl)-1H-imidazol-4-yl]-phenyl}-benzenesulfonamide

ESI-MS [M+H⁺] = 435

### Beispiel 60:

### Thiophene-2-sulfonic acid {3-[2-amino-1-(2-methoxy-benzyl)-1H-imidazol-4-yl]-phenyl}-amide

ESI-MS [M+H⁺] = 441

### Beispiel 61:

### 1-(2-Methoxy-benzyl)-4-pyridin-3-yl-1H-imidazol-2-ylamine

ESI-MS [M+H⁺] = 281

### Beispiel 62:

### 5-(2-Methoxy-benzyl)-2-(4-methoxy-phenyl)-3H-imidazol-4-ylamine hydrochlorid

ESI-MS [M+H⁺] = 310

### Beispiel 63:

### Biphenyl-4-sulfonic acid {3-[2-amino-1-(2-methoxy-benzyl)-1H-imidazol-4-yl]-phenyl}-amide

ESI-MS [M+H⁺] = 511

### Beispiel 64:

### Propane-2-sulfonic acid {3-(2-amino-1-(2-methoxy-benzyl)-1H-imidazol-4-yl]-phenyl}-amide

ESI-MS [M+H⁺] = 401

### Biologische Tests

### 1. [³H]5-CT Bindungsassay h5-HT_{5A}

Membranen von HEK293-Zellen, die das h5-HT_{5A-}Rezeptorgen permanent exprimieren, werden in 100 mM Tris-HCl-Puffer (pH 7,7), der 1 mM EDTA enthält, in Gegenwart von 2,5 nM [³H]5-CT inkubiert (600 µl Gesamtvolumen). Die Gesamtbindung ist definiert durch die Bindung, die beobachtet wird, wenn die Membranen in Gegenwart des Radioliganden allein inkubiert werden. Die durch die Verbindung induzierte Hemmung wird bestimmt durch Inkubieren von Zellmembranen in Gegenwart des Radioliganden und von verschiedenen Konzentrationen der interessierenden Verbindung. Die unspezifische Bindung ist definiert durch die [³H]5-CT-Bindung, die durch Inkubieren der Membranen wie für die Gesamtbindung, aber in Gegenwart von 10 µM Methiothepine erhalten wird. Im Anschluss an eine Inkubation von 75 min bei 30°C wird die Membransuspension durch GF/B-Filter, eingehüllt mit 0,03 % PEI, filtriert, wobei ein Skatron^{R}-Erntesystem verwendet wird. Die in dem Filter zurückgehaltene Radioaktivität wird durch Flüssigszintillationszählung quantifiziert.

### 2. Funktioneller Assay für menschliche 5-HT5A-Rezeptorliganden -Serotonin-induzierte Zunahme der GTP-Europium-Bindung

### Allgemeine Beschreibung:

Eine Stimulierung von G Protein-gekoppelten Rezeptoren durch geeignete Agonisten führt zur Bindung von GTP an die α-Untereinheit von trimeren G-Proteinen, gefolgt von der Dissoziation der GTP-gebundenen α-Untereinheit von den βγ-Untereinheiten und der Aktivierung der Signaltransduktion. Durch Verwenden eines Europium-markierten GTP-Analogons, GTP-Eu, kann die Aktivierung eines G-Protein-gekoppelten Rezeptors durch einen Agonisten als eine Zunahme der Bindung von GTP-Eu an den Rezeptor-G-Protein-Komplex verfolgt werden. Nach dem Entfernen von ungebundenem GTP-Eu kann gebundenes GTP-Eu durch Messen der zeitaufgelösten Fluoreszenzemission in geeigneten Nachweisvorrichtungen quantifiziert werden.

Zellinie: h5HT5A_18.2_SH.sy.5y, eine menschliche Neuroblastomzelllinie, die den menschlichen 5-HT5A-Rezeptor stabil exprimiert.

Membranpräparation: Zellmembranen werden gemäß einer Standardvorschrift in Gegenwart von Proteaseinhibitoren hergestellt und werden durch zwei aufeinanderfolgende Zentrifugationsschritte bei 40000xg teilweise aufgereinigt. Aliquots werden bei -80°C aufbewahrt.

### Assay:

Der Assay wird in Filterplatten mit 96 Vertiefungen (AcroWell-96, Pall Corp.) durchgeführt. Die in Assaypuffer (2,5 µM GDP, 100 mM NaCl, 3 mM MgCl₂, 50 mM HEPES pH 7,4) verdünnten Rezeptormembranen werden zu der Filterplatte zugegeben (5 µg Rezeptormembran/Vertiefung). Testverbindungen werden in 100 % DMSO gelöst und Reihenverdünnungen werden zu den Rezeptormembranen zugegeben (DMSO-Endkonzentration 0,5 %). Die Reaktion wird durch die Zugabe von Serotonin (Endkonzentration 1 µM, gesamtes Assayvolumen 100 µl) gestartet. Nach einem ersten Inkubationszeitraum von 30 min bei 30°C wird GTP-Eu (Endkonzentration 10 nM) zugegeben, gefolgt von einem zweiten Inkubationszeitraum von 30 min bei 30°C. Die Reaktion wird durch schnelle Vakuumfiltration angehalten und die Vertiefungen werden zweimal mit eiskaltem Assaypuffer gewaschen. Gebundenes GTP-Eu wird in einem VICTOR-Multilabel-Counter (PerkinElmerCorp.) unter Verwendung der zeitaufgelösten Europium-Einstellungen gemessen. Die Daten werden bezüglich der unspezifischen Bindung korrigiert und IC50-Werte werden mit PRISM4.0 (GraphPad Inc.) unter Verwendung von standardmäßigen nicht-linearen Kurvenanpassungsalgorithmen berechnet. Kb-Werte werden aus IC50-Werten unter Verwendung der Cheng-Prusoff-Näherung berechnet.

In beiden Assays werden verschiedene Konzentrationen der Testsubstanzen eingesetzt, und die Ki-bzw. die IC50-Werte bestimmt. Die Affinität ausgewählter Verbindungen ist in nachfolgender Tabelle gezeigt:

**Table 1 Affinität ausgewählter Verbindungen an 5-HT5A (Ki)**

| **Beispiel #** | **Bindungsaffinität 5-HT5A (Ki)** |
|---|---|
| 1 | ++ |
| 2 | ++ |
| 3 | ++ |
| 4 | + |
| 5 | + |
| 6 | + |
| 7 | + |
| 8 | ++ |
| 9 | + |
| 10 | ++ |
| 11 | + |
| 12 | ++ |
| 13 | ++ |
| 14 | ++ |
| 15 | ++ |
| 16 | ++ |
| 17 | ++ |
| 18 | ++ |
| 19 | ++ |
| 20 | ++ |
| 21 | + |
| 22 | ++ |
| 23 | ++ |
| 24 | ++ |
| 25 | ++ |
| 26 | ++ |
| 27 | ++ |
| 28 | ++ |
| 29 | ++ |
| 30 | + |
| 31 | ++ |
| 32 | ++ |
| 33 | ++ |
| 34 | ++ |
| 35 | ++ |
| 36 | ++ |
| 37 | ++ |
| 38 | ++ |
| 39 | ++ |
| 40 | ++ |
| 41 | ++ |
| 42 | ++ |
| 43 | ++ |
| 44 | ++ |
| 45 | ++ |
| 46 | ++ |
| 47 | ++ |
| 48 | ++ |
| 49 | ++ |
| 50 | ++ |
| 51 | ++ |
| 52 | ++ |
| 53 | ++ |
| 54 | + |
| 55 | ++ |
| 56 | ++ |
| 57 | ++ |
| 58 | ++ |
| 59 | ++ |
| 60 | ++ |
| 61 | ++ |
| 62 | + |
| 63 | ++ |
| 64 | ++ |

| | |
|---|---|
| + bedeutet eine Affinität > 600 nM ++ bedeutet eine Affinität < 600 nM | |

## Patentansprüche

1. Mindestens eine 5-Ring Heteroaromaten-Verbindung der allgemeinen Formel I 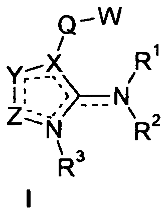 , und/oder entsprechende enantiomere, diastereomere und/ oder tautomere Formen davon und/oder pharmazeutisch annehmbare Salze davon, wobei die angegebenen Reste die folgenden Definitionen besitzen
**W**: einen Rest der allgemeinen Formel **W1** oder **W2** 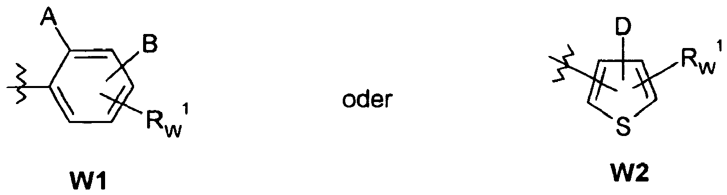 worin
**A**: NO₂, NH₂, CN, CF₃, OCF₃, CHF₂, OCHF₂, COOH, O-CH₂-COOH, Halogen, SH, oder jeweils gegebenenfalls substituiertes O-C₁-C₆-Alkyl, S-C₁-C₆-Alkyl, -O-CO-C₁-C₆-Alkyl, -O-CO-Aryl, -O-CO-Hetaryl, -O-COO-C₁-C₆-Alkyl oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-AlkylenHetaryl oder C₁-C₄-Alkylen-Aryl, oder
jeweils gegebenenfalls substituiertes O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³_{;}
**R_{A}¹:** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl, C₁-C₆-Alkylen-Hetaryl;
**R_{A}²**: Wasserstoff, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-AlkylenHetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³**: jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-AlkylenHetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste **R_{A}²** und **R_{A}³** bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome aus der Gruppe O, N, S enthalten kann; wobei gegebenenfalls zwei an diesem Heterocyclus substituierte Reste zusammen einen 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls 3 bis 7-gliedriger substituierter Cyclus ankondensiert sein kann;
**R_{A}⁴** Wasserstoff, oder
jeweils gegebenenfalls substituiertes C₁-C₆-Alkyl, C₂-C₆-Alkenyl, C₂-C₁₂-Alkinyl, C₃-C₇-Cycloalkyl, Aryl, C₁-C₄-Alkylen-Aryl, C₁-C₆-Alkylen-O-C₁-C₆-Alkyl, Hetaryl;
**B:** Wasserstoff oder unabhängig von Rest **A** wie Rest **A** definiert ist, oder zwei der Reste **A, B** oder **R_{W}¹** bilden jeweils unabhängig voneinander zusammen mit den anhängenden C-Atomen einen 3 bis 7-gliedrigen, jeweils gegebenenfalls substituierten, gesättigten, ungesättigten oder aromatischen Carbocyclus oder einen jeweils gegebenenfalls 3 bis 7-gliedrigen substituierten, gesättigten, ungesättigten oder aromatischen Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann;
**R_{W}¹:** Wasserstoff, OH, Halogen, NO₂, NH₂, CN, CF₃, CHF₂, CH₂F, OCF₃, OCHF₂, OCH₂F oder
jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
gegebenenfalls mit der Maßgabe, dass wenn W = W1, dann R_{w}¹ oder B nicht ein in 4-Position (para-Position) zur Veknüpfungsposition mit Q stehender gegebenenfalls substituierter N-Pyrrolidinyl-Rest sein soll;
**D**: Wasserstoff oder unabhängig von A wie Rest **A** definiert ist;
**Q**: einen Rest der allgemeinen Formel **Q1** 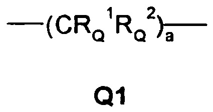 mit dem Index
a=1
**R_{Q}¹, R_{Q}²** unabhängig voneinander :
Wasserstoff, Halogen, OH oder gegebenenfalls substituiertes C₁-C₆-Alkyl;
**R¹, R²** unabhängig voneinander:
Wasserstoff, OH, CN, oder
jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-AlkylenAryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
**R¹** und **R²** bilden zusammen mit dem Stickstoff einen 5 bis 7-gliedrigen, jeweils gegebenenfalls substituierten, gesättigten oder ungesättigten Heterocyclus, der ein, zwei oder drei weitere verschiedene oder gleiche Heteroatome aus der Gruppe O, N, S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen jeweils gegebenenfalls substituierten Carbocyclus oder Heterocyclus bilden können, wobei der Heterocyclus bis zu drei verschiedene oder gleiche Heteroatome ausgewählt aus der Gruppe bestehend aus O, N, und S enthalten kann und wobei der gebildete Cyclus gegebenenfalls substituiert sein kann;
**R³** Freies Elektronenpaar oder Wasserstoff;
**X, Y, Z** jeweils zusammen folgende Bedeutung haben:
X = N, Y = CR⁴, Z = CR⁶, oder
X = C, Y = NR⁵ Z = CR⁶, oder
X = C, Y = CR⁴, Z = NR⁷, oder
X = N, Y = N, Z = CR⁶, oder
X = N, Y = CR, Z = N
**R⁴, R⁶** jeweils unabhängig voneinander einen Rest, ausgewählt aus der gleichen oder verschiedenen der Gruppen **1.), 3.), 4.), 5.)** oder **6**.) und
**R⁵, R⁷** jeweils unabhängig voneinander einen Rest, ausgewählt aus der gleichen oder verschiedenen der Gruppen **2.), 3.), 4.)** oder **5.)**
**1.)** Wasserstoff, NO₂, NH₂, CN, CF₃, CHF₂, OCF₃, OCHF₂, COOH, O-CH₂-COOH, Halogen, oder
jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂NR_{Z,Y}⁶R_{Z,Y}⁷ oder CO-NR_{Z,Y}⁶R_{Z,Y}⁷;
**2.)** Freies Elektronenpaar oder Wasserstoff, CH₂-CF₃, CH₂-CHF₂, jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆,-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
**3.)** Phenyl, 1-Naphthyl oder 2-Naphthyl, die jeweils mit einem, zwei oder drei Resten ausgewählt aus R_{Z,Y}¹, R_{Z,Y}² und **R_{Z,Y}³** substituiert sind oder sein können, wobei
**R_{Z,Y}¹, R_{Z,Y}²** und **R_{Z,Y}³** jeweils unabhängig voneinander einen Substituenten aus der folgenden Gruppe darstellen:
Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₈-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
O-R_{Z,Y}⁴, S-R_{Z,Y}⁴, NR_{Z,Y}⁶R_{Z,Y}⁷, ,CO-OR_{Z,Y}⁵, NR_{Z,Y}⁷₋CO-O-R_{Z,Y}⁵, O-CH₂-COO-R_{7,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}7-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ oder CO-NR_{Z,Y}⁶R_{Z,Y}⁷, oder
jeweils zwei der Reste ausgewählt aus der Gruppe bestehend aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen 3 bis 7-gliedrigen, jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei gegebenenfalls jeweils gegebenenfalls zwei an diesem Carbo- oder Heterocyclus substituierte Reste zusammen einen 3 bis 7-gliedrigen, anellierten, gesättigten, ungesättigten oder aromatischen Carbocyclus oder Heterocyclus bilden können, wobei der Heterocyclus 1, 2, oder bis zu drei verschiedene oder gleiche Heteroatome ausgewählt aus der Gruppe bestehend aus O, N, S enthalten kann und wobei der gebildete Cyclus gegebenenfalls substituiert sein kann und/oder an diesem Cyclus ein weiterer, 3 bis 7-gliedriger, gegebenenfalls substituierter Cyclus ankondensiert sein kann;
**R_{Z,Y}⁴** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, C₁-C₄-Alkylen-Hetaryl, Hetaryl, C₁-C₄-Alkylen-Aryl, Aryl, oder
C₁-C₆-Alkyl, das jeweils gegebenenfalls substituiert ist mit einem Substituenten ausgewählt aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, bedeutet;
**R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, Benzofuranyl, Dihydrobenzofuranyl, Indanyl, oder
jeweils gegebenenfalls 1, 2 oder bis zu 3-fach substituiertes Aryl oder Hetaryl, wobei die Substituenten unabhängig voneinander ausgewählt sind aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, sowie COOH, O-CH₂-COOH, SH, oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder jeweils gegebenenfalls substituiertes O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³, bedeutet;
**R_{Z,Y}⁶** Wasserstoff, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl; bedeutet;
**R_{Z,Y}⁷** Wasserstoff oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl; bedeutet;
oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann, bilden; und jeweils gegebenenfalls zwei an diesem Heterocyclus substituierte Reste zusammen einen 3 bis 7-gliedrigen, anellierten, gesättigten, ungesättigten oder aromatischen Carbocyclus oder Heterocyclus bilden können, wobei der Heterocyclus 1, 2 oder bis zu drei verschiedene oder gleiche Heteroatome ausgewählt aus der Gruppe bestehend aus O, N, und S enthalten kann, und der gebildete Cyclus gegebenenfalls substituiert sein kann oder an diesem Cyclus ein weiterer, gegebenenfalls substituierter 3 bis 7-gliedriger Cyclus ankondensiert sein kann;
**4.)** einen jeweils gegebenenfalls substituierten 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** substituiert sein kann, wobei **R_{Z,Y}¹, R_{Z,Y}²** und **R_{Z,Y}³** unabhängig voneinander die vorstehend unter 3.) genannten Bedeutungen haben ;
**5.)** einen jeweils gegebenenfalls substituierten C₅₋C₁₈ bi- oder tricyclischen, gesättigten Kohlenwasserstoffrest.
**6.) R⁴** und **R⁶** bilden zusammen mit X und Y einen jeweils gegebenenfalls substituierten C₆-C₁₀-gliedrigen gesättigten, ungesättigten oder aromatischen Carbocyclus, bevorzugt einen Benzo-Rest, die jeweils mit einem, zwei oder bis zu drei Resten substituiert sein können, welche jeweils unabhängig voneinander ausgewählt sind aus **R_{Z,Y}⁸.**
**R_{Z,Y}⁸**: Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-AlkylenHetaryl, oder jeweils gegebenenfalls substituierten O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷
, gegebenenfalls mit einer, zwei oder drei der Maßgaben ausgewählt aus Gruppe bestehend aus den nachstehend genannten Maßgaben (i), (ii) und (III):
(i) mit der Maßgabe, dass wenn R⁴ und R⁶ die in der Gruppe 6.) genannten Bedeutungen haben, dann die Reste **A, B, Q, R_{Q}¹** und **R_{Q}²** wie folgt definiert sind:
**A** sitzt in W1 wie angegeben in 2 Position und bedeutet NH₂, OCF₃, OCHF₂, O-CH₂-COOH, O-C₁-C₆-Alkyl, S-C₁-C₆-Alkyl, oder O-R_{A}¹, CO-R_{A}¹, S-R_{A}¹, SO-R_{A}¹, SO₂-R_{A}¹, SO₂-NR_{A}²R_{A}³;
**B** Sitzt in W1 in 6 Position und bedeutet Halogen, CN, CF₃,-CHF₂, OCF₃, OCHF₂, oder
jeweils gegebenenfalls substituiertes C₁-C₆-Alkyl oder C₂-C₆-Alkenyl, -O-CH₂-COO-R_{A}¹, - O-R_{A}¹, S-R_{A}¹, NR_{A}²R_{A}³, -NR_{A}⁴-CO-R_{A}¹ oder-CO-NR_{A}²R_{A}³; NR_{A}⁴-SO₂-R_{A}¹;
oder B bildet zusammen **R_{W}¹** einen der Reste -(CH₂)₃-, -O-CH₂-O-, -O-(CH₂) ₂-O-, -CH=CH-CH=CH-, -O-(CH₂)₂-,-(CH₂)₂-O-;
**Q** -CR_{Q}¹R_{Q}²-
**R_{Q}¹, R_{Q}²** unabhängig voneinander: Wasserstoff, Halogen, gegebenenfalls substituiertes C₁-C₆-Alkyl;
(ii) mit der Maßgabe, dass wenn W = W1, dann R_{w}¹ oder B nicht ein in 4-Position (para-Position) zur Veknüpfungsposition mit Q stehender gegebenenfalls substituierter N-Pyrrolidinyl-Rest sein soll;
(iii) mit der Maßgabe, dass die Summe aus a, b und c 1, 2, 3, 4 oder 5 ist, dann wenn X = N, Y = CR⁴, und Z = CR⁶;

2. Mindestens eine 5-Ring-Heteroaromaten-Verbindung der allgemeinen Formel I nach Anspruch 1, und/oder entsprechende enantiomere, diastereomere und/oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², R₁, R₂, R₃, X, Y, Z,** soweit nachstehend nichts anderslautendes ausgeführt ist die gleiche Bedeutung wie in Anspruch 1 haben und die nachfolgenden Reste folgende Definitionen besitzen:
**R⁴, R⁶** jeweils unabhängig voneinander einen Rest, ausgewählt aus der gleichen oder verschiedenen der Gruppen **1.), 3.), 4.) oder 5.)** und
**R⁵, R⁷** jeweils unabhängig voneinander einen Rest, ausgewählt aus der gleichen oder verschiedenen der Gruppen **2.), 3.) 4.)** oder **5.)**
wobei die Gruppen 1.) - 5.) jeweils die in Anspruch 1 genannten Bedeutungen haben sollen;

3. Mindestens eine 5-Ring-Heteroaromaten-Verbindung der allgemeinen Formel I nach einem der Ansprüche 1 oder 2, und/oder entsprechende enantiomere, diastereomere und/ oder tautomere Formen davon und/oder pharmazeutisch annehmbare Salze davon, wobei die Reste **Q, R_{Q}¹, R_{Q}², R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 oder 2 genannt haben und die nachfolgenden Reste folgende Definition besitzen:
**W:** einen Rest der allgemeinen Formel **W1, W21** oder **W22** 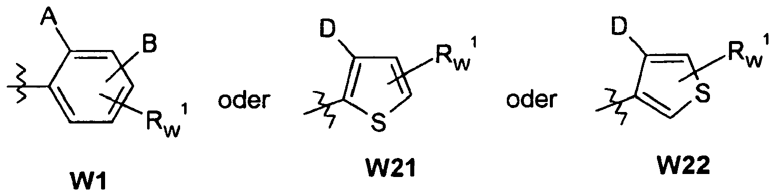 bedeutet, worin die Rest A, B, D und R_{W}¹ unabhängig voneinander und jeweils unabhängig von ihrem jeweiligen Auftreten eine der nachstehend genannten Bedeutungen aufweisen können:
**A**: Halogen, NH₂, CN, CF₃, OCF₃, OCHF₂, COOH, O-CH₂-COOH, SH, O-C₁-C₆-Alkyl, S-C₁-C₆-Alkyl, C₁-C₆-Alkyl oder jeweils gegebenenfalls substituiertes
O-R_{A}¹, CO-R_{A}¹, S-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, SO₂-NR_{A}²R_{A}³ oder CO-NR_{A}²R_{A}³;
**R_{A}¹**: jeweils gegebenenfalls substituiertes C₁-C₆-Alkyl, C₃-C₇-Cycloalkyl, Phenyl oder Benzyl;
**R_{A}² R_{A}³** unabhängig voneinander
Wasserstoff, oder
jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₆-Alkyl, SO₂-Aryl, SO₂-Hetaryl, oder SO₂-C₁-C₄-Alkylen-Aryl;
oder beide Reste **R_{A}²** und **R_{A}³** bilden zusammen mit dem Stickstoff einen jeweils gegebenenfalls substituierten 5-oder 6-gliedrigen gesättigten oder ungesättigten Ring, der ein oder bis zu zwei gleiche oder verschiedene Heteroatome ausgewählt aus der Gruppe bestehend aus O und N enthalten kann.
**R_{A}⁴:** Wasserstoff oder einen gegebenenfalls substituierten C₁-C₆-Alkylrest;
**B:** Wasserstoff, Halogen, CN, CF₃, -CHF₂, OCF₃, OCHF₂, oder jeweils gegebenenfalls substituiertes C₁-C₆-Alkyl oder C₂-C₆-Alkenyl, oder jeweils gegebenenfalls substituierten -O-CH₂₋COO-R_{A}¹, - O-R_{A}¹, S-R_{A}¹, NR_{A}²R_{A}³, -NRA⁴-CO-R_{A}¹ oder-CO-NR_{A}²R_{A}³_{;} NR_{A}⁴-SO₂-R_{A}¹;
oder B bildet zusammen **R_{W}¹** einen der Reste -(CH₂)₃-, -O-CH₂-O-, - O-(CH₂)₂-O-, -CH=CH-CH=CH-, -O-(CH₂)₂-, -(CH₂)₂-O-;
**R_{W}¹:** Wasserstoff, F, Cl, CN, CF₃, O-CF₃, oder
jeweils gegebenenfalls substituiertes C₁-C₆-Alkyl, Aryl, C₁-C₆-Alkylamino oder C₁-C₆-Dialkylamino;
**D**: Wasserstoff oder unabhängig von A wie Rest **A** definiert ist;

4. Mindestens eine 5-Ring-Heteroaromaten-Verbindung der allgemeinen Formel I nach einem der Ansprüche 1 bis 3, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a R_{Q}¹, R_{Q}², X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 bis 3 genannt haben und die nachfolgenden Reste folgende Definition besitzen:
**R¹, R²**, unabhängig voneinander:
Wasserstoff, OH, CN, C₁-C₆-Alkyl, C₁-C₆-Alkylen-O-C₁-C₆-Alkyl, substituiertes Aryl, Benzyl, CO-C₁-C₆-Alkyl, CO-Aryl, oder CO-C₁-C₄-Alkylen-Aryl;
**R³** Freies Elektronenpaar oder Wasserstoff.

5. Mindestens eine 5-Ring-Heteroaromaten-Verbindung der allgemeinen Formel I nach einem der Ansprüche 1 bis 4, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z},_{Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 bis 4 genannt haben und die nachfolgenden Reste folgende Definition besitzen:
**X, Y, Z** X = N, Y = CR⁴, Z = CR⁶, oder
X = C, Y = CR⁴, Z = NR⁷, oder
X = N, Y = N, _{Z} = CR⁶

6. Mindestens eine 5-Ring-Heteroaromaten-Verbindung der allgemeinen Formel I nach einem der Ansprüche 1 bis 5, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹ R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷_{,} R_{Z,Y}⁸** soweit nachstehend nichts anderslautendes ausgeführt ist die gleiche Bedeutung wie in einem der Ansprüche 1 bis 5 genannt haben und die nachfolgenden Reste folgende Definition besitzen:
**X, Y, Z** X = N, Y = CR⁴, Z = CR⁶, oder
X = N, Y = N, Z = CR⁶

7. Mindestens eine 5-Ring-Heteroaromaten-Verbindung der allgemeinen Formel I nach einem der Ansprüche 1 bis 6, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², R₁, R₂, R₃, X, Y, Z,** soweit nachstehend nichts anderslautendes ausgeführt ist die gleiche Bedeutung wie in einem der Ansprüche 1 bis 6 genannt haben und die nachfolgenden Reste folgende Definition besitzen:
**R⁴, R⁶** jeweils unabhängig voneinander einen Rest, ausgewählt aus der gleichen oder verschiedenen der Gruppen **1.), 3.), 4.)** oder **5.)** und
**R⁵, R⁷** jeweils unabhängig voneinander einen Rest, ausgewählt aus der gleichen oder verschiedenen der Gruppen **2.), 3.)** oder **4.)** oder **5.)**
wobei die Gruppen 1.), 2.), 3.), 4.) und 5.) wie folgt definiert sind:
1.) Wasserstoff, CN, CF₃, CHF₂, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cydoalkyl, C₁-C₄-Alkylen-Aryl, C₁-C₄-Alkylen-Hetaryl, oder jeweils gegebenenfalls substituiertes
O-R_{Z,Y}⁴, NR_{Z,Y}⁶R_{Z,Y}⁷, CO-OR_{Z,Y}⁵, NR_{Z,Y}⁷ -CO-O-R_{Z,Y}⁵, O-CH₂-COO-R_{Z,Y}⁵, NR_{Z,Y}⁷-CO-R_{Z,Y}⁵, SO₂R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
2.) Freies Elektronenpaar oder Wasserstoff,
jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₆-Alkyl oder jeweils gegebenenfalls substituiertes SO₂-R_{Z,Y}⁵, SO₂NH₂, CONH₂, SO₂-NR_{Z,Y}⁶R_{Z,Y}^{z} oder CO-NR_{Z,Y}⁶R_{Z,Y}⁷;
3.) Phenyl, 1-Naphthyl oder 2-Naphthyl, die jeweils mit **R_{Z,Y}¹, R_{Z,Y}²** und **R_{Z,Y}³** substituiert sind, wobei
**R_{Z,Y}³, R_{Z,Y}²** und **R_{Z,Y}³** jeweils unabhängig voneinander einen Substituenten aus der folgenden Gruppe darstellen:
Wasserstoff, NH₂, CN, CF₃, CHF₂, OCF₃, OCHF₂, O-CH₂-COOH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, **SO₂-R**_{Z,Y}**⁵,** NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}³R_{Z,Y}⁷, oder
jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
**R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, C₁-C₄-Alkylen-Aryl, oder
C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
**R_{Z,Y}⁵** jeweils gegebenenfalls substituiertes C₁-C₈-Alkyl, C₂-C₆-Alkenyl, C₂-C₆-Alkinyl, C₃-C₇-Cycloalkyl, C₁-C₄-Alkylen-C₃-C₇ Cycloalkyl, C₁-C₄-Alkylen-Heterocycloalkyl, Heterocycloalkyl oder C₁-Cₐ-Alkylen-O-C₁-C₆-Alkyl, Dioxymethylenphenyl, oder jeweils gegebenenfalls 1-, 2- oder bis zu 3-fach substituiertes Aryl oder Hetaryl, wobei die Substituenten unabhängig voneinander ausgewählt sind aus der Gruppe bestehend aus Halogen, NO₂, NH₂, OH, CN, CF₃, CHF₂, OCF₃, OCHF₂, NH-(C₁-C₆-Alkyl) oder N(C₁-C₆-Alkyl)₂, COOH, O-CH₂-COOH, SH, oder
jeweils gegebenenfalls substituiertes C₁-C₆-Alkyl, C₃-C₇-Cycloalkyl, Aryl, Hetaryl, Heterocycloalkyl, oder jeweils gegebenenfalls substituiertes
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
**R_{Z,Y}⁶** Wasserstoff, oder
jeweils gegebenenfalls substituiertes C₁-C₆-Alkyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₆-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, SO₂-C₁-C₈-Alkyl, SO₂-Aryl, SO₂-Hetaryl, SO₂-C₁-C₄-Alkylen-Aryl oder SO₂-C₁-C₄-AlkylenHetaryl;
**R_{Z,Y}⁷** Wasserstoff oder gegebenenfalls substituiertes C₁-C₆-Alkyl;
oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
**4.)** einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** substituiert sein kann;
**5.)** einen gegebenenfalls substituierten C₅-C₁₀- bi- oder tricyclischen, gesättigten Kohlenwasserstoffrest.

8. Mindestens eine 5-Ring-Heteroaromaten-Verbindung der allgemeinen Formel I nach einem der Ansprüche 1 bis 7, und/oder entsprechende enantiomere, diastereomere und/ oder tautomere Formen davon und/oder pharmazeutisch annehmbare Salze davon, wobei die Reste **Q, a, R_{Q}¹ R_{Q}², R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 bis 7 genannt haben und die nachfolgenden Reste folgende Definition besitzen:
**W:** einen Rest der allgemeinen Formel 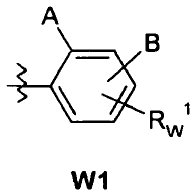
**A:** F, Cl, OCF₃, OCHF₂, jeweils gegebenenfalls substituiertes C₁-C₆-Alkyl, O-C₁-C₆-Alkyl, S-C₁-C₈-Alkyl oder OR_{A}¹;
**B:** Wasserstoff, F, Cl, CF₃, OCF₃, OCHF₂, jeweils gegebenenfalls substituiertes C₁-C₆-Alkyl, O-C₁-C₆-Alkyl oder S-C₁-C₆-Alkyl;
**Rw¹:** Wasserstoff, F, Cl, CN, CF₃ oder O-CF₃;
**R_{A}¹:** jeweils gegebenenfalls substituiertes C₁-C₆-Alkyl oder Phenyl.

9. Mindestens eine 5-Ring-Heteroaromaten-Verbindung der allgemeinen Formel I nach einem der Ansprüche 1 bis 8, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, R₁, R₂, R₃, X, Y, Z, R₄, R₅, R₆, R₇, R_{Z,Y}¹, R_{Z,Y}¹, R_{Z,Y}³, R_{Z,Y}⁴_{,} R_{Z,Y}⁴, R_{Z,Y}⁵, R_{Z,Y}⁷, R_{Z,Y}⁸** soweit nachstehend nichts anderslautendes ausgeführt ist die gleiche Bedeutung wie in einem der Ansprüche 1 bis 8 genannt haben und die nachfolgenden Reste folgende Definition besitzen:
**Q:** -CR_{Q}¹R_{Q}²-;
**R_{Q}^{'1} R_{Q}²** jeweils unabhängig voneinander Wasserstoff, F, oder CH₃.

10. Mindestens eine 5-Ring-Heteroaromaten-Verbindung der allgemeinen Formel I nach einem der Ansprüche 1 bis 9, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁵, R_{Z,Y}⁷, R_{Z,Y}⁸** soweit nachstehend nichts anderslautendes ausgeführt ist die gleiche Bedeutung wie in einem der Ansprüche 1 bis 9 genannt haben und die nachfolgenden Reste folgende Definition besitzen:
**R¹ R²** unabhängig voneinander:
Wasserstoff, OH, CN, O-Methyl, O-Phenyl, Acetyl, Benzoyl, O-Acetyl, oder O-Benzoyl;
**R³** Freies Elektronenpaar oder Wasserstoff

11. Mindestens eine 5-Ring-Heteroaromaten-Verbindung der allgemeinen Formel I nach einem der Ansprüche 1 bis 10, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, Rw¹, D, Q, a, R_{Q}¹, R_{Q}², X, Y, Z, R₄, R_{5,} R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 bis 10 genannt haben und die nachfolgenden Reste die folgende Definition besitzen:
**R¹, R²** Wasserstoff
**R³** Freies Elektronenpaar oder Wasserstoff.

12. **Mindestens** eine 5-Ring-Heteroaromaten-Verbindung der allgemeinen Formel I nach einem der Ansprüche 1 bis 11, entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z},_{Y}⁵, R_{Z,Y}⁶, R_{Z,Y}⁷, R_{Z,Y}⁸** soweit nachstehend nichts anderslautendes ausgeführt ist die gleiche Bedeutung wie in einem der Ansprüche 1 bis 11 genannt haben und die nachfolgenden Reste die folgende Definition besitzen:
X,Y,Z X=N,Y=CR⁴,Z=CR⁶

13. Mindestens eine 5-Ring-Heteroaromaten-Verbindung der allgemeinen Formel I nach einem der Ansprüche 1 bis 12, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹ D, Q, a, R_{Q}¹, R_{Q}², R₁, R₂, R₃, X, Y, Z** soweit nachstehend nichts anderslautendes ausgeführt ist die gleiche Bedeutung wie in einem der Ansprüche 1 bis 12 genannt haben und die nachfolgenden Reste folgende Definition besitzen:
**R⁴, R⁶** jeweils unabhängig voneinander einen Rest, ausgewählt aus der gleichen oder verschiedenen der Gruppen **1.), 3.), 4.)** oder 5.) und
**R⁵, R⁷** einen Rest, ausgewählt aus der gleichen oder verschiedenen der Gruppen **2.) oder 3.),** wobei die Gruppen 1.), 2.), 3.), 4.) und 5.) wie folgt definiert sind:
**1.)** Wasserstoff, CF₃, CHF₂, oder
jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, oder jeweils gegebenenfalls substituiertes NR_{Z,Y}⁷-CO-O-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶,R_{Z,Y}⁷;
**2.)** Freies Elektronenpaar oder Wasserstoff, oder
jeweils gegebenenfalls substituiertes C₁-C₁₀-Alkyl, C₃-C₇-Cycloalkyl;
3.) Phenyl oder Naphthyl, die jeweils mit **R_{Z,Y}¹, R_{Z,Y}²** und **R_{Z,Y}³** substituiert sind, wobei
**R_{Z,Y}¹, R_{Z,Y}²** und **R_{Z,Y}³** jeweils unabhängig voneinander einen Substituenten aus der folgenden Gruppe darstellen:
Wasserstoff, CN, NH₂, CF₃, CHF₂, OCF₃, OCHF₂, O-CH₂-COOH, Halogen, oder
jeweils gegebenenfalls substituiertes Aryl, Hetaryl, Heterocycloalkyl, C₁-C₆-Alkyl, C₃-C₇-Cycloalkyl oder jeweils gegebenenfalls substituiertes
O-R_{Z,Y}⁴, NR_{Z,Y}⁶R_{Z,Y}⁷, SO₂-R_{Z,Y}⁶R_{Z,Y}⁷, NR_{Z,Y}⁷-SO₂-R_{Z,Y}⁵ oder
jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend O, N, und S enthalten kann; wobei
**R_{Z,Y}⁴** jeweils gegebenenfalls substituiertes Hetaryl, Aryl,
oder
C₁-C₆-Alkyl, das gegebenenfalls substituiert ist mit einem Substituenten ausgewählt aus der Gruppe, bestehend aus Halogen, CN, CF₃, CHF₂, OCF₃, OCHF₂, gegebenenfalls substituiertes NH-(C₁-C₆-Alkyl) und gegebenenfalls substituiertes N(C₁-C₆-Alkyl)₂;
**R_{Z,Y}⁵** gegebenenfalls substituiertes C₁-C₆-Alkyl, oder
jeweils gegebenenfalls 1-, 2- oder bis zu 3-fach substituiertes Aryl oder Hetaryl, wobei die Substituenten unabhängig voneinander ausgewählt sind aus der Gruppe bestehend aus Halogen, NH₂, CN, CF₃, CHF₂, OCF₃, OCHF₂, NH-(C₁-C₆-Alkyl) oder N(C₁-C₆-Alkyl)₂, oder
gegebenenfalls substituiertes C₁-C₆-Alkyl, oder jeweils gegebenenfalls substituiertes
O-R_{A}¹ NR_{A}²R_{A}³, SO₂NH₂, SO₂-R_{A}¹, NR_{A}⁴-SO₂-R_{A}¹, oder SO₂-NR_{A}²R_{A}³.
**R_{Z,Y}⁶** Wasserstoff, oder
jeweils gegebenenfalls substituiertes C₁-C₆-Alkyl, Aryl, C₁-C₄-Alkylen-Aryl, C₁-C₄-Alkylen-Hetaryl, SO₂-C₁-C₆-Alkyl, SO₂-Aryl, SO₂-Hetaryl oder SO₂-C₁-C₄-Alkylen-Aryl;
**R_{Z,Y}⁷** Wasserstoff oder gegebenenfalls substituiertes C₁-C₈-Alkyl;
oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, **N**, und S enthalten kann;
**4.)** einen 5- oder 6-gliedrigen aromatischen Heterocyclus der ein, zwei oder drei verschiedene oder gleiche Heteroatome ausgewählt aus der Gruppe bestehend aus O, **N**, und S enthalten kann und der jeweils mit **R_{Z,Y}¹, R_{Z,Y}²** und **R_{Z,Y}³** substituiert sein kann;
**5.)** gegebenenfalls substituiertes Adamantyl;
bedeuten.

14. Mindestens eine 5-Ring-Heteroaromaten-Verbindung der allgemeinen Formel I nach einem der Ansprüche 1 bis 13, und/oder entsprechende enantiomere, diastereomere und/ oder tautomere Formen davon und/oder pharmazeutisch annehmbare Salze davon, wobei die Reste **R_{Q}¹, R_{Q}², R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** soweit nachstehend nichts anderslautendes ausgeführt ist die nachfolgenden Definitionen besitzen:
W: einen Rest der allgemeinen Formel 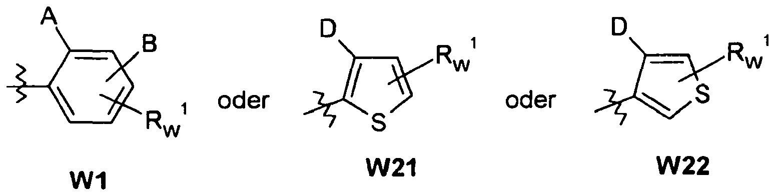
**A:** NH₂, OCF₃, OCHF₂, COOH, O-CH₂-COOH, SH, oder jeweils gegebenenfalls substituiertes O-C₁-C₆-Alkyl, S-C₁-C₆-Alkyl, C₁-C₆-Alkyl, oder jeweils gegebenenfalls substituiertes
O-R_{A}¹, CO-R_{A}¹, S-R_{A}¹, O-CH₂-COO-R_{A}¹, NR_{A}²R_{A}³, CONH₂, SO₂NH₂, SO2-R_{A}¹, NR_{A}⁴-SO₂-R_{A}¹, SO₂-NR_{A}²R_{A}³ oder CO-NR_{A}²R_{A}³; wobei
**R_{A}¹:** jeweils gegebenenfalls substituiertes C₁-C₆-Alkyl, C₃-C₇-cloalkyl, Phenyl oder Benzyl;
**R_{A}²; R_{A}³** unabhängig voneinander
Wasserstoff, oder
jeweils gegebenenfalls substituiertes C₁-C₆-Alkyl, Phenyl, Benzyl, Phenethyl, CO-C₁-C₆-Alkyl, CO-Aryl, CO-O-C₁-C₆-Alkyl, SO₂-C₁-C₄-Alkyl, SO₂-Aryl, SO₂-Hetaryl, oder SO₂-C₁-C₄-Alkylen-Aryl;
oder beide Reste **R_{A}²** und **R_{A}³** bilden zusammen mit dem Stickstoff einen jeweils gegebenenfalls substituierten 5-oder 6-gliedrigen gesättigten oder ungesättigten Ring, der ein oder bis zu zwei gleiche oder verschiedene Heteroatome ausgewählt aus der Gruppe bestehend aus O und N enthalten kann;
**R_{A}⁴:** Wasserstoff oder einen gegebenenfalls substituierten C₁-C₆-Alkylrest;
**B:** Wasserstoff, Halogen, CN, CF₃, -CHF₂, OCF₃, OCHF₂, oder jeweils gegebenenfalls substituiertes C₁-C₆-Alkyl oder C₂-C₆-Alkenyl, oder jeweils gegebenenfalls substituiertes -O-CH₂₋COO-R_{A}¹, - O-R_{A}¹, S-R_{A}¹, NR_{A}²R_{A}³, -NR_{A}⁴-CO-R_{A}¹oder-CO-NR_{A}²R_{A}³; NR_{A}⁴-SO₂-R_{A}¹;
oder B bildet zusammen mit **R_{W}¹** einen der Reste -(CH₂)₃-, -O-CH₂-O- -O-(CH₂) ₂-O-, -CH=CH-CH=CH-, -O-(CH₂)₂-, -(CH₂)₂-O-;
**R_{W}¹:** Wasserstoff, F, Cl, CN, CF₃, O-CF₃, oder
jeweils gegebenenfalls substituiertes C₁-C₆-Alkyl, Aryl, C₁-C₆-Alkylamino oder C₁-C₆-Dialkylamino;
**Q:**
**R_{Q}¹ R_{Q}²** unabhängig voneinander:
Wasserstoff, Halogen, OH, gegebenenfalls substituiertes C₁-C₆-Alkyl;
**R¹ R²,** unabhängig voneinander:
Wasserstoff, OH, CN, oder jeweils gegebenenfalls substituiertes C₁-C₆-Alkyl, C₁-C₆-Alkylen-O-C₁-C₆-Alkyl, substituiertes Aryl, Benzyl, CO-C₁-C₆-Alkyl, CO-Aryl, oder CO-C₁-C₄-Alkylen-Aryl;
**R³** Freies Elektronenpaar oder Wasserstoff;
**X, Y, Z** X = N, Y = CR⁴, Z = CR⁶ oder
X = C, Y = CR⁴, Z = NR⁷, oder
X = N, Y = N, Z = CR⁶
**R⁴, R⁶** jeweils unabhängig voneinander einen Rest, ausgewählt aus der gleichen oder verschiedenen der Gruppen **1.), 3.), 4.)** oder **5.)** und
**R⁵, R⁷** jeweils unabhängig voneinander einen Rest, ausgewählt aus der gleichen oder verschiedenen der Gruppen **2.), 3.)** oder **4.)** oder **5.),** mit den folgenden Bedeutungen für die Gruppen 1.) bis 5.):
**1.)** Wasserstoff, CN, CF₃, CHF₂, O-CH₂-COOH, Halogen, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, oder C₁-C₄-Alkylen-Hetaryl, oder
jeweils gegebenenfalls substituiertes O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
**2.)** Freies Elektronenpaar oder Wasserstoff,
jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₆-Alkyl oder
SO₂-R_{Z,Y}⁵, SO₂NH₂, CONH₂, oder jeweils gegebenenfalls substituiertes SO₂-NR_{Z,Y}⁶,R_{Z,Y}⁷ oder CO-NR_{Z,Y}⁶R_{Z,Y}⁷;
**3.)** Phenyl, 1-Naphthyl oder 2-Naphthyl, die jeweils mit **R_{Z,Y}¹, R_{Z,Y}²** und **R_{Z,Y}³** substituiert sind oder sein können, wobei
**R_{Z,Y}¹, R_{Z,Y}²** und **R_{Z,Y}³** jeweils unabhängig voneinander einen Substituenten ausgewählt aus der folgenden Gruppe darstellen:
Wasserstoff, NH₂, CN, CF₃, CHF₂, OCF₃, OCHF₂, O-CH₂-COOH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
O-R_{Z,Y}⁴, S-R_{Z,Y}⁴, NR_{Z,Y}⁶R_{Z,Y}⁷, NR_{Z,Y}⁷,-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶,R_{Z,Y}⁷, CO₂NH₂, oder CO-NR_{Z,Y}⁶R_{Z,Y}⁷, oder
jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder
ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
**R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, C₁-C₄-Alkylen-Aryl, oder
C₁-C₆-Alkyl, das gegebenenfalls substituiert ist mit einem Substituenten ausgewählt aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) und gegebenenfalls substituiertes N(C₁-C₆-Alkyl)₂;
**R_{Z,Y}⁵,** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, oder jeweils gegebenenfalls 1-, 2- oder bis zu 3-fach substituiertes Aryl oder Hetaryl, wobei die Substituenten unabhängig voneinander ausgewählt sind aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl₂, COOH, O-CH₂-COOH, SH, oder jeweils gegebenenfalls substituiertes C₁-C₆-Alkyl, C₃-C₇-Cycloalkyl, Aryl, Hetaryl, Heterocycloalkyl, oder
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
**R_{Z,Y}⁶** Wasserstoff, oder
jeweils gegebenenfalls substituiertes C₁-C₆-Alkyl, C₃-C₇ Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, S02-C₁-C₆-Alkyl, SO₂-Aryl, SO₂-Hetaryl, SO₂-C₁-C₄-Alkylen-Aryl oder SO₂-C₁-C₄-AlkylenHetaryl;
**R_{Z,Y}⁷** Wasserstoff oder gegebenenfalls substituiertes C₁-C₆-Alkyl;
oder die Reste R_{Z,Y}⁶ und R_{Z,Y}⁷bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
**4.)** einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** wie vorstehend definiert substituiert sein kann;
**5.)** einen gegebenenfalls substituierten C₅-C₁₀- bi- oder tricyclischen, gesättigten Kohlenwasserstoffrest;
bedeuten.

15. Mindestens eine 5-Ring-Heteroaromaten-Verbindung der allgemeinen Formel I nach einem der Ansprüche 1 bis 14, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}² R₁, R₂, R₃, X, Y, Z** soweit nachstehend nichts anderslautendes ausgeführt ist die nachfolgenden Definitionen besitzen:
W: einen Rest der allgemeinen Formel 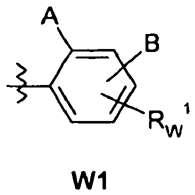
**A:** F, Cl, OCF₃, OCHF₂, jeweils gegebenenfalls substituiertes C₁-C₆-Alkyl, O-C₁-C₆-Alkyl oder S-C₁-C₆-Alkyl oder gegebenenfalls substituiertes OR_{A}¹;
**R_{A}¹:** jeweils gegebenenfalls substituiertes C₁-C₆-Alkyl oder Phenyl
**B:** Wasserstoff, F, Cl, CF₃, OCF₃, OCHF₂, oder jeweils gegebenenfalls substituiertes C₁-C₆-Alkyl, O-C₁-C₆-Alkyl oder S-C₁-C₆-Alkyl;
**R_{W}¹:** Wasserstoff, F, Cl, CN, CF₃ oder O-CF₃;
**Q:** -CR_{Q}¹R_{Q}²-;
**R_{Q}¹,** R_{Q}² jeweils unabhängig voneinander Wasserstoff, F, CH₃;
**R¹, R²** unabhängig voneinander:
Wasserstoff, OH, CN, O-Methyl, O-Phenyl, Acetyl, Benzoyl, O-Acetyl, O-Benzoyl;
**R³** Freies Elektronenpaar oder Wasserstoff
**X, Y, Z** X=N, Y = CR⁴ Z = CR⁶ oder
X = N, Y = N, Z = CR⁶
**R⁴, R⁶** jeweils unabhängig voneinander einen Rest, ausgewählt aus der gleichen oder verschiedenen der Gruppen **1.), 3.), 4.)** oder **5.)** und
**R⁶** einen Rest, ausgewählt aus der gleichen oder verschiedenen
der Gruppen **2.)** oder **3.),** wobei die Gruppen 1.) bis 5.) bedeuten:
**1.)** Wasserstoff, CN, CF₃, CHF₂, O-CH₂-COOH, Halogen, oder jeweils gegebenenfalls substituiertes C₁-Cₗ₁₀-Alkyl, C₂-C₁₀-Alkenyl, C₂-C₁₀-Alkinyl, C₃-C₇-Cycloalkyl, C₁-C₆-Alkylen-C₃-C₇-Cydoalkyl, C₁-C₄-Alkylen-Aryl, C₁-C₄-Alkylen-Hetaryl, oder jeweils gegebenenfalls substituiertes
O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
**2.)** Freies Elektronenpaar oder Wasserstoff,
jeweils gegebenenfalls substituiertes C₁-C₁₀-Alkyl, C₃-C₇-Cycloalkyl, C₁-C₆-Alkylen-C₃-C₇-Cycloalkyl, C₁-C₄-Alkylen-Aryl, C₁-C₄-AlkylenHetaryl, COO-C₁-C₄-Alkyl oder
SO₂-R_{Z,Y}⁵, SO₂NH₂, CONH₂, gegebenenfalls substituiertes SO₂-NR_{Z,Y}⁶R_{Z,Y}⁷ oder gegebenenfalls substituiertes CO-NR_{Z,Y}⁶R_{Z,Y}⁷;
**3.)** Phenyl, 1-Naphthyl oder 2-Naphthyl, die jeweils mit **R_{Z,Y}¹, R_{Z,Y}²** und **R_{Z,Y}³** substituiert sein können, wobei
**R_{Z,Y}¹, R_{Z,Y}²** und **R_{Z,Y}³** jeweils unabhängig voneinander einen Substituenten aus der folgenden Gruppe darstellen:
Wasserstoff, NH₂, CN, CF₃, CHF₂, OCF₃, OCHF₂, O-CH₂-COOH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
O-R_{Z,Y}⁴, S-R_{Z,Y}⁴, NR_{Z,Y}⁶R_{Z,Y}⁷ NR_{Z,Y}⁷-CO-O-R_{Z,Y}⁵, NR_{Z,Y}⁷-CO-R_{Z,Y}⁵, **SO₂-R**_{Z,Y}**⁵,** NR_{Z,Y}⁷-SO₂-R_{Z,Y}⁵, SO₂NH₂, SO₂-NR_{Z,Y}⁶R_{Z,Y}⁷ oder CO-NR_{Z,Y}⁶R_{Z,Y}⁷, oder
jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann;
**R_{Z,Y}⁴** jeweils gegebenenfalls substituiertes C₁-C₄-Alkylen-C₃-C₇-Cycloalkyl, C₁-C₄-Alkylen-Heterocycloalkyl, Heterocycloalkyl, Hetaryl, Aryl, C₁-C₄-Alkylen-Hetaryl, Hateryl, C₁-C₄-Alkylen-Aryl, oder
C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) und gegebenenfalls substituiertes N(C₁-Ce-Alkyl)₂;
**R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, oder jeweils gegebenenfalls 1-, 2- oder bis zu 3-fach substituiertes Aryl oder Hetaryl, wobei die Substituenten unabhängig voneinander ausgewählt sind aus der Gruppe bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, COOH, O-CH₂-COOH, SH, oder jeweils gegebenenfalls substituiertes C₁-C₆-Alkyl, C₃-C₇-Cycloalkyl, Aryl, Hetaryl, Heterocycloalkyl, oder jeweils gegebenenfalls substituiertes
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, oder SO₂-NR_{A}²R_{A}³;
**R_{Z,Y}⁶** Wasserstoff, oder
jeweils gegebenenfalls substituiertes C₁-C₆-Alkyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-AlkylenHetaryl;
**R_{Z,Y}⁷** Wasserstoff oder gegebenenfalls substituiertes C₁-C₆-Alkyl;
oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann;
**4.)** einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹, R_{Z,Y}²** und **R_{Z,Y}³** wie vorstehend definiert substituiert sein kann;
**5.)** gegebenenfalls substituiertes Adamantyl.

16. Mindestens eine 5-Ring-Heteroaromaten-Verbindung der allgemeinen Formel I nach einem der Ansprüche 1 bis 15, und/oder entsprechende enantiomere, diastereomere und/ oder tautomere Formen davon und/oder pharmazeutisch annehmbare Salze davon, wobei die Reste **W, A, R_{A}¹, R_{A}², R_{A}³, R_{A}⁴, B, R_{W}¹, D, Q, a, R_{Q}¹, R_{Q}², R₁, R₂, R₃, X, Y, Z** soweit nachstehend nichts anderslautendes ausgeführt ist die nachfolgenden Definitionen besitzen:
**W:** einen Rest der allgemeinen Formel 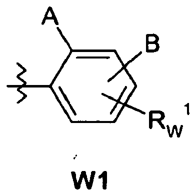
**A:** OCF₃, OCHF₂, OCH₃, Methyl, O-Ethyl, O-Propyl oder O-iPropyl;
**B:** Wasserstoff, F, Cl, CF₃, OCF₃, OCHF₂, gegebenenfalls substituiertes C₁-C₆-Alkyl oder gegebenenfalls substituiertes O-C₁-C₈-Alkyl;
**R_{W}¹:** Wasserstoff, F, Cl;
**Q:** -CH₂-;
**R¹, R²** Wasserstoff,
**R³** Freies Elektronenpaar oder Wasserstoff
**X** N
**Y** CR⁴
**Z** CR⁶
**R⁴, R⁶** jeweils unabhängig voneinander einen Rest, ausgewählt aus der gleichen oder verschiedenen der Gruppen **1.), 2.)** oder **3.),** wobei die Gruppen 1.) bis 3.) bedeuten:
**1.)** Wasserstoff, CF₃, CHF₂, oder
jeweils gegebenenfalls substituiertes C₁-C₁₀-Alkyl, oder
2.) Phenyl, 1-Naphthyl oder 2-Naphthyl, die jeweils mit **R_{Z,Y}¹, R_{Z,Y}²** und **R_{Z,Y}³** substituiert sind, wobei
R_{Z,Y}¹, **R_{Z,Y}²** und R_{Z,Y}³ jeweils unabhängig voneinander einen Substituenten aus der folgenden Gruppe darstellen:
Wasserstoff, CN, CF₃, CHF₂, OCF₃, OCHF₂, O-CH₂-COOH, Halogen, oder
jeweils gegebenenfalls substituiertes C₁-C₆-Alkyl, Aryl, oder jeweils gegebenenfalls substituiertes O-R_{Z,Y}⁴, NR_{Z,Y}⁶R_{Z,Y}⁷ ,NR_{Z,Y}⁷-SO₂-R_{Z,Y}⁵ oder
jeweils zwei der Reste aus **R_{Z,Y}¹, R_{Z,Y}²** oder **R_{Z,Y}³** bilden zusammen einen der Reste
-O-CH₂-O-, -O-CH₂-CH₂-O-, -O-CH₂-CH₂- oder -CH₂-CH₂-CH₂-;
R_{Z,Y}⁴ jeweils gegebenenfalls substituiertes Hetaryl, Aryl,
oder C₁-C₆-Alkyl;
R_{Z,Y}⁵ gegebenenfalls substituiertes C₁-C₆-Alkyl, oder
jeweils gegebenenfalls 1-, 2- oder bis zu 3-fach substituiertes Aryl oder Hetaryl, wobei die Substituenten unabhängig voneinander ausgewählt sind aus der Gruppe bestehend aus Halogen, NH₂, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, oder
gegebenenfalls substituiertes C₁-C₆-Alkyl, oder
jeweils gegebenenfalls substituiertes O-R_{A}¹, NR_{A}²R_{A}³, SO₂NH₂, SO₂-R_{A}¹, NR_{A}⁴-SO₂-R_{A}¹, oder SO₂-NR_{A}²R_{A}³;
R_{Z,Y}⁶ Wasserstoff, oder
jeweils gegebenenfalls substituiertes C₁-C₆-Alkyl, Aryl, SO₂-C₁-C₆-Alkyl, SO₂-Aryl;
**R_{Z,Y}⁷** Wasserstoff oder gegebenenfalls substituiertes C₁-C₆-Alkyl;
**3.)** Benzothiophenyl, Benzofuranyl, Chinolinyl, Isochinolinyl, Thienyl, Furanyl, Pyridinyl, Pyrimidinyl, oder Thiazolyl die jeweils mit R_{Z,Y}¹, **R_{Z,Y}²** und R_{Z,Y}³ substituiert sein können.

17. Mindestens eine 5-Ring-Heteroaromaten-Verbindung der allgemeinen Formel I nach einem der Ansprüche 1 bis 16, und/oder entsprechende enantiomere, diastereomere und/ oder tautomere Formen davon und/oder pharmazeutisch annehmbare Salze davon, wobei die Reste R¹, R², R³, Q, W, X, Y und Z die in einem der Ansprüche 1 bis 16 genannten Bedeutungen haben sollen, zur Verwendung als Arzneimittel.

18. Pharmazeutische Zusammensetzung, enthaltend mindestens eine 5-Ring-Heteroaromaten-Verbindung der allgemeinen Formel I nach einem der Ansprüche 1 bis 16, und/oder entsprechende enantiomere, diastereomere und/ oder tautomere Formen davon und/oder pharmazeutisch annehmbare Salze davon, wobei die Reste R¹, R², R³, Q, W, X, Y und Z die in einem der Ansprüche 1 bis 16 genannten Bedeutungen haben sollen, sowie gegebenenfalls mindestens einen pharmazeutisch annehmbaren Träger und/oder Verdünnungsmittel.

19. Verwendung von mindestens einer 5-Ring-Heteroaromaten-Verbindung der allgemeinen Formel **I** 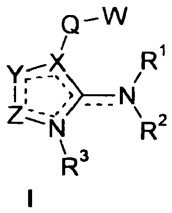 , und/oder entsprechende enantiomere, diastereomere und/ oder tautomere Formen davon und/oder pharmazeutisch annehmbare Salze davon, wobei die Reste R¹, R², R³, Q, W, X, Y und Z wie folgt definiert sind:
**W:** einen Rest der allgemeinen Formel **W11** oder **W2** 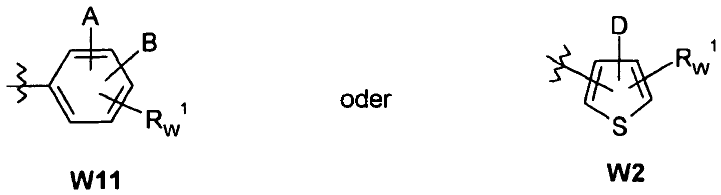
worin
**A:** NO₂, NH₂, CN, CF₃, OCF₃, CHF₂, OCHF₂, COOH, O-CH₂-COOH, Halogen, SH, oder jeweils gegebenenfalls substituiertes O-C₁-C₆-Alkyl, S-C₁-C₆-Alkyl, -O-CO-C₁-C₆-Alkyl, -O-CO-Aryl, -O-CO-Hetaryl, -O-COO-C₁-C₆-Alkyl oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-AlkylenHetaryl oder C₁-C₄-Alkylen-Aryl, oder jeweils gegebenenfalls substituiertes
O-R_{A}¹, CO-R_{A}¹, S-R_{A}¹, SO-R_{A}¹, CO-O-R_{A}¹, NRA⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
**R_{A}¹**: jeweils gegebenenfalls substituiertes C₁-C₈-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²**: Wasserstoff, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-AlkylenHetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
R_{A}³: jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-AlkylenHetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, S0₂-Hetaryl, SO₂-C₁-C₄-Alkylen-Aryl oder SO₂-C₁-C₄-Alkylen-Hetaryl;
oder die Reste **R_{A}²** und **R_{A}³** bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann; wobei gegebenenfalls zwei an diesem Heterocyclus substituierte Reste zusammen einen jeweils gegebenenfalls substituierten 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome ausgewählt aus der Gruppe bestehend aus O, N, und S enthalten kann, und wobei der dadurch gebildete Cyclus gegebenenfalls substituiert sein kann oder an diesem Cyclus ein weiterer, gegebenenfalls 3 bis 7-gliedriger jeweils gegebenenfalls substituierter Cyclus ankondensiert sein kann;
**R_{A}⁴**: Wasserstoff, oder
jeweils gegebenenfalls substituiertes C₁-C₆-Alkyl, C₂-C₆-Alkenyl, C₂-C₁₂-Alkinyl, C₃-C₇-Cycloalkyl, Aryl, C₁-C₄-Alkylen-Aryl, C₁-C₆-Alkylen-O-C₁-C₆-Alkyl, Hetaryl;
**B**: Wasserstoff, OH oder unabhängig von **A** wie Rest **A** definiert ist, oder zwei der Reste **A**, **B** oder **R_{W}¹** bilden jeweils unabhängig voneinander zusammen mit den anhängenden C-Atomen einen 3 bis 7-gliedrigen, jeweils gegebenenfalls substituierten, gesättigten, ungesättigten oder aromatischen Carbocyclus oder einen jeweils gegebenenfalls substituierten 3 bis 7-gliedrigen jeweils gegebenenfalls substituierten, gesättigten, ungesättigten oder aromatischen Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen jeweils gegebenenfalls substituierten 3 bis 7-gliedrigen anellierten, gesättigten, ungesättigten oder aromatischen Carbocyclus oder Heterocyclus bilden können, wobei der Heterocyclus bis zu drei verschiedene oder gleiche Heteroatome ausgewählt aus der Gruppe bestehend aus O, N, und S enthalten kann und wobei der gebildete Cyclus gegebenenfalls substituiert sein kann;
**R_{W}¹**: Wasserstoff, OH, Halogen, NO₂, NH₂, CN, CF₃, CHF₂, CH₂F, OCF₃, OCHF₂, OCH₂F oder
jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D**: unabhängig von A wie Rest **A** definiert ist;
**Q**: einen Rest der allgemeinen Formel **Q1** 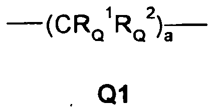 mit dem Index
a=1
**R_{Q}¹, R_{Q}²** unabhängig voneinander:
Wasserstoff, Halogen, OH, oder gegebenenfalls substituiertes C₁-C₆-Alkyl;
**R¹, R²** unabhängig voneinander:
Wasserstoff, OH, CN, oder
jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-AlkylenAryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₈-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
**R¹** und **R²** bilden zusammen mit dem Stickstoff einen 5 bis 7-gliedrigen, jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen anellierten, gesättigten, ungesättigten oder aromatischen jeweils gegebenenfalls substituierten Carbocyclus oder Heterocyclus bilden können, wobei der Heterocyclus ein, zwei oder bis zu drei verschiedene oder gleiche Heteroatome ausgewählt aus der Gruppe bestehend aus O, N, und S enthalten kann und wobei der gebildete Cyclus gegebenenfalls substituiert sein kann;
**R³** Freies Elektronenpaar oder Wasserstoff;
**X,Y,Z** X = N, Y = CR⁴, Z = CR⁶, oder
X = C, Y = NR⁵, Z = CR⁶, oder
X = C, Y = CR⁴, Z = NR⁷, oder
X = N, Y = N, Z = CR⁶, oder
X = N,Y = CR⁴, Z = N
**R⁴, R⁶** jeweils unabhängig voneinander einen Rest, ausgewählt aus der gleichen oder verschiedenen Gruppen **1.), 3.), 4.),5.)** oder **6.)** und
**R⁵, R⁷** jeweils unabhängig voneinander einen Rest, ausgewählt aus der gleichen oder verschiedenen Gruppen **2.), 3.),4.)** oder **5.)**, wobei die Gruppen 1.) bis 6.) wie folgt definiert sind:
**1.)** Wasserstoff, NO₂, NH₂, CN, CF₃, CHF₂, OCF₃, OCHF₂, COOH, O-CH₂-COOH, Halogen, oder
jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵ SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
**2.)** Freies Elektronenpaar oder Wasserstoff, CH₂-CF₃, CH₂-CHF₂, jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₆-Alkyl oder C₁-C₄-Alkylen-COO-C₁-C₆-Alkyl, oder jeweils gegebenenfalls substituiertes CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷;
**3.)** Phenyl, 1-Naphthyl oder 2-Naphthyl, die jeweils mit **R_{Z,Y}¹, R_{Z,Y}²** und **R_{Z,Y}³** substituiert sein können, wobei
**R_{Z,Y}¹, R_{Z,Y}²** und **R_{Z,Y}³** jeweils unabhängig voneinander einen Substituenten aus der folgenden Gruppe darstellen:
Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder jeweils gegebenenfalls substituiertes
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}-CO-R_{Z,Y}⁵, NR_{Z,Y}⁷-CO-NR_{Z,Y}⁵, SO₂-R_{Z,Y}⁵, NR_{Z,Y}⁷-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ oder CO-NR_{Z,Y}⁶R_{Z,Y}⁷, oder
jeweils zwei der Reste aus **R_{Z,Y}¹, R_{Z,Y}²** oder **R_{Z,Y}³** bilden zusammen mit den Ringatomen, die diese Substituenten tragen, einen 3 bis 7-gliedrigen, jeweils gegebenenfalls substituierten, gesättigten oder ungesättigten Carbocyclus, oder einen jeweils gegebenenfalls substituierten, gesättigten oder ungesättigten Heterocyclus, der ein, zwei oder drei weitere verschiedene oder gleiche Heteroatome ausgewählt aus der Gruppe bestehend aus O, N, und S enthalten kann, wobei jeweils gegebenenfalls zwei an diesem Carbo- oder Heterocyclus substituierte Reste zusammen einen 3 bis 7-gliedrigen, anellierten, gesättigten, ungesättigten oder aromatischen jeweils gegebenenfalls substituierten Carbocyclus oder Heterocyclus bilden können, wobei der Heterocyclus ein, zwei oder bis zu drei verschiedene oder gleiche Heteroatome ausgewählt aus der Gruppe bestehend aus O, N, und S enthalten kann und wobei der gebildete Cyclus gegebenenfalls substituiert sein kann und/oder an diesem Cyclus ein weiterer, 3 bis 7-gliedrigen, gegebenenfalls substituierter Cyclus ankondensiert sein kann;
**R_{Z,Y}⁴** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Hetaryl, Aryl, C₁-C₄-AlkylenHetaryl, C₁-C₄-Alkylen-Aryl, oder C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂;
**R_{Z,Y}⁵** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Heterocycloalkyl oder C₁-C₆-Alkylen-O-C₁-C₆-Alkyl, Dioxymethylenphenyl, Benzofuranyl, Dihydrobenzofuranyl, Indanyl, oder
jeweils gegebenenfalls 1-, 2- oder bis zu 3-fach substituiertes Aryl oder Hetaryl, wobei die Substituenten unabhängig voneinander ausgewählt sind aus der Gruppe, bestehend aus Halogen, NO₂, NH₂, OH, CN, CF₃, CHF₂, OCF₃, OCHF₂, gegebenenfalls substituiertes NH-(C₁-C₆-Alkyl) oder gegebenenfalls substituiertes N(C₁-C₆-Alkyl)₂, COOH, O-CH₂-COOH, SH, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder jeweils gegebenenfalls substituiertes O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
**R_{Z,Y}⁶** Wasserstoff, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl,
**R_{Z,Y}⁷** Wasserstoff oder jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl,
oder die Reste R_{Z,Y}⁶ und R_{Z,Y}⁷ bilden zusammen mit dem Stickstoff einen 3 bis 7-gliedrigen, jeweils gegebenenfalls substituierten, gesättigten oder aromatischen Heterocyclus, der ein, zwei oder drei weitere verschiedene oder gleiche Heteroatome ausgewählt aus der Gruppe bestehend aus O, N, und S enthalten kann, bilden; und jeweils gegebenenfalls zwei an diesem Heterocyclus substituierte Reste zusammen einen 3 bis 7-gliedrigen, anellierten, gesättigten, ungesättigten oder aromatischen jeweils gegebenenfalls substituierten Carbocyclus oder Heterocyclus bilden können, wobei der Heterocyclus bis zu drei verschiedene oder gleiche Heteroatome ausgewählt aus der Gruppe bestehend aus O, N, und S enthalten kann, und der gebildete Cyclus gegebenenfalls substituiert sein kann und/oder an diesem Cyclus ein weiterer, gegebenenfalls substituierter 3 bis 7-gliedriger Cyclus ankondensiert sein kann;
**4.)** einen 5- oder 6-gliedrigen aromatischen Heterocyclus der ein, zwei oder drei verschiedene oder gleiche Heteroatome ausgewählt aus der Gruppe bestehend aus O, N, und S enthalten kann und der jeweils mit **R_{Z,Y}¹**, R_{Z,Y}² und **R_{Z,Y}³,** welche unabhängig voneinander wie vorstehend definiert sind, substituiert sein kann;
**5.)** einen jeweils gegebenenfalls substituierten C₅-C₁₈- bi- oder tricyclischen, gesättigten Kohlenwasserstoffrest
**6.) R⁴** und **R⁶** bilden zusammen mit X und Y einen C₆-C₁₀-gliedrigen gesättigten, ungesättigten oder aromatischen gegebenenfalls substituierten Carbocyclus, bevorzugt einen gegebenenfalls substituierten *bis* Benzo-Rest, die jeweils mit einem, zwei oder bis zu drei Resten substituiert sein können, unabhängig voneinander ausgewählt aus der Gruppe **R_{Z,Y}⁸.**
R_{Z,Y}⁸: Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-AlkylenHetaryl, oder jeweils gegebenenfalls substituiertes O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ oder CO-NR_{Z,Y}⁶R_{Z,Y}⁷
zur Herstellung eines Medikaments zur Behandlung und/oder Prophylaxe von ZNS Erkrankungen oder von ZNS bezogenen Erkrankungen.

20. Verwendung von mindestens einer 5-Ring-Heteroaromaten-Verbindung der allgemeinen Formel I nach einem der Ansprüche 1 bis 16 oder Anspruch 19, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikaments zur Behandlung und/oder Prophylaxe von ZNS Erkrankungen und/oder von ZNS bezogenen Erkrankungen.

21. Verwendung von mindestens einer 5-Ring-Heteroaromaten-Verbindung der allgemeinen Formel I nach einem der Ansprüche 1 bis 16 oder Anspruch 19 oder 20, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikaments zur Behandlung und/oder Prophylaxe von neuropathologischen, neuropsychiatrischen und/oder neurodegenerativen Störungen, Symptomen und/oder Fehlfunktionen.

22. Verwendung von mindestens einer 5-Ring-Heteroaromaten-Verbindung der allgemeinen Formel I nach einem der Ansprüche 1 bis 16 oder 19, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von Migräne und/oder Gehirnschädigungen.

23. Verwendung von mindestens einer 5-Ring-Heteroaromaten-Verbindung der allgemeinen Formel I nach einem der Ansprüche 1 bis 16 oder 19, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von neuropathologischen, neuropsychiatrischen und/oder neurodegenerativen Krankheiten ausgewählt aus der Gruppe bestehend aus cerebraler Ischämie, Schlaganfall, Epilepsie und Anfälle im allgemeinen, Psychosen, Schizophrenie, Autismus, OCD-Syndrom, cognitiven Erkrankungen, Aufmerksamkeitsstörungen, Depressionen, bipolaren und/oder unipolaren Depressionen, Angstzuständen, Demenz, seniler Demenz, Alzheimer Demenz, demyelinisierenden Erkrankungen, Multipler Sklerose und Gehirntumoren.

24. Verwendung von mindestens einer 5-Ring-Heteroaromaten-Verbindung der allgemeinen Formel I nach einem der Ansprüche 1 bis 16 oder 19, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von Krankheiten ausgewählt aus der Gruppe bestehend aus cerebrovaskulären Störungen, Schmerz, Schmerz-bedingten Störungen, Abhängigkeit, Drogen-bedingten Störungen, Amnesie, Alkoholmissbrauch, Drogenmissbrauch, Störungen des circadianen Rhythmus und Cushing Syndrom.

25. Verwendung von mindestens einer 5-Ring-Heteroaromaten-Verbindung der allgemeinen Formel I nach einem der Ansprüche 1 bis 16 oder einem der Ansprüche 19 bis 24, und/oder entsprechende enantiomere, diastereomere und/oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von Krankheiten, die durch eine 5-HT5-Rezeptoraktivität moduliert werden.

26. Verwendung von mindestens einer 5-Ring-Heteroaromaten-Verbindung der allgemeinen Formel I nach einem der Ansprüche 1 bis 16 oder einem der Ansprüche 19 bis 25, und/oder entsprechende enantiomere, diastereomere und/oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor beruht.

27. Verwendung von mindestens einer 5-Ring-Heteroaromaten-Verbindung der allgemeinen Formel I einem der Ansprüche 1 bis 16 oder einem der Ansprüche 19 bis 25, und/oder entsprechende enantiomere, diastereomere und/ oder tautomere Formen davon und/oder pharmazeutisch annehmbare Salze davon, wobei die Reste R¹, R², R³, Q, W, X, Y und Z wie in einem der Ansprüche 1 bis 16 oder 19 ausgeführt definiert sind, zur Herstellung eines Medikamentes zur Behandlung und/oder Prophylaxe von Krankheiten, die durch eine 5-HT5-Rezeptoraktivität moduliert werden und wobei die Behandlung und/oder Prophylaxe auf einer Selektivität zum 5-HT5A-Rezeptor mit einer Bindungsaffinität (Ki) von kleiner oder gleich 600 nM beruht.

28. Verwendung nach einem der Ansprüche 19 bis 27, wobei die Behandlung und/oder Prophylaxe in einem menschlichen oder nicht menschlichen Säugetier erfolgt.

## Claims

1. At least one 5-membered ring heteroaromatic compound of the general formula I 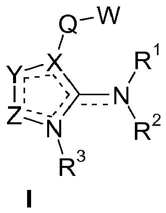 and/or corresponding enantiomeric, diastereomeric and/or tautomeric forms thereof and/or pharmaceutically acceptable salts thereof, where the radicals indicated possess the following definitions
**W** is: a radical of the general formula **W1** or **W2** 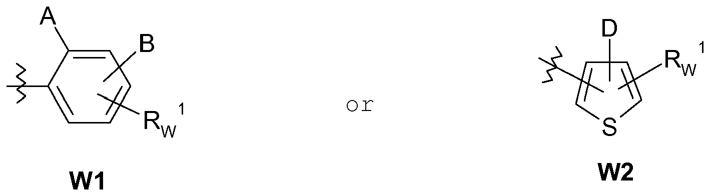 in which
**A** is: NO₂, NH₂, CN, CF₃, OCF₃, CHF₂, OCHF₂, COOH, 0-CH₂-COOH, halogen, SH, or in each case optionally substituted O-C₁-C₆-alkyl, S-C₁-C₆-alkyl, -CO-C₁-C₆-alkyl, -0-CO-aryl, -0-CO-hetaryl, -O-COO-C₁-C₆-alkyl or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-hetaryl or C₁-C₄-alkylene-aryl, or
in each case optionally substituted O-R_{A}¹, CO-R_{A}¹, S-R_{A}¹, SO-R_{A}¹ CO-R_{A}¹, NR_{A}⁴-CO-O-R_{A}¹, O-CH₂-COO-R_{A}¹, NR_{A}²R_{A}³, CONH₂, SO₂NH₂, NR_{A}⁴-COR_{A}¹, SO₂-R_{A}¹, NR_{A}⁴-SO₂-R_{A}¹, SO₂-NR_{A}²R_{A}³ or CO-NR_{A}²R_{A}³ ;
**R_{A}¹** is: in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₂-C₆-alkenylene-aryl, C₁-C₆-alkylene-hetaryl;
**R_{A}²** is : hydrogen, or
in each case optionally substituted C₁-C₆-alkyl, C₂-C₆-alkenyl, C₂-C₆-alkynyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₁-C₄-alkylene-hetaryl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{A}³** is: in each case optionally substituted C₁-C₆-alkyl, C₂-C₆-alkenyl, C₂-C₆-alkynyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₁-C₄-alkylene-hetaryl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
or the radicals **R_{A}²** and **R_{A}³** together with the nitrogen form a 3- to 7-membered, in each case optionally substituted, saturated or aromatic heterocycle, which can contain one, two or three further different or identical heteroatoms from the group 0, N, S; where optionally two radicals substituted on this heterocycle together can form a 3- to 7-membered fused, saturated, unsaturated or aromatic carbocycle or heterocycle, where the heterocycle can contain up to three different or identical heteroatoms 0, N, S, and where the cycle formed thereby can optionally be substituted or a further, optionally 3- to 7-membered substituted cycle can be fused to this cycle;
**R_{A}⁴** is: hydrogen, or
in each case optionally substituted C₁-C₆-alkyl, C₂-C₆-alkenyl, C₂-C₁₂-alkynyl, C₃-C₇-cycloalkyl, aryl, C₁-C₄-alkylene-aryl, C₁-C₆-alkylene-O-C₁-C₆-alkyl, hetaryl;
**B** is: hydrogen or independently of radical **A** is defined as radical **A**,
or two of the radicals **A**, **B** or **R_{W}¹** in each case independently of one another together with the attached C atoms form a 3- to 7-membered, in each case optionally substituted, saturated, unsaturated or aromatic carbocycle or an in each case optionally 3- to 7-membered substituted, saturated, unsaturated or aromatic heterocycle, which can contain one, two or three further different or identical heteroatoms selected from the group consisting of 0, N, and S, where in each case optionally two radicals substituted on this carbo- or heterocycle together can form a 3- to 7-membered fused, saturated, unsaturated or aromatic carbocycle or heterocycle, where the heterocycle can contain up to three different or identical heteroatoms 0, N, S and where the cycle formed can optionally be substituted;
**R_{W}¹** is: hydrogen, OH, halogen, NO₂, NH₂, CN, CF₃, CHF₂, CH₂F, OCF₃, OCHF₂, OCH₂F or
in each case optionally substituted C₁-C₆-alkyl, C₃-C₇-cycloalkyl, C₁-C₆-alkylene-O-C₁-C₆-alkyl, C₁-C₆-thioalkyl, aryl, hetaryl, 0-C₁-C₆-alkyl, O-benzyl, C₁-C₆-alkylamino, C₁-C₆-dialkylamino, pyrrolidinyl, piperidinyl, morpholinyl, CO-C₁-C₆-alkyl, SO₂-C₁-C₆-alkyl, CO-aryl, SO₂-aryl, CO-C₁-C₄-alkylene-aryl, SO₂-C₁-C₄-alkylene-aryl, SO-aryl, CONH₂, CONH-C₁-C₆-alkyl, SO₂NH-C₁-C₆-alkyl, CON-(C₁-C₆-alkyl)₂, SO₂N-(C₁-C₆-alkyl)₂, NH-SO₂-C₁-C₆-alkyl or NH-CO-C₁-C₆-alkyl;
optionally with the proviso that if W = W1, then R_{W}¹ or B should not be an optionally substituted N-pyrrolidinyl radical standing in the 4-position (para-position) to the linkage position with Q;
**D:** is hydrogen or independently of **A** is defined as radical **A;**
**Q** is: a radical of the general formula **Q1** 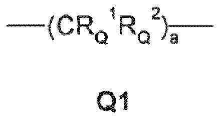 having the index
a = 1
**R_{Q}¹, R_{Q}²** independently of one another are :
hydrogen, halogen, OH or optionally substituted C₁-C₆-alkyl;
**R¹**, **R²** independently of one another are:
hydrogen, OH, CN, or
in each case optionally substituted C₁-C₆-alkyl, O-C₁-C₆-alkyl, C₁-C₆-alkylene-O-C₁-C₆-alkyl, C₃-C₇-cycloalkyl, O-C₃-C₇-cycloalkyl, aryl, hetaryl, C₁-C₄-alkylene-aryl, C₁-C₄-alkylene-hetaryl, O-aryl, O-C₁-C₄-alkylene-aryl, 0-hetaryl, O-C₁-C₄-alkylene-hetaryl, CO-C₁-C₆-alkyl, CO-aryl, CO-hetaryl, CO-C₁-C₄-alkylene-aryl, CO-C₁-C₄-alkylene-hetaryl, COO-C₁-C₆-alkyl, CO-O-aryl, CO-0-hetaryl, CO-O-C₁-C₄-alkylene-aryl, SO₂-C₁-C₆-alkyl, SO₂-aryl, SO₂-hetaryl, SO₂-C₁-C₄-alkylene-aryl, OCO-C₁-C₆-alkyl, OCO-aryl, OCO-hetaryl, OCO-C₁-C₄-alkylene-aryl, OCO-C₁-C₄-alkylene-hetaryl, SO₂-C₁-C₆-alkyl, SO₂-aryl, SO₂-hetaryl or SO₂-C₁-C₄-alkylene-aryl, or
**R¹** and **R²** together with the nitrogen form a 5-to 7-membered, in each case optionally substituted, saturated or unsaturated heterocycle, which can contain one, two or three further different or identical heteroatoms from the group 0, N, S, where in each case optionally two radicals substituted on this carbo- or heterocycle together can form a 3- to 7-membered fused, saturated, unsaturated or aromatic in each case optionally substituted carbocycle or heterocycle, where the heterocycle can contain up to three different or identical heteroatoms selected from the group consisting of 0, N, and S and where the cycle formed can optionally be substituted;
**R³** is a free electron pair or hydrogen;
**X, Y, Z** in each case together have the following meaning:
X = N, Y = CR⁴, Z = CR⁶, or
X = C, Y = NR⁵, Z = CR⁶, or
X = C, Y = CR⁴, Z = NR⁷, or
X = N, Y = N, Z = CR⁶, or
X = N, Y = CR⁴, Z = N
**R⁴, R⁶** in each case independently of one another are a radical, selected from the identical or different radicals of the groups **1.), 3.)**, **4.)**, **5.)** or **6.)** and
**R⁵, R⁷** in each case independently of one another are a radical selected from the identical or different radicals of the groups **2.), 3.), 4.)** or **5.)**
**1.)** hydrogen, NO₂, NH₂, CN, CF₃, CHF₂, OCF₃, OCHF₂, COOH, O-CH₂-COOH, halogen, or in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, C₁-C₆-alkylene-O-C₁-C₆-alkyl, C₁-C₆-alkylene-O-aryl, COO-C₁-C₆-alkyl or C₁-C₄-alkylene-COO-C₁-C₆-alkyl, or in each case optionally substituted
O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-O-R_{Z,Y}⁵, O-CH₂-COO-R_{Z,Y}⁵, NR_{Z,Y}⁷-COR_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
**2.)** a free electron pair or hydrogen, CH₂-CF₃, CH₂-CHF₂,
in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, C₁-C₆-alkylene-O-C₁-C₆-alkyl, C₁-C₆-alkylene-O-aryl, COO-C₁-C₆-alkyl or C₁-C₄-alkylene-COO-C₁-C₆-alkyl, or in each case optionally substituted
CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ or CO-NR_{Z,Y}⁶ R_{Z,Y}⁷ ;
**3.)** phenyl, 1-naphthyl or 2-naphthyl, which in each case are or can be substituted by one, two or three radicals selected from R_{Z,Y}¹, R_{Z,Y}² and **R_{Z},_{Y}³ ,** where
**R_{Z},Y¹, R_{Z,Y}²** and **R_{Z,Y}³** in each case independently of one another represent a substituent from the following group:
hydrogen, NO₂, NH₂, OH, CN, CF₃, CHF₂, OCF₃, OCHF₂, COOH, O-CH₂-COOH, SH, halogen, or in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵_{,} NR_{Z,Y}⁷-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ or CO-NR_{Z,Y}⁶R_{Z,Y}⁷, or
in each case two of the radicals selected from the group consisting of **R_{Z,Y}¹, R_{Z,Y}²** or **R_{Z,Y}³** together with the ring atoms which carry this substituent form a 3- to 7-membered, in each case optionally substituted, saturated or unsaturated carbocycle, or a 3- to 7-membered in each case optionally substituted, saturated or unsaturated heterocycle, which can contain one, two or three further different or identical heteroatoms selected from the group consisting of 0, N, and S, where optionally in each case optionally two radicals substituted on this carbo- or heterocycle together can form a 3- to 7-membered, fused, saturated, unsaturated or aromatic carbocycle or heterocycle, where the heterocycle can contain 1, 2 or up to three different or identical heteroatoms selected from the group consisting of O, N, S and where the cycle formed can optionally be substituted and/or a further, 3- to 7-membered, optionally substituted cycle can be fused to this cycle;
**R_{Z,Y}⁴** is in each case optionally substituted C₂-C₆-alkenyl, C₂-C₆-alkynyl, C₁-C₄-alkylene-C₃-C₇-cycloalkyl, C₁-C₄-alkylene-heterocycloalkyl, heterocycloalkyl, C₁-C₄-alkylenehetaryl, hetaryl, C₁-C₄-alkylenearyl, aryl, or C₁-C₆-alkyl which is optionally substituted by a substituent selected from the group consisting of halogen, NO₂, NH₂, OH, CN, CF₃, CHF₂, OCF₃, OCHF₂, optionally substituted NH- (C₁-C₆-alkyl) or optionally substituted N (C₁-C₆-alkyl) ₂;
**R_{Z,Y}⁵** is in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, heterocycloalkyl or C₁-C₆-alkylene-O-C₁-C₆-alkyl, dioxymethylenephenyl, benzofuranyl, dihydrobenzofuranyl, indanyl, or
in each case aryl or hetaryl which are optionally substituted 1, 2 or up to 3 times, the substituents being independently of one another selected from the group consisting of halogen, NO₂, NH₂, OH, CN, CF₃, CHF₂, OCF₃, OCHF₂, optionally substituted NH- (C₁-C₆-alkyl) or optionally substituted N (C₁-C₆-alkyl)₂, COOH, O-CH₂-COOH, SH, or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-hetaryl or C₁-C₄-alkylene-aryl, or in each case optionally substituted
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A} ¹ NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NRA₂R_{A}³ or CO-NR_{A}²R_{A}³;
**R_{Z,Y}⁶** is hydrogen, or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, C₁-C₄-alkylene-aryl, C₁-C₄-alkylene-hetaryl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, CO-O-C₁-C₄-alkylene-aryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{Z,Y}⁷** is hydrogen or in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, Cₗ-C₄-alkylene-heterocycloalkyl, aryl, hetaryl, heterocycloalkyl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-O-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
or the radicals R_{Z,Y}⁶ and R_{Z,Y}⁷ together with the nitrogen form a 3- to 7-membered, in each case optionally substituted, saturated or aromatic heterocycle, which can contain one, two or three further different or identical heteroatoms selected from the group consisting of 0, N, and S; and in each case optionally two radicals substituted on this heterocycle together can form a 3- to 7-membered, fused, saturated, unsaturated or aromatic carbocycle or heterocycle, where the heterocycle can contain 1, 2 or up to three different or identical heteroatoms selected from the group consisting of 0, N, and S, and the cycle formed can optionally be substituted or a further, optionally substituted 3- to 7-membered cycle can be fused to this cycle;
**4.)** an in each case optionally substituted 5- or 6-membered aromatic heterocycle which can contain one, two or three different or identical heteroatoms selected from the group consisting of 0, N, and S and which in each case can be substituted by **R_{Z,Y}¹, R_{Z,Y}²** and **R_{Z,Y}³**, where R_{Z,Y}¹_{,} R_{Z,Y}² and R_{Z,Y}³ independently of one another have the meanings mentioned above under 3.);
**5.)** an in each case optionally substituted C₅-C₁₈- bi- or tricyclic, saturated hydrocarbon radical.
**6.) R⁴** and **R⁵** together with X and Y form an in each case optionally substituted C₆-C₁₀-membered saturated, unsaturated or aromatic carbocycle, preferably a benzo radical, which in each case can be substituted by one, two or up to three radicals, independently of one another selected from the group **R_{Z,Y}⁸.**
**R_{Z,Y}⁸:** hydrogen, NO₂, NH₂, OH, CN, CF₃, CHF₂, OCF₃, OCHF₂, COOH, O-CH₂-COOH, SH, halogen, or
in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵_{,} NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷,
optionally with one, two or three of the provisos selected from the group consisting of the provisos (i), (ii) and (iii) mentioned below:
(i) with the proviso that if R⁴ and R⁶ have the meanings mentioned in group 6.), then the radicals **A, B, Q, R_{Q}¹** and **R_{Q}²** are defined as follows:
**A** is located in W1 as indicated in the 2 position and is NH₂, OCF₃, OCHF₂, O-CH₂-COOH, O-C₁-C₆-alkyl, S-C₁-C₆-alkyl, or 0-R_{A}¹, CO-R_{A}¹, S-R_{A}¹, SO-R_{A}¹, SO₂-R_{A}¹, SO₂-NR_{A}²R_{A}³ ;
**B** is located in W1 in the 6 position and is halogen, CN, CF₃, -CHF₂, OCF₃, OCHF₂, or
in each case optionally substituted C₁-C₆-alkyl or C₂-C₆-alkenyl, -O-CH₂-COO-R_{A}¹, - O-R_{A}¹, S-R_{A}¹, NR_{A}²R_{A}³, -NR_{A}⁴-CO-R_{A}¹ or -CO-NR_{A}²R_{A}³_{;} NR_{A}⁴-SO₂-R_{A}¹;
or B together with **R_{W}¹** forms one of the radicals - (CH₂)₃-, -O-CH₂-O-, -O-(CH₂) ₂-O-, -CH=CH-CH=CH-, -O- (CH₂)₂-, - (CH₂)₂-O-;
**Q** is -CR_{Q}¹R_{Q}²-
**R_{Q}¹, R_{Q}²** independently of one another are: hydrogen, halogen, optionally substituted C₁-C₆-alkyl;
(ii) with the proviso that if W = W1, then R_{W}¹ or B should not be an optionally substituted N-pyrrolidinyl radical standing in the 4-position (para-position) to the linkage position with Q;
(iii) with the proviso that the sum of a, b and c is then 1, 2, 3, 4 or 5 if X = N, Y = CR⁴, and Z = CR⁶.

2. At least one 5-membered ring heteroaromatic compound of the general formula I according to Claim 1, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, X, Y, Z,** unless specified otherwise hereinbelow have the same meaning as described in Claim 1 and the radicals below possess the following definitions:
**R⁴, R⁶ in** each case independently of one another are a radical selected from the identical or different radicals of the groups **1.), 3.), 4.),** or **5.)** and
**R⁵, R⁷** in each case independently of one another are a radical selected from the identical or different radicals of the groups **2.), 3.), 4.)** or **5.)**
where the groups 1.) - 5.) should in each case have the meanings given in Claim 1.

3. At least one 5-membered ring heteroaromatic compound of the general formula I according to either of Claims 1 and 2, and/or corresponding enantiomeric, diastereomeric and/or tautomeric forms thereof and/or pharmaceutically acceptable salts thereof, where the radicals **Q, R_{Q}¹, R_{Q}², R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow have the same meaning as described in either of Claims 1 and 2 and the radicals below possess the following definition:
**W** is: a radical of the general formula **W1, W21** or **W22** 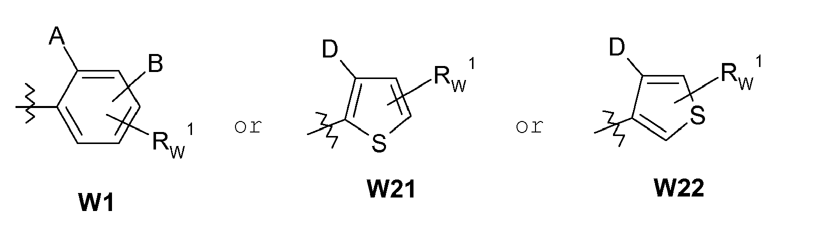
**W**1 **W21 W22**
where the radicals A, B, D and R_{W}¹ independently of one another and in each case independently of their respective occurrence may have one of the following meanings:
**A** is: halogen, NH₂, CN, CF₃, OCF₃, OCHF₂, COOH, 0-CH₂-COOH, SH, O-C₁-C₆-alkyl, S-C₁-C₆-alkyl, C₁-C₆-alkyl or in each case optionally substituted
O-R_{A}¹_{,} CO-R_{A}¹, S-R_{A}¹, O-CH₂-COO-R_{A}¹, NR_{A}²R_{A}³, CONH₂, SO₂NH₂, SO₂₋R_{A}¹_{,} NR_{A}⁴-SO₂-R_{A}¹, SO₂-NR_{A}²R_{A}³ or CO-NR_{A}²R_{A}³;
**R_{A}¹** is: in each case optionally substituted C₁-C₆-alkyl, C₃-C₇-cycloalkyl, phenyl or benzyl;
**R_{A}²**; **R_{A}³** independently of one another are
hydrogen, or
in each case optionally substituted C₁-C₆-alkyl, phenyl, benzyl, phenethyl, CO-C₁-C₆-alkyl, CO-aryl, CO-o-C₁-C₆-alkyl, SiO₂-C₁-C₆-alkyl, SO₂-aryl, SO₂-hetaryl, or SO₂-C₁-C₄-alkylene-aryl;
or both radicals **R_{A}²** and **R_{A}³** together with the nitrogen form an in each case optionally substituted 5- or 6-membered saturated or unsaturated ring, which can contain one or up to two identical or different heteroatoms selected from the group consisting of 0 and N.
**R_{A}⁴** is: hydrogen or an optionally substituted C₁-C₆-alkyl radical;
**B** is: hydrogen, halogen, CN, CF₃, -CHF₂, OCF₃, OCHF₂, or
in each case optionally substituted C₁-C₆-alkyl or C₂-C₆-alkenyl, or in each case optionally substituted
-O-CH₂-COO-R_{A}¹, - O-R_{A}¹, S-R_{A}¹, NR_{A}²R_{A}³, -NR_{A}⁴-CO-R_{A}¹ or -CO-NR_{A}²R_{A}³; NR_{A}⁴-SO₂-R_{A}¹;
or B together with **R_{W}¹** forms one of the radicals - (CH₂)₃-, -O-CH₂-O-, -O-(CH₂)₂-O-, - CH=CH-CH=CH-, -O- (CH₂)₂-, - (CH₂)₂-O-;
**R_{W}¹** is: hydrogen, F, Cl, CN, CF₃, O-CF₃, or in each case optionally substituted C₁-C₆-alkyl, aryl, C₁-C₆-alkylamino or C₁-C₆-dialkylamino;
**D** is: hydrogen or independently of A is defined as radical **A.**

4. At least one 5-membered ring heteroaromatic compound of the general formula I according to one of Claims 1 to 3, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹**, **R_{Q}², X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow have the same meaning as described in one of Claims 1 to 3 and the radicals below possess the following definition:
**R¹, R²**, independently of one another are:
hydrogen, OH, CN, C₁-C₆-alkyl, C₁-C₆-alkylene-O-C₁-C₆-alkyl, substituted aryl, benzyl, CO-C₁-C₆-alkyl, CO-aryl, or CO-C₁-C₄-alkylene-aryl;
**R³** is a free electron pair or hydrogen.

5. At least one 5-membered ring heteroaromatic compound of the general formula I according to one of Claims 1 to 4, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}**⁴ , **R_{Z,Y}⁵, R_{Z,Y}⁶, R_{Z,Y}⁷, R_{Z,Y} ⁸** unless specified otherwise hereinbelow have the same meaning as described in one of Claims 1 to 4 and the radicals below possess the following definition:
**X, Y, Z** X = N, Y = CR⁴, Z = CR⁶, or
X = C, Y = CR⁴, Z = NR⁷, or
X = N, Y = N, Z = CR⁶

6. At least one 5-membered ring heteroaromatic compound of the general formula I according to one of Claims 1 to 5, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, RZ,_{Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow have the same meaning as described in one of Claims 1 to 5 and the radicals below possess the following definition:
**X, Y, Z** X = N, Y = CR⁴, Z = CR⁶, or
X = N, Y = N, Z = CR⁶

7. At least one 5-membered ring heteroaromatic compound of the general formula I according to one of Claims 1 to 6, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, RQ¹, R_{Q}², R₁, R₂, R₃, X, Y, Z,** unless specified otherwise hereinbelow have the same meaning as described in one of Claims 1 to 6 and the radicals below possess the following definition:
**R⁴, R⁶** in each case independently of one another are a radical selected from the identical or different radicals of the groups **1.), 3.), 4.)** or **5.)** and
**R⁵, R⁷ in** each case independently of one another are a radical selected from the identical or different radicals of the groups **2.), 3.), 4.)** or **5.),**
where the groups 1.), 2.), 3.), 4.) and 5.) are defined as follows:
**1.)** hydrogen, CN, CF₃, CHF₂, O-CH₂-COOH, halogen, or
in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, or in each case optionally substituted O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}, NR_{Z,Y}⁷-CO-R_{Z,Y}⁵, SO₂-R_{Z,Y}⁵, NR_{Z,Y}7-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
**2.)** a free electron pair or hydrogen,
in each case optionally substituted C₁-C₁₀-alkyl, C₃-C₇-cycloalkyl, C₁-C₆-alkylene-C₃-C₇-cycloalkyl, C₁-C₄-alkylene-aryl, C₁-C₄-alkylene-hetaryl, COO-C₁-C₆-alkyl or in each case optionally substituted
SO₂-R_{Z,Y}⁵, SO₂NH₂, CONH₂, SO₂-NR_{Z,Y}⁶R_{Z,Y}⁷ or CO-NR_{Z,Y}⁶R_{Z,Y} ;
**3.)** phenyl, 1-naphthyl or 2-naphthyl, which in each case are substituted by **R_{Z,Y}¹, R_{Z,Y}²** and **R_{Z,Y}³,** where
**R_{Z,Y}¹, R_{Z,Y}²** and **R_{Z,Y}³** in each case independently of one another represent a substituent from the following group:
hydrogen, NH₂, CN, CF₃, CHF₂, OCF₃, OCHF₂, 0-CH₂-COOH, halogen, or
in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷, or
in each case two of the radicals from **R_{Z,Y}¹, R_{Z,Y}²** or **R_{Z,Y}³** together with the ring atoms which carry this substituent form a 3- to 7-membered, in each case optionally substituted, saturated or unsaturated carbocycle, or an in each case optionally substituted, saturated or unsaturated heterocycle, which can contain one, two or three further different or identical heteroatoms selected from the group consisting of 0, N, and S;
**R_{Z,Y}⁴** is in each case optionally substituted C₁-C₄-alkylene-C₃-C₇-cycloalkyl, C₁-C₄-alkylene-heterocycloalkyl, heterocycloalkyl, hetaryl, aryl, C₁-C₄-alkylene-hetaryl, C₁-C₄-alkylene-aryl, or
C₁-C₆-alkyl, which optionally is substituted by a substituent from the group, consisting of halogen, NO₂, NH₂, OH, CN, CF₃, CHF₂, OCF₃, OCHF₂, NH- (C₁-C₆-alkyl) and N (C₁-C₆-alkyl) ₂;
**R_{Z,Y}⁵** is in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, heterocycloalkyl or C₁-C₆-alkylene-O-C₁-C₆-alkyl, dioxymethylenephenyl, or
in each case aryl or hetaryl optionally substituted 1, 2 or up to 3 times here by substituents independently of one another selected from the group consisting of halogen, NO₂, NH₂, OH, CN, CF₃, CHF₂, OCF₃, OCHF₂, NH- (C₁-C₆-alkyl) or N (C₁-C₆-alkyl) ₂, COOH, O-CH₂-COOH, SH, or
in each case optionally substituted C₁-C₆-alkyl, C₃-C₇-cycloalkyl, aryl, hetaryl, heterocycloalkyl, or in each case optionally substituted O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, or SO₂-NR_{A}²R_{A}³;
**R_{Z,Y}⁶** is hydrogen, or
in each case optionally substituted C₁-C₆-alkyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, C₁-C₄-alkylene-aryl, C₁-C₄-alkylene-hetaryl, CO-aryl, CO-hetaryl, CO-C₁-C₄-alkylene-aryl, CO-C₁-C₆-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, CO-O-C₁-C₄-alkylene-aryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{Z,Y}⁷** is hydrogen or optionally substituted C₁-C₆-alkyl;
or the radicals R_{Z,Y}⁶ and R_{Z,Y}⁷ together with the nitrogen form a 3- to 7-membered, in each case optionally substituted, saturated or aromatic heterocycle which can contain one, two or three further different or identical heteroatoms selected from the group consisting of 0, N, and S;
**4.)** a 5- or 6-membered aromatic heterocycle which can contain one, two or three different or identical heteroatoms selected from the group consisting of 0, N, and S and which in each case can be substituted by **R_{Z,Y}¹, R_{Z,Y}²** and **R_{Z,Y}³**;
**5.)** an optionally substituted C₅-C₁₀- bi- or tricyclic, saturated hydrocarbon radical.

8. At least one 5-membered ring heteroaromatic compound of the general formula I according to one of Claims 1 to 7, and/or corresponding enantiomeric, diastereomeric and/or tautomeric forms thereof and/or pharmaceutically acceptable salts thereof, where the radicals **Q, a, R_{Q}¹, R_{Q}², R₁, R₂, R₃, X, Y, Z, R₄, R₅, R₆, R₇, R_{Z,Y}¹, R_{Z,Y}², R_{Z,Y}³ , R_{Z,Y}⁴, R_{Z,Y}⁵, R_{Z,Y}⁶, R_{Z,Y}⁷, R_{Z,Y}⁸** unless specified otherwise hereinbelow have the same meaning as described in one of Claims 1 to 7 and the radicals below possess the following definition:
**W** is: a radical of the general formula 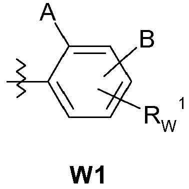
**A** is : F, Cl, OCF₃, OCHF₂, in each case optionally substituted C₁-C₆-alkyl, O-C₁-C₆-alkyl, S-C₁-C₆-alkyl or OR_{A}¹;
**B** is : hydrogen, F, Cl, CF₃, OCF₃, OCHF₂, in each case optionally substituted C₁-C₆-alkyl, 0-C₁-C₆-alkyl or S-C₁-C₆-alkyl;
**R_{W}¹** is: hydrogen, F, Cl, CN, CF₃ or O-CF₃;
**R_{A}¹** is: in each case optionally substituted C₁-C₆-alkyl or phenyl.

9. At least one 5-membered ring heteroaromatic compound of the general formula I according to one of Claims 1 to 8, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z},_{Y}⁸** unless specified otherwise hereinbelow have the same meaning as described in one of Claims 1 to 8 and the radicals below possess the following definition:
**Q** is : -CR_{Q}¹R_{Q}²-;
**R_{Q}¹, R_{Q}²** in each case independently of one another are hydrogen, F, or CH₃.

10. At least one 5-membered ring heteroaromatic compound of the general formula I as described in one of Claims 1 to 9, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², X, Y, Z, R₄, R₅, R₆, R₇, R_{Z,Y}¹, R_{Z,Y}², R_{Z,Y}³, R_{Z,Y}⁴_{,} R_{Z,Y}⁵_{,} R_{Z,Y}⁶, R_{Z,Y}⁷, R_{Z,Y}⁸** unless specified otherwise hereinbelow have the same meaning as described in one of Claims 1 to 9 and the radicals below possess the following definition:
**R¹, R²** independently of one another are:
hydrogen, OH, CN, O-methyl, 0-phenyl, acetyl, benzoyl, O-acetyl, or O-benzoyl;
R³ is a free electron pair or hydrogen.

11. At least one 5-membered ring heteroaromatic compound of the general formula I as described in one of Claims 1 to 10, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow have the same meaning as described in one of Claims 1 to 10 and the radicals below possess the following definition:
**R¹, R²** are hydrogen
**R³** is a free electron pair or hydrogen.

12. At least one 5-membered ring heteroaromatic compound of the general formula I as described in one of Claims 1 to 11, corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow have the same meaning as described in one of Claims 1 to 11 and the radicals below possess the following definition:
**X, Y, Z** X = N, Y = CR⁴, Z = CR⁶.

13. At least one 5-membered ring heteroaromatic compound of the general formula I according to one of Claims 1 to 12, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, X, Y, Z** unless specified otherwise hereinbelow have the same meaning as described in one of Claims 1 to 12 and the radicals below possess the following definition:
**R⁴, R⁶** in each case independently of one another are a radical selected from the identical or different radicals of the groups **1.)**, **3.), 4.)** or **5.)** and
**R⁵, R⁷** is a radical selected from the identical or different
radicals of the groups **2.)** or **3.)**, where the groups 1.), 2.), 3.), 4.) and 5.) are defined as follows:
1.) hydrogen, CF₃, CHF₂, or
in each case optionally substituted C₁-C₁₀-alkyl, C₃-C₇-Cycloalkyl, C₁-C₆-alkylene-C₃-C₇-cycloalkyl, C₁-C₄-alkylene-aryl, C₁-C₄-alkylene-hetaryl, or in each case optionally substituted
NR_{Z,Y}⁷-CO-O-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
2.) a free electron pair or hydrogen, or in each case optionally substituted C₁-C₁₀-alkyl, C₃-C₇-cycloalkyl;
3.) phenyl or naphthyl, which in each case is substituted by **R_{Z,Y}¹, R_{Z,Y}²** and **R_{Z,Y}³,** where
**R_{Z,Y}¹, R_{Z,Y}²** and **R_{Z,Y}³** in each case independently of one another represent a substituent from the following group:
hydrogen, CN, NH₂, CF₃, CHF₂, OCF₃, OCHF₂, 0-CH₂-COOH, halogen, or
in each case optionally substituted aryl, hetaryl, heterocycloalkyl, C₁-C₆-alkyl, C₃-C₇-cycloalkyl or in each case optionally substituted
O-R_{Z,Y}⁴, NR_{Z,Y}⁶R_{Z,Y}⁷, SO₂-R_{Z,Y}⁶R_{Z,Y}⁷, NR_{Z,Y}⁷-SO₂-R_{Z,Y}⁵ or
in each case two of the radicals from **R_{Z,Y}¹**, **R_{Z,Y}²** or **R_{Z,Y}³** together with the ring atoms which carry these substituents form a 3- to 7-membered, in each case optionally substituted, saturated or unsaturated carbocycle, or an in each case optionally substituted, saturated or unsaturated heterocycle, which can contain one, two or three further different or identical heteroatoms selected from the group consisting of 0, N, and S; where
**R_{Z,Y}⁴** is in each case optionally substituted hetaryl, aryl,
or
C₁-C₆-alkyl, which is optionally substituted by a substituent selected from the group consisting of halogen, CN, CF₃, CHF₂, OCF₃, OCHF₂, optionally substituted NH- (C₁-C₆-alkyl) and optionally substituted N(C₁-C₆-alkyl)₂;
**R_{Z,Y}⁵** is optionally substituted C₁-C₆-alkyl,
or
in each case aryl or hetaryl optionally substituted 1, 2 or up to 3 times, with substitution here by substituents independently of one another selected from the group consisting of halogen, NH₂, CN, CF₃, CHF₂, OCF₃, OCHF₂, NH-(C₁-C₆-alkyl) or N(C₁-C₆-alkyl)₂, or optionally substituted C₁-C₆-alkyl, or in each case optionally substituted O-R_{A}¹, NR_{A}²R_{A}³, SO₂NH₂, SO₂-R_{A}¹, NR_{A}⁴-SO₂-R_{A}¹, or SO₂-NR_{A}²R_{A}³;
**R_{Z,Y}⁶** is hydrogen, or
in each case optionally substituted C₁-C₆-alkyl, aryl, C₁-C₄-alkylene-aryl, C₁-C₄-alkylene-hetaryl, SO₂-C₁-C₆-alkyl, SO₂-aryl, SO₂-hetaryl or SO₂-C₁-C₄-alkylene-aryl;
**R_{Z,Y}⁷** is hydrogen or optionally substituted C₁-C₆-alkyl;
or the radicals R_{Z,Y}⁶ and R_{Z,Y}⁷ together with the nitrogen form a 3- to 7-membered, in each case optionally substituted, saturated or aromatic heterocycle which can contain one, two or three further different or
identical heteroatoms selected from the group consisting of 0, N, and S;
4.) a 5- or 6-membered aromatic heterocycle which can contain one, two or three different or identical heteroatoms selected from the group consisting of 0, N, and S and which in each case can be substituted by **R_{Z,Y}1, R_{Z,Y}²**, and **R_{Z,Y}³**;
5.) optionally substituted adamantyl.

14. At least one 5-membered ring heteroaromatic compound of the general formula I according to one of Claims 1 to 13, and/or corresponding enantiomeric, diastereomeric and/or tautomeric forms thereof and/or pharmaceutically acceptable salts thereof, where the radicals **R_{Q}¹, R_{Q}², R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** unless specified otherwise hereinbelow possess the following definitions:
**W** is: a radical of the general formula 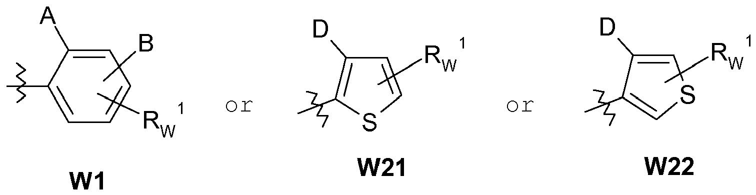
**A** is: NH₂, OCF₃, OCHF₂, COOH, O-CH₂-COOH, SH, or in each case optionally substituted O-C₁-C₆-alkyl, S-C₁-C₆-alkyl, C₁-C₆-alkyl, or in each case optionally substituted O-R_{A}¹, CO-RA¹, S-R_{A}¹, O-CH₂-COO-R_{A}1, NR_{A}²R_{A}³, CONH₂, SO₂NH₂, SO₂-R_{A}¹, NR_{A}⁴-SO₂-R_{A}¹, SO₂-NR_{A}²R_{A}³ or CO-NR_{A}²R_{A}³; where
**R_{A}¹** is: in each case optionally substituted C₁-C₆-alkyl, C₃-C₇-cycloalkyl, phenyl or benzyl;
**RA²**; **R_{A}³** independently of one another are
hydrogen, or
in each case optionally substituted C₁-C₆-alkyl, phenyl, benzyl, phenethyl, CO-C₁-C₆-alkyl, CO-aryl, CO-O-C₁-C₆-alkyl, SO₂-C₁-C₄-alkyl, SO₂-aryl, SO₂-hetaryl, or SO₂-C₁-C₄-alkylene-aryl;
or both radicals **R_{A}²** and **R_{A}³** together with the nitrogen form an in each case optionally substituted 5- or 6-membered saturated or unsaturated ring, which can contain one or up to two identical or different heteroatoms selected from the group consisting of 0 and N;
**R_{A}⁴** is: hydrogen or an optionally substituted C₁-C₆-alkyl radical;
**B** is: hydrogen, halogen, CN, CF₃, -CHF₂, OCF₃, OCHF₂, or
in each case optionally substituted C₁-C₆-alkyl or C₂-C₆-alkenyl, or in each case optionally substituted
-O-CH₂-COO-R_{A}¹, -O-R_{A}¹, S-R_{A}¹, NR_{A}²R_{A}³, -NR_{A}⁴-CO-R_{A}¹ or -CO-NR_{A}²R_{A}3; NR_{A}⁴-SO₂-R_{A}¹;
or B together with **R_{W}¹** forms one of the radicals - (CH₂)₃-, -O-CH₂-O-, -O-(CH₂) ₂-O-, -CH=CH-CH=CH-, -O-(CH₂)₂-, -(CH₂)₂-O;
**R_{W}¹** is: hydrogen, F, Cl, CN, CF₃, O-CF₃, or
in each case optionally substituted C₁-C₆-alkyl, aryl, C₁-C₆-alkylamino or C₁-C₆-dialkylamino;
**Q :**
**R_{Q}¹, R_{Q}²**, independently of one another are :
hydrogen, halogen, OH, optionally substituted C₁-C₆-alkyl;
**R¹, R²,** independently of one another are :
hydrogen, OH, CN, or in each case optionally substituted C₁-C₆-alkyl, C₁-C₆-alkylene-O-C₁-C₆-alkyl, substituted aryl, benzyl, CO-C₁-C₆-alkyl, CO-aryl, or CO-C₁-C₄-alkylene-aryl;
**R³** is a free electron pair or hydrogen;
**X, Y, Z** X = N, Y = CR⁴, Z = CR⁶, or
X = C, Y = CR⁴, Z = NR⁷, or
X = N, Y = N, Z = CR⁶
**R⁴, R⁶** in each case independently of one another are a radical selected from the identical or different radicals of the groups **1.), 3.), 4.)** or **5.)** and
**R⁵, R**⁷ in each case independently of one another are a radical selected from the identical or different radicals of the groups **2.), 3.)** or **4.)** or **5.),** with the following meanings for the groups 1.) to 5.):
1.) hydrogen, CN, CF₃, CHF₂, O-CH₂-COOH, halogen, or
in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, or C₁-C₄-alkylene-hetaryl, or
in each case optionally substituted O-R_{Z,Y}⁴, NR_{Z,Y}⁶, R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y} ⁶R_{Z,Y}⁷ ;
2.) a free electron pair or hydrogen,
in each case optionally substituted C₁-C₁₀-alkyl, C₃-C₇-cycloalkyl, C₁-C₆-alkylene-C₃-C₇-cycloalkyl, C₁-C₄-alkylene-aryl, C₁-C₄-alkylene-hetaryl, COO-C₁-C₆-alkyl or SO₂-R_{Z,Y}R⁵, SO₂NH₂, CONH₂, or in each case optionally substituted SO₂-NR_{Z,Y}⁶R_{Z,Y}⁷ or CO-NR_{Z,Y} ⁶R_{Z,Y}⁷ ;
3.) phenyl, 1-naphthyl or 2-naphthyl, which in each case are or can be substituted by **R_{Z,Y}¹, R_{Z,Y}²** and **R_{Z,Y}³,** where
**R_{Z,Y}¹, R_{Z,Y}²** and **R_{Z,Y}³** in each case independently of one another represent a substituent selected from the following group:
hydrogen, NH₂, CN, CF₃, CHF₂, OCF₃, OCHF₂, 0-CH₂-COOH, halogen, or
in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷, CO₂NH₂, or CO-NR_{Z,Y}⁶R_{Z,Y}⁷, or
in each case two of the radicals from **R_{Z,Y}¹**, **R_{Z,Y}²** or **R_{Z,Y}³** together with the ring atoms which carry this substituent form a 3- to 7-membered, in each case optionally substituted, saturated or unsaturated carbocycle, or an in each case optionally substituted, saturated or unsaturated heterocycle, which can contain one, two or three further different or identical heteroatoms selected from the group consisting of 0, N, and S;
**R_{Z,Y}⁴** is in each case optionally substituted C₁-C₄-alkylene-C₃-C₇-cycloalkyl, C₁-C₄-alkylene-heterocycloalkyl, heterocycloalkyl, hetaryl, aryl, C₁-C₄-alkylene-hetaryl, C₁-C₄-alkylene-aryl, or
C₁-C₆-alkyl, which optionally is substituted by a substituent selected from the group, consisting of halogen, NO₂, NH₂, OH, CN, CF₃, CHF₂, OCF₃, OCHF₂, optionally substituted NH-(C₁-C₆-alkyl) and optionally substituted N(C₁-C₆-alkyl)₂;
**R⁵_{Z,Y}** is in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, heterocycloalkyl or C₁-C₆-alkylene-O-C₁-C₆-alkyl, dioxymethylenephenyl, or
in each case aryl or hetaryl optionally substituted 1, 2 or up to 3 times, with substitution by substituents independently of one another selected from the group consisting of halogen, NO₂, NH₂, OH, CN, CF₃, CHF₂, OCF₃, OCHF₂, optionally substituted NH-(C₁-C₆-alkyl) or optionally substituted N(C₁-C₆-alkyl)₂, COOH, O-CH₂-COOH, SH, or
in each case optionally substituted C₁-C₆-alkyl, C₃-C₇-cycloalkyl, aryle, hetaryl, heterocycloalkyl, or
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, or SO₂-NR_{A}²R_{A}³;
**R_{Z,Y}⁶** is hydrogen, or
in each case optionally substituted C₁-C₆-alkyl, C₃-C₇-Cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, C₁-C₄-alkylene-aryl, C₁-C₄-alkylene-hetaryl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-C₁-C₄-alkylene-hetaryl, CO-O-C₁-C₄-alkylene-aryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{Z,Y}⁷** is hydrogen or optionally substituted C₁-C₆-alkyl;
or the radicals R_{Z,Y}⁶ and R_{Z,Y}⁷ together with the nitrogen form a 3- to 7-membered, in each case optionally substituted, saturated or aromatic heterocycle which can contain one, two or three further different or identical heteroatoms selected from the group consisting of 0, N, and S;
4.) a 5- or 6-membered aromatic heterocycle which can contain one, two or three different or identical heteroatoms selected from the group consisting of 0, N, and S and which in each case can be substituted by **R_{Z,Y}¹, R_{Z,Y}²** and **R_{Z,Y}³** as defined above;
5.) an optionally substituted C₅-C₁₀- bi- or tricyclic, saturated hydrocarbon radical.

15. At least one 5-membered ring heteroaromatic compound of the general formula I according to one of Claims 1 to 14, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, X, Y, Z** unless specified otherwise hereinbelow possess the following definitions:
**W** is: a radical of the general formula 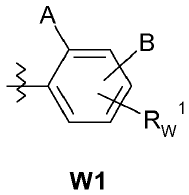
**A** is: F, Cl, OCF₃, OCHF₂, in each case optionally substituted C₁-C₆-alkyl, O-C₁-C₆-alkyl or S-C₁-C₆-alkyl or optionally substituted OR_{A}¹;
**R_{A}¹** is: in each case optionally substituted C₁-C₆-alkyl or phenyl
**B** is: hydrogen, F, Cl, CF₃, OCF₃, OCHF₂, or in each case optionally substituted C₁-C₆-alkyl, O-C₁-C₆-alkyl or S-C₁-C₆-alkyl;
**R_{W}¹** is: hydrogen, F, Cl, CN, CF₃ or O-CF₃;
**Q** is: -CR_{Q}¹R_{Q}²-;
**R_{Q}¹, R_{Q}²** in each case independently of one another are hydrogen, F, CH₃;
**R¹, R²** independently of one another are: hydrogen, OH, CN, O-methyl, 0-phenyl, acetyl, benzoyl, O-acetyl, O-benzoyl;
**R³** is a free electron pair or hydrogen
**X, Y, Z** X = N, Y = CR⁴, Z = CR⁶, or
X = N, Y = N, Z = CR⁶
**R⁴, R⁶** in each case independently of one another are a radical selected from the identical or different radicals of the groups **1.), 3.), 4.)** or **5.)** and
**R⁵** is a radical selected from the identical or different
radicals of the groups **2.)** or **3.),** where the groups 1.) to 5.) are:
1.) hydrogen, CN, CF₃, CHF₂, O-CH₂-COOH, halogen, or
in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, or in each case optionally substituted O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
2.) a free electron pair or hydrogen,
in each case optionally substituted C₁-C₁₀-alkyl, C₃-C₇-cycloalkyl, C₁-C₆-alkylene-C₃-C₇-cycloalkyl, C₁-C₄-alkylene-aryl, C₁-C₄-alkylene-hetaryl, COO-C₁-C₄-alkyl or SO₂-R⁵, SO₂NH₂, CONH₂, optionally substituted SO₂-NR_{Z,Y}⁶R_{Z,Y}⁷ or optionally substituted CO-NR_{Z,Y}⁶R_{Z,Y}⁷;
3.) phenyl, 1-naphthyl or 2-naphthyl, which can in each case be substituted by **R_{Z,Y}¹, R_{Z,Y}²** and **R_{Z,Y}³,** where
**R_{Z,Y}¹, R_{Z,Y}²** and **R_{Z,Y}³** in each case independently of one another represent a substituent from the following group:
hydrogen, NH₂, CN, CF₃, CHF₂, OCF₃, OCHF₂, 0-CH₂-COOH, halogen, or
in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷ or CO-NR_{Z,Y}⁶R_{Z,Y}⁷, or
in each case two of the radicals from **R_{Z,Y}¹, R_{Z,Y}²** or **R_{Z,Y}³** together with the ring atoms which carry these substituents form a 3- to 7-membered, in each case optionally substituted, saturated or unsaturated carbocycle, or an in each case optionally substituted, saturated or unsaturated heterocycle, which can contain one, two or three further different or identical heteroatoms selected from the group consisting of 0, N, and S;
**R_{Z,Y}⁴** is in each case optionally substituted C₁-C₄-alkylene-C₃-C₇-cycloalkyl, C₁-C₄-alkylene-heterocycloalkyl, heterocycloalkyl, hetaryl, aryl, C₁-C₄-alkylene-hetaryl, C₁-C₄-alkylene-aryl, or
C₁-C₆-alkyl, which optionally is substituted by a substituent from the group, consisting of halogen, NO₂, NH₂, OH, CN, CF₃, CHF₂, OCF₃, OCHF₂, optionally substituted NH-(C₁-C₆-alkyl) and optionally substituted N(C₁-C₆-alkyl)₂;
**R_{Z,Y}⁵** is in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, heterocycloalkyl or C₁-C₆-alkylene-O-C₁-C₆-alkyl, dioxymethylenephenyl, or
in each case aryl or hetaryl optionally substituted 1, 2 or up to 3 times, being substituted by substituents independently of one another selected from the group consisting of halogen, NO₂, NH₂, OH, CN, CF₃, CHF₂, OCF₃, OCHF₂, optionally substituted NH-(C₁-C₆-alkyl) or optionally substituted N(C₁-C₆-alkyl)₂, COOH, O-CH₂-COOH, SH, or
in each case optionally substituted C₁-C₆-alkyl, C₃-C₇-cycloalkyl, aryl, hetaryl, heterocycloalkyl, or in each case optionally substituted O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, or SO₂-NR_{A}²R_{A}³;
**R_{Z,Y}⁶** is hydrogen, or in each case optionally substituted C₁-C₆-alkyl, C₃-C₇-Cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, C₁-C₄-alkylene-aryl, C₁-C₄-alkylene-hetaryl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, CO-O-C₁-C₄-alkylene-aryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{Z,Y}⁷** is hydrogen or optionally substituted C₁-C₆-alkyl;
or the radicals R_{Z,Y}⁶ and R_{Z,Y}⁷ together with the nitrogen form a 3- to 7-membered, in each case optionally substituted, saturated or aromatic heterocycle which can contain one, two or three further different or identical heteroatoms selected from the group consisting of 0, N, and S;
4.) a 5- or 6-membered aromatic heterocycle which can contain one, two or three different or identical heteroatoms selected from the group consisting of 0, N, and S and which in each case can be substituted by **R_{Z,Y}¹, R_{Z,Y}²** and **R_{Z,Y}³** as defined above;
5.) optionally substituted adamantyl.

16. At least one 5-membered ring heteroaromatic compound of the general formula I according to one of Claims 1 to 15, and/or corresponding enantiomeric, diastereomeric and/or tautomeric forms thereof and/or pharmaceutically acceptable salts thereof, where the radicals **W, A, R_{A}¹, R_{A}², R_{A}³, R_{A}⁴, B, R_{W}¹, D, Q, a, R_{Q}¹, R_{Q}², R₁, R₂, R₃, X, Y, Z** unless specified otherwise hereinbelow possess the following definitions:
**W** is: a radical of the general formula 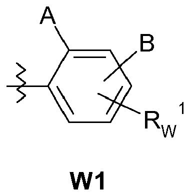
**A** is: OCF₃, OCHF₂, OCH₃, methyl, O-ethyl, 0-propyl or O-i-propyl;
**B** is: hydrogen, F, Cl, CF₃, OCF₃, OCHF₂, optionally substituted C₁-C₆-alkyl or optionally substituted O-C₁-C₆-alkyl;
**R_{W}¹** is: hydrogen, F, Cl;
**Q** is: -CH₂-;
**R¹, R²** are hydrogen,
**R³** is a free electron pair or hydrogen
**X** is N
**Y** is CR⁴
**Z** is CR⁶
**R⁴, R⁶** in each case independently of one another are a radical selected from the identical or different radicals of the groups **1.)**, **2.)** or **3.)**, where the groups 1.) to 3.) are:
1.) hydrogen, CF₃, CHF₂, or
in each case optionally substituted C₁-C₁₀-alkyl, or
2.) phenyl, 1-naphthyl or 2-naphthyl, which in each case are substituted by **R_{Z,Y}1, R_{Z,Y}²** and **R_{Z,Y}³,** where
**R_{Z,Y}¹, R_{Z,Y}²** and **R_{Z,Y}³** in each case independently of one another represent a substituent from the following group:
hydrogen, CN, CF₃, CHF₂, OCF₃, OCHF₂, O-CH₂-COOH, halogen, or
in each case optionally substituted C₁-C₆-alkyl, aryl, or
in each case optionally substituted O-R_{Z,Y}⁴, NR_{Z,Y}⁶ R_{Z,Y}7, NR_{Z,Y}⁷-SO₂-R_{Z,Y}⁵ or
in each case two of the radicals from **R_{Z,Y}¹**, **R_{Z,Y}²** or **R_{Z,Y}³** together form one of the radicals
-O-CH₂-O-, -O-CH₂-CH₂-O-, -O-CH₂-CH₂- or - CH₂-CH₂-CH₂-;
**R_{Z,Y}⁴** is in each case optionally substituted hetaryl, aryl,
or C₁-C₆-alkyl;
**R_{Z,Y}⁵** optionally substituted C₁-C₆-alkyl, or in each case aryl or hetaryl optionally substituted 1, 2 or up to 3 times, being substituted by substituents independently of one another selected from the group consisting of halogen, NH₂, CN, CF₃, CHF₂, OCF₃, OCHF₂, optionally substituted NH-(C₁-C₆-alkyl) or optionally substituted N(C₁-C₆-alkyl)₂, or
optionally substituted C₁-C₆-alkyl, or
in each case optionally substituted O-R_{A}¹, NR_{A}²R_{A}³, SO₂NH₂, SO₂-R_{A}¹, NR_{A}⁴-SO₂-R_{A}¹, or SO₂-NR_{A}²R_{A}³;
**R_{Z,Y}⁶** is hydrogen, or in each case optionally substituted C₁-C₆-alkyl, aryl, SO₂-C₁-C₆-alkyl, SO₂-aryl;
**R_{Z,Y}⁷** is hydrogen or optionally substituted C₁-C₆-alkyl;
3.) benzothiophenyl, benzofuranyl, quinolinyl, isoquinolinyl, thienyl, furanyl, pyridinyl, pyrimidinyl, or thiazolyl which in each case can be substituted by **R_{Z,Y}¹, R_{Z,Y}²** and **R_{Z,Y}³.**

17. At least one 5-membered ring heteroaromatic compound of the general formula I according to one of Claims 1 to 16, and/or corresponding enantiomeric, diastereomeric and/or tautomeric forms thereof and/or pharmaceutically acceptable salts thereof, where the radicals R¹, R², R³, Q, W, X, Y and Z should have the meanings as mentioned in one of Claims 1 to 16, for use as medicaments.

18. Pharmaceutical composition comprising at least one 5-membered ring heteroaromatic compound of the general formula I according to one of Claims 1 to 16, and/or corresponding enantiomeric, diastereomeric and/or tautomeric forms thereof and/or pharmaceutically acceptable salts thereof, where the radicals R¹, R², R³, Q, W, X, Y and Z should have the meanings as mentioned in one of Claims 1 to 16, and optionally at least one pharmaceutically acceptable carrier and/or diluent.

19. Use of at least one 5-membered ring heteroaromatic compound of the general formula I 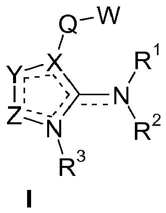 and/or corresponding enantiomeric, diastereomeric and/or tautomeric forms thereof and/or pharmaceutically acceptable salts thereof, where the radicals R¹, R², R³, Q, W, X, Y and Z are defined as follows:
**W** is: a radical of the general formula **W11** or **W2** 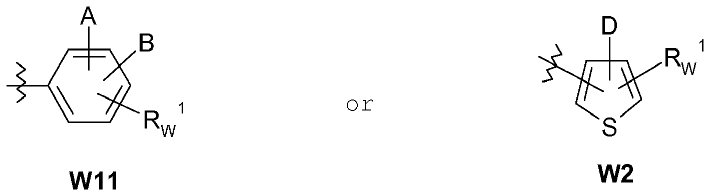
in which
**A** is: NO₂, NH₂, CN, CF₃, OCF₃, CHF₂, OCHF₂, COOH, 0-CH₂-COOH, halogen, SH, or in each case optionally substituted O-C₁-C₆-alkyl, S-C₁-C₆-alkyl, -O-CO-C₁-C₆-alkyl, -0-CO-aryl, -O-CO-hetaryl, -O-COO-C₁-C₆-alkyl or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-hetaryl or C₁-C₄-alkylene-aryl, or in each case optionally substituted
O-R_{A}¹, CO-R_{A}¹, S-R_{A}¹, SO-R_{A}¹, CO-O-R_{A}¹, NR_{A}⁴-COO-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ or CO-NR_{A}²R_{A}³ ;
**R_{A}¹** is:in each case optionally substituted C₁-C₆-Alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₂-C₆-alkenylene-aryl or C₁-C₆-alkylene-hetaryl;
**R_{A}²** is:hydrogen, or
in each case optionally substituted C₁-C₆-alkyl, C₂-C₆-alkenyl, C₂-C₆-alkynyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₁-C₄-alkylene-hetaryl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, COO-C₁-C₆-alkyl, CO-O-aryl, CO-O-C₁-C₄-alkylene-aryl, CO-0-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{A}³** is:in each case optionally substituted C₁-C₆-alkyl, C₂-C₆-alkenyl, C₂-C₆-alkynyl, C₁-C₄-alkylene-C₃-C₇-cycloalkyl, C₁-C₄-alkylene-heterocycloalkyl, aryl, hetaryl, heterocycloalkyl, C₁-C₄-alkylene-aryl, C₁-C₄-alkylene-hetaryl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, COO-C₁-C₆-alkyl, CO-O-aryl, CO-O-C₁-C₄-alkylene-aryl, CO-0-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
or the radicals **R_{A}²** and **R_{A}³** together with the nitrogen form a 3- to 7-membered, in each case optionally substituted, saturated or aromatic heterocycle, which can contain one, two or three further different or identical heteroatoms selected from the group consisting of 0, N, and S; where optionally two radicals substituted on this heterocycle together can form an optionally substituted 3- to 7-membered fused, saturated, unsaturated or aromatic carbocycle or heterocycle, where the heterocycle can contain up to three different or identical heteroatoms selected from the group consisting of 0, N, and S, and where the cycle formed thereby can optionally be substituted or a further, optionally 3- to 7-membered in each case optionally substituted cycle can be fused to this cycle;
**R_{A}⁴** is: hydrogen, or
in each case optionally substituted C₁-C₆-alkyl, C₂-C₆-alkenyl, C₂-C₁₂-alkynyl, C₃-C₇-cycloalkyl, aryl, C₁-C₄-alkylene-aryl, C₁-C₆-alkylene-O-C₁-C₆-alkyl, hetaryl;
**B** is: hydrogen, OH or independently of **A** is
defined as radical **A,**
or two of the radicals **A, B** or **R_{W}¹** in each case independently of one another together with the attached C atoms form a 3- to 7-membered, in each case optionally substituted, saturated, unsaturated or aromatic carbocycle or an in each case optionally substituted 3- to 7-membered in each case optionally substituted, saturated, unsaturated or aromatic heterocycle, which can contain one, two or three further different or identical heteroatoms selected from the group consisting of 0, N, and S, where in each case optionally two radicals substituted on this carbo- or heterocycle together can form an in each case optionally substituted 3- to 7-membered fused, saturated, unsaturated or aromatic carbocycle or heterocycle, where the heterocycle can contain up to three different or identical heteroatoms selected from the group consisting of 0, N, and S and where the cycle formed can optionally be substituted;
**R_{W}¹** is: hydrogen, OH, halogen, NO₂, NH₂, CN, CF₃, CHF₂, CH₂F, OCF₃, OCHF₂, OCH₂F or
in each case optionally substituted C₁-C₆-alkyl, C₃-C₇-cycloalkyl, C₁-C₆-alkylene-O-C₁-C₆-alkyl, C₁-C₆-thioalkyl, aryl, hetaryl, O-C₁-C₆-alkyl, O-benzyl, C₁-C₆-alkylamino, C₁-C₆-dialkylamino, pyrrolidinyl, piperidinyl, morpholinyl, CO-C₁-C₆-alkyl, SO₂-C₁-C₆-alkyl, CO-aryl, SO₂-aryl, CO-C₁-C₄-alkylene-aryl, SO₂-C₁-C₄-alkylene-aryl, SO-aryl, CONH₂, CONH-C₁-C₆-alkyl, SO₂NH-C₁-C₆-alkyl, CON-(C₁-C₆-alkyl)₂, SO₂N-(C₁-C₆-alkyl)₂, NH-SO₂-C₁-C₆-alkyl or NH-CO-C₁-C₆-alkyl ;
**D:** independently of A is defined as radical **A;**
**Q** is: a radical of the general formula **Q1** 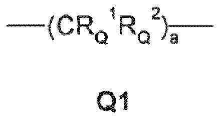 having the index
a = 1
**R_{Q}¹, R_{Q}²** independently of one another are :
hydrogen, halogen, OH, or optionally substituted C₁-C₆-alkyl;
**R¹**, **R²** independently of one another are:
hydrogen, OH, CN, or
in each case optionally substituted C₁-C₆-alkyl, O-C₁-C₆-alkyl, C₁-C₆-alkylene-O-C₁-C₆-alkyl, C₃-C₇-cycloalkyl, O-C₃-C₇-cycloalkyl, aryl, hetaryl, C₁-C₄-alkylene-aryl, C₁-C₄-alkylene-hetaryl, O-aryl, O-C₁-C₄-alkylene-aryl, 0-hetaryl, O-C₁-C₄-alkylene-hetaryl, CO-C₁-C₆-alkyl, CO-aryl, CO-hetaryl, CO-C₁-C₄-alkylene-aryl, CO-C₁-C₄-alkylene-hetaryl, COO-C₁-C₆-alkyl, CO-O-aryl, CO-0-hetaryl, CO-O-C₁-C₄-alkylene-aryl, SO₂-C₁-C₆-alkyl, SO₂-aryl, SO₂-hetaryl, SO₂-C₁-C₄-alkylene-aryl, OCO-C₁-C₆-alkyl, OCO-aryl, OCO-hetaryl, OCO-C₁-C₄-alkylene-aryl, OCO-C₁-C₄-alkylene-hetaryl, SO₂-C₁-C₆-alkyl, SO₂-aryl, SO₂-hetaryl or SO₂-C₁-C₄-alkylene-aryl, or
**R¹** and **R²** together with the nitrogen form a 5-to 7-membered, in each case optionally substituted, saturated or unsaturated heterocycle, which can contain one, two or three further different or identical heteroatoms selected from the group consisting of 0, N, and S, where in each case optionally two radicals substituted on this carbo- or heterocycle together can form a 3-to 7-membered fused, saturated, unsaturated or aromatic in each case optionally substituted carbocycle or heterocycle, where the heterocycle can contain one, two or up to three different or identical heteroatoms selected from the group consisting of 0, N, and S and where the cycle formed can optionally be substituted;
**R³** is a free electron pair or hydrogen;
**X, Y, Z** X = N, Y = CR⁴, Z = CR⁶, or
X = C, Y = NR⁵, Z = CR⁶, or
X = C, Y = CR⁴, Z = NR⁷, or
X = N, Y = N, Z = CR⁶, or
X = N, Y = CR⁴, Z = N
**R⁴, R⁶** in each case independently of one another are a radical selected from the identical or different radicals of the groups **1.), 3.)**, **4.), 5.)** or **6.)** and
**R⁵, R⁷** in each case independently of one another are a radical selected from the identical or different radicals of the groups **2.), 3.), 4.)** or **5.),** where the groups 1.) to 6.) are defined as follows:
**1.)** hydrogen, NO₂, NH₂, CN, CF₃, CHF₂, OCF₃, OCHF₂, COOH, O-CH₂-COOH, halogen, or in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, C₁-C₆-alkylene-O-C₁-C₆-alkyl, C₁-C₆-alkylene-O-aryl, COO-C₁-C₆-alkyl or C₁-C₄-alkylene-COO-C₁-C₆-alkyl, or in each case optionally substituted
O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-O-R_{Z,Y}⁵, O-CH₂-COO-R_{Z,Y}⁵, NR_{Z,Y}⁷-COR_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
**2.)** a free electron pair or hydrogen, CH₂-CF₃, CH₂-CHF₂,
in each case optionally substituted C₁-C₁₀-alkyl, C₂-C₁₀-alkenyl, C₂-C₁₀-alkynyl, C₃-C₇-cycloalkyl, C₁-C₆-alkylene-C₃-C₇-cycloalkyl, C₁-C₄-alkylene-aryl, C₁-C₄-alkylene-hetaryl, C₁-C₆-alkylene-O-C₁-C₆-alkyl, C₁-C₆-alkylene-O-aryl, COO-C₁-C₆-alkyl or C₁-C₄-alkylene-COO-C₁-C₆-alkyl, or in each case optionally substituted
CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷ or CO-NR_{Z,Y}⁶R_{Z,Y}⁷;
**3.)** phenyl, 1-naphthyl or 2-naphthyl, which can in each case be substituted by **R_{Z,Y}¹**, **R_{Z,Y}²** and **R_{Z,Y}³**, where
**R_{Z,Y}¹**, **R_{Z,Y}²** and **R_{Z,Y}³** i n each case independently of one another represent a substituent from the following group:
hydrogen, NO₂, NH₂, OH, CN, CF₃, CHF₂, OCF₃, OCHF₂, COOH, O-CH₂-COOH, SH, halogen, or in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}⁷-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ or CO-NR_{Z,Y}⁶R_{Z,Y}⁷, or
in each case two of the radicals from **R_{Z,Y}¹**, **R_{Z,Y}²** or **R_{Z,Y}³** together with the ring atoms which carry these substituents form a 3- to 7-membered, in each case optionally substituted, saturated or unsaturated carbocycle, or an in each case optionally substituted, saturated or unsaturated heterocycle, which can contain one, two or three further different or identical heteroatoms selected from the group consisting of 0, N, and S, where in each case optionally two radicals substituted on this carbo- or heterocycle together can form a 3- to 7-membered, fused, saturated, unsaturated or aromatic in each case optionally substituted carbocycle or heterocycle, where the heterocycle can contain one, two or up to three different or identical heteroatoms selected from the group consisting of 0, N, and S and where the cycle formed can optionally be substituted and/or a further, 3- to 7-membered, optionally substituted cycle can be fused to this cycle;
**R_{Z,Y}⁴** is in each case optionally substituted C₂-C₆-alkenyl, C₂-C₆-alkynyl, C₁-C₄-alkylene-C₃-C₇-cycloalkyl, C₁-C₄-alkylene-heterocycloalkyl, heterocycloalkyl, hetaryl, aryl, C₁-C₄-alkylene-hetaryl, C₁-C₄-alkylene-aryl, or
C₁-C₆-alkyl which is optionally substituted by a substituent from the group consisting of halogen, NO₂, NH₂, OH, CN, CF₃, CHF₂, OCF₃, OCHF₂, optionally substituted NH-(C₁-C₆-alkyl) or optionally substituted N(C₁-C₆-alkyl)₂;
**R_{Z,Y}⁵** is in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, heterocycloalkyl or C₁-C₆-alkylene-O-C₁-C₆-alkyl, dioxymethylenephenyl, benzofuranyl, dihydrobenzofuranyl, indanyl, or
in each case aryl or hetaryl optionally substituted 1, 2 or up to 3 times, with substitution by substituents independently of one another selected from the group consisting of halogen, NO₂, NH₂, OH, CN, CF₃, CHF₂, OCF₃, OCHF₂, optionally substituted NH-(C₁-C₆-alkyl) or optionally substituted N(C₁-C₆-alkyl)₂, COOH, O-CH₂-COOH, SH, or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₄-alkylene-hetaryl or C₁-C₄-alkylene-aryl, or in each case optionally substituted O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ or CO-NR_{A}²R_{A}³;
**R_{Z,Y}⁶** is hydrogen, or
in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, C₁-C₄-alkylene-aryl, C₁-C₄-alkylene-hetaryl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-0-hetaryl, CO-O-C₁-C₄-alkylene-hetaryl, CO-O-C₁-C₄-alkylene-aryl, SO₂-C₁-C₆-alkyl, SO₂-aryl, SO₂-hetaryl, SO₂-C₁-C₄-alkylene-aryl or SO₂-C₁-C₄-alkylene-hetaryl;
**R_{Z,Y}⁷** is hydrogen or in each case optionally substituted C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, aryl, hetaryl, heterocycloalkyl, C₁-C₆-alkylene-O-C₁-C₆-alkyl, CO-C₁-C₆-alkyl, CO-aryl, CO-hetaryl, CO-C₁-C₄-alkylene-aryl, CO-C₁-C₄-alkylene-hetaryl, CO-O-C₁-C₆-alkyl, CO-O-aryl, CO-O-C₁-C₄-alkylene-aryl, CO-O-hetaryl, CO-O-C₁-C₄-alkylene-hetaryl, SO₂-C₁-C₆-alkyl, SO₂-aryl, SO₂-hetaryl, SO₂-C₁-C₄-alkylene-aryl or SO₂-C₁-C₄-alkylene-hetaryl;
or the radicals R_{Z,Y}⁶ and R_{Z,Y}⁷ together with the nitrogen form a 3- to 7-membered, in each case optionally substituted, saturated or aromatic heterocycle, which can contain one, two or three further different or identical heteroatoms selected from the group consisting of 0, N, and S; and in each case optionally two radicals substituted on this heterocycle together can form a 3- to 7-membered, fused, saturated, unsaturated or aromatic in each case optionally substituted carbocycle or heterocycle, where the heterocycle can contain up to three different or identical heteroatoms selected from the group consisting of 0, N, and S, and the cycle formed can optionally be substituted and/or a further, optionally substituted 3- to 7-membered cycle can be fused to this cycle;
**4.)** a 5- or 6-membered aromatic heterocycle which can contain one, two or three different or identical heteroatoms selected from the group consisting of 0, N, and S and which in each case can be substituted by **R_{Z,Y}¹**, **R_{Z,Y}²** and **R_{Z,Y}³**, which independently of one another are as defined above;
**5.)** an in each case optionally substituted C₅-C₁₈- bi- or tricyclic, saturated hydrocarbon radical
**6.) R⁴** and **R⁶** together with X and Y form a C₆-C₁₀-membered saturated, unsaturated or aromatic optionally substituted carbocycle, preferably an optionally substituted benzo radical, which in each case can be substituted by one, two or up to three radicals, independently of one another selected from the group **R_{Z,Y}⁸**.
**R_{Z,Y}⁸:** hydrogen, NO₂, NH₂, OH, CN, CF₃, CHF₂, OCF₃, OCHF₂, COOH, O-CH₂-COOH, SH, halogen, or
in each case optionally substituted aryl, hetaryl, heterocycloalkyl, C₁-C₆-alkyl, C₂-C₆-alkenyl, C₂-C₆-alkynyl, C₃-C₇-cycloalkyl, C₁-C₄-alkylene-C₃-C₇-cycloalkyl, C₁-C₄-alkylene-heterocycloalkyl, C₁-C₄-alkylene-aryl or C₁-C₄-alkylene-hetaryl, or in each case optionally substituted
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ or CO-NR_{Z,Y}⁶R_{Z,Y}⁷
for the preparation of a medicament for the treatment and/or prophylaxis of CNS disorders or of CNS related disorders.

20. Use of at least one 5-membered ring heteroaromatic compound of the general formula I according to one of Claims 1 to 16 or Claim 19, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of CNS disorders and/or of CNS related disorders.

21. Use of at least one 5-membered ring heteroaromatic compound of the general formula I according to one of Claims 1 to 16 or Claim 19 or 20, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of neuropathological, neuropsychiatric and/or neurodegenerative disorders, symptoms and/or dysfunctions.

22. Use of at least one 5-membered ring heteroaromatic compound of the general formula I according to one of Claims 1 to 16 or 19, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of migraine and/or brain damage.

23. Use of at least one 5-membered ring heteroaromatic compound of the general formula I according to one of Claims 1 to 16 or 19, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis, of neuropathological, neuropsychiatric and/or neurodegenerative illnesses selected from the group consisting of cerebral ischemia, stroke, epilepsy and attacks in general, psychoses, schizophrenia, autism, OCD syndrome, cognitive disorders, attention disorders, depression, bipolar and/or unipolar depression, anxiety states, dementia, senile dementia, Alzheimer's dementia, demyelinizing disorders, multiple sclerosis and brain tumors.

24. Use of at least one 5-membered ring heteroaromatic compound of the general formula I according to one of Claims 1 to 16 or 19, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of illnesses selected from the group consisting of cerebrovascular disorders, pain, pain-related disorders, dependence, drug-related disorders, amnesia, alcohol abuse, drug abuse, disorders of the circadian rhythm and Cushing's syndrome.

25. Use of at least one 5-membered ring heteroaromatic compound of the general formula I according to one of Claims 1 to 16 or one of Claims 19 to 24, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of illnesses which are modulated by a 5-HT5 receptor activity.

26. Use of at least one 5-membered ring heteroaromatic compound of the general formula I according to one of Claims 1 to 16 or one of Claims 19 to 25, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of illnesses, which are modulated by a 5-HT5 receptor activity and where the treatment and/or prophylaxis is based on a selectivity for the 5-HT5A receptor.

27. Use of at least one 5-membered ring heteroaromatic compound of the general formula I according to one of Claims 1 to 16 or one of Claims 19 to 25, and/or corresponding enantiomeric, diastereomeric and/or tautomeric forms thereof and/or pharmaceutically acceptable salts thereof, where the radicals R¹, R², R³, Q, W, X, Y and Z are defined as explained in one of Claims 1 to 16 or 19, for the preparation of a medicament for the treatment and/or prophylaxis of illnesses which are modulated by a 5-HT5-receptor activity and where the treatment and/or prophylaxis is based on a selectivity for the 5-HT5A receptor with a binding affinity (Ki) of less than or equal to 600 nM.

28. Use according to one of Claims 19 to 27, where the treatment and/or prophylaxis is carried out in a human or nonhuman mammal.

## Revendications

1. Composé ou composés hétéroaromatiques à cycle à 5 éléments de formule générale I 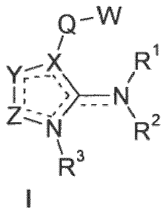 et/ou leurs formes énantiomères, diastéréomères et/ou tautomères correspondantes et/ou leurs sels pharmaceutiquement acceptables, les radicaux donnés ayant les définitions suivantes :
**W** : un radical de formule générale **W1** ou **W2** 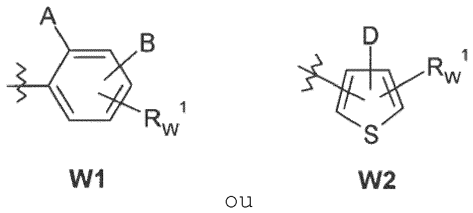 dans lesquelles
**A :** NO₂, NH₂, CN, CF₃, OCF₃, CHF₂, OCHF₂, COOH, O-CH₂-COOH, halogène, SH ; ou O-alkyle en C₁-C₆, S-alkyle en C₁-C₆, -O-CO-alkyle en C₁-C₆, -0-CO-aryle, -O-CO-hétaryle, -O-COO-alkyle en C₁-C₆, à chaque fois éventuellement substitué, ou
alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-hétaryle ou alkylène en C₁-C₄-aryle, à chaque fois éventuellement substitué, ou
O-R_{A}¹, CO-R_{A}¹, S-R_{A}¹, SO-R_{A}¹, CO-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ ou CO-NR_{A}²R_{A}³, à chaque fois éventuellement substitué ;
**R_{A}¹ :** alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alcénylène en C₂-C₆-aryle, alkylène en C₁-C₆-hétaryle, à chaque fois éventuellement substitué ;
**R_{A}² :** hydrogène, ou
alkyle en C₁-C₆, alcényle en C₂-C₆, alcynyle en C₂-C₆, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alkylène en C₁-C₄-hétaryle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{A}³ :** alkyle en C₁-C₆, alcényle en C₂-C₆, alcynyle en C₂-C₆, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alkylène en C₁-C₄-hétaryle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
ou les radicaux **R_{A}²** et **R_{A}³** forment ensemble avec l'azote un hétérocycle saturé ou aromatique, de 3 à 7 éléments, à chaque fois éventuellement substitué, qui peut contenir un, deux ou trois hétéroatomes supplémentaires identiques ou différents du groupe 0, N, S ; deux radicaux substitués sur cet hétérocycle pouvant éventuellement former ensemble un carbocycle ou hétérocycle saturé, insaturé ou aromatique, annelé, de 3 à 7 éléments, l'hétérocycle pouvant contenir jusqu'à trois hétéroatomes 0, N, S identiques ou différents, et le cycle ainsi formé pouvant éventuellement être substitué, ou un cycle supplémentaire de 3 à 7 éléments, éventuellement substitué, pouvant être condensé sur ce cycle ;
**R_{A}⁴ :** hydrogène ou
alkyle en C₁-C₆, alcényle en C₂-C₆, alcynyle en C₂-C₁₂, cycloalkyle en C₃-C₇, aryle, alkylène en C₁-C₄-aryle, alkylène en C₁-C₆-O-alkyle en C₁-C₆, hétaryle, à chaque fois éventuellement substitué ;
**B :** hydrogène ou, indépendamment du radical **A**, est défini comme le radical **A**,
ou deux des radicaux **A**, **B** ou **R_{W}¹** forment, à chaque fois indépendamment les uns des autres, ensemble avec les atomes C attachés un carbocycle saturé, insaturé ou aromatique, de 3 à 7 éléments, à chaque fois éventuellement substitué, ou un hétérocycle saturé, insaturé ou aromatique, de 3 à 7 éléments, à chaque fois éventuellement substitué, qui peut contenir un, deux ou trois hétéroatomes supplémentaires identiques ou différents choisis dans le groupe constitué par 0, N et S, deux radicaux substitués sur ce carbo- ou hétérocycle pouvant à chaque fois éventuellement former ensemble un carbocycle ou hétérocycle saturé, insaturé ou aromatique, annelé, de 3 à 7 éléments, l'hétérocycle pouvant contenir jusqu'à trois hétéroatomes 0, N, S identiques ou différents et le cycle formé pouvant éventuellement être substitué ;
**R_{W}¹ :** hydrogène, OH, halogène, NO₂, NH₂, CN, CF₃, CHF₂, CH₂F, OCF₃, OCHF₂, OCH₂F ou
alkyle en C₁-C₆, cycloalkyle en C₃-C₇, alkylène en C₁-C₆-O-alkyle en C₁-C₆, thioalkyle en C₁-C₆, aryle, hétaryle, O-alkyle en C₁-C₆, O-benzyle, alkylamino en C₁-C₆, dialkylamino en C₁-C₆, pyrrolidinyle, pipéridinyle, morpholinyle, CO-alkyle en C₁-C₆, SO₂-alkyle en C₁-C₆, CO-aryle, SO₂-aryle, CO-alkylène en C₁-C₄-aryle, SO₂-alkylène en C₁-C₄-aryle, SO-aryle, CONH₂, CONH-alkyle en C₁-C₆, SO₂NH-alkyle en C₁-C₆, CON-(alkyle en C₁-C₆)₂, SO₂N-(alkyle en C₁-C₆)₂, NH-SO₂-alkyle en C₁-C₆ ou NH-CO-alkyle en C₁-C₆, à chaque fois éventuellement substitué ;
éventuellement à condition que lorsque W = W1, alors R_{W}¹ ou B ne puisse pas être un radical N-pyrrolidinyle éventuellement substitué en position 4 (position para) par rapport à la position de liaison avec Q ;
**D** : hydrogène ou, indépendamment de A, est défini comme le radical A ;
**Q** : un radical de formule générale **Q1**
- (CR_{Q}¹R_{Q}²)ₐ- **Q1**
avec l'indice
a ₌ 1
**R_{Q}¹, R_{Q}²,** indépendamment l'un de l'autre :
hydrogène, halogène, OH ou alkyle en C₁-C₆ éventuellement substitué ;
**R¹**, **R²**, indépendamment l'un de l'autre :
hydrogène, OH, CN, ou
alkyle en C₁-C₆, O-alkyle en C₁-C₆, alkylène en C₁-C₆-O-alkyle en C₁-C₆, cycloalkyle en C₃-C₇, O-cycloalkyle en C₃-C₇, aryle, hétaryle, alkylène en C₁-C₄-aryle, alkylène en C₁-C₄-hétaryle, 0-aryle, O-alkylène en C₁-C₄-aryle, 0-hétaryle, O-alkylène en C₁-C₄-hétaryle, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-hétaryle, CO-0-alkylène en C₁-C₄-aryle, SO₂-alkyle en C₁-C₆, SO₂-aryle, SO₂-hétaryle, SO₂-alkylène en C₁-C₄-aryle, OCO-alkyle en C₁-C₆, OCO-aryle, OCO-hétaryle, OCO-alkylène en C₁-C₄-aryle, OCO-alkylène en C₁-C₄-hétaryle, SO₂-alkyle en C₁-C₆, SO₂-aryle, SO₂-hétaryle ou SO₂-alkylène en C₁-C₄-aryle, à chaque fois éventuellement substitué, ou
**R¹** et **R²** forment ensemble avec l'azote un hétérocycle saturé ou insaturé, à chaque fois éventuellement substitué, de 5 à 7 éléments, qui peut contenir un, deux ou trois hétéroatomes supplémentaires identiques ou différents du groupe 0, N, S, deux radicaux substitués sur ce carbo- ou hétérocycle pouvant à chaque fois éventuellement former ensemble un carbocycle ou hétérocycle saturé, insaturé ou aromatique, annelé, de 3 à 7 éléments, à chaque fois éventuellement substitué, l'hétérocycle pouvant contenir jusqu'à trois hétéroatomes identiques ou différents choisis dans le groupe constitué par 0, N et S, et le cycle formé pouvant éventuellement être substitué ;
**R³** paire d'électrons libres ou hydrogène ;
**X, Y, Z** ont à chaque fois ensemble la signification suivante :
X = N, Y = CR⁴, Z = CR⁶ ou
X = C, Y = NR⁵, Z = CR⁶ ou
X = C, Y = CR⁴, Z = NR⁷ ou
X = N, Y = N, Z = CR⁶ ou
X = N, Y = CR⁴, Z = N,
**R⁴, R⁶** à chaque fois indépendamment l'un de l'autre, un radical choisi dans des groupes identiques ou différents parmi les groupes **1.), 3.), 4.), 5.)** ou **6.)** et
**R⁵**, **R⁷** à chaque fois indépendamment l'un de l'autre, un radical choisi dans des groupes identiques ou différents parmi les groupes **2.)**, **3.)**, **4.)** ou **5.),**
**1.)** hydrogène, NO₂, NH₂, CN, CF₃, CHF₂, OCF₃, OCHF₂, COOH, O-CH₂-COOH, halogène, ou
alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, alkylène en C₁-C₆-O-alkyle en C₁-C₆, alkylène en C₁-C₆-O-aryle, COO-alkyle en C₁-C₆ ou alkylène en C₁-C₄-COO-alkyle en C₁-C₆, à chaque fois éventuellement substitué, ou
O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷-CO-OR_{Z,Y}⁵, O-CH₂-COO-R_{Z,Y}⁵, NR_{Z,Y}⁷-CO-R_{Z,Y}⁵, SO₂-R_{Z,Y}5, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**2.)** paire d'électrons libres ou hydrogène, CH₂-CF₃, CH₂-CHF₂ ; alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, alkylène en C₁-C₆-O-alkyle en C₁-C₆, alkylène en C₁-C₆-O-aryle, COO-alkyle en C₁-C₆ ou alkylène en C₁-C₄-COO-alkyle en C₁-C₆, à chaque fois éventuellement substitué, ou
CO-OR_{Z,Y}⁵, CO-R_{Z,Y}⁵, SO₂-R_{Z,Y}⁵, SO-R_{Z,Y}⁵, SO₂NH₂, CONH₂, SO₂-NR_{Z,Y}⁶R_{Z,Y}⁷, ou CO-NR_{Z,Y}⁶R_{Z,Y}⁷, à chaque fois éventuellement substitué ;
**3.)** phényle, 1-naphtyle ou 2-naphtyle, qui à chaque fois sont ou peuvent être substitués avec un, deux ou trois radicaux choisis parmi R_{Z,Y}¹, R_{Z,Y}² et **R_{Z,Y}³**,
**R_{Z,Y}¹**, **R_{Z,Y}²** et **R_{Z,Y}³** représentant à chaque fois indépendamment les uns des autres un substituant du groupe suivant :
hydrogène, NO₂, NH₂, OH, CN, CF₃, CHF₂, OCF₃, OCHF₂, COOH, O-CH₂-COOH, SH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}⁷-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué, ou à chaque fois deux des radicaux choisis dans le groupe constitué par **R_{Z,Y}¹**, **R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, de 3 à 7 éléments, à chaque fois éventuellement substitué, qui peut contenir un, deux ou trois hétéroatomes supplémentaires identiques ou différents choisis dans le groupe constitué par 0, N et S, deux radicaux substitués sur ce carbo- ou hétérocycle pouvant à chaque fois éventuellement former ensemble un carbocycle ou hétérocycle saturé, insaturé ou aromatique, annelé, de 3 à 7 éléments, l'hétérocycle pouvant contenir 1, 2 ou jusqu'à trois hétéroatomes identiques ou différents choisis dans le groupe constitué par 0, N, S, et le cycle formé pouvant éventuellement être substitué et/ou un cycle supplémentaire d e 3 à 7 éléments, éventuellement substitué, pouvant être condensé sur ce cycle ;
**R_{Z,Y}⁴** signifiant alcényle en C₂-C₆, alcynyle en C₂-C₆, alkylène en C₁-C₄-cycloalkyle en C₃-C₇, alkylène en C₁-C₄-hétérocycloalkyle, hétérocycloalkyle, alkylène en C₁-C₄-hétaryle, hétaryle, alkylène en C₁-C₄-aryle, aryle, à chaque fois éventuellement substitué, ou
alkyle en C₁-C₆, qui est à chaque fois éventuellement substitué avec un substituant choisi dans le groupe constitué par halogène, NO₂, NH₂, OH, CN, CF₃, CHF₂, OCF₃, OCHF₂, NH-(alkyle en C₁-C₆) éventuellement substitué ou N(alkyle en C₁-C₆)₂ éventuellement substitué ;
**R_{Z,Y}⁵** signifiant alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, hétérocycloalkyle ou alkylène en C₁-C₆-O-alk yle e n C₁-C₆, dioxyméthylènephényle, benzofuranyle, dihydrobenzofuranyle, indanyle, à chaque fois éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NO₂, NH₂, OH, CN, CF₃, CHF₂, OCF₃, OCHF₂, NH- (alkyle en C₁-C₆) éventuellement substitué ou N(alkyle en C₁-C₆)₂ éventuellement substitué, ainsi que COOH, O-CH₂-COOH, SH ; ou alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-hétaryle ou alkylène en C₁-C₄-aryle, à chaque fois éventuellement substitué, ou
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ ou CO-NR_{A}²R_{A}³, à chaque fois éventuellement substitué ;
**R_{Z,Y}⁶** signifiant hydrogène, ou
alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, alkylène en C₁-C₄-aryle, alkylène en C₁-C₄-hétaryle, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, CO-0-alkylène en C₁-C₄-aryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{Z,Y}⁷** signifiant hydrogène ; ou alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-O-alkyle en C₁-C₆, CO-O-aryle, CO-O-alkylène en C₁-C₄-aryle, CO-O-hétaryle, CO-O-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
ou les radicaux R_{z,Y}⁶ et R_{Z,Y}⁷ formant ensemble avec l'azote un hétérocycle saturé ou aromatique, de 3 à 7 éléments, à chaque fois éventuellement substitué, qui peut contenir un, deux ou trois hétéroatomes supplémentaires identiques ou différents choisis dans le groupe constitué par 0, N et S ; et deux radicaux substitués sur cet hétérocycle pouvant à chaque fois éventuellement former ensemble un carbocycle ou hétérocycle saturé, insaturé ou aromatique, annelé, de 3 à 7 éléments, l'hétérocycle pouvant contenir 1, 2 ou jusqu'à trois hétéroatomes identiques ou différents choisis dans le groupe constitué par 0, N et S, et le cycle formé pouvant éventuellement être substitué ou un cycle supplémentaire de 3 à 7 éléments, éventuellement substitué, pouvant être condensé sur ce cycle ;
**4.)** un hétérocycle aromatique de 5 ou 6 éléments, à chaque fois éventuellement substitué, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³** ; **R_{Z,Y}¹, R_{Z,Y}² et R_{Z,Y}³** ayant indépendamment les uns des autres les significations données précédemment en 3.) ;
**5.)** un radical hydrocarboné saturé bi- ou tricyclique en C₅-C₁₈, à chaque fois éventuellement substitué ;
**6.) R⁴** et **R⁶** forment ensemble avec X et Y un carbocycle saturé, insaturé ou aromatique, en C₆-C₁₀, à chaque fois éventuellement substitué, de préférence un radical benzo, qui peuvent à chaque fois être substitués avec un, deux ou jusqu'à trois radicaux choisis à chaque fois indépendamment les uns des autres parmi **R_{Z,Y}⁸** ;
**R_{Z,Y}⁸:** hydrogène, NO₂, NH₂, OH, CN, CF₃, CHF₂, OCF₃, OCHF₂, COOH, O-CH₂-COOH, SH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué,
éventuellement avec une, deux ou trois des conditions choisies dans le groupe constitué par les conditions (i), (ii) et (iii) suivantes :
(i) à condition que lorsque R⁴ et R⁶ ont les significations données dans le groupe 6.), alors les radicaux **A, B, Q, R_{Q}¹** et **R_{Q}²** sont définis de la manière suivante :
**A** se trouve dans W1 tel que donné en position 2 et signifie NH₂, OCF₃, OCHF₂, O-CH₂-COOH, O-alkyle en C₁-C₆, S-alkyle en C₁-C₆ ou O-R_{A}¹, CO-R_{A}¹, S-R_{A}¹, SO-R_{A}¹, SO₂-R_{A}¹, SO₂-NR_{A}²R_{A}³ ;
**B** se trouve dans W1 en position 6 et signifie halogène, CN, CF₃, -CHF₂, OCF₃, OCHF₂, ou
alkyle en C₁-C₆ ou alcényle en C₂-C₆, -O-CH₂-COO-R_{A}¹, -OR_{A}¹, S-R_{A}¹, NR_{A}²R_{A}³, -NR_{A}⁴-CO-R_{A}¹ ou -CO-NR_{A}²R_{A}³ ; NR_{A}⁴-SO₂-R_{A}¹ à chaque fois éventuellement substitué ;
ou B forme avec **R_{w}¹** un des radicaux - (CH₂)₃-, -O-CH₂-O-, -O-(CH₂)₂-O-, -CH=CH-CH=CH-, -O-(CH₂)₂-, -(CH₂)₂-O- ;
**Q** -CR_{Q}¹R_{Q}²-
**R_{Q}¹**, **R_{Q}²** indépendamment l'un de l'autre :
hydrogène, halogène, alkyle en C₁-C₆ éventuellement substitué ;
(ii) à condition que lorsque W = W1, alors R_{W}¹ ou B ne doit pas être un radical N-pyrrolidinyle éventuellement substitué en position 4 (position para) par rapport à la position de liaison avec Q ;
(iii) à condition que la somme de a, b et c soit de 1, 2, 3, 4 ou 5, lorsque X = N, Y = CR⁴ et Z = CR⁶.

2. Composé ou composés hétéroaromatiques à cycle à 5 éléments de formule générale I selon la revendication 1, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}², R₁, R₂, R₃, X, Y, Z** ayant, sauf indication contraire ci-après, la même signification que dans la revendication 1 et les radicaux suivants ayant les définitions suivantes :
**R⁴, R⁶** à chaque fois indépendamment l'un de l'autre, un radical choisi dans des groupes identiques ou différents parmi les groupes **1.), 3.), 4.) ou 5.)** et
**R⁵, R⁷** à chaque fois indépendamment l'un de l'autre, un radical choisi dans des groupes identiques ou différents parmi les groupes **2.)**, **3.)**, **4.) ou 5.)**, les groupes 1.) à 5.) devant à chaque fois avoir les significations données dans la revendication 1.

3. Composé ou composés hétéroaromatiques à cycle à 5 éléments de formule générale I selon l'une quelconque des revendications 1 ou 2, et/ou leurs formes énantiomères, diastéréomères et/ou tautomères correspondantes et/ou leurs sels pharmaceutiquement acceptables, les radicaux **Q, R_{Q}¹, R_{Q}², R₁, R₂, R₃, X, Y, Z, R₄, R₅, R₆, R₇ R_{Z,Y}¹, R_{Z,Y}², R_{Z,Y}³, R_{Z,Y}⁴, R_{Z,Y}⁵, R_{Z},_{Y}⁶**, **R_{Z,Y}⁷, R_{Z,Y}⁸** ayant, sauf indication contraire ci-après, la même signification que dans l'une quelconque des revendications 1 ou 2 et les radicaux suivants ayant la définition suivante :
**W :** signifie un radical de formule générale **W1, W21** ou **W22** 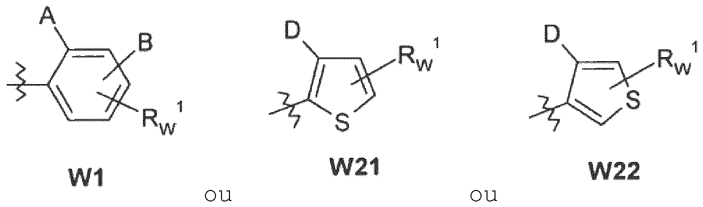 dans lesquelles les radicaux A, B, D et R_{W}¹ peuvent présenter indépendamment les uns des autres et à chaque fois indépendamment de leur occurrence respective une des significations suivantes :
**A :** halogène, NH₂, CN, CF₃, OCF₃, OCHF₂, COOH, O-CH₂-COOH, SH, O-alkyle en C₁-C₆, S-alkyle en C₁-C₆, ou
O-R_{A}¹, CO-R_{A}¹, S-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹, SO₂-NR_{A}²R_{A}³ ou CO-NR_{A}²R_{A}³, à chaque fois éventuellement substitué ;
**R_{A}¹ :** alkyle en C₁-C₆, cycloalkyle en C₃-C₇, phényle ou benzyle, à chaque fois éventuellement substitué ;
**R_{A}², R_{A}³ :** indépendamment l'un de l'autre hydrogène, ou
alkyle en C₁-C₆, phényle, benzyle, phénéthyle, CO-alkyle en C₁-C₆, CO-aryle, CO-0-alkyle en C₁-C₆, SO₂-alkyle en C₁-C₆, SO₂-aryle, SO₂-hétaryle ou SO₂-alkylène en C₁-C₄-aryle, à chaque fois éventuellement substitué ;
ou les deux radicaux **R_{A}²** et **R_{A}³** forment ensemble avec l'azote un cycle saturé ou insaturé de 5 ou 6 éléments, à chaque fois éventuellement substitué, qui peut contenir un ou jusqu'à deux hétéroatomes identiques ou différents choisis dans le groupe constitué par 0 et N ;
**R_{A}⁴ :** hydrogène ou un radical alkyle en C₁-C₆ éventuellement substitué ;
**B :** hydrogène, halogène, CN, CF₃, -CHF₂, OCF₃, OCHF₂ ; ou alkyle en C₁-C₆ ou alcényle en C₂-C₆ à chaque fois éventuellement substitué, ou
-O-CH₂-COO-R_{A}¹, -O-R_{A}¹, S-R_{A}¹, NR_{A}²R_{A}³, -NR_{A}⁴-CO-R_{A}¹ ou -CO-NR_{A}²R_{A}³ ; NR_{A}⁴-SO₂-R_{A}¹, à chaque fois éventuellement substitué ;
ou B forme avec **R_{W}¹** un des radicaux -(CH₂)₃-, -O-CH₂-O-, -O-(CH₂)₂-O-, -CH=CH-CH=CH-, -O-(CH₂)₂-, -(CH₂)₂-O- ;
**R_{W}¹ :** hydrogène, F, Cl, CN, CF₃, O-CF₃, ou
alkyle en C₁-C₆, aryle, alkylamino en C₁-C₆ ou dialkylamino en C₁-C₆, à chaque fois éventuellement substitué ;
**D:** hydrogène ou, indépendamment de A, est défini comme le radical **A.**

4. Composé ou composés hétéroaromatiques à cycle à 5 éléments de formule générale I selon l'une quelconque des revendications 1 à 3, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}1, R_{Q}², X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 3 et les radicaux suivants ayant la définition suivante :
**R¹, R²,** indépendamment l'un de l'autre
hydrogène, OH, CN, alkyle en C₁-C₆, alkylène en C₁-C₆-O-alkyle en C₁-C₆, aryle substitué, benzyle, CO-alkyle en C₁-C₆, CO-aryle ou CO-alkylène en C₁-C₄-aryle ;
**R³** paire d'électrons libres ou hydrogène.

5. Composé ou composés hétéroaromatiques à cycle à 5 éléments de formule générale I selon l'une quelconque des revendications 1 à 4, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 4 et les radicaux suivants ayant la définition suivante :
**X, Y, Z** X = N, Y = CR⁴, Z = CR⁶, ou
X = C, Y = CR⁴, Z = NR⁷, ou
X = N, Y = N, Z = CR⁶.

6. Composé ou composés hétéroaromatiques à cycle à 5 éléments de formule générale I selon l'une quelconque des revendications 1 à 5, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 5 et les radicaux suivants ayant la définition suivante :
**X, Y, Z** X = N, Y = CR⁴, Z = CR⁶, ou
X = N, Y = N, Z = CR⁶.

7. Composé ou composés hétéroaromatiques à cycle à 5 éléments de formule générale I selon l'une quelconque des revendications 1 à 6, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}², R₁, R₂, R₃, X, Y, Z** ayant, sauf indication contraire ci-après, la même signification que dans l'une quelconque des revendications 1 à 6 et les radicaux suivants ayant la définition suivante :
**R⁴, R⁶** à chaque fois indépendamment l'un de l'autre, un radical choisi dans des groupes identiques ou différents parmi les groupes **1.), 3.), 4.)** ou **5.)** et
**R⁵, R⁷** à chaque fois indépendamment l'un de l'autre, un radical choisi dans des groupes identiques ou différents parmi les groupes **2.), 3.)** ou **4.)** ou **5.),**
les groupes **1.), 2.), 3.), 4.)** et **5.)** étant définis de la manière suivante :
**1.)** hydrogène, CN, CF₃, CHF₂, O-CH₂-COOH, halogène, ou alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, à chaque fois éventuellement substitué, ou O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**2.)** paire d'électrons libres ou hydrogène,
alkyle en C₁-C₁₀, cycloalkyle en C₃-C₇, alkylène en C₁-C₆-cycloalkyle en C₃-C₇, alkylène en C₁-C₄-aryle, alkylène en C₁-C₄-hétaryle, COO-alkyle en C₁-C₆, à chaque fois éventuellement substitué, ou
SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**3.)** phényle, 1-naphtyle ou 2-naphtyle, qui à chaque fois sont substitués avec **R_{Z,Y}¹, R_{Z,Y}²** et **R_{Z,Y}**³ ;
**R_{Z,Y}¹, R_{Z,Y}²** et **R_{Z,Y}³** représentant à chaque fois indépendamment les uns des autres un substituant du groupe suivant :
hydrogène, NH₂, CN, CF₃, CHF₂, OCF₃, OCHF₂, O-CH₂-COOH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴, S-R_{Z,Y}⁴ NR_{Z,Y}⁶R_{Z,Y}⁷, NR_{Z,Y}-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué, ou à chaque fois deux des radicaux **R_{Z,Y}¹, R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, à chaque fois éventuellement substitué, qui peut contenir un, deux ou trois hétéroatomes supplémentaires identiques ou différents choisis dans le groupe constitué par 0, N et S ;
**R_{Z,Y}⁴** signifiant alkylène en C₁-C₄-cycloalkyle en C₃-C₇, alkylène en C₁-C₄-hétérocycloalkyle, hétérocycloalkyle, hétaryle, aryle, alkylène en C₁-C₄-hétaryle, alkylène en C₁-C₄-aryle, à chaque fois éventuellement substitué, ou alkyle en C₁-C₆, qui est éventuellement substitué avec un substituant du groupe constitué par halogène, NO₂, NH₂, OH, CN, CF₃, CHF₂, OCF₃, OCHF₂, NH-(alkyle en C₁-C₆) et N (alkyle en C₁-C₆)₂ ;
**R_{Z,Y}⁵** signifiant alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, hétérocycloalkyle ou alkylène en C₁-C₆-O-alkyle en C₁-C₆, dioxyméthylènephényle, à chaque fois éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NO₂, NH₂, OH, CN, CF₃, CHF₂, OCF₃, OCHF₂, NH-(alkyle en C₁-C₆) ou N (alkyle en C₁-C₆)₂, COOH, O-CH₂-COOH, SH, ou
alkyle en C₁-C₆, cycloalkyle en C₃-C₇, aryle, hétaryle, hétérocycloalkyle, à chaque fois éventuellement substitué, ou
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹ ou SO₂-NR_{A}²R_{A}³, à chaque fois éventuellement substitué ;
**R_{Z,Y}⁶** signifiant hydrogène, ou
alkyle en C₁-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, alkylène en C₁-C₄-aryle, alkylène en C₁-C₄-hétaryle, CO-aryle, CO-hétaryle, CO-alkylène en C₁-C₄-aryle, CO-alkylène en C₁-C₆-hétaryle, CO-0-alkyle en C₁-C₆, CO-O-aryle, CO-0-alkylène en C₁-C₄-aryle, CO-O-hétaryle, CO-O-alkylène en C₁-C₄-hétaryle, CO-0-alkylène en C₁-C₄-aryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{Z,Y}⁷** signifiant hydrogène, ou alkyle en C₁-C₆ éventuellement substitué ;
ou les radicaux R_{Z,Y}⁶ et R_{Z,Y}⁷ formant ensemble avec l'azote un hétérocycle saturé ou aromatique de 3 à 7 éléments, à chaque fois éventuellement substitué, qui peut contenir un, deux ou trois hétéroatomes supplémentaires identiques ou différents choisis dans le groupe constitué par 0, N et S ;
**4.)** un hétérocycle aromatique de 5 ou 6 éléments, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³** ;
**5.)** un radical hydrocarboné saturé bi- ou tricyclique en C₅-C₁₀, éventuellement substitué.

8. Composé ou composés hétéroaromatiques à cycle à 5 éléments de formule générale I selon l'une quelconque des revendications 1 à 7, et/ou leurs formes énantiomères, diastéréomères et/ou tautomères correspondantes et/ou leurs sels pharmaceutiquement acceptables, les radicaux **Q, a, R_{Q}¹, R_{Q}², R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 7 et les radicaux suivants ayant la définition suivante :
**W :** un radical de formule générale 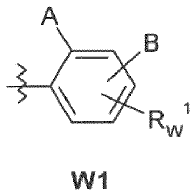
**A :** F, Cl, OCF₃, OCHF₂ ; alkyle en C₁-C₆, O-alkyle en C₁-C₆, S-alkyle en C₁-C₆ ou OR_{A}¹ à chaque fois éventuellement substitué ;
**B :** hydrogène, F, Cl, CF₃, OCF₃, OCHF₂ ; alkyle en C₁-C₆,
O-alkyle en C₁-C₆ ou S-alkyle en C₁-C₆ à chaque fois éventuellement substitué ;
**R_{W}¹ :** hydrogène, F, Cl, CN, CF₃ ou O-CF₃ ;
**R_{A}¹:** alkyle en C₁-C₆ ou phényle, à chaque fois éventuellement substitué.

9. Composé ou composés hétéroaromatiques à cycle à 5 éléments de formule générale I selon l'une quelconque des revendications 1 à 8 , et/ou leurs formes énantiomères, diastéréomères et/ou tautomères correspondantes et/ou leurs sels pharmaceutiquement acceptables, les radicau**x W, A, R_{A}¹, R_{A}², R_{A}³, R_{A}⁴, B, R_{W}¹, D, R₁, R₂, R₃, X, Y, Z, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 8 et les radicaux suivants ayant la définition suivante :
**Q :** -CR_{Q}¹R_{Q}²- ;
**R_{Q}¹, R_{Q}²** à chaque fois indépendamment l'un de l'autre, hydrogène, F ou CH₃.

10. Composé ou composés hétéroaromatiques à cycle à 5 éléments de formule générale I selon l'une quelconque des revendications 1 à 9, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}², X, Y, Z, R₄, R₅, R₆, R₇, R_{Z,Y}¹, R_{z},_{Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 9 et les radicaux suivants ayant la définition suivante :
**R¹, R²** indépendamment l'un de l'autre :
hydrogène, OH, CN, O-méthyle, O-phényle, acétyle, benzoyle, 0-acétyle ou O-benzoyle ;
**R³** paire d'électrons libres ou hydrogène.

11. Composé ou composés hétéroaromatiques à cycle à 5 éléments de formule générale I selon l'une quelconque des revendications 1 à 10, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R**_{W}¹, **D, Q, a, R_{Q}¹, R_{Q}², X, Y, Z, R₄, R₅, R₆, R₇, R_{Z,Y}¹, R_{Z,Y}²_{,} R_{z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 10 et les radicaux suivants ayant la définition suivante :
**R¹, R²** hydrogène ;
**R³** paire d'électrons libres ou hydrogène.

12. Composé ou composés hétéroaromatiques à cycle à 5 éléments de formule générale I selon l'une quelconque des revendications 1 à 11, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, RQ¹, R_{Q}², R₁, R₂, R₃, R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, la même signification que dans l'une quelconque des revendications 1 à 11 et les radicaux suivants ayant la définition suivante :
**X, Y, Z** X = N, Y = CR⁴, Z = CR⁶.

13. Composé ou composés hétéroaromatiques à cycle à 5 éléments de formule générale I selon l'une quelconque des revendications 1 à 12, et/ou leurs formes énantiomères, diastéréomères et/ou tautomères correspondantes et/ou leurs sels pharmaceutiquement acceptables, les radicaux **W, A, R_{A}¹, R_{A}², RA³, R_{A}⁴, B, R_{W}**¹, **D , Q , a, R_{Q}¹, R_{Q}² , R₁, R₂, R₃, X, Y, Z** ayant, sauf indication contraire ci-après, la même signification que dans l'une quelconque des revendications 1 à 12 et les radicaux suivants ayant la définition suivante :
**R⁴, R⁶** à chaque fois indépendamment l'un de l'autre, un radical choisi dans des groupes identiques ou différents parmi les groupes **1.), 3.), 4.)** ou **5.)** et
**R⁵, R⁷** à chaque fois indépendamment l'un de l'autre, un radical choisi dans des groupes identiques ou différents parmi les groupes **2.) ou 3.),** les groupes **1.), 2.), 3.), 4.)** et **5.)** étant définis de la manière suivante :
**1.)** hydrogène, CF₃, CHF₂, ou
alkyle en C₁-C₁₀, cycloalkyle en C₃-C₇, alkylène en C₁-C₆-cycloalkyle en C₃-C₇, alkylène en C₁-C₄-aryle, alkylène en C₁-C₄-hétaryle, à chaque fois éventuellement substitué, ou
NR_{Z,Y}⁷-CO-O-R_{Z,Y}, SO₂-R_{Z,Y}⁵, NR_{Z,Y}-SO₂-R_{Z,Y}, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**2.)** paire d'électrons libres ou hydrogène, ou
alkyle en C₁-C₁₀, cycloalkyle en C₃-C₇, à chaque fois éventuellement substitué ;
**3.)** phényle ou naphtyle, qui à chaque fois sont substitués avec **R_{Z,Y}¹, R_{Z,Y}²** et **R_{Z,Y}³;**
**R_{Z,Y}¹, R_{Z,Y}²** et **R_{Z,Y}³** représentant à chaque fois indépendamment les uns des autres un substituant du groupe suivant :
hydrogène, CN, NH₂, CF₃, CHF₂, OCF₃, OCHF₂, O-CH₂-COOH, halogène, ou
aryle, hétaryle, hétérocycloalkyle, alkyle en C₁-C₆, cycloalkyle en C₃-C₇, à chaque fois éventuellement substitué, ou
O-R_{Z,Y}⁴, NR_{Z,Y}R_{Z,Y}⁷, SO₂-R_{Z,Y}⁶R_{Z,Y}⁷, NR_{Z,Y}-SO₂-R_{Z,Y}⁵, à Chaque fois éventuellement substitué, ou
à chaque fois deux des radicaux **R_{Z,Y}¹, R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, à chaque fois éventuellement substitué, qui peut contenir un, deux ou trois hétéroatomes supplémentaires identiques ou différents choisis dans le groupe constitué par 0, N et S ;
**R_{Z,Y}⁴** signifiant hétaryle, aryle, à chaque fois éventuellement substitué, ou
alkyle en C₁-C₆, qui est éventuellement substitué avec un substituant choisi dans le groupe constitué par halogène, CN, CF₃, CHF₂, OCF₃, OCHF₂, NH- (alkyle en C₁-C₆) éventuellement substitué et N(alkyle en C₁-C₆)₂ éventuellement substitué ;
**R_{Z,Y}⁵** signifiant alkyle en C₁-C₆ éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NH₂, CN, CF₃, CHF₂, OCF₃, OCHF₂, NH- (alkyle en C₁-C₆) ou N(alkyle en C₁-C₆) ₂, ou alkyle en C₁-C₆ éventuellement substitué, ou
O-R_{A}¹, NR_{A}²R_{A}³, SO₂NH₂, SO₂-R_{A}¹, NR_{A}⁴-SO₂-R_{A}¹ ou SO₂-NR_{A}²R_{A}³, à chaque fois éventuellement substitué ;
**R_{Z,Y}⁶** signifiant hydrogène, ou
alkyle en C₁-C₆, aryle, alkylène en C₁-C₄-aryle, alkylène en C₁-C₄-hétaryle, SO₂-alkyle en C₁-C₆, SO₂-aryle, SO₂-hétaryle ou SO₂-alkylène en C₁-C₄-aryle, à chaque fois éventuellement substitué ;
**R_{Z,Y}⁷** signifiant hydrogène, ou alkyle en C₁-C₆ éventuellement substitué ;
ou les radicaux R_{Z,Y}⁶ et R_{Z,Y}⁷ formant ensemble avec l'azote un hétérocycle saturé ou aromatique de 3 à 7 éléments, à chaque fois éventuellement substitué, qui peut contenir un, deux ou trois hétéroatomes supplémentaires identiques ou différents choisis dans le groupe constitué par 0, N et S ;
**4.)** un hétérocycle aromatique de 5 ou 6 éléments, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³ ;**
**5.)** adamantyle éventuellement substitué.

14. Composé ou composés hétéroaromatiques à cycle à 5 éléments de formule générale I selon l'une quelconque des revendications 1 à 13, et/ou leurs formes énantiomères, diastéréomères et/ou tautomères correspondantes et/ou leurs sels pharmaceutiquement acceptables, les radicaux **R_{Q}¹, R_{Q}², R₄, R₅, R₆, R₇, R_{Z,Y}¹, R_{Z,Y}², R_{Z,Y}³, R_{Z,Y}⁴, R_{Z,Y}⁵, R_{Z,Y}⁶, R_{Z,Y}⁷, R_{Z,Y}⁸** ayant, sauf indication contraire ci-après, les définitions suivantes :
**W :** un radical de formule générale 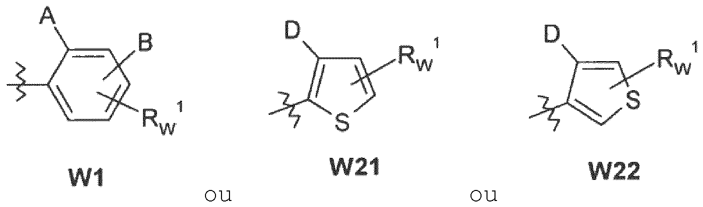
**A :** NH₂, OCF₃, OCHF₂, COOH, O-CH₂-COOH, SH ; ou O-alkyle en C₁-C₆, S-alkyle en C₁-C₆, alkyle en C₁-C₆ à chaque fois éventuellement substitué, ou
O-R_{A}¹, CO-R_{A}¹, S-R_{A}¹, O-CH₂-COO-R_{A}, NR_{A}²R_{A}³, CONH₂, SO₂NH₂, SO₂-R_{A}¹, NR_{A}⁴-SO₂-RA¹, SO₂-NR_{A}²R_{A}³ ou CO-NR_{A}²R_{A}³, à chaque fois éventuellement substitué ;
**R_{A}¹ :** alkyle en C₁-C₆, cycloalkyle en C₃-C₇, phényle ou benzyle, à chaque fois éventuellement substitué ;
**R_{A}², R_{A}³ :** indépendamment l'un de l'autre hydrogène, ou
alkyle en C₁-C₆, phényle, benzyle, phénéthyle, CO-alkyle en C₁-C₆, CO-aryle, CO-0-alkyle en C₁-C₆, SO₂-alkyle en C₁-C₄, SO₂-aryle, SO₂-hétaryle ou SO₂-alkylène en C₁-C₄-aryle, à chaque fois éventuellement substitué ;
ou les deux radicaux **R_{A}²** et **R_{A}³** forment ensemble avec l'azote un cycle saturé ou insaturé de 5 ou 6 éléments, à chaque fois éventuellement substitué, qui peut contenir un ou jusqu'à deux hétéroatomes identiques ou différents choisis dans le groupe constitué par 0 et N ;
**R_{A}⁴:** hydrogène ou un radical alkyle en C₁-C₆ éventuellement substitué ;
**B :** hydrogène, halogène, CN, CF₃, -CHF₂, OCF₃, OCHF₂ ; ou alkyle en C₁-C₆ ou alcényle en C₂-C₆ à chaque fois éventuellement substitué, ou
-O-CH₂-COO-R_{A}¹, -O-R_{A}¹, S-R_{A}¹, NR_{A}²R_{A}³, -NR_{A}⁴-CO-R_{A}¹ ou -CO-NR_{A}²R_{A}³ ; NR_{A}⁴-SO₂-R_{A}¹, à chaque fois éventuellement substitué ;
ou B forme avec **R_{W}¹** un des radicaux -(CH₂)₃-, -O-CH₂-O-, -O-(CH₂)₂-O-, -CH=CH-CH=CH-, -O-(CH₂)₂-, -(CH₂)₂-O- ;
**R_{W}¹ :** hydrogène, F, Cl, CN, CF₃, O-CF₃, ou
alkyle en C₁-C₆, aryle, alkylamino en C₁-C₆ ou
dialkylamino en C₁-C₆, à chaque fois éventuellement substitué ;
**Q :**
**R_{Q}¹, R_{Q}²,** indépendamment l'un de l'autre:
hydrogène, halogène, OH, alkyle en C₁-C₆ éventuellement substitué ;
**R¹, R²,** indépendamment l'un de l'autre:
hydrogène, OH, CN ; ou alkyle en C₁-C₆, alkylène en C₁-C₆-O-alkyle en C₁-C₆, aryle substitué, benzyle, CO-alkyle en C₁-C₆, CO-aryle ou CO-alkylène en C₁-C₄-aryle, à chaque fois éventuellement substitué ;
**R³** paire d'électrons libres ou hydrogène ;
**X, Y, Z** X = N, Y = CR⁴, Z = CR⁶, ou
X = C, Y = CR⁴, Z = NR⁷, ou
X = N, Y = N, Z = CR⁶ ;
**R⁴, R⁶** à chaque fois indépendamment l'un de l'autre, un radical choisi dans des groupes identiques ou différents parmi les groupes **1.), 3.), 4.)** ou **5.)** et
**R⁵, R⁷** à chaque fois indépendamment l'un de l'autre, un radical choisi dans des groupes identiques ou différents parmi les groupes **2.), 3.)** ou **4.)** ou **5.),** avec les significations suivantes pour les groupes 1.) à 5.) :
**1.)** hydrogène, CN, CF₃, CHF₂, O-CH₂-COOH, halogène, ou alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle ou alkylène en C₁-C₄-hétaryle, à chaque fois éventuellement substitué, ou O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}, à chaque fois éventuellement substitué ;
**2.)** paire d'électrons libres ou hydrogène,
alkyle en C₁-C₁₀, cycloalkyle en C₃-C₇, alkylène en C₁-C₆-cycloalkyle en C₃-C₇, alkylène en C₁-C₄-aryle, alkylène en C₁-C₄-hétaryle, COO-alkyle en C₁-C₆, à chaque fois éventuellement substitué, ou
SO₂-R_{Z,Y}⁵, SO₂NH₂, CONH₂ ; ou SO₂-NR_{Z,Y}⁶R_{Z,Y}⁷ ou CO-NR_{Z,Y}⁶R_{Z,Y}⁷, à chaque fois éventuellement substitué ;
**3.)** phényle, 1-naphtyle ou 2-naphtyle, qui à chaque fois sont ou peuvent être substitués avec **R_{Z,Y}¹, R_{Z,Y}²** et **R_{Z,Y}³,**
**R_{Z,Y}¹, R_{Z,Y}²** et **R_{Z,Y}³** représentant à chaque fois indépendamment les uns des autres un substituant choisi dans le groupe suivant :
hydrogène, NH₂, CN, CF₃, CHF₂, OCF₃, OCHF₂, O-CH₂-COOH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷, CO₂NH₂ ou CO-NR_{Z,Y}⁶R_{Z,Y}⁷, à chaque fois éventuellement substitué, ou à chaque fois deux des radicaux **R_{Z,Y}¹, R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, à chaque fois éventuellement substitué, qui peut contenir un, deux ou trois hétéroatomes supplémentaires identiques ou différents choisis dans le groupe constitué par 0, N et S ;
**R_{Z,Y}⁴** signifiant alkylène en C₁-C₄-cycloalkyle en C₃-C₇, alkylène en C₁-C₄-hétérocycloalkyle, hétérocycloalkyle, hétaryle, aryle, alkylène en C₁-C₄-hétaryle, alkylène en C₁-C₄-aryle, à chaque fois éventuellement substitué, ou alkyle en C₁-C₆, qui est éventuellement substitué avec un substituant choisi dans le groupe constitué par halogène, NO₂, NH₂, OH, CN, CF₃, CHF₂, OCF₃, OCHF₂, NH-(alkyle en C₁-C₆) éventuellement substitué et N(alkyle en C₁-C₆)₂ éventuellement substitué ;
**R_{Z,Y}⁵** signifiant alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, hétérocycloalkyle ou alkylène en C₁-C₆-O-alkyle en C₁-C₆, dioxyméthylènephényle, à chaque fois éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NO₂, NH₂, OH, CN, CF₃, CHF₂, OCF₃, OCHF₂, NH-(alkyle en C₁-C₆) éventuellement substitué ou N(alkyle en C₁-C₆)₂ éventuellement substitué, COOH, O-CH₂-COOH, SH ; ou alkyle en C₁-C₆, cycloalkyle en C₃-C₇, aryle, hétaryle, hétérocycloalkyle, à chaque fois éventuellement substitué, ou
O-R_{A}¹, S-R_{A}¹, NR_{A}⁴-CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹ ou SO₂-NR_{A}²R_{A}³ ;
**R_{Z,Y}⁶** signifiant hydrogène, ou
alkyle en C₁-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, alkylène en C₁-C₄-aryle, alkylène en C₁-C₄-hétaryle, CO-aryle, CO-hétaryle, CO-alkylène en C₁-C₄-aryle, CO-alkylène en C₁-C₄-hétaryle, CO-0-alkyle en C₁-C₆, CO-O-aryle, CO-0-alkylène en C₁-C₄-aryle, CO-0-hétaryle, CO-O-alkylène en C₁-C₄-hétaryle, CO-0-alkylène en C₁-C₄-aryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{Z,Y}⁷** signifiant hydrogène, ou alkyle en C₁-C₆ éventuellement substitué ;
ou les radicaux R_{Z,Y}⁶ et R_{Z,Y}⁷ formant ensemble avec l'azote un hétérocycle saturé ou aromatique de 3 à 7 éléments, à chaque fois éventuellement substitué, qui peut contenir un, deux ou trois hétéroatomes supplémentaires identiques ou différents choisis dans le groupe constitué par 0, N et S ;
**4.)** un hétérocycle aromatique de 5 ou 6 éléments, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³** tels que définis précédemment ;
**5.)** un radical hydrocarboné saturé bi- ou tricyclique en C₅-C₁₀, éventuellement substitué.

15. Composé ou composés hétéroaromatiques à cycle à 5 éléments de formule générale I selon l'une quelconque des revendications 1 à 14, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}² , R₁, R₂, R₃, X, Y, Z** ayant, sauf indication contraire ci-après, les définitions suivantes :
**W :** un radical de formule générale 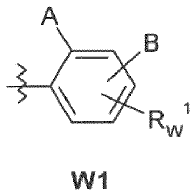
**A :** F, Cl, OCF₃, OCHF₂ ; alkyle en C₁-C₆, O-alkyle en C₁-C₆ ou S-alkyle en C₁-C₆, à chaque fois éventuellement substitué, ou OR_{A}¹ éventuellement substitué ;
**R_{A}¹ :** alkyle en C₁-C₆ ou phényle, à chaque fois éventuellement substitué
**B :** hydrogène, F, Cl, CF₃, OCF₃, OCHF₂ ; ou alkyle en C₁-C₆, O-alkyle en C₁-C₆ ou S-alkyle en C₁-C₆, à chaque fois éventuellement substitué ;
**R_{W}¹ :** hydrogène, F, Cl, CN, CF₃ ou O-CF₃ ;
**Q** : - CR_{Q}¹R_{Q}²- ;
**R_{Q}¹, R_{Q}²** à chaque fois indépendamment l'un de l'autre, hydrogène, F, CH₃ ;
**R¹, R²** indépendamment l'un de l'autre:
hydrogène, OH, CN, O-méthyle, O-phényle, acétyle, benzoyle, 0-acétyle, O-benzoyle ;
**R³** paire d'électrons libres ou hydrogène ;
**X, Y, Z** X = N, Y = CR⁴, Z = CR⁶, ou
X = N, Y = N, Z = CR⁶ ;
**R⁴, R⁶** à chaque fois indépendamment l'un de l'autre, un radical choisi dans des groupes identiques ou différents parmi les groupes **1.), 3.), 4.)** ou **5.)** et
**R⁵** un radical choisi dans des groupes identiques ou différents parmi les groupes **2.)** ou **3.),** les groupes 1.) à 5.) signifiant :
**1.)** hydrogène, CN, CF₃, CHF₂, O-CH₂-COOH, halogène, ou alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, à chaque fois éventuellement substitué, ou O-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**2.)** paire d'électrons libres ou hydrogène,
alkyle en C₁-C₁₀, cycloalkyle en C₃-C₇, alkylène en C₁-C₆-cycloalkyle en C₃-C₇, alkylène en C₁-C₄-aryle, alkylène en C₁-C₄-hétaryle, COO-alkyle en C₁-C₄, à chaque fois éventuellement substitué, ou SO₂-R_{Z,Y}⁵, S O₂NH₂, CONH₂, SO₂-NR_{Z,Y}⁶R_{Z,Y}⁷, éventuellement substitué ou CO-NR_{Z,Y}⁶R_{Z,Y}⁷ éventuellement substitué ;
**3.)** phényle, 1-naphtyle ou 2-naphtyle, qui à chaque fois peuvent être substitués avec **R_{Z,Y}¹, R_{Z,Y}²** et **R_{Z,Y}³; R_{Z,Y}¹, R_{Z,Y}²** et **R_{Z,Y}³** représentant à chaque fois indépendamment les uns des autres un substituant du groupe suivant :
hydrogène, NH₂, CN, CF₃, CHF₂, OCF₃, OCHF₂, O-CH₂-COOH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴, S-R_{Z,Y}⁴, NR_{Z,Y}⁶R_{Z,Y}⁷, NR_{Z,Y}⁷-CO-O-R_{Z,Y}⁵, NR_{Z,Y}⁷-CO-R_{Z,Y}⁵, SO₂-R_{Z,Y}⁵, NR_{Z,Y}⁷-SO₂-R_{Z,Y}⁵, SO₂NH₂, SO₂-NR_{Z,Y}⁶R_{Z,Y}⁷ ou CO-NR_{Z,Y}⁶R_{Z,Y}⁷, à chaque fois éventuellement substitué, ou à chaque fois deux des radicaux **R_{Z,Y}¹, R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, à chaque fois éventuellement substitué, qui peut contenir un, deux ou trois hétéroatomes supplémentaires identiques ou différents choisis dans le groupe constitué par 0, N et S ;
**R_{Z,Y}⁴** signifiant alkylène en C₁-C₄-cycloalkyle en C₃-C₇, alkylène en C₁-C₄-hétérocycloalkyle, hétérocycloalkyle, hétaryle, aryle, alkylène en C₁-C₄-hétaryle, alkylène en C₁-C₄-aryle, à chaque fois éventuellement substitué, ou alkyle en C₁-C₆, qui est éventuellement substitué avec un substituant du groupe constitué par halogène, NO₂, NH₂, OH, CN, CF₃, CHF₂, OCF₃, OCHF₂, NH-(alkyle en C₁-C₆) éventuellement substitué et N(alkyle en C₁-C₆)₂ éventuellement substitué ;
**R_{Z,Y}⁵** signifiant alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, hétérocycloalkyle ou alkylène en C₁-C₆-O-alkyle en C₁-C₆, dioxyméthylènephényle, à chaque fois éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NO₂, NH₂, OH, CN, CF₃, CHF₂, OCF₃, OCHF₂, NH-(alkyle en C₁-C₆) éventuellement substitué ou N(alkyle en C₁-C₆)₂ éventuellement substitué, COOH, O-CH₂-COOH, SH ; ou alkyle en C₁-C₆, cycloalkyle en C₃-C₇, aryle, hétaryle, hétérocycloalkyle, à chaque fois éventuellement substitué, ou
O-R_{A}¹, S-R_{A}¹, NR_{A}⁴ -CO-O-R_{A}¹, O-CH₂-COO-R_{A}¹, NR_{A}²R_{A}³, CONH₂, SO₂NH₂, SO₂-R_{A}¹, NR_{A}⁴-SO₂-R_{A}¹ ou SO₂-NR_{A}²R_{A}³, à chaque fois éventuellement substitué ;
**R_{Z,Y}⁶** signifiant hydrogène, ou
alkyle en C₁-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, alkylène en C₁-C₄-aryle, alkylène en C₁-C₄-hétaryle, CO-aryle, CO-hétaryle, CO-alkylène en C₁-C₄-aryle, CO-alkylène en C₁-C₄-hétaryle, CO-0-alkyle en C₁-C₆, CO-O-aryle, CO-0-alkylène en C₁-C₄-aryle, CO-0-hétaryle, CO-O-alkylène en C₁-C₄-hétaryle, CO-0-alkylène en C₁-C₄-aryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{Z,Y}⁷** signifiant hydrogène, ou alkyle en C₁-C₆ éventuellement substitué ;
ou les radicaux R_{Z,Y}⁶ et R_{Z,Y}⁷ formant ensemble avec l'azote un hétérocycle saturé ou aromatique de 3 à 7 éléments, à chaque fois éventuellement substitué, qui peut contenir un, deux ou trois hétéroatomes supplémentaires identiques ou différents choisis dans le groupe constitué par 0, N et S ;
**4.)** un hétérocycle aromatique de 5 ou 6 éléments, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³** tels que définis précédemment ;
5.) adamantyle éventuellement substitué.

16. Composé ou composés hétéroaromatiques à cycle à 5 éléments de formule générale I selon l'une quelconque des revendications 1 à 15, et/ou leurs formes énantiomères, diastéréomères et/ou tautomères correspondantes et/ou leurs sels pharmaceutiquement acceptables, les radicaux **W, A, R_{A}¹, R_{A}², R_{A}³, R_{A}⁴, B, R_{W}¹, D, Q, a, R_{Q}¹, R_{Q}² , R₁, R₂, R₃, X, Y, Z** ayant, sauf indication contraire ci-après, les définitions suivantes :
**W :** un radical de formule générale 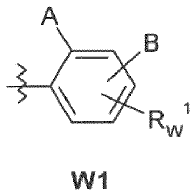
**A :** OCF₃, OCHF₂, OCH₃, méthyle, O-éthyle, O-propyle ou 0-ipropyle ;
**B :** hydrogène, F, Cl, CF₃, OCF₃, OCHF₂, alkyle en C₁-C₆ éventuellement substitué ou O-alkyle en C₁-C₆ éventuellement substitué ;
**R_{W}¹ :** hydrogène, F, Cl ;
**Q :** -CH₂- ;
**R¹, R²** hydrogène ;
**R³** paire d'électrons libres ou hydrogène ;
**X** N ;
**Y** CR⁴ ;
**Z** CR⁶ ;
**R⁴, R⁶** à chaque fois indépendamment l'un de l'autre, un radical choisi dans des groupes identiques ou différents parmi les groupes **1.), 2.)** ou **3.),** les groupes 1.) à 3.) signifiant :
**1.)** hydrogène, CF₃, CHF₂, ou
alkyle en C₁-C₁₀ à chaque fois éventuellement substitué, ou
**2.)** phényle, 1-naphtyle ou 2-naphtyle, qui à chaque fois sont substitués avec **R_{Z,Y}¹, R_{Z,Y}² et R_{Z,Y}³;**
**R_{Z,Y}¹, R_{Z,Y}²** et **R_{Z,Y}³** représentant à chaque fois indépendamment les uns des autres un substituant du groupe suivant :
hydrogène, CN, CF₃, CHF₂, OCF₃, OCHF₂, O-CH₂-COOH, halogène, ou
alkyle en C₁-C₆, aryle, à chaque fois éventuellement substitué, ou
O-R_{Z,Y}⁴, NR_{Z,Y}⁶R_{Z,Y}⁷, NR_{Z,Y}⁷- SO₂-R_{Z,Y}⁵, à chaque fois éventuellement substitué, ou
à chaque fois deux des radicaux **R_{Z,Y}¹, R_{Z,Y}²** ou **R_{Z,Y}³** formant ensemble un des radicaux
-O-CH₂-O-, -O-CH₂-CH₂-O-, -O-CH₂-CH₂- ou -CH₂-CH₂-CH₂- ;
**R_{Z,Y}⁴** hétaryle, aryle, à chaque fois éventuellement substitué,
ou alkyle en C₁-C₆ ;
**R_{Z,Y}⁵** alkyle en C₁-C₆ éventuellement substitué, ou aryle ou hétaryle, à chaque fois éventuellement substitué 1, 2 ou jusqu'à 3 fois, avec des substituants choisis indépendamment les uns des autres dans le groupe constitué par halogène, NH₂, CN, CF₃, CHF₂, OCF₃, OCHF₂, NH-(alkyle en C₁-C₆) éventuellement substitué ou N(alkyle en C₁-C₆)₂ éventuellement substitué, ou alkyle en C₁-C₆ éventuellement substitué, ou
O-R_{A}¹, NR_{A}²R_{A}³, SO₂NH₂, SO₂-R_{A}¹, NR_{A}⁴-SO₂-R_{A}¹ ou SO₂-NR_{A}²R_{A}³, à chaque fois éventuellement substitué ;
**R_{Z,Y}⁶** hydrogène, ou
alkyle en C₁-C₆, aryle, SO₂-alkyle en C₁-C₆, SO₂-aryle, à chaque fois éventuellement substitué ;
**R_{Z,Y}⁷** hydrogène ou alkyle en C₁-C₆ éventuellement substitué ;
**3.)** benzothiophényle, benzofuranyle, quinolinyle, isoquinolinyle, thiényle, furanyle, pyridinyle, pyrimidinyle ou thiazolyl, qui peuvent à chaque fois être substitués avec **R_{Z,Y}¹, R_{Z,Y}²** et **R_{Z,Y}³.**

17. Composé ou composés hétéroaromatiques à cycle à 5 éléments de formule générale I selon l'une quelconque des revendications 1 à 16, et/ou leurs formes énantiomères, diastéréomères et/ou tautomères correspondantes et/ou leurs sels pharmaceutiquement acceptables, les radicaux R¹, R², R³, Q, W, X, Y et Z devant avoir les significations données dans l'une quelconque des revendications 1 à 16, destinés à être utilisés en tant que médicament.

18. Composition pharmaceutique, contenant au moins un composé hétéroaromatique à cycle à 5 éléments de formule générale I selon l'une quelconque des revendications 1 à 16, et/ou ses formes énantiomères, diastéréomères et/ou tautomères correspondantes et/ou ses sels pharmaceutiquement acceptables, les radicaux R¹, R², R³, Q, W, X, Y et Z devant avoir les significations données dans l'une quelconque des revendications 1 à 16, ainsi qu'éventuellement au moins un véhicule e t / o u diluant pharmaceutiquement acceptable.

19. Utilisation d'au moins un composé hétéroaromatique à cycle à 5 éléments de formule générale I 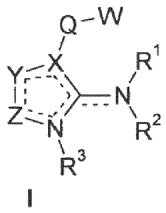 et/ou ses formes énantiomères, diastéréomères et/ou tautomères correspondantes et/ou ses sels pharmaceutiquement acceptables, les radicaux R¹, R², R³, Q, W, X, Y et Z étant définis de la manière suivante :
**W :** un radical de formule générale **W11** ou **W2** 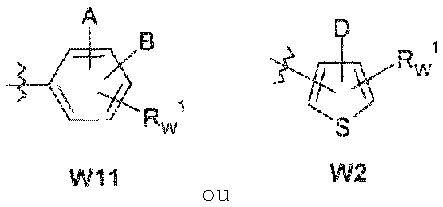 dans lesquelles
**A :** NO₂, NH₂, CN, CF₃, OCF₃, CHF₂, OCHF₂, COOH, O-CH₂-COOH, halogène, SH ; ou, O-alkyle en C₁-C₆, S-alkyle en C₁-C₆, -0-CO-alkyle en C₁-C₆, -0-CO-aryle, -O-CO-hétaryle, -O-COO-alkyle en C₁-C₆, à chaque fois éventuellement substitué, ou
alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-hétaryle ou alkylène en C₁-C₄-aryle, à chaque fois éventuellement substitué, ou
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ ou CO-NR_{A}²R_{A}³, à chaque fois éventuellement substitué ;
**R_{A}¹ :** alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alcénylène en C₂-C₆-aryle, alkylène en C₁-C₆-hétaryle, à chaque fois éventuellement substitué ;
**R_{A}² :** hydrogène, ou
alkyle en C₁-C₆, alcényle en C₂-C₆, alcynyle en C₂-C₆, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alkylène en C₁-C₄-hétaryle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{A}³** : alkyle en C₁-C₆, alcényle en C₂-C₆, alcynyle en C₂-C₆, alkylène en C₁-C₄-cycloalkyle en C₃-C₇, alkylène en C₁-C₄-hétérocycloalkyle, aryle, hétaryle, hétérocycloalkyle, alkylène en C₁-C₄-aryle, alkylène en C₁-C₄-hétaryle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
ou les radicaux **R_{A}²** et **R_{A}³** forment ensemble avec l'azote un hétérocycle saturé ou aromatique de 3 à 7 éléments, à chaque fois éventuellement substitué, qui peut contenir un, deux ou trois hétéroatomes supplémentaires identiques ou différents du groupe constitué par 0, N, S ; deux radicaux substitués sur cet hétérocycle pouvant éventuellement former ensemble un carbocycle ou hétérocycle saturé, insaturé ou aromatique, annelé, de 3 à 7 éléments, l'hétérocycle pouvant contenir jusqu'à trois hétéroatomes identiques ou différents choisis dans le groupe constitué par 0, N et S, et le cycle ainsi formé pouvant éventuellement être substitué ou un cycle supplémentaire éventuellement de 3 à 7 éléments, à chaque fois éventuellement substitué, pouvant être condensé sur ce cycle ;
**R_{A}⁴** : hydrogène ou
alkyle en C₁-C₆, alcényle en C₂-C₆, alcynyle en C₂-C₁₂, cycloalkyle en C₃-C₇, aryle, alkylène en C₁-C₄-aryle, alkylène en C₁-C₆-O-alkyle en C₁-C₆, hétaryle, à chaque fois éventuellement substitué ;
**B** : hydrogène, OH ou, indépendamment de **A,** est défini comme le radical **A,** ou deux des radicaux **A, B** ou **R_{W}¹** forment, à chaque fois indépendamment les uns des autres, ensemble avec les atomes C attachés un carbocycle saturé, insaturé ou aromatique, à chaque fois éventuellement substitué, de 3 à 7 éléments, ou un hétérocycle saturé, insaturé ou aromatique, de 3 à 7 éléments, à chaque fois éventuellement substitué, qui peut contenir un, deux ou trois hétéroatomes supplémentaires identiques ou différents choisis dans le groupe constitué par 0, N et S, deux radicaux substitués sur ce carbo- ou hétérocycle pouvant à chaque fois éventuellement former ensemble un carbocycle ou hétérocycle saturé, insaturé ou aromatique, annelé, de 3 à 7 éléments, à chaque fois éventuellement substitué, l'hétérocycle pouvant contenir jusqu'à trois hétéroatomes identiques ou différents choisis dans le groupe constitué par 0, N et S, et le cycle formé pouvant éventuellement être substitué ;
**R_{W}¹ :** hydrogène, OH, halogène, NO₂, NH₂, CN, CF₃, CHF₂, CH₂F, OCF₃, OCHF₂, OCH₂F ou
alkyle en C₁-C₆, cycloalkyle en C₃-C₇, alkylène en C₁-C₆-O-alkyle en C₁-C₆, thioalkyle en C₁-C₆, aryle, hétaryle, O-alkyle en C₁-C₆, O-benzyle, alkylamino en C₁-C₆, dialkylamino en C₁-C₆, pyrrolidinyle, pipéridinyle, morpholinyle, CO-alkyle en C₁-C₆, SO₂-alkyle en C₁-C₆, CO-aryle, SO₂-aryle, CO-alkylène en C₁-C₄-aryle, SO₂-alkylène en C₁-C₄-aryle, SO-aryle, CONH₂, CONH-alkyle en C₁-C₆, SO₂NH-alkyle en C₁-C₆, CON-(alkyle en C₁-C₆)₂, SO₂N-(alkyle en C₁-C₆)₂, NH-SO₂-alkyle en C₁-C₆ ou NH-CO-alkyle en C₁-C₆, à chaque fois éventuellement substitué ;
D : indépendamment de **A,** est défini comme le radical **A ;**
Q : un radical de formule générale **Q1**
-(CR_{Q}¹R_{Q}²)ₐ- **Q1**
avec l'indice
a = 1
**R_{Q}¹, R_{Q}²,** indépendamment l'un de l'autre :
hydrogène, halogène, OH ou alkyle en C₁-C₆ éventuellement substitué ;
**R¹, R²,** indépendamment l'un de l'autre:
hydrogène, OH, CN, ou
alkyle en C₁-C₆, O-alkyle en C₁-C₆, alkylène en C₁-C₆-O-alkyle en C₁-C₆, cycloalkyle en C₃-C₇, O-cycloalkyle en C₃-C₇, aryle, hétaryle, alkylène en C₁-C₄-aryle, alkylène en C₁-C₄-hétaryle, 0-aryle, O-alkylène en C₁-C₄-aryle, 0-hétaryle, O-alkylène en C₁-C₄-hétaryle, CO-alkyle en C₁-C₆, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-hétaryle, CO-0-alkylène en C₁-C₄-aryle, SO₂-alkyle en C₁-C₆, SO₂-aryle, SO₂-hétaryle, SO₂-alkylène en C₁-C₄-aryle, OCO-alkyle en C₁-C₆, OCO-aryle, OCO-hétaryle, OCO-alkylène en C₁-C₄-aryle, OCO-alkylène en C₁-C₄-hétaryle, SO₂-alkyle en C₁-C₆, SO₂-aryle, SO₂-hétaryle ou SO₂-alkylène en C₁-C₄-aryle, à chaque fois éventuellement substitué, ou
**R¹** et **R²** forment ensemble avec l'azote un hétérocycle saturé ou insaturé, à chaque fois éventuellement substitué, de 5 à 7 éléments, qui peut contenir un, deux ou trois hétéroatomes supplémentaires identiques ou différents choisis dans le groupe constitué par 0, N et S, deux des radicaux substitués sur ce carbo- ou hétérocycle pouvant à chaque fois éventuellement former ensemble un carbocycle ou hétérocycle saturé, insaturé ou aromatique, annelé, de 3 à 7 éléments, à chaque fois éventuellement substitué, l'hétérocycle pouvant contenir un, deux ou jusqu'à trois hétéroatomes identiques ou différents choisis dans le groupe constitué par 0, N et S, et le cycle formé pouvant éventuellement être substitué ;
**R³** paire d'électrons libres ou hydrogène ;
**X, Y, Z** X = N, Y = CR⁴, Z = CR⁶ ou
X = C, Y = NR⁵, Z = CR⁶ ou
X = C, Y = CR⁴, Z = NR⁷ ou
X = N, Y = N, Z = CR⁶ ou
X = N, Y = CR⁴, Z = N,
**R⁴, R⁶** à chaque fois indépendamment l'un de l'autre, un radical choisi dans des groupes identiques ou différents parmi les groupes **1.), 3.), 4.), 5.)** ou **6.)** et
**R⁵, R⁷** à chaque fois indépendamment l'un de l'autre, un radical choisi dans des groupes identiques ou différents parmi les groupes **2.), 3.), 4.)** ou **5.),** les groupes 1.) à 6.) étant définis de la manière suivante :
**1.)** hydrogène, NO₂, NH₂, CN, CF₃, CHF₂, OCF₃, OCHF₂, COOH, O-CH₂-COOH, halogène, ou
alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, alkylène en C₁-C₆-O-alkyle en C₁-C₆, alkylène en C₁-C₆-O-aryle, COO-alkyle en C₁-C₆ ou alkylène en C₁-C₄-COO-alkyle en C₁-C₆, à chaque fois éventuellement substitué, ou
O-R_{Z,Y}⁴, S-R_{Z,Y}⁴, NR_{Z,Y}⁶R_{Z,Y}⁷, CO-OR_{Z,Y}⁵, CO-R_{Z,Y}⁵, NR_{Z,Y}⁷- CO-OR_{Z,Y}⁵, O-CH₂-COO-R_{Z,Y}⁵, NR_{Z,Y}⁷-CO-R_{Z,Y}⁵, SO₂-R_{Z,Y}⁵, SO-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**2.)** paire d'électrons libres ou hydrogène, CH₂-CF₃, CH₂-CHF₂ ; alkyle en C₁-C₁₀, alcényle en C₂-C₁₀, alcynyle en C₂-C₁₀, cycloalkyle en C₃-C₇, alkylène en C₁-C₆-cycloalkyle en C₃-C₇, alkylène en C₁-C₄-aryle, alkylène en C₁-C₄-hétaryle, alkylène en C₁-C₆-O-alkyle en C₁-C₆, alkylène en C₁-C₆-O-aryle, COO-alkyle en C₁-C₆ ou alkylène en C₁-C₄-COO-alkyle en C₁-C₆, à chaque fois éventuellement substitué, ou
CO-OR_{Z,Y}⁵, CO-R_{Z,Y}⁵_{,} SO₂-R_{Z,Y}⁵, SO-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué ;
**3.)** phényle, 1-naphtyle ou 2-naphtyle, qui à chaque fois peuvent être substitués avec **R_{Z,Y}¹, R_{Z,Y}²** et **R_{Z,Y}³; R_{Z,Y}¹, R_{Z,Y}²** et **R_{Z,Y}³** représentant à chaque fois indépendamment les uns des autres un substituant du groupe suivant :
hydrogène, NO₂, NH₂, OH, CN, CF₃, CHF₂, OCF₃, OCHF₂, COOH, O-CH₂-COOH, SH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}, S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵, NR_{Z,Y}⁷-CO-NR_{Z,Y}⁵, SO₂-R_{Z,Y}⁵, NR_{Z,Y}-SO₂-R_{Z,Y}⁵, NR_{Z,Y}⁷-SO₂-NR_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué, ou
à chaque fois deux des radicaux **R_{Z,Y}¹, R_{Z,Y}²** ou **R_{Z,Y}³** formant ensemble avec les atomes de cycle qui portent ces substituants un carbocycle saturé ou insaturé, de 3 à 7 éléments, à chaque fois éventuellement substitué, ou un hétérocycle saturé ou insaturé, à chaque fois éventuellement substitué, qui peut contenir un, deux ou trois hétéroatomes supplémentaires identiques ou différents choisis dans le groupe constitué par 0, N et S, deux radicaux substitués sur ce carbo- ou hétérocycle pouvant à chaque fois éventuellement former ensemble un carbocycle ou hétérocycle saturé, insaturé ou aromatique, annelé, de 3 à 7 éléments, à chaque fois éventuellement substitué, l'hétérocycle pouvant contenir un, deux ou jusqu'à trois hétéroatomes identiques ou différents choisis dans le groupe constitué par 0, N et S, et le cycle formé pouvant éventuellement être substitué et/ou un cycle supplémentaire d e 3 à 7 éléments, éventuellement substitué, pouvant être condensé sur ce cycle ;
**R_{Z,Y}⁴** signifiant alcényle en C₂-C₆, alcynyle en C₂-C₆, alkylène en C₁-C₄-cycloalkyle en C₃-C₇, alkylène en C₁-C₄-hétérocycloalkyle, hétérocycloalkyle, hétaryle, aryle, alkylène en C₁-C₄-hétaryle, alkylène en C₁-C₄-aryle, à chaque fois éventuellement substitué, ou
alkyle en C₁-C₆, qui est éventuellement substitué avec un substituant du groupe constitué par halogène, NO₂, NH₂, OH, CN, CF₃, CHF₂, OCF₃, OCHF₂, NH- (alkyle en C₁-C₆) éventuellement substitué ou N (alkylène en C₁-C₆)₂ éventuellement substitué ;
**R_{Z,Y}⁵** signifiant alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, hétérocycloalkyle ou alkylène en C₁-C₆-O-alkyle en C₁-C₆, dioxyméthylènephényle, benzofuranyle, dihydrobenzofuranyle, indanyle, à chaque fois éventuellement substitué, ou
aryle ou hétaryle, à chaque fois éventuellement substitué 1, 2 ou jusqu'à 3 fois, avec des substituants choisis indépendamment les uns des autres dans le groupe constitué par halogène, NO₂, NH₂, OH, CN, CF₃, CHF₂, OCF₃, OCHF₂, NH-(alkyle en C₁-C₆) éventuellement substitué ou N(alkylène en C₁-C₆)₂ éventuellement substitué, COOH, O-CH₂-COOH, SH, ou
alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₄-hétaryle ou alkylène en C₁-C₄-aryle, à chaque fois éventuellement substitué, ou
O-RA¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ ou CO-NR_{A}²R_{A}³, à chaque fois éventuellement substitué ;
**R_{Z,Y}⁶** signifiant hydrogène, ou
alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, alkylène en C₁-C₄-aryle, alkylène en C₁-C₄-hétaryle, CO-aryle, CO-hétaryle, CO-alkylène en C₁-C₄-aryle, CO-alkylène en C₁-C₄-hétaryle, CO-0-alkyle en C₁-C₆, CO-0-aryle, CO-0-alkylène en C₁-C₄-aryle, CO-O-hétaryle, CO-0-alkylène en C₁-C₄-hétaryle, CO-0-alkylène en C₁-C₄-aryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
**R_{Z,Y}⁷** signifiant hydrogène, ou alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, aryle, hétaryle, hétérocycloalkyle, alkylène en C₁-C₆-O-alkyle en C₁-C₆, CO-alkyle en C₁-C₆, CO-aryle, CO-hétaryle, CO-alkylène en C₁-C₄-aryle, CO-alkylène en C₁-C₄-hétaryle, CO-O-alkyle en C₁-C₆, CO-0-aryle, CO-0-alkylène en C₁-C₄-aryle, CO-O- hétaryle, CO-0-alkylène en C₁-C₄-hétaryle, SO₂-alkyle en C₁-C₆, SO₂-aryle, SO₂-hétaryle, SO₂-alkylène en C₁-C₄-aryle ou SO₂-alkylène en C₁-C₄-hétaryle, à chaque fois éventuellement substitué ;
ou les radicaux R_{Z,Y}⁶ et R_{Z,Y}⁷ formant ensemble avec l'azote un hétérocycle saturé ou aromatique de 3 à 7 éléments, à chaque fois éventuellement substitué, qui peut contenir un, deux ou trois hétéroatomes supplémentaires identiques ou différents choisis dans le groupe constitué par 0, N, S ; et deux radicaux substitués sur cet hétérocycle pouvant à chaque fois éventuellement former ensemble un carbocycle ou hétérocycle saturé, insaturé ou aromatique, annelé, de 3 à 7 éléments, à chaque fois éventuellement substitué, l'hétérocycle pouvant contenir jusqu'à trois hétéroatomes identiques ou différents choisis dans le groupe constitué par 0, N et S, et le cycle formé pouvant éventuellement être substitué et/ou un cycle supplémentaire d e 3 à 7 éléments, éventuellement substitué, pouvant être condensé sur ce cycle ;
4.) un hétérocycle aromatique de 5 ou 6 éléments, qui peut contenir un, deux ou trois hétéroatomes identiques ou différents choisis dans le groupe constitué par 0, N et S, et qui peut à chaque fois être substitué avec **R_{Z,Y}¹, R_{Z,Y}²** et **R_{Z,Y}³,** qui sont définis indépendamment les uns des autres tel que précédemment ;
5.) un radical hydrocarboné saturé bi- ou tricyclique en C₅-C₁₈, à chaque fois éventuellement substitué ;
6.) **R⁴** et **R⁶** forment ensemble avec X et Y un carbocycle saturé, insaturé ou aromatique, e n C₆-C₁₀, éventuellement substitué, de préférence un radical benzo éventuellement substitué, qui peuvent à chaque fois être substitués avec un, deux ou jusqu'à trois radicaux choisis indépendamment les uns des autres dans le groupe **R_{Z,Y}⁸** ;
**R_{Z,Y}⁸ :** hydrogène, NO₂, NH₂, OH, CN, CF₃, CHF₂, OCF₃, OCHF₂, COOH, O-CH₂-COOH, SH, halogène, ou
aryle, hétaryle, hétérocycloalkyle, alkyle en C₁-C₆, alcényle en C₂-C₆, alcynyle en C₂-C₆, cycloalkyle en C₃-C₇, alkylène en C₁-C₄-cycloalkyle en C₃-C₇, alkylène en C₁-C₄-hétérocycloalkyle, alkylène en C₁-C₄-aryle ou alkylène en C₁-C₄-hétaryle, à chaque fois éventuellement substitué, ou
O-R_{Z,Y}⁴ S-R_{Z,Y}⁴, NR_{Z,Y}⁶R_{Z,Y}⁷, CO-OR_{Z,Y}⁵, NR_{Z,Y}⁷-CO-O-R_{Z,Y}⁵, O-CH₂-COO-R_{Z,Y}⁵, NR_{Z,Y}⁷-CO-R_{Z,Y}⁵ SO₂-R_{Z,Y}⁵, NR_{Z,Y}⁷-SO₂-R_{Z,Y}⁵, SO₂NH₂, CONH₂, SO₂-NR_{Z,Y}⁶R_{Z,Y}⁷ ou CO-NR_{Z,Y}⁶R_{Z,Y}⁷, à chaque fois éventuellement substitué,
pour la fabrication d'un médicament pour le traitement et/ou la prophylaxie de maladies du SNC ou de maladies relatives au SNC.

20. Utilisation d'au moins un composé hétéroaromatique à cycle à 5 éléments de formule générale I selon l'une quelconque des revendications 1 à 16 ou selon la revendication 19, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies du SNC et/ou de maladies relatives au SNC.

21. Utilisation d'au moins un composé hétéroaromatique à cycle à 5 éléments de formule générale I selon l'une quelconque des revendications 1 à 16 ou selon la revendication 19 ou 20, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de troubles, symptômes et/ou dysfonctionnements neuropathologiques, neuropsychiatriques et/ou neurodégénératifs.

22. Utilisation d'au moins un composé hétéroaromatique à cycle à 5 éléments de formule générale I selon l'une quelconque des revendications 1 à 16 ou 19, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de la migraine et/ou des lésions cérébrales.

23. Utilisation d'au moins un composé hétéroaromatique à cycle à 5 éléments de formule générale I selon l'une quelconque des revendications 1 à 16 ou 19, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies neuropathologiques, neuropsychiatriques et/ou neurodégénératives choisies dans le groupe constitué p a r l'ischémie cérébrale, l'attaque cérébrale, l'épilepsie et les attaques en général, les psychoses, la schizophrénie, l'autisme, le syndrome TOC, les maladies cognitives, les troubles de l'attention, les dépressions, les dépressions bipolaires et/ou unipolaires, les états d'anxiété, la démence, la démence sénile, la démence d'Alzheimer, les maladies démyélinisantes, la sclérose en plaques et les tumeurs cérébrales.

24. Utilisation d'au moins un composé hétéroaromatique à cycle à 5 éléments de formule générale I selon l'une quelconque des revendications 1 à 16 ou 19, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies choisies dans le groupe constitué par les troubles cérébro-vasculaires, la douleur, les troubles liés à la douleur, la dépendance, les troubles liés aux drogues, l'amnésie, l'abus d'alcool, l'abus de drogues, les troubles du rythme circadien et le syndrome de Cushing.

25. Utilisation d'au moins un composé hétéroaromatique à cycle à 5 éléments de formule générale I selon l'une quelconque des revendications 1 à 16 ou l'une quelconque des revendications 19 à 24, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies qui sont modulées par une activité des récepteurs 5-HT5.

26. Utilisation d'au moins un composé hétéroaromatique à cycle à 5 éléments de formule générale I selon l'une quelconque des revendications 1 à 16 ou l'une quelconque des revendications 19 à 25, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies qui sont modulées par une activité des récepteurs 5-HT5, le traitement et/ou la prophylaxie reposant sur une sélectivité pour le récepteur 5-HT5A.

27. Utilisation d'au moins un composé hétéroaromatique à cycle à 5 éléments de formule générale I selon l'une quelconque des revendications 1 à 16 ou l'une quelconque des revendications 19 à 25, et/ou ses formes énantiomères, diastéréomères et/ou tautomères correspondantes et/ou ses sels pharmaceutiquement acceptables, les radicaux R¹, R², R³, Q, W, X, Y et Z étant définis selon l'une quelconque des revendications 1 à 16 ou 19, pour la fabrication d'un médicament pour le traitement et/ou la prophylaxie de maladies qui sont modulées par une activité des récepteurs 5-HT5, le traitement et/ou la prophylaxie reposant sur une sélectivité pour le récepteur 5-HT5A avec une affinité de liaison (Ki) inférieure ou égale à 600 nM.

28. Utilisation selon l'une quelconque des revendications 19 à 27, dans laquelle le traitement et/ou la prophylaxie ont lieu chez un mammifère humain ou non humain.
